# EUROPEAN PATENT APPLICATION

(11) **EP 4 640 274 A1**
(43) Date of publication of application: **29.10.2025**
(21) Application number: 25171680.9
(22) Date of filing: 22.04.2025
(51) Int. Cl.: A61P 19/00, C07D 401/04, C07D 401/14, C07D 405/14, C07D 409/14, C07D 413/14, C07D 417/14, C07D 493/04, A61K 31/5355, A61K 31/454

(54) **LPAR1 INHIBITORS**

(30) Priority: 22.04.2024 US 202463637200 P
(71) Applicant: AbbVie Inc., North Chicago, IL 60064 (US)
(72) Inventor: Korman, Henry, Vernon Hills, IL, 60061 (US); Krueger, Allan C., Gurnee, IL, 60031 (US); Breinlinger, Eric C, Charlton, MA, 01507 (US); Wilson, Noel S., Kenosha, WI, 53142 (US); Owens, Kyle R., Highland Park, IL (US); Heyman, Howard R., Deerfield, IL (US); Newton, James N., Lake Bluff, IL (US); Shrestha, Anurupa, Vernon Hills, IL (US); Chavez, Martin Garcia, Lake Bluff, IL (US); Zhao, Gang, Northbrook, IL, 60062 (US); Liu, Bo, Chicago, IL (US); Hamza, Daniel, Nottingham, Nottinghamshire, UK (GB); Ackrill, Thomas D, Nottingham, Nottinghamshire, UK (GB); McLellan, Jayde A., Nottingham, Nottinghamshire, UK (GB); Leeder, Alexander J., Nottingham, Nottinghamshire, UK (GB)
(74) Representative: J A Kemp LLP

(57) **Abstract**

Provided herein are compounds of Formula (I), compounds of Formula (II) and pharmaceutically acceptable salts thereof, useful as LPAR1 inhibitors, and pharmaceutical compositions comprising the same. Also provided are methods of treating or preventing a disease or condition comprising administering to a subject in need thereof a therapeutically effective amount of a compound of Formula (I) or a compound of Formula (II).

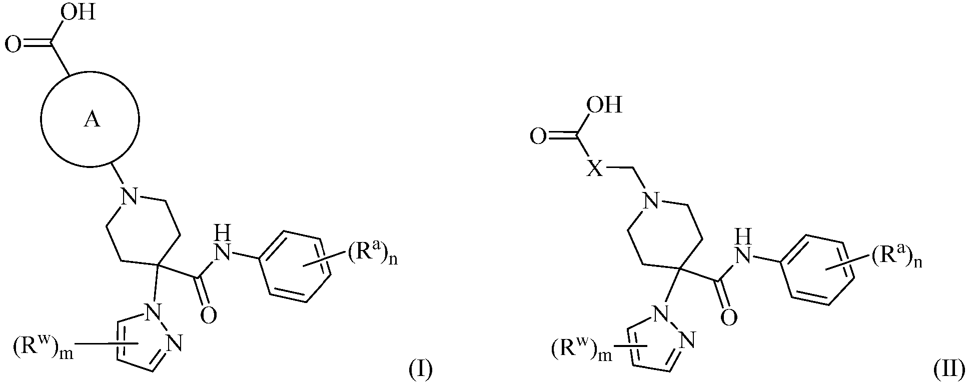

## Description

### CROSS-REFERENCE TO RELATED APPLICATIONS

The present application claims priority to U.S. Provisional Application No. 63/637,200, filed April 22, 2024, the entirety of which is incorporated herein by reference.

### BACKGROUND

Lysophosphatidic acid receptor 1 (LPAR1) signaling has been shown to mediate diverse biologic functions, including proliferation, platelet aggregation, smooth muscle contraction, inhibition of neuroblastoma cell differentiation, chemotaxis, and tumor cell invasion. Accordingly, LPAR1 antagonists may be useful for treating or preventing diseases and disorders associated with LPAR1 signaling.

### SUMMARY

In certain aspects, provided is a compound of Formula (I): 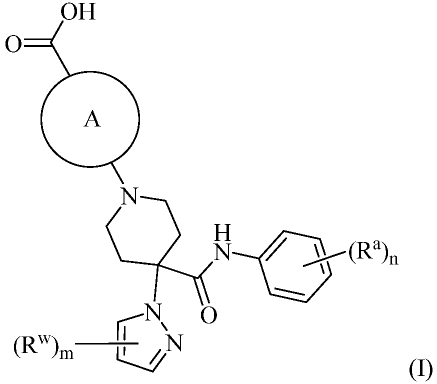 or a pharmaceutically acceptable salt thereof,
wherein:
each R^{a} is independently selected from the group consisting of halo, -O(C₁₋₆alkyl), -O(C₁₋₆haloalkyl), -C₁₋₆alkyl, and -C₁₋₆haloalkyl;
n is 0, 1, or 2;
each R^{W} is independently selected from the group consisting of halo, -OR^{W2}, -NZ₂, optionally substituted C₁₋₆alkyl, optionally substituted C₁₋₆haloalkyl, optionally substituted 3-6-membered carbocyclyl, and optionally substituted 3-6-membered heterocyclyl; or
   two vicinal R^{W} groups, taken together with the atoms to which they are attached, form a fused optionally substituted 4-6 membered carbocyclyl or fused optionally substituted 4-6 membered heterocyclyl;
m is 0, 1, 2, or 3;
Ring A is an optionally substituted non-aromatic 3-7-membered carbocyclylene or a non-aromatic 3-7-membered heterocyclylene;
R^{W2} is hydrogen, optionally substituted C₁₋₆alkyl, optionally substituted C₁₋₆haloalkyl, optionally substituted 3-6-membered carbocyclyl, or optionally substituted 3-6-membered heterocyclyl; and
each Z is independently selected from the group consisting of hydrogen, optionally substituted C₁₋₆alkyl, optionally substituted C₁₋₆haloalkyl, optionally substituted 3-6-membered carbocyclyl, and optionally substituted 3-6-membered heterocyclyl.

In some embodiments, Ring A is an optionally substituted 4-membered carbocyclyene.

In some embodiments, Ring A is a 4-membered carbocyclylene, optionally substituted with 1-3 substituents each independently selected from the group consisting of halo, C₁₋₆alkyl, C₁₋₆haloalkyl, -O(C₁₋₆alkyl), and -O(C₁₋₆haloalkyl).

In some aspects, provided is a compound of Formula (II): 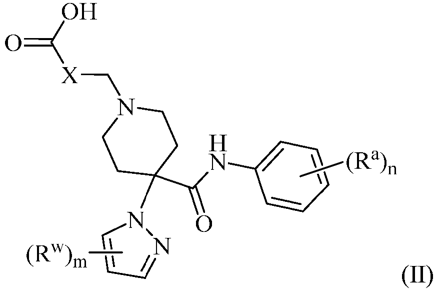 or a pharmaceutically acceptable salt thereof,
wherein:
each R^{a} is independently selected from the group consisting of halo, -O(C₁₋₆alkyl), -O(C₁₋₆haloalkyl), -C₁₋₆alkyl, and -C₁₋₆haloalkyl;
n is 0, 1, or 2;
each R^{W} is independently selected from the group consisting of halo, -OR^{W2}, -NZ₂, optionally substituted C₁₋₆alkyl, optionally substituted C₁₋₆haloalkyl, optionally substituted 3-6-membered carbocyclyl, and optionally substituted 3-6-membered heterocyclyl; or
   two vicinal R^{W} groups, taken together with the atoms to which they are attached, form a fused optionally substituted 4-6 membered carbocyclyl or fused optionally substituted 4-6 membered heterocyclyl;
m is 0, 1, 2, or 3;
X is a bond or an optionally substituted 3-7-membered carbocyclylene, 3-7-membered heterocyclylene, 3-7-membered arylene, 3-7-membered heteroarylene, C₁₋₆alkylene, C₂₋₆alkenylene, -CH(3-7-membered carbocyclyl)-, -CH(3-7-membered heterocyclyl)-, - CH(3-7-membered heterocyclyl)alkyl)-, -CH(3-7-membered carbocyclyl)alkyl)-, - CH(heteroaryl)-, -CH(aryl)-, -CH(heteroarylalkyl)-, -CH(arylalkyl)-, -CH(C₁₋₆alkyl)-, - CH(C₁₋₆haloalkyl)-, -CH(C₁₋₆hydroxyalkyl)-, -CH(C₁₋₆alkoxy)-, -CH(C₁₋₆alkoxyC₁₋₆alkyl)-, or -C(OH)(C₁₋₆alkyl)-;
R^{W2} is hydrogen, optionally substituted C₁₋₆alkyl, optionally substituted C₁₋₆haloalkyl, optionally substituted 3-6-membered carbocyclyl, or optionally substituted 3-6-membered heterocyclyl; and
each Z is independently selected from the group consisting of hydrogen, optionally substituted C₁₋₆alkyl, optionally substituted C₁₋₆haloalkyl, optionally substituted 3-6-membered carbocyclyl, and optionally substituted 3-6-membered heterocyclyl.

In some embodiments, X is an optionally substituted 3-7-membered carbocyclylene, 3-7-membered heterocyclylene, -CH(3-7-membered carbocyclyl)-, -CH(3-7-membered heterocyclyl)-, -CH(C₁₋₆alkyl), -CH(C₁₋₆haloalkyl), or -CH(C₁₋₆alkoxyC₁₋₆alkyl).

In some embodiments, X is an optionally substituted 3-7-membered heterocyclylene, - CH(3-7-membered heterocyclyl)-, or -CH(C₁₋₆alkoxyC₁₋₆alkyl).

In some embodiments, each R^{a} is independently selected from the group consisting of - Cl and -OCHF₂, and n is 2.

In some aspects, the compound has the structure of Formula (Ib): 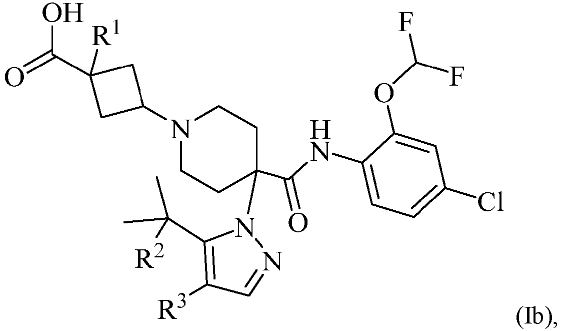 or a pharmaceutically acceptable salt thereof,
wherein:
R¹ is hydrogen or C₁₋₆alkyl; and
R² and R³ are each independently hydrogen, halo, optionally substituted (C₁₋₃alkyl), or optionally substituted alkoxy(C₁₋₃alkyl); or
R² and R³ taken together with the atoms to which they are attached, form a fused optionally substituted 4-6 membered carbocyclyl or fused optionally substituted 4-6 membered heterocyclyl.

In some embodiments, R¹ is hydrogen or methyl.

In some embodiments, R² and R³ are each hydrogen.

In some embodiments, the compound is selected from the following table:

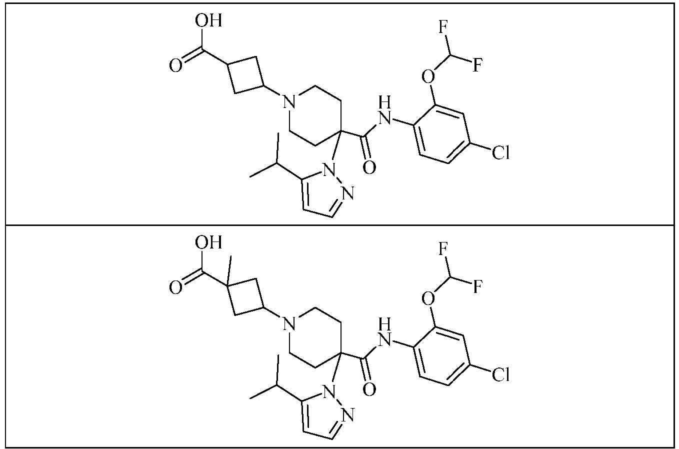

In some embodiments, the compound is selected from the following table:

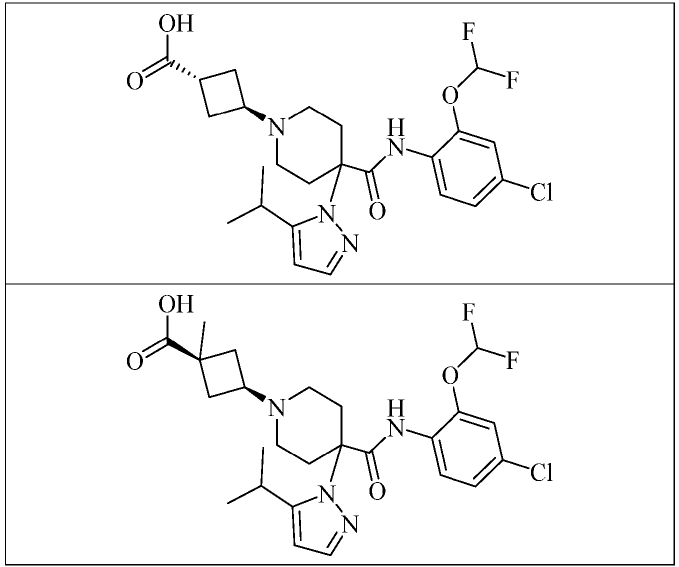

In some embodiments, the compound is: 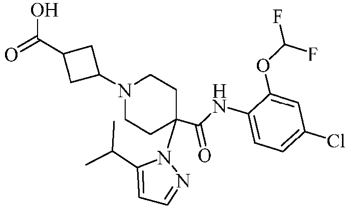 or a pharmaceutically acceptable salt thereof.

In some embodiments, the compound is:

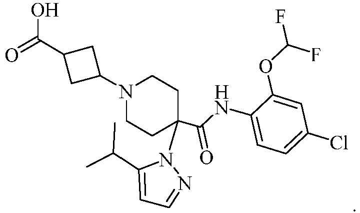

In some embodiments, the compound is: 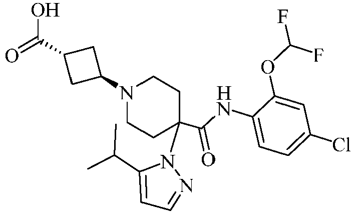 or a pharmaceutically acceptable salt thereof.

In some embodiments, the compound is:

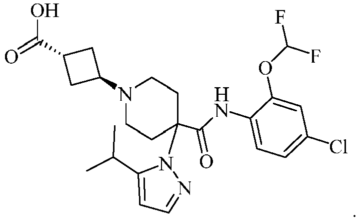

In some embodiments, the compound is a pharmaceutically acceptable salt of the compound:

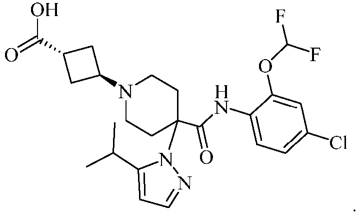

In some aspects, the compound has the structure of Formula (IIb): 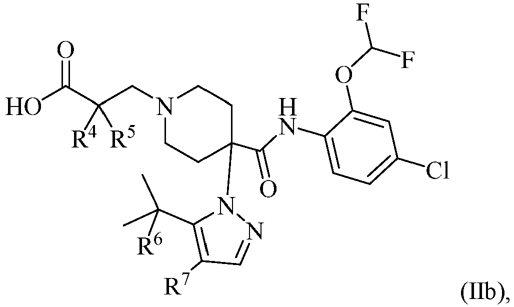 or a pharmaceutically acceptable salt thereof,
wherein:
R⁴ is hydrogen and R⁵ is an optionally substituted 3-7-membered carbocyclyl, 3-7-membered heterocyclyl, C₁₋₆alkyl, C₁₋₆haloalkyl, or C₁₋₆alkoxyC₁₋₆alkyl; or
R⁴ and R⁵ taken together with the atom to which they are attached form an optionally substituted 3-7-membered carbocyclylene or an optionally susbstituted 3-7-membered heterocyclylene; and
R⁶ and R⁷ are each independently hydrogen, halo, optionally substituted (C₁₋₃alkyl), or optionally substituted alkoxy(C₁₋₃alkyl); or
R⁶ and R⁷ taken together with the atoms to which they are attached, form a fused optionally substituted 4-6 membered carbocyclyl or fused optionally substituted 4-6 membered heterocyclyl.

In some embodiments, R⁴ is hydrogen and R⁵ is tetrahydropyranyl, or -CH₂OCH₃; or R⁴ and R⁵ taken together with the atom to which they are attached form a tetrahydropyran ring. In some embodiments, R⁶ and R⁷ are each independently hydrogen or fluoro; or R⁶ and R⁷ taken together with the atoms to which they are attached form a fused 5-membered heterocyclyl.

In some embodiments, the compound is selected from the following table:

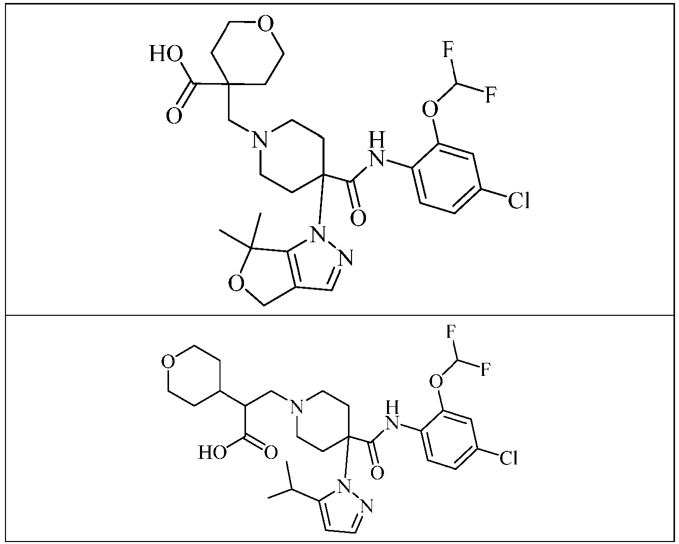

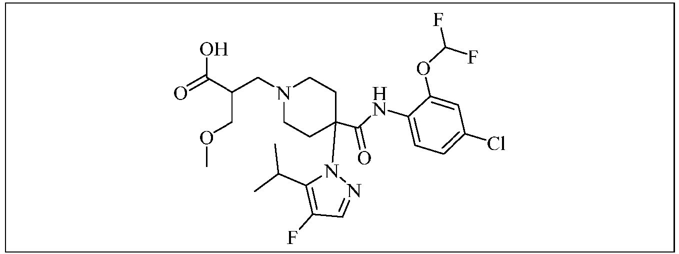

In some embodiments, the compound is selected from the following table:

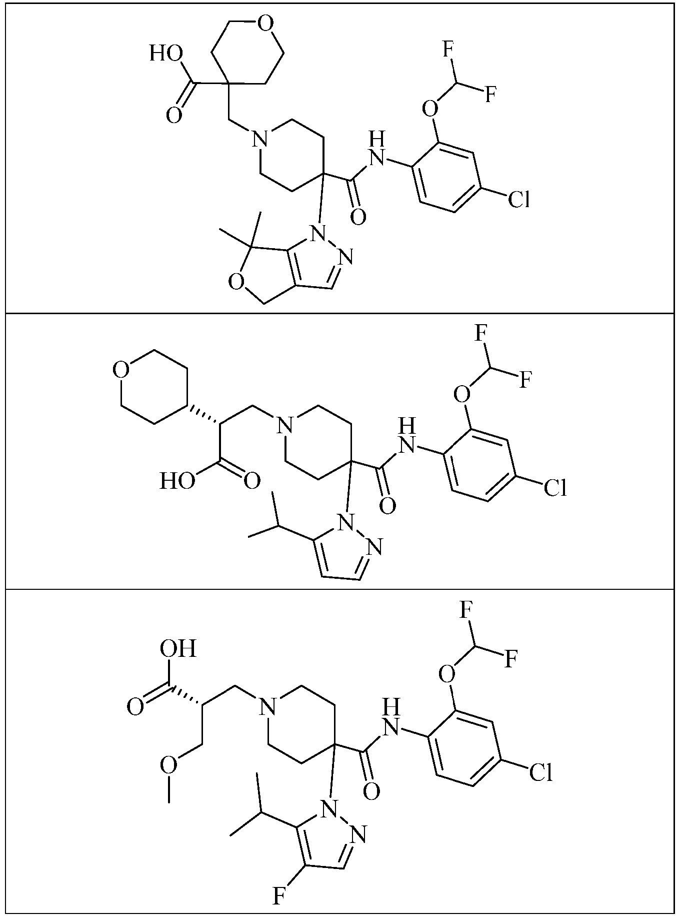

In some embodiments, the compound is: 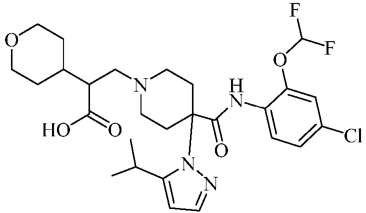 or a pharmaceutically acceptable salt thereof.

In some embodiments, the compound is:

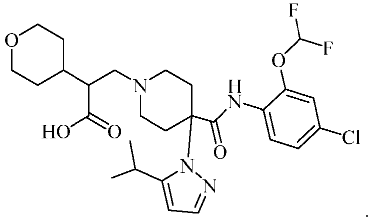

In some embodiments, the compound is: 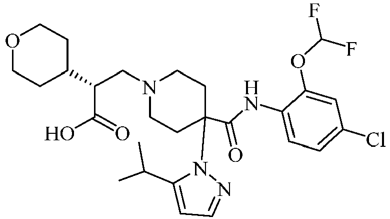 or a pharmaceutically acceptable salt thereof.

In some embodiments, the compound is:

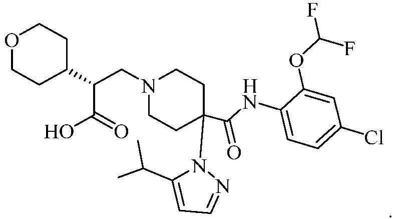

In some embodiments, the compound is a pharmaceutically acceptable salt of the compound:

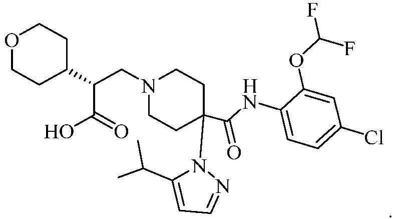

In some embodiments, the compound is: 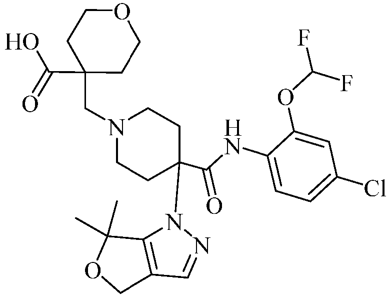 or a pharmaceutically acceptable salt thereof.

In some embodiments, the compound is:

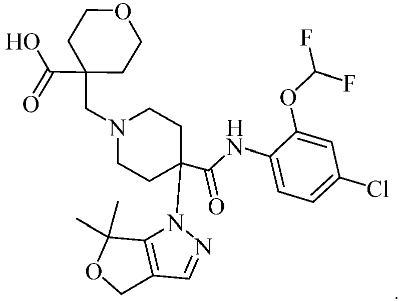

In some embodiments, the compound is a pharmaceutically acceptable salt of the compound:

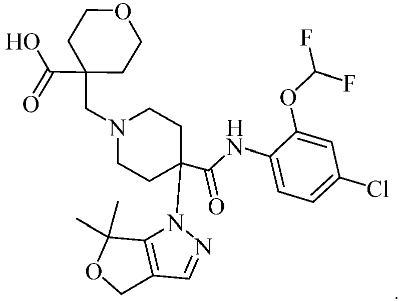

In certain aspects, provided herein is a pharmaceutical composition, comprising a compound of Formula (I) or a compound of Formula (II) and a pharmaceutically acceptable carrier.

In some aspects, provided herein is a method of treating or preventing a disease or condition comprising administering to a subject in need thereof a therapeutically effective amount of a compound of Formula (I) or a compound of Formula (II), wherein the disease or condition is treated by inhibiting LPAR1. In some aspects, provided herein is a compound of Formula (I) or a compound of Formula (II) for use in a method of treating or preventing a disease or condition comprising administering to a subject in need thereof a therapeutically effective amount of the compound of Formula (I) or the compound of Formula (II), wherein the disease or condition is treated by inhibiting LPAR1.

In some aspects, provided herein is a method of treating a disease or condition comprising administering to a subject in need thereof a therapeutically effective amount of a compound of Formula (I) or a compound of Formula (II), wherein the disease or condition is treated by inhibiting LPAR1. In some embodiments, the disease or condition is systemic sclerosis. In some aspects, provided herein is a compound of Formula (I) or a compound of Formula (II) for use in a method of treating a disease or condition comprising administering to a subject in need thereof a therapeutically effective amount of the compound of Formula (I) or the compound of Formula (II), wherein the disease or condition is treated by inhibiting LPAR1. In some embodiments, the disease or condition is systemic sclerosis.

In certain aspects, provided herein is a method of inhibiting LPAR1 comprising administering to a subject an effective amount of a compound of Formula (I) or a compound of Formula (II). In certain aspects, provided herein is a compound of Formula (I) or a compound of Formula (II) for use in a method of inhibiting LPAR1 comprising administering to a subject an effective amount of the compound of Formula (I) or the compound of Formula (II).

In certain aspects, provided herein is a method of treating systemic sclerosis in a subject in need thereof, comprising administering to the subject an effective amount of a compound of the present disclosure or a pharmaceutically acceptable salt thereof. In some embodiments, provided herein is a method of treating systemic sclerosis in a subject in need thereof, comprising administering to the subject an effective amount of a compound of the present disclosure. In some embodiments, provided herein is a method of treating systemic sclerosis in a subject in need thereof, comprising administering to the subject an effective amount of a pharmaceutically acceptable salt of a compound of the present disclosure. In certain aspects, provided herein is a compound of the present disclosure or a pharmaceutically acceptable salt thereof for use in a method of treating systemic sclerosis in a subject in need thereof, comprising administering to the subject an effective amount of the compound of the present disclosure or the pharmaceutically acceptable salt thereof. In some embodiments, provided herein is a compound of the present disclosure for use in a method of treating systemic sclerosis in a subject in need thereof, comprising administering to the subject an effective amount of the compound of the present disclosure. In some embodiments, provided herein is a pharmaceutically acceptable salt of a compound of the present disclosure for use in a method of treating systemic sclerosis in a subject in need thereof, comprising administering to the subject an effective amount of the pharmaceutically acceptable salt of a compound of the present disclosure.

In some embodiments, provided herein is a method of treating systemic sclerosis in a subject in need thereof, comprising administering to the subject an effective amount of Compound 15 or a pharmaceutically acceptable salt thereof. In some embodiments, provided herein is a method of treating systemic sclerosis in a subject in need thereof, comprising administering to the subject an effective amount of Compound 15. In some embodiments, provided herein is a method of treating systemic sclerosis in a subject in need thereof, comprising administering to the subject an effective amount of a pharmaceutically acceptable salt of Compound 15. In some embodiments, provided herein is Compound 15 or a pharmaceutically acceptable salt thereof for use in a method of treating systemic sclerosis in a subject in need thereof, comprising administering to the subject an effective amount of Compound 15 or the pharmaceutically acceptable salt thereof. In some embodiments, provided herein is Compound 15 for use in a method of treating systemic sclerosis in a subject in need thereof, comprising administering to the subject an effective amount of Compound 15. In some embodiments, provided herein is a pharmaceutically acceptable salt of Compound 15 for use in a method of treating systemic sclerosis in a subject in need thereof, comprising administering to the subject an effective amount of the pharmaceutically acceptable salt of Compound 15.

### DEFINITIONS

Definitions of specific functional groups and chemical terms are described in more detail below. The chemical elements are identified in accordance with the Periodic Table of the Elements, CAS version, Handbook of Chemistry and Physics, 75^{th} Ed., inside cover, and specific functional groups are generally defined as described therein. Additionally, general principles of organic chemistry, as well as specific functional moieties and reactivity, are described in Organic Chemistry, Thomas Sorrell, University Science Books, Sausalito, 1999; Smith and March March's Advanced Organic Chemistry, 5th Edition, John Wiley & Sons, Inc., New York, 2001; Larock, Comprehensive Organic Transformations, VCH Publishers, Inc., New York, 1989; and Carruthers, Some Modern Methods of Organic Synthesis, 3rd Edition, Cambridge University Press, Cambridge, 1987.

Compounds described herein may comprise one or more asymmetric centers, and thus may exist in various stereoisomeric forms, *e.g.,* enantiomers and/or diastereomers and/or geometric (cis/trans or E/Z) isomers in a composition. For example, compositions may comprise a mixture of stereoisomers, including racemic (equal) mixtures, mixtures enriched in one or more stereoisomer, or may comprise an individual stereoisomer in substantially pure (>99%) form. As used herein, "enriched" refers to a composition which comprises greater than (>) 50% of one stereoisomer over the sum total of other stereoisomer(s) which may be present in the composition. In certain embodiments, a composition may comprise >60%, >65%, >70%, >75%, >80%, >85%, >90%, >91%, >92%, >93%, >94%, >95%, >96%, >97%, >98%, >99%, >99.5%, >99.9%, or even up to 100% of one stereoisomer over the sum total of other stereoisomer(s) which may be present in the composition; or may comprise 0% or less than (<) 0.1%, <0.5%, <1%, <2%, <3%, <4%, <5%, <6%, <7%, <8%, <9%, <10%, <15%, <20%, <25%, <30%, <35%, <40%, <45%, or <50% of one stereoisomer over the sum total of other stereoisomer(s) which may be present in the composition. For simplicity, calculating enriched amounts of any of the stereoisomer(s), if provided as pharmaceutically acceptable salt(s) in a composition, are based on the hypothetical amount of free base form. In certain embodiments, a composition is enriched in its (*S*)-enantiomer. In other embodiments, a composition is enriched in its (*R*)-enantiomer.

Unless otherwise stated, structures depicted herein are also meant to include compounds that differ only in the presence of one or more isotopically enriched atoms. For example, compounds having the present structures except for the replacement of hydrogen by deuterium or tritium, replacement of ¹⁹F with ¹⁸F, replacement of a carbon by a ¹³C- or ¹⁴C-enriched carbon, and/or replacement of an oxygen atom with ¹⁸O, are within the scope of the disclosure.

When a range of values is listed, it is intended to encompass each value and sub-range within the range. For example, "C₁₋₆ alkyl" is intended to encompass, C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆ alkyl.

"Heteroatom" refers to an atom of any element other than carbon or hydrogen. Illustrative heteroatoms include boron, nitrogen, oxygen, phosphorus, sulfur and selenium, and alternatively oxygen, nitrogen or sulfur.

"Alkyl" refers to saturated aliphatic groups, including straight-chain alkyl groups, branched-chain alkyl groups, cycloalkyl (alicyclic) groups, alkyl substituted cycloalkyl groups, and cycloalkyl substituted alkyl groups. In certain embodiments, a straight-chain or branched-chain alkyl has about 30 or fewer carbon atoms in its backbone (e.g., C₁-₃₀ for straight chain, C₃-₃₀ for branched chain), and alternatively, about 20 or fewer, or 10 or fewer. In certain embodiments, the term "alkyl" refers to a C₁-₁₀ alkyl group. In certain embodiments, the term "alkyl" refers to a C₁-₆ alkyl group, for example a C₁-₆ straight-chain alkyl group. In certain embodiments, the term "alkyl" refers to a C₃-₁₂ branched-chain alkyl group. In certain embodiments, the term "alkyl" refers to a C₃-₈ branched-chain alkyl group. Representative examples of alkyl include, but are not limited to, methyl, ethyl, n-propyl, iso-propyl, n-butyl, sec-butyl, iso-butyl, tert-butyl, n-pentyl, isopentyl, neopentyl, and n-hexyl.

"Alkylene" refers to a divalent alkyl group.

"Haloalkyl" refers to an alkyl group, as defined herein, wherein some or all of the hydrogens are replaced with a halogen, *e.g.,* fluoro, bromo, chloro, or iodo.

"Carbocyclyl" or "carbocyclic" refers to a monocyclic or multicyclic (e.g., bicyclic, tricyclic, etc.) hydrocarbon radical containing from 3 to 12 carbon atoms (i.e., 3-12-membered) that is completely saturated or has one or more unsaturated bonds, and for the avoidance of doubt, the degree of unsaturation does not result in an aromatic ring system (e.g., phenyl). In some embodiments, the carbocyclyl is 3-6-membered, 3-7-membered, 4-6-membered, or 4-membered. Examples of monocyclic carbocyclyls include cyclopropyl, cyclobutyl, cyclopentyl, cyclopentenyl, cyclohexyl, cyclohexenyl, cycloheptyl, and cyclooctyl. Bicyclic carbocyclyl ring systems include bridged monocyclic rings, fused bicyclic rings, and spirocyclyl bicyclic rings. Bridged monocyclic rings contain a monocyclic carbocyclyl ring where two non-adj acent carbon atoms of the monocyclic ring are linked by an alkylene bridge of between one and three additional carbon atoms (*i.e., a* bridging group of the form -(CH₂)*_{w}*-, where *w* is 1, 2, or 3). Representative examples of bicyclic ring systems include, but are not limited to, bicyclo[3.1.1]heptane, bicyclo[2.2.1]heptane, bicyclo[2.2.2]octane, bicyclo[3.2.2]nonane, bicyclo[3.3.1]nonane, and bicyclo[4.2.1]nonane. Fused bicyclic carbocyclyl ring systems contain a monocyclic carbocyclyl ring fused to either an aryl, a monocyclic carbocyclyl, a monocyclic heterocyclyl, or a monocyclic heteroaryl. Spirocyclyl bicyclic ring systems refer to a bicyclic cycloalkyl ring system in which the two rings are linked by a single atom, such as a quaternary carbon atom. The bridged or fused or spirocyclyl bicyclic carbocyclyl is attached to the parent molecular moiety through any carbon atom contained within the carbocyclyl ring system. Exemplary spirocyclyl bicyclic ring systems include, but are not limited to, spiro[2.2]pentane, spiro[3.3]heptane, spiro[4.4.]nonane, spiro[2.3]hexane, and spiro[3.4]octane.

"Carbocyclylene" refers to a divalent carbocyclyl group.

"Heterocyclyl" or "heterocyclic" refers to a radical of a non-aromatic ring system, including, but not limited to, monocyclic, bicyclic, and tricyclic rings, which can be completely saturated or which can contain one or more units of unsaturation, for the avoidance of doubt, the degree of unsaturation does not result in an aromatic ring system, and having 3 to 12 atoms including at least one heteroatom, such as nitrogen, oxygen, or sulfur. In some embodiments, the heterocyclyl is 3-6-membered, 3-7-membered, 4-6-membered, or 4-membered. Bicyclic heterocyclyl ring systems include bridged monocyclic rings, fused bicyclic rings, and spirocyclyl bicyclic rings. Fused bicyclic heterocyclyl ring systems contain a monocyclic heterocyclyl ring fused to either an aryl, a monocyclic carbocyclyl, a monocyclic heterocyclyl, or a monocyclic heteroaryl. Spirocyclyl bicyclic ring systems refer to a bicyclic heterocycloalkyl ring system in which the two rings are linked by a single atom, such as a quaternary carbon atom. The bridged or fused or spirocyclyl bicyclic heterocyclyl is attached to the parent molecular moiety through any atom contained within the heterocyclyl ring system. For purposes of exemplification, which should not be construed as limiting the scope of this invention, the following are examples of heterocyclic rings: aziridinyl, azirinyl, oxiranyl, thiiranyl, thiirenyl, dioxiranyl, diazirinyl, diazepanyl, 1,3-dioxanyl, 1,3-dioxolanyl, 1,3-dithiolanyl, 1,3-dithianyl, imidazolidinyl, isothiazolinyl, isothiazolidinyl, isoxazolinyl, isoxazolidinyl, azetyl, oxetanyl, oxetyl, thietanyl, thietyl, diazetidinyl, dioxetanyl, dioxetenyl, dithietanyl, dithietyl, dioxalanyl, oxazolyl, thiazolyl, triazinyl, isothiazolyl, isoxazolyl, azepines, azetidinyl, morpholinyl, oxadiazolinyl, oxadiazolidinyl, oxazolinyl, oxazolidinyl, oxopiperidinyl, oxopyrrolidinyl, piperazinyl, piperidinyl, pyranyl, pyrazolinyl, pyrazolidinyl, pyrrolinyl, pyrrolidinyl, quinuclidinyl, thiomorpholinyl, tetrahydropyranyl, tetrahydrofuranyl, tetrahydrothienyl, thiadiazolinyl, thiadiazolidinyl, thiazolinyl, thiazolidinyl, thiomorpholinyl, 1,1-dioxidothiomorpholinyl (thiomorpholine sulfone), thiopyranyl, trithianyl, and 2-azobicyclo[3.1.0]hexane.

The term "heterocyclylene" refers to a divalent heterocyclyl group.

"Aryl" refers to a monocyclic, bicyclic and polycyclic aromatic hydrocarbon groups, for example, benzene, naphthalene, anthracene, and pyrene. In some embodiments, an aryl group contains from 3-7 carbon ring atoms (i.e., 3-7-membered aryl). The term "aryl" also includes polycyclic ring systems having two or more cyclic rings in which two or more carbons are common to two adjoining rings (the rings are "fused rings") wherein at least one of the rings is an aromatic hydrocarbon, e.g., the other cyclic rings may be carbocyclyls, aryls, heteroaryls, and/or heterocyclyls. In certain embodiments, the term "aryl" refers to a phenyl group.

The term "arylene" as used herein pertains to a diradical obtained by removing two hydrogen atoms of an aryl group, as defined above. Arylene includes, without limitation, 1,2-phenylene, 1,3-phenylene, and 1,4-phenylene, as depicted below:

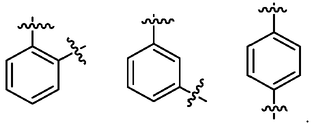

"Heteroaryl" refers to a monocyclic, bicyclic, and polycyclic aromatic group having 3 to 12 total atoms including one or more heteroatoms such as nitrogen, oxygen, or sulfur in the ring structure. In some embodiments, the heteroaryl is 3-7-membered. Exemplary heteroaryl groups include azaindolyl, benzo(b)thienyl, benzimidazolyl, benzofuranyl, benzoxazolyl, benzothiazolyl, benzothiadiazolyl, benzotriazolyl, benzoxadiazolyl, furanyl, imidazolyl, imidazopyridinyl, indolyl, indolinyl, indazolyl, isoindolinyl, isoxazolyl, isothiazolyl, isoquinolinyl, oxadiazolyl, oxazolyl, purinyl, pyranyl, pyrazinyl, pyrazolyl, pyridinyl, pyrimidinyl, pyrrolyl, pyrrolo[2,3-d]pyrimidinyl, pyrazolo[3,4-d]pyrimidinyl, quinolinyl, quinazolinyl, triazolyl, thiazolyl, thiophenyl, tetrahydroindolyl, tetrazolyl, thiadiazolyl, thienyl, thiomorpholinyl, triazolyl or tropanyl, and the like. The term "heteroaryl" also includes polycyclic ring systems having two or more cyclic rings in which two or more atoms are common to two adjoining rings (the rings are "fused rings") wherein at least one of the rings is an aromatic group having one or more heteroatoms in the ring structure, e.g., the other cyclic rings may be carbocyclyls, aryls, heteroaryls, and/or heterocyclyls.

The term "heteroarylene" as used herein pertains to a diradical obtained by removing two hydrogen atoms of a heteroaryl group, as defined above. Heteroarylene includes, without limitation, the divalent heteroarylene groups depicted below:

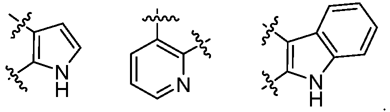

"Alkenyl" refers to a a straight or branched chain hydrocarbon radical containing from 2 to 10 carbons and containing at least one carbon-carbon double bond formed by the removal of two hydrogens. Representative examples of alkenyl include, but are not limited to, ethenyl, 2-propenyl, 2-methyl-2-propenyl, 3-butenyl, 4-pentenyl, 5-hexenyl, 2-heptenyl, 2-methyl-1-heptenyl, and 3-decenyl. The unsaturated bond(s) of the alkenyl group can be located anywhere in the moiety and can have either the (Z) or the (E) configuration about the double bond(s).

"Alkenylene" refers to a divalent alkenyl group.

"(Heterocyclyl)alkyl" refers to an alkyl group substituted with one or more heterocyclyl groups.

"(Carbocyclyl)alkyl" refers to an alkyl group substituted with one or more carbocyclyl groups.

"Arylalkyl" refers to an alkyl group substituted with an aryl group.

"Heteroarylalkyl" refers to an alkyl group substituted with a heteroaryl group.

"Alkoxy" refers to an alkyl group appended to the parent molecular moiety through an oxygen atom. Representative examples of alkoxy include, but are not limited to, methoxy, ethoxy, propoxy, 2-propoxy, butoxy, tert-butoxy, pentyloxy, and hexyloxy.

"Hydroxy" refers to -OH.

"Cyano" refers to -CN.

"Hydroxyalkyl" refers to at least one hydroxy group appended to the parent molecular moiety through an alkyl group. Representative examples of hydroxyalkyl include, but are not limited to, hydroxymethyl, 2-hydroxyethyl, 3-hydroxypropyl, 2,3-dihydroxypentyl, and 2-ethyl-4-hydroxyheptyl.

"Halo" refers to fluorine (fluoro, -F), chlorine (chloro, -Cl), bromine (bromo, -Br), or iodine (iodo, -I).

Certain compounds contained in compositions of the present invention may exist in particular geometric or stereoisomeric forms. In addition, compounds of the present invention may also be optically active. The present invention contemplates all such compounds, including *cis-* and *trans*-isomers, (*R*)- and (*S*)-enantiomers, diastereoisomers, (D)-isomers, (L)-isomers, the racemic mixtures thereof, and other mixtures thereof, as falling within the scope of the invention. Additional asymmetric carbon atoms may be present in a substituent such as an alkyl group. All such isomers, as well as mixtures thereof, are intended to be included in this invention.

If, for instance, a particular enantiomer of compound of the present invention is desired, it may be prepared by asymmetric synthesis, or by derivation with a chiral auxiliary, where the resulting diastereomeric mixture is separated and the auxiliary group cleaved to provide the pure desired enantiomers. Alternatively, where the molecule contains a basic functional group, such as amino, or an acidic functional group, such as carboxyl, diastereomeric salts are formed with an appropriate optically-active acid or base, followed by resolution of the diastereomers thus formed by fractional crystallization or chromatographic means well known in the art, and subsequent recovery of the pure enantiomers.

"Substitution" or "substituted with" includes the implicit proviso that such substitution is in accordance with permitted valence of the substituted atom and the substituent, and that the substitution results in a stable compound, e.g., which does not spontaneously undergo transformation such as by rearrangement, fragmentation, decomposition, cyclization, elimination, or other reaction.

"Substituted" is contemplated to include all permissible substituents of organic compounds. In a broad aspect, the permissible substituents include acyclic and cyclic, branched and unbranched, carbocyclic and heterocyclic, aromatic and nonaromatic substituents of organic compounds. Illustrative substituents include, for example, those described herein above. The permissible substituents may be one or more and the same or different for appropriate organic compounds. For purposes of this disclosure, the heteroatoms such as nitrogen may have hydrogen substituents and/or any permissible substituents of organic compounds described herein which satisfy the valences of the heteroatoms. This disclosure is not intended to be limited in any manner by the permissible substituents of organic compounds.

In certain embodiments, the optional substituents contemplated in this disclosure include halogen, azide, oxo (=O), alkyl, alkenyl, alkynyl, aryl, arylalkyl, heteroaryl, heteroarylalkyl, carbocyclyl, (carbocyclyl)alkyl, heterocyclyl, (heterocyclyl)alkyl, hydroxyl, alkoxyl, amino, aminoalkyl, nitro, sulfhydryl, imino, amido, phosphonate, phosphinate, carbonyl, carboxyl, carbamato (e.g., -NHC(O)Oalkyl), silyl, ether (e.g., -alkylene-O(alkyl)), alkylthio, sulfonyl, sulfonamido (e.g., -NHSO₂alkyl), ketone (e.g., -CO(alkyl)), aldehyde (-C(O)H), ester (e.g., -COO(alkyl)), haloalkyl, hydroxyalkyl, alkoxyalkyl, haloalkoxy, haloalkoxyalkyl, and cyano.

As used herein, the term "optionally substituted" or "substituted or unsubstituted" when it precedes a list of chemical moieties means that the list of chemical moieities that follow are each substituted or unsubstituted. For example, "substituted or unsubstituted aryl, heteroaryl, and cycloalkyl" or "optionally substituted aryl, heteroaryl, and cycloalkyl" means substituted or unsubstituted aryl, substituted or unsubstituted heteroaryl, and substituted or unsubstituted cycloalkyl.

"Salt" refers to any and all salts, and is produced from the ionic complexation of a basic compound with an inorganic or organic acid, or an acidic compound with an inorganic or organic base, to provide a compound which is electronically neutral. "Pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. See also Berge et al., J. Pharmaceutical Sciences (1977) 66:1-19. A "free base" of a compound is the neutral and salt-free form of the compound. In certain embodiments, a compound of Formula (**I**) may be a salt (*e.g.,* a pharmaceutically acceptable salt). In certain embodiments, *e.g.,* in the absence of reference to a pharmaceutically acceptable salt, a compound of Formula (**I**) may be present as the free base form.

Hydroxyl protecting groups are well known in the art and include those described in detail in Protecting Groups in Organic Synthesis, T. W. Greene and P. G. M. Wuts, 3rd edition, John Wiley & Sons, 1999. Exemplary "hydroxyl protecting groups", as used herein, include but are not limited to, methoxylmethyl (MOM), methylthiomethyl (MTM), *t*-butylthiomethyl, (phenyldimethylsilyl)methoxymethyl (SMOM), benzyloxymethyl (BOM), *p-*methoxybenzyloxymethyl (PMBM), (4-methoxyphenoxy)methyl (*p*-AOM), guaiacolmethyl (GUM), *t*-butoxymethyl, 4-pentenyloxymethyl (POM), siloxymethyl, 2-methoxyethoxymethyl (MEM), 2,2,2-trichloroethoxymethyl, bis(2-chloroethoxy)methyl, 2-(trimethylsilyl)ethoxymethyl (SEMOR), tetrahydropyranyl (THP), 3-bromotetrahydropyranyl, tetrahydrothiopyranyl, 1-methoxycyclohexyl, 4-methoxytetrahydropyranyl (MTHP), 4-methoxytetrahydrothiopyranyl, 1-[(2-chloro-4-methyl)phenyl]-4-methoxypiperidin-4-yl (CTMP), allyl, benzyl (Bn), *p-*methoxybenzyl, 3,4-dimethoxybenzyl, *o*-nitrobenzyl, *p*-nitrobenzyl, *p*-halobenzyl, 2,6-dichlorobenzyl, *p*-cyanobenzyl, *p*-phenylbenzyl, diphenylmethyl, triphenylmethyl, trimethylsilyl (TMS), triethylsilyl (TES), triisopropylsilyl (TIPS), dimethylisopropylsilyl (IPDMS), diethylisopropylsilyl (DEIPS), dimethylhexylsilyl, *t*-butyldimethylsilyl (TBDMS), *t-*butyldiphenylsilyl (TBDPS), tribenzylsilyl, triphenylsilyl, diphenylmethylsilyl (DPMS), *t-*butylmethoxyphenylsilyl (TBMPS), acetate, chloroacetate, dichloroacetate, trichloroacetate, trifluoroacetate, methoxyacetate, triphenylmethoxyacetate, phenoxyacetate, *p-*chlorophenoxyacetate, methyl carbonate, 9-fluorenylmethyl carbonate (Fmoc), ethyl carbonate, 2,2,2-trichloroethyl carbonate (Troc), 2-(trimethylsilyl)ethyl carbonate (TMSEC), 2-(phenylsulfonyl) ethyl carbonate (Psec), 2-(triphenylphosphonio) ethyl carbonate (Peoc), isobutyl carbonate, vinyl carbonate, allyl carbonate, *t*-butyl carbonate (BOC), *p*-nitrophenyl carbonate, benzyl carbonate, *p*-methoxybenzyl carbonate, 3,4-dimethoxybenzyl carbonate, o-nitrobenzyl carbonate, and *p*-nitrobenzyl carbonate.

A "subject" refers to a mammal, and includes, but is not limited to, humans (*i.e.,* a male or female of any age group, *e.g.,* a pediatric subject (*e.g.,* infant, child, adolescent) or adult subject (*e.g.,* young adult, middle-aged adult or senior adult)) and/or other non-human mammals, for example, primates (*e.g.,* cynomolgus monkeys, rhesus monkeys), cats, and/or dogs.

"Treat," "treating" and "treatment" refers to an action that occurs while a subject is suffering from the disease, and which reduces the severity of the disease, or retards or slows the progression of the disease or associated symptoms.

An "effective amount" of a compound, or a pharmaceutically acceptable salt thereof, is an amount, alone or in combination with other therapies, which provides a therapeutic benefit in the treatment of a disease from which the subject suffers, or to delay or minimize one or more symptoms associated with the disease from which the subject suffers.

### DETAILED DESCRIPTION OF THE INVENTION

### Compounds

In certain aspects, provided is a compound of Formula (I): 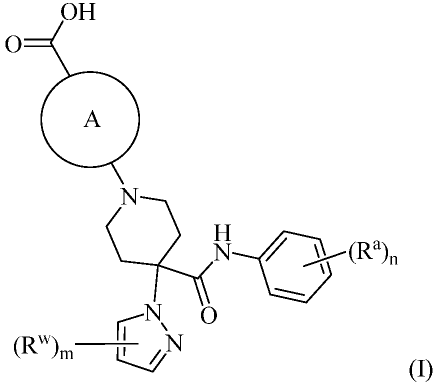 or a pharmaceutically acceptable salt thereof,
wherein:
each R^{a} is independently selected from the group consisting of halo, -O(C₁₋₆alkyl), -O(C₁₋₆haloalkyl), -C₁₋₆alkyl, and -C₁₋₆haloalkyl;
n is 0, 1, or 2;
each R^{W} is independently selected from the group consisting of halo, -OR^{W2}, -NZ₂, optionally substituted C₁₋₆alkyl, optionally substituted C₁₋₆haloalkyl, optionally substituted 3-6-membered carbocyclyl, and optionally substituted 3-6-membered heterocyclyl; or
   two vicinal R^{W} groups, taken together with the atoms to which they are attached, form a fused optionally substituted 4-6 membered carbocyclyl or fused optionally substituted 4-6 membered heterocyclyl;
m is 0, 1, 2, or 3;
Ring A is an optionally substituted non-aromatic 3-7-membered carbocyclylene or non-aromatic 3-7-membered heterocyclylene;
R^{W2} is hydrogen, an optionally substituted C₁₋₆alkyl, optionally substituted C₁₋₆haloalkyl, optionally substituted 3-6-membered carbocyclyl, or optionally substituted 3-6-membered heterocyclyl; and
each Z is independently selected from the group consisting of hydrogen, optionally substituted C₁₋₆alkyl, optionally substituted C₁₋₆haloalkyl, optionally substituted 3-6-membered carbocyclyl, and optionally substituted 3-6-membered heterocyclyl.

In some aspects, provided is a compound of Formula (II): 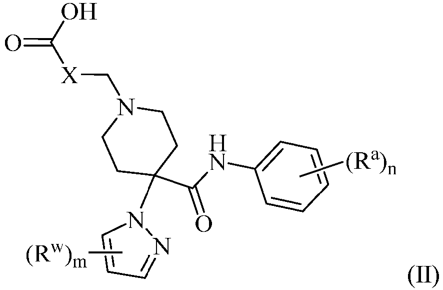 or a pharmaceutically acceptable salt thereof,
wherein:
each R^{a} is independently selected from the group consisting of halo, -O(C₁₋₆alkyl), -O(C₁₋₆haloalkyl), -C₁₋₆alkyl, and -C₁₋₆haloalkyl;
n is 0, 1, or 2;
each R^{W} is independently selected from the group consisting of halo, -OR^{W2}, -NZ₂, optionally substituted C₁₋₆alkyl, optionally substituted C₁₋₆haloalkyl, optionally substituted 3-6-membered carbocyclyl, and optionally substituted 3-6-membered heterocyclyl; or
   two vicinal R^{W} groups, taken together with the atoms to which they are attached, form a fused optionally substituted 4-6 membered carbocyclyl or fused optionally substituted 4-6 membered heterocyclyl;
m is 0, 1, 2, or 3;
X is a bond or an optionally substituted 3-7-membered carbocyclylene, 3-7-membered heterocyclylene, 3-7-membered arylene, 3-7-membered heteroarylene, C₁₋₆alkylene, C₂₋₆alkenylene, -CH(3-7-membered carbocyclyl)-, -CH(3-7-membered heterocyclyl)-, - CH(3-7-membered heterocyclyl)alkyl)-, -CH(3-7-membered carbocyclyl)alkyl)-, - CH(heteroaryl)-, -CH(aryl)-, -CH(heteroarylalkyl)-, -CH(arylalkyl)-, -CH(C₁₋₆alkyl)-, - CH(C₁₋₆haloalkyl)-, -CH(C₁₋₆hydroxyalkyl)-, -CH(C₁₋₆alkoxy)-, -CH(C₁₋₆alkoxyC₁₋₆alkyl)-, or -C(OH)(C₁₋₆alkyl)-;
R^{W2} is hydrogen, an optionally substituted C₁₋₆alkyl, optionally substituted C₁₋₆haloalkyl, optionally substituted 3-6-membered carbocyclyl, or optionally substituted 3-6-membered heterocyclyl; and
each Z is independently selected from the group consisting of hydrogen, optionally substituted C₁₋₆alkyl, optionally substituted C₁₋₆haloalkyl, optionally substituted 3-6-membered carbocyclyl, and optionally substituted 3-6-membered heterocyclyl.

In some aspects, the compound is selected from the following table. Nuclear magnetic resonance (NMR) data was experimentally determined on the synthesized compounds below:

| **Structure** | **IUPAC NAME** | **¹H NMR** |
|---|---|---|
| 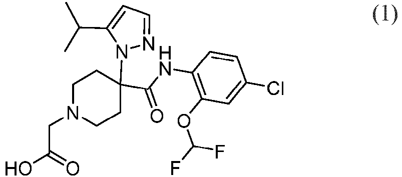 | 2-[4-[[4-chloro-2-(difluoromethoxy)phenyl] carbamoyl]-4-(5-isopropylpyrazol-1-yl)-1-piperidyl]acetic acid | ¹H NMR (400 MHz, DMSO) δ 7.90 (d, J = 9.3 Hz, 1H), 7.49 (d, J = 1.6 Hz, 1H), 7.26 (dt, J = 7.1, 1.8 Hz, 2H), 7.13 (t, J = 73.2, 1.4 Hz, 1H), 6.27 (t, J = 1.7 Hz, 1H), 3.05 (s, 2H), 2.97 (p, J = 6.7 Hz, 3H), 2.83 (dd, J = 11.1, 5.2 Hz, 2H), 2.61 - 2.50 (m, 4H), 1.09 (dd, J = 6.7, 1.2 Hz, 6H). |
| 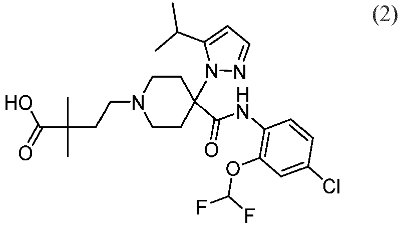 | 4-[4-[[4-chloro-2-(difluoromethoxy)phenyl] carbamoyl]-4-(5-isopropylpyrazol-1-yl)-1-piperidyl]-2,2-dimethyl-butanoic acid | ¹H NMR (500 MHz, DMSO) δ 8.13 (s, 1H), 7.86 - 7.79 (m, 1H), 7.53 (d, *J =* 1.9 Hz, 1H), 7.34 - 7.30 (m, 2H), 7.15 (t, *J =* 73.2 Hz, 1H), 6.32 (d, *J* = 1.9 Hz, 1H), 2.99 - 2.88 (m, 1H), 2.78 - 2.71 (m, 2H), 2.52 - 2.45 (m, 2H), 2.45 - 2.32 (m, 4H), 2.27 (t, *J =* 7.5 Hz, 2H), 1.63 (t, *J =* 7.4 Hz, 2H), 1.13 - 1.07 (m, 12H). |
| 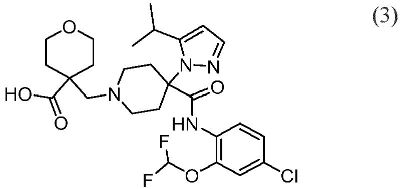 | 4-[[4-[[4-chloro-2-(difluoromethoxy)phenyl] carbamoyl]-4-(5-isopropylpyrazol-1-yl)-1-piperidyl]methyl]tetrahyd ropyran-4-carboxylic acid | ¹H NMR (500 MHz, DMSO-d6) δ 12.55 (bs, 1H), 8.11 (s, 1H), 7.80 (d, *J* = 9.4 Hz, 1H), 7.52 (d, *J* = 1.8 Hz, 1H), 7.32 (m, 2H), 7.14 (t, *J =* 73.2 Hz, 1H), 6.32 (d, J = 1.8 Hz, 1H), 3.69 (m, 2H), 3.37 (m, 4H), 2.91 (m, 1H), 2.72 (m, 2H), 2.45 (m, 4H), 2.35 (m, 2H), 1.87 (m, 2H), 1.46 (m, 2H), 1.09 (d, *J =* 6.7 Hz, 6H). |
| 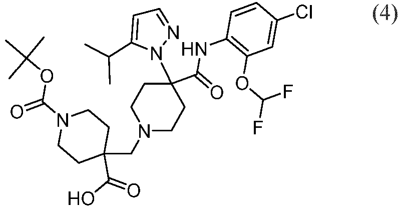 | 1-*tert*-butoxycarbonyl-4-[[4-[[4-chloro-2-(difluoromethoxy)phenyl] carbamoyl]-4-(5-isopropylpyrazol-1-yl)-1-piperidyl]methyl]piperidi ne-4-carboxylic acid | ¹H NMR (500 MHz, DMSO-d6) δ 8.04 (bs, 1H), 7.83 (d, *J* = 9.4 Hz, 1H), 7.51 (d, *J* = 1.8 Hz, 1H), 7.31 (m, 2H), 7.14 (t, *J* = 73.2 Hz, 1H), 6.31 (d, J = 1.8 Hz, 1H), 3.61 (m, 4H), 2.91 (m, 1H), 2.71 (m, 2H), 2.40 (m, 6H), 1.87 (m, 2H), 1.76 (m, 4H), 1.38 (s, 9H), 1.08 (d, *J =* 6.7 Hz, 6H). |
| 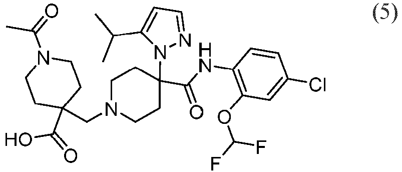 | 1-acetyl-4-[[4-[[4-chloro-2-(difluoromethoxy)phenyl] carbamoyl]-4-(5-isopropylpyrazol-1-yl)-1-piperidyl]methyl]piperidi ne-4-carboxylic acid | ¹H NMR (600 MHz, DMSO-d6) δ 8.12 (s, 1H), 7.80 (d, *J =* 9.4 Hz, 1H), 7.52 (d, *J =* 1.8 Hz, 1H), 7.32 (m, 2H), 7.14 (t, *J* = 73.2 Hz, 1H), 6.32 (d, J = 1.8 Hz, 1H), 4.01 (m, 1H), 3.62 (m, 1H), 3.15 (m, 1H), 2.91 (m, 1H), 2.77 (m, 1H), 2.72 (m, 2H), 2.46 (m, 3H), 2.34 (m, 2H), 1.97 (s, 3H), 1.96 (m, 1H), 1.88 (m, 1H), 1.41 (m, 1H), 1.28 (m, 1H), 1.09 (d, *J =* 6.7 Hz, 6H). |
| 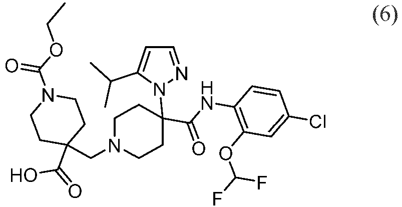 | 4-[[4-[[4-chloro-2-(difluoromethoxy)phenyl] carbamoyl]-4-(5-isopropylpyrazol-1-yl)-1-piperidyl]methyl]-1-ethoxycarbonyl-piperidine-4-carboxylic acid | ¹H NMR (600 MHz, DMSO-d6) δ 8.11 (s, 1H), 7.80 (d, *J =* 9.4 Hz, 1H), 7.52 (d, *J =* 1.8 Hz, 1H), 7.32 (m, 2H), 7.14 (t, *J* = 73.2 Hz, 1H), 6.32 (d, J = 1.8 Hz, 1H), 4.01 (d, J = 7.1 Hz, 2H), 3.73 (m, 2H), 3.17 (s, 2H), 2.95 (m, 2H), 2.92 (m, 1H), 2.72 (m, 2H), 2.44 (m, 4H), 2.35 (m, 2H), 1.90 (m, 2H), 1.35 (m, 2H), 1.16 (t, J = 7.1 Hz, 3H), 1.09 (d, *J =* 6.7 Hz, 6H). |
| 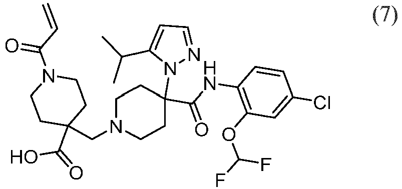 | 4-[[4-[[4-chloro-2-(difluoromethoxy)phenyl] carbamoyl]-4-(5-isopropylpyrazol-1-yl)-1-piperidyl]methyl]-1-prop-2-enoyl-piperidine-4-carboxylic acid | ¹H NMR (600 MHz, DMSO-d6) δ 8.11 (s, 1H), 7.80 (d, *J =* 9.4 Hz, 1H), 7.52 (d, *J =* 1.8 Hz, 1H), 7.32 (m, 2H), 7.14 (t, *J* = 73.2 Hz, 1H), 6.77 (q, J = 16.7, 10.5 Hz, 1H), 6.32 (d, J = 1.8 Hz, 1H), 6.07 (q, J = 16.7, 2.5 Hz, 1H), 5.64 (q, J = 10.5, 2.5 Hz, 1H), 4.09 (m, 1H), 3.84 (m, 1H), 3.21 (m, 1H), 2.91 (m, 2H), 2.72 (m, 2H), 2.45 (m, 6H), 2.34 (m, 2H), 1.94 (m, 2H), 1.35 (m, 2H), 1.09 (d, *J =* 6.7 Hz, 6H). |
| 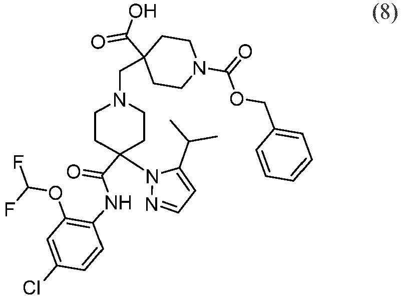 | 1-benzyloxycarbonyl-4-[[4-[[4-chloro-2-(difluoromethoxy)phenyl] carbamoyl]-4-(5-isopropylpyrazol-1-yl)-1-piperidyl]methyl]piperidi ne-4-carboxylic acid | ¹H NMR (500 MHz, DMSO-d6) δ 12.60 (bs, 1H), 8.12 (s, 1H), 7.80 (d, *J* = 9.5 Hz, 1H), 7.52 (d, *J =* 1.8 Hz, 1H), 7.34 (m, 7H), 7.14 (t, *J* = 73.2 Hz, 1H), 6.32 (d, J = 1.9 Hz, 1H), 5.06 (s, 2H), 3.77 (m, 2H), 3.00 (m, 2H), 2.91 (m, 3H), 2.71 (m, 2H), 2.46 (m, 6H), 2.35 (m, 2H), 1.91 (m, 2H), 1.37 (m, 2H), 1.09 (d, *J =* 6.6 Hz, 6H). |
| 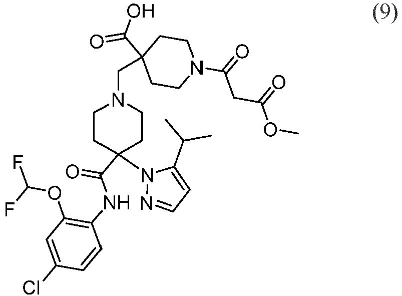 | 4-[[4-[[4-chloro-2-(difluoromethoxy)phenyl] carbamoyl]-4-(5-isopropylpyrazol-1-yl)-1-piperidyl]methyl]-1-(3-methoxy-3-oxo-propanoyl)piperidine-4-carboxylic acid | ¹H NMR (600 MHz, DMSO-d6) δ 8.11 (s, 1H), 7.80 (d, *J =* 9.4 Hz, 1H), 7.52 (d, *J =* 1.8 Hz, 1H), 7.32 (m, 2H), 7.14 (t, *J* = 73.2 Hz, 1H), 6.32 (d, J = 1.8 Hz, 1H), 4.01 (m, 1H), 3.62 (s, 3H), 3.58 (m, 1H), 3.52 (s, 2H), 3.26 (m, 2H), 2.84 (m, 1H), 2.71 (m, 2H), 2.72 (m, 2H), 2.47 (m, 4H), 2.34 (m, 2H), 1.92 (m, 2H), 1.43 (m, 1H), 1.32 (m, 1H), 1.24 (m, 1H), 1.09 (d, *J =* 6.7 Hz, 6H). |
| 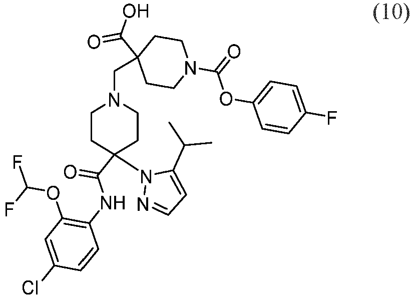 | 4-[[4-[[4-chloro-2-(difluoromethoxy)phenyl] carbamoyl]-4-(5-isopropylpyrazol-1-yl)-1-piperidyl]methyl]-1-(4-fluorophenoxy)carbonyl-piperidine-4-carboxylic acid | ¹H NMR (400 MHz, DMSO-d6) δ 8.13 (s, 1H), 7.80 (d, *J =* 9.4 Hz, 1H), 7.53 (d, *J =* 1.9 Hz, 1H), 7.33 (m, 2H), 7.18 (m, 5H), 6.33 (d, J = 2.0 Hz, 1H), 3.93 (m, 1H), 3.79 (m, 1H), 3.03 (m, 1H), 2.92 (m, 1H), 2.75 (m, 2H), 2.41 (m, 7H), 1.99 (m, 2H), 1.48 (m, 2H), 1.23 (m, 2H), 1.09 (d, *J =* 6.7 Hz, 6H). |
| 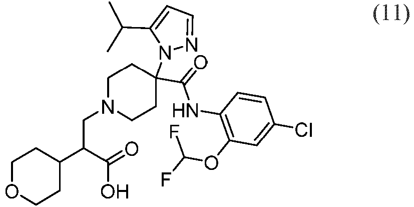 | 3-[4-[[4-chloro-2-(difluoromethoxy)phenyl] carbamoyl]-4-(5-isopropylpyrazol-1-yl)-1-piperidyl]-2-tetrahydropyran-4-yl-propanoic acid | ¹H NMR (600 MHz, DMSO-d6) δ 8.14 (s, 1H), 7.80 (d, *J =* 9.4 Hz, 1H), 7.52 (t, *J* = 1.9 Hz, 1H), 7.32 (m, 2H), 7.15 (t, *J* = 73.1 Hz, 1H), 6.32 (s, 1H), 3.81 (m, 2H), 3.24 (m, 2H), 2.93 (m, 1H), 2.85 (m, 1H), 2.63 (m, 1H), 2.55 (m, 1H), 2.47 (m, 3H), 2.36 (m, 3H), 2.26 (m, 1H), 1.71 (m, 1H), 1.56 (m, 1H), 1.41 (m, 1H), 1.28 (m, 3H), 1.10 (d, *J* = 6.7 Hz, 6H). |
| 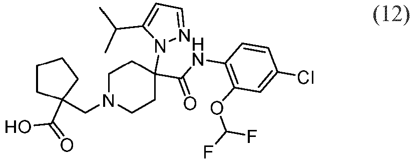 | 1-[[4-[[4-chloro-2-(difluoromethoxy)phenyl] carbamoyl]-4-(5-isopropylpyrazol-1-yl)-1-piperidyl]methyl]cyclope ntanecarboxylic acid | ¹H NMR (400 MHz, DMSO-d6) δ 8.15 (s, 1H), 7.80 (d, *J =* 9.4 Hz, 1H), 7.53 (d, *J =* 1.8 Hz, 1H), 7.32 (m, 2H), 7.15 (t, *J* = 73.2 Hz, 1H), 6.32 (d, J = 1.8 Hz, 1H), 2.92 (m, 1H), 2.76 (m, 2H), 2.49 (m, 7H), 2.35 (m, 2H), 1.96 (m, 2H), 1.51 (m, 5H), 1.09 (d, *J =* 6.7 Hz, 6H). |
| 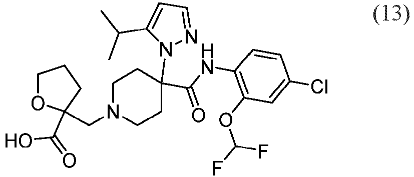 | 2-[[4-[[4-chloro-2-(difluoromethoxy)phenyl] carbamoyl]-4-(5-isopropylpyrazol-1-yl)-1-piperidyl]methyl]tetrahyd rofuran-2-carboxylic acid | ¹H NMR (500 MHz, DMSO-d6) δ 8.14 (s, 1H), 7.80 (d, *J =* 9.4 Hz, 1H), 7.53 (d, *J =* 1.8 Hz, 1H), 7.32 (m, 2H), 7.15 (t, *J* = 73.2 Hz, 1H), 6.32 (d, J = 1.8 Hz, 1H), 3.79 (m, 2H), 3.17 (m, 1H), 3.06 (m, 1H), 2.91 (m, 1H), 2.79 (m, 2H), 2.65 (m, 1H), 2.46 (m, 3H), 2.35 (m, 2H), 2.13 (m, 1H), 1.78 (m, 3H), 1.09 (d, *J =* 6.7 Hz, 6H). |
| 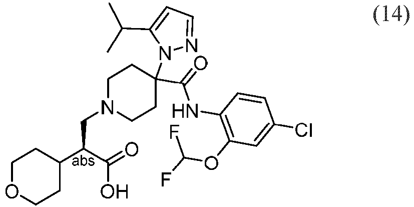 | (2*S*)-3-[4-[[4-chloro-2-(difluoromethoxy)phenyl] carbamoyl]-4-(5-isopropylpyrazol-1-yl)-1-piperidyl]-2-tetrahydropyran-4-yl-propanoic acid | ¹H NMR (400 MHz, DMSO-d6) δ 8.14 (s, 1H), 7.80 (d, *J =* 9.4 Hz, 1H), 7.52 (t, *J* = 1.9 Hz, 1H), 7.32 (m, 2H), 7.15 (t, *J =* 73.1 Hz, 1H), 6.32 (s, 1H), 3.81 (m, 2H), 3.24 (m, 2H), 2.91 (m, 1H), 2.86 (m, 1H), 2.63 (m, 1H), 2.51 (m, 4H), 2.34 (m, 4H), 1.72 (m, 1H), 1.56 (m, 1H), 1.41 (m, 1H), 1.29 (m, 3H), 1.10 (d, *J* = 6.7 Hz, 6H). |
| 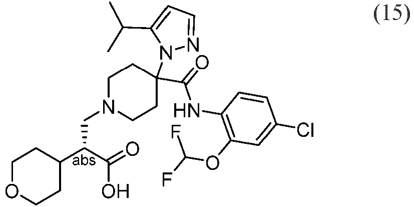 | (2*R*)-3-[4-[[4-chloro-2-(difluoromethoxy)phenyl] carbamoyl]-4-(5-isopropylpyrazol-1-yl)-1-piperidyl]-2-tetrahydropyran-4-yl-propanoic acid | ¹H NMR (400 MHz, DMSO-d6) δ 8.14 (s, 1H), 7.81 (d, *J* = 9.4 Hz, 1H), 7.53 (t, *J =* 1.9 Hz, 1H), 7.32 (m, 2H), 7.15 (t, *J* = 73.1 Hz, 1H), 6.32 (s, 1H), 3.82 (m, 2H), 3.23 (m, 2H), 2.93 (m, 1H), 2.86 (m, 1H), 2.63 (m, 1H), 2.51 (m, 4H), 2.35 (m, 4H), 1.72 (m, 1H), 1.57 (m, 1H), 1.41 (m, 1H), 1.29 (m, 3H), 1.10 (d, *J* = 6.7 Hz, 6H). |
| 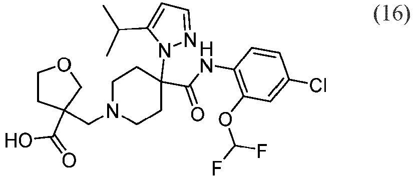 | 3-[[4-[[4-chloro-2-(difluoromethoxy)phenyl] carbamoyl]-4-(5-isopropylpyrazol-1-yl)-1-piperidyl]methyl]tetrahyd rofuran-3-carboxylic acid | ¹H NMR (400 MHz, DMSO-d6) δ 8.16 (s, 1H), 7.79 (d, *J* = 9.4 Hz, 1H), 7.53 (d, *J =* 1.8 Hz, 1H), 7.32 (m, 2H), 7.15 (t, *J* = 73.2 Hz, 1H), 6.32 (d, J = 1.8 Hz, 1H), 3.96 (d, J = 8.5 Hz, 1H), 3.71 (m, 2H), 3.49 (d, J = 8.6 Hz, 1H), 2.92 (m, 1H), 2.74 (m, 2H), 2.62 (s, 2H), 2.45 (m, 2H), 2.36 (m, 2H), 2.26 (m, 2H), 1.82 (m, 2H), 1.09 (d, *J =* 6.7 Hz, 6H). |
| 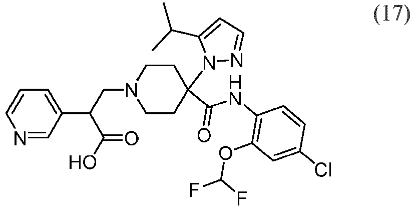 | 3-[4-[[4-chloro-2-(difluoromethoxy)phenyl] carbamoyl]-4-(5-isopropylpyrazol-1-yl)-1-piperidyl]-2-(3-pyridyl)propanoic acid | ¹H NMR (600 MHz, DMSO) δ 8.50 (dd, J = 2.3, 0.9 Hz, 1H), 8.45 (dd, J = 4.8, 1.6 Hz, 1H), 8.20 (s, 1H), 7.77 (d, J = 9.3 Hz, 1H), 7.73 (dt, J = 7.9, 2.0 Hz, 1H), 7.52 (d, J = 1.5 Hz, 1H), 7.34 (ddd, J = 7.9, 4.8, 0.9 Hz, 1H), 7.33 - 7.31 (m, 1H), 7.15 (t, J = 73.2 Hz, 1H), 6.32 (d, J = 1.9 Hz, 1H), 3.89 (dd, J = 9.3, 6.5 Hz, 1H), 2.99 (dd, J = 12.5, 9.4 Hz, 1H), 2.95 - 2.86 (m, 2H), 2.79 (dd, J = 11.4, 5.6 Hz, 1H), 2.57 (dd, J = 12.4, 6.5 Hz, 1H), 2.54 - 2.41 (m, 5H), 2.40 - 2.28 (m, 2H), 1.10 (dd, J = 6.9, 1.4 Hz, 6H). |
| 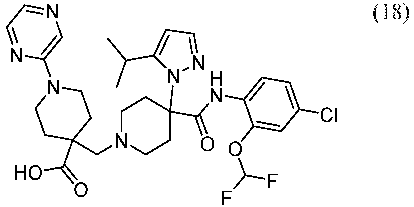 | 4-[[4-[[4-chloro-2-(difluoromethoxy)phenyl] carbamoyl]-4-(5-isopropylpyrazol-1-yl)-1-piperidyl]methyl]-1-pyrazin-2-yl-piperidine-4-carboxylic acid | ¹H NMR (500 MHz, DMSO) δ 1.15 (d, J = 6.7 Hz, 6H), 1.74 (ddd, J = 13.4, 9.1, 3.9 Hz, 2H), 2.07 - 2.15 (m, 2H), 2.80 (q, J = 12.8 Hz, 4H), 2.89 - 2.97 (m, 1H), 3.63 - 3.43 (m, 10H), 3.85 - 3.92 (m, 2H), 6.39 (d, J = 1.9 Hz, 1H), 7.13 (t, J = 73.2 Hz, 1H), 7.30 - 7.36 (m, 2H), 7.60 (s, 1H), 7.78 (d, J = 9.1 Hz, 1H), 7.82 (d, J = 2.6 Hz, 1H), 8.07 (dd, J = 2.6, 1.5 Hz, 1H), 8.30 (d, J = 1.5 Hz, 1H). |
| 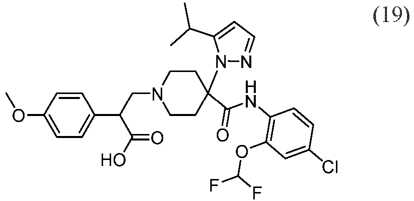 | 3-[4-[[4-chloro-2-(difluoromethoxy)phenyl] carbamoyl]-4-(5-isopropylpyrazol-1-yl)-1-piperidyl]-2-(4-methoxyphenyl)propanoi c acid | ¹H NMR (400 MHz, DMSO) δ 8.17 (s, 1H), 7.79 (d, *J* = 9.4 Hz, 1H), 7.58 - 7.49 (m, 1H), 7.37 - 7.28 (m, 2H), 7.26 - 7.19 (m, 2H), 7.15 (t, *J =* 73.3 Hz, 1H) 6.90 - 6.82 (m, 2H), 6.38 - 6.29 (m, 1H), 3.72 (s, 4H), 2.94 (ddd, *J =* 18.2, 13.1, 8.5 Hz, 4H), 2.78 - 2.70 (m, 1H), 2.49 - 2.31 (m, 7H), 1.15 - 1.06 (m, 6H). |
| 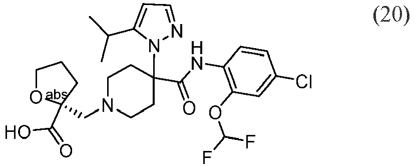 | (2*R*)-2-[[4-[[4-chloro-2-(difluoromethoxy)phenyl] carbamoyl]-4-(5-isopropylpyrazol-1-yl)-1-piperidyl]methyl]tetrahyd rofuran-2-carboxylic acid | ¹H NMR (600 MHz, DMSO-d6) δ 8.14 (s, 1H), 7.80 (d, *J =* 9.4 Hz, 1H), 7.53 (d, *J =* 1.8 Hz, 1H), 7.32 (m, 2H), 7.15 (t, *J* = 73.2 Hz, 1H), 6.32 (d, J = 1.8 Hz, 1H), 3.79 (m, 2H), 3.17 (m, 1H), 3.06 (m, 1H), 2.92 (m, 1H), 2.79 (m, 2H), 2.65 (m, 1H), 2.46 (m, 3H), 2.35 (m, 2H), 2.13 (m, 1H), 1.77 (m, 3H), 1.09 (d, *J =* 6.7 Hz, 6H). |
| 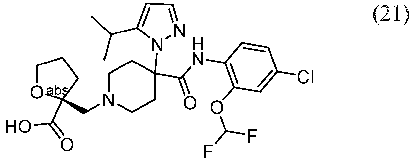 | (2*S*)-2-[[4-[[4-chloro-2-(difluoromethoxy)phenyl] carbamoyl]-4-(5-isopropylpyrazol-1-yl)-1-piperidyl]methyl]tetrahyd rofuran-2-carboxylic acid | ¹H NMR (600 MHz, DMSO-d6) δ 8.14 (s, 1H), 7.80 (d, *J =* 9.4 Hz, 1H), 7.53 (d, *J =* 1.8 Hz, 1H), 7.32 (m, 2H), 7.15 (t, *J* = 73.2 Hz, 1H), 6.32 (d, J = 1.8 Hz, 1H), 3.79 (m, 2H), 3.17 (m, 1H), 3.06 (m, 1H), 2.92 (m, 1H), 2.79 (m, 2H), 2.65 (m, 1H), 2.46 (m, 3H), 2.35 (m, 2H), 2.13 (m, 1H), 1.77 (m, 3H), 1.09 (d, *J =* 6.7 Hz, 6H). |
| 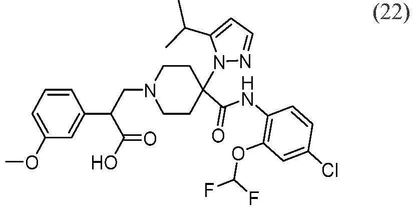 | 3-[4-[[4-chloro-2-(difluoromethoxy)phenyl] carbamoyl]-4-(5-isopropylpyrazol-1-yl)-1-piperidyl]-2-(3-methoxyphenyl)propanoi c acid | ¹H NMR (400 MHz, DMSO) δ 7.82 (s, 1H), 7.58 - 7.42 (m, 1H), 7.33 - 7.22 (m, 3H), 7.15 (t, 73.3 Hz, 1H), 7.06 (d, *J* = 6.0 Hz, 1H), 6.87 (dd, *J* = 4.5, 2.4 Hz, 3H), 6.80 (ddd, *J =* 8.3, 2.5, 1.0 Hz, 1H), 6.28 (d, *J* = 1.9 Hz, 1H), 3.78 - 3.74 (m, 3H), 3.67 - 3.57 (m, 2H), 3.24 - 3.04 (m, 5H), 2.98 - 2.87 (m, 4H), 2.75 (d, *J* = 5.2 Hz, 1H), 1.16 - 1.09 (m, 6H). |
| 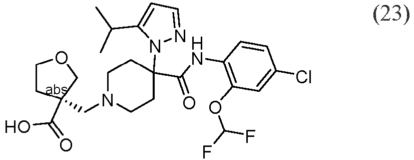 | (3*R*)-3-[[4-[[4-chloro-2-(difluoromethoxy)phenyl] carbamoyl]-4-(5-isopropylpyrazol-1-yl)-1-piperidyl]methyl]tetrahyd rofuran-3-carboxylic acid | ¹H NMR (400 MHz, DMSO) δ 7.82 (s, 1H), 7.58 - 7.42 (m, 1H), 7.33 - 7.22 (m, 3H), 7.15 (t, 73.3 Hz, 1H), 7.06 (d, *J* = 6.0 Hz, 1H), 6.87 (dd, *J* = 4.5, 2.4 Hz, 3H), 6.80 (ddd, *J =* 8.3, 2.5, 1.0 Hz, 1H), 6.28 (d, *J* = 1.9 Hz, 1H), 3.78 - 3.74 (m, 3H), 3.67 - 3.57 (m, 2H), 3.24 - 3.04 (m, 5H), 2.98 - 2.87 (m, 4H), 2.75 (d, *J* = 5.2 Hz, 1H), 1.16 - 1.09 (m, 6H). |
| 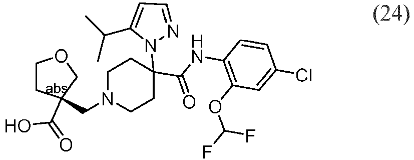 | (3*S*)-3-[[4-[[4-chloro-2-(difluoromethoxy)phenyl] carbamoyl]-4-(5-isopropylpyrazol-1-yl)-1-piperidyl]methyl]tetrahyd rofuran-3-carboxylic acid | ¹H NMR (400 MHz, DMSO-d6) δ 8.16 (s, 1H), 7.79 (d, *J* = 9.4 Hz, 1H), 7.53 (d, *J =* 1.8 Hz, 1H), 7.32 (m, 2H), 7.15 (t, *J* = 73.2 Hz, 1H), 6.32 (d, J = 1.8 Hz, 1H), 3.96 (d, J = 8.5 Hz, 1H), 3.71 (m, 2H), 3.49 (d, J = 8.6 Hz, 1H), 2.92 (m, 1H), 2.74 (m, 2H), 2.62 (s, 2H), 2.45 (m, 2H), 2.36 (m, 2H), 2.26 (m, 2H), 1.82 (m, 2H), 1.09 (d, *J =* 6.7 Hz, 6H). |
| 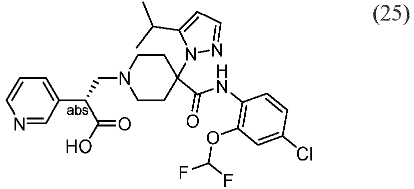 | (2*R*)-3-[4-[[4-chloro-2-(difluoromethoxy)phenyl] carbamoyl]-4-(5-isopropylpyrazol-1-yl)-1-piperidyl]-2-(3-pyridyl)propanoic acid | ¹H NMR (600 MHz, DMSO) δ 8.50 (dd, J = 2.3, 0.9 Hz, 1H), 8.45 (dd, J = 4.8, 1.6 Hz, 1H), 8.20 (s, 1H), 7.77 (d, J = 9.3 Hz, 1H), 7.73 (dt, J = 7.9, 2.0 Hz, 1H), 7.52 (d, J = 1.5 Hz, 1H), 7.34 (ddd, J = 7.9, 4.8, 0.9 Hz, 1H), 7.33 - 7.31 (m, 1H), 7.15 (t, J = 73.2 Hz, 1H), 6.32 (d, J = 1.9 Hz, 1H), 3.89 (dd, J = 9.3, 6.5 Hz, 1H), 2.99 (dd, J = 12.5, 9.4 Hz, 1H), 2.95 - 2.86 (m, 2H), 2.79 (dd, J = 11.4, 5.6 Hz, 1H), 2.57 (dd, J = 12.4, 6.5 Hz, 1H), 2.54 - 2.41 (m, 5H), 2.40 - 2.28 (m, 2H), 1.10 (dd, J = 6.9, 1.4 Hz, 6H). |
| 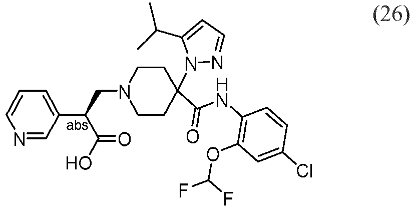 | (2*S*)-3-[4-[[4-chloro-2-(difluoromethoxy)phenyl] carbamoyl]-4-(5-isopropylpyrazol-1-yl)-1-piperidyl]-2-(3-pyridyl)propanoic acid | ¹H NMR (600 MHz, DMSO) δ 8.50 (dd, J = 2.3, 0.9 Hz, 1H), 8.45 (dd, J = 4.8, 1.6 Hz, 1H), 8.20 (s, 1H), 7.77 (d, J = 9.3 Hz, 1H), 7.73 (dt, J = 7.9, 2.0 Hz, 1H), 7.52 (d, J = 1.5 Hz, 1H), 7.34 (ddd, J = 7.9, 4.8, 0.9 Hz, 1H), 7.33 - 7.31 (m, 1H), 7.15 (t, J = 73.2 Hz, 1H), 6.32 (d, J = 1.9 Hz, 1H), 3.89 (dd, J = 9.3, 6.5 Hz, 1H), 2.99 (dd, J = 12.5, 9.4 Hz, 1H), 2.95 - 2.86 (m, 2H), 2.79 (dd, J = 11.4, 5.6 Hz, 1H), 2.57 (dd, J = 12.4, 6.5 Hz, 1H), 2.54 - 2.41 (m, 5H), 2.40 - 2.28 (m, 2H), 1.10 (dd, J = 6.9, 1.4 Hz, 6H). |
| 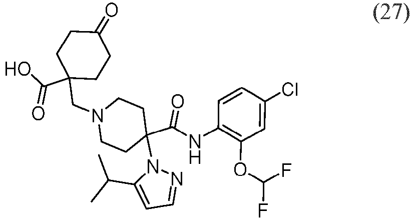 | 1-[[4-[[4-chloro-2-(difluoromethoxy)phenyl] carbamoyl]-4-(5-isopropylpyrazol-1-yl)-1-piperidyl]methyl]-4-oxo-cyclohexanecarboxylic acid | ¹H NMR (500 MHz, DMSO-d6) δ 8.13 (s, 1H), 7.80 (d, *J =* 9.4 Hz, 1H), 7.53 (d, *J =* 1.8 Hz, 1H), 7.33 (m, 2H), 7.14 (t, *J* = 73.2 Hz, 1H), 6.32 (d, J = 1.8 Hz, 1H), 2.92 (m, 1H), 2.75 (m, 2H), 2.54 (m, 4H), 2.46 (m, 2H), 2.35 (m, 4H), 2.20 (m, 4H), 1.70 (m, 2H), 1.09 (d, *J =* 6.7 Hz, 6H). |
| 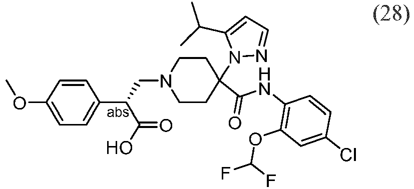 | (2*R*)-3-[4-[[4-chloro-2-(difluoromethoxy)phenyl] carbamoyl]-4-(5-isopropylpyrazol-1-yl)-1-piperidyl]-2-(4-methoxyphenyl)propanoi c acid | ¹H NMR (400 MHz, DMSO) δ 8.17 (s, 1H), 7.79 (d, J = 9.4 Hz, 1H), 7.58 - 7.49 (m, 1H), 7.37 - 7.28 (m, 2H), 7.26 - 7.19 (m, 2H), 7.15 (t, J = 73.3 Hz, 1H) 6.90 - 6.82 (m, 2H), 6.38 - 6.29 (m, 1H), 3.72 (s, 4H), 2.94 (ddd, J = 18.2, 13.1, 8.5 Hz, 4H), 2.78 - 2.70 (m, 1H), 2.49 - 2.31 (m, 7H), 1.15 - 1.06 (m, 6H). |
| 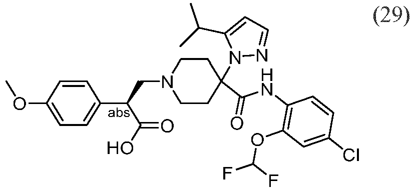 | (2*S*)-3-[4-[[4-chloro-2-(difluoromethoxy)phenyl] carbamoyl]-4-(5-isopropylpyrazol-1-yl)-1-piperidyl]-2-(4-methoxyphenyl)propanoi c acid | ¹H NMR (400 MHz, DMSO) δ 8.17 (s, 1H), 7.79 (d, *J* = 9.4 Hz, 1H), 7.58 - 7.49 (m, 1H), 7.37 - 7.28 (m, 2H), 7.26 - 7.19 (m, 2H), 7.15 (t, *J =* 73.3 Hz, 1H) 6.90 - 6.82 (m, 2H), 6.38 - 6.29 (m, 1H), 3.72 (s, 4H), 2.94 (ddd, *J =* 18.2, 13.1, 8.5 Hz, 4H), 2.78 - 2.70 (m, 1H), 2.49 - 2.31 (m, 7H), 1.15 - 1.06 (m, 6H). |
| 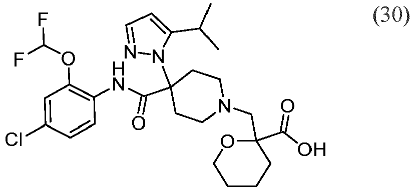 | 2-[[4-[[4-chloro-2-(difluoromethoxy)phenyl] carbamoyl]-4-(5-isopropylpyrazol-1-yl)-1-piperidyl]methyl]tetrahyd ropyran-2-carboxylic acid | ¹H NMR (400 MHz, DMSO) δ 8.17 (s, 1H), 7.79 (d, *J* = 9.4 Hz, 1H), 7.58 - 7.49 (m, 1H), 7.37 - 7.28 (m, 2H), 7.26 - 7.19 (m, 2H), 7.15 (t, *J =* 73.3 Hz, 1H) 6.90 - 6.82 (m, 2H), 6.38 - 6.29 (m, 1H), 3.72 (s, 4H), 2.94 (ddd, *J =* 18.2, 13.1, 8.5 Hz, 4H), 2.78 - 2.70 (m, 1H), 2.49 - 2.31 (m, 7H), 1.15 - 1.06 (m, 6H). |
| 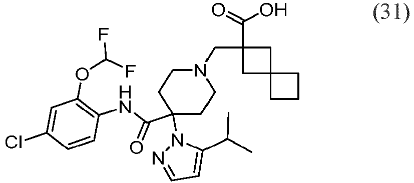 | 2-[[4-[[4-chloro-2-(difluoromethoxy)phenyl] carbamoyl]-4-(5-isopropylpyrazol-1-yl)-1-piperidyl]methyl]spiro[3. 3]heptane-2-carboxylic acid | ¹H NMR (400 MHz, DMSO) δ 8.07 (s, 1H), 7.79 (d, J = 9.3 Hz, 1H), 7.68 - 7.48 (m, 3H), 7.34 - 7.23 (m, 1H), 7.08 (s, 1H), 6.35 (d, J = 1.9 Hz, 1H), 3.36 - 3.25 (m, 1H), 3.08 (t, J = 10.8 Hz, 2H), 2.93 (p, J = 6.7 Hz, 1H), 2.77 - 2.58 (m, 4H), 2.50 - 2.37 (m, 7H), 2.18 - 2.07 (m, 3H), 1.80 - 1.69 (m, 3H), 1.13 (d, J = 6.7 Hz, 6H). |
| 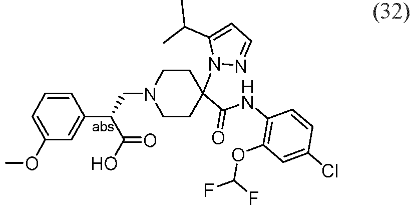 | (2*R*)-3-[4-[[4-chloro-2-(difluoromethoxy)phenyl] carbamoyl]-4-(5-isopropylpyrazol-1-yl)-1-piperidyl]-2-(3-methoxyphenyl)propanoi c acid | ¹H NMR (400 MHz, DMSO) δ 7.82 (s, 1H), 7.58 - 7.42 (m, 1H), 7.33 - 7.22 (m, 3H), 7.15 (t, 73.3 Hz, 1H), 7.06 (d, J = 6.0 Hz, 1H), 6.87 (dd, J = 4.5, 2.4 Hz, 3H), 6.80 (ddd, J = 8.3, 2.5, 1.0 Hz, 1H), 6.28 (d, J = 1.9 Hz, 1H), 3.78 - 3.74 (m, 3H), 3.67 - 3.57 (m, 2H), 3.24 - 3.04 (m, 5H), 2.98 - 2.87 (m, 4H), 2.75 (d, J = 5.2 Hz, 1H), 1.16 - 1.09 (m, 6H). |
| 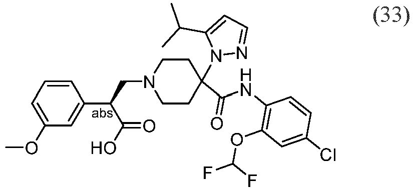 | (2*S*)-3-[4-[[4-chloro-2-(difluoromethoxy)phenyl] carbamoyl]-4-(5-isopropylpyrazol-1-yl)-1-piperidyl]-2-(3-methoxyphenyl)propanoi c acid | ¹H NMR (400 MHz, DMSO) δ 7.82 (s, 1H), 7.58 - 7.42 (m, 1H), 7.33 - 7.22 (m, 3H), 7.15 (t, 73.3 Hz, 1H), 7.06 (d, J = 6.0 Hz, 1H), 6.87 (dd, J = 4.5, 2.4 Hz, 3H), 6.80 (ddd, J = 8.3, 2.5, 1.0 Hz, 1H), 6.28 (d, J = 1.9 Hz, 1H), 3.78 - 3.74 (m, 3H), 3.67 - 3.57 (m, 2H), 3.24 - 3.04 (m, 5H), 2.98 - 2.87 (m, 4H), 2.75 (d, J = 5.2 Hz, 1H), 1.16 - 1.09 (m, 6H). |
| 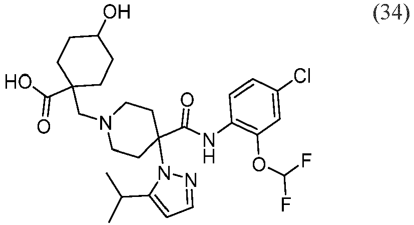 | 1-[[4-[[4-chloro-2-(difluoromethoxy)phenyl] carbamoyl]-4-(5-isopropylpyrazol-1-yl)-1-piperidyl]methyl]-4-hydroxy-cyclohexanecarboxylic acid | ¹H NMR (600 MHz, DMSO-d6) δ 12.12 (bs, 1H), 8.08 (s, 1H), 7.82 (d, *J* = 9.4 Hz, 1H), 7.52 (d, *J* = 1.8 Hz, 1H), 7.32 (m, 2H), 7.14 (t, *J* = 73.2 Hz, 1H), 6.32 (d, J = 1.8 Hz, 1H), 4.42 (m, 1H), 2.91 (m, 1H), 2.72 (m, 2H), 2.45 (m, 4H), 2.35 (m, 3H), 2.00 (m, 2H), 1.66 (m, 2H), 1.24 (m, 2H), 1.14 (m, 2H), 1.09 (d, *J* = 6.7 Hz, 6H). |
| 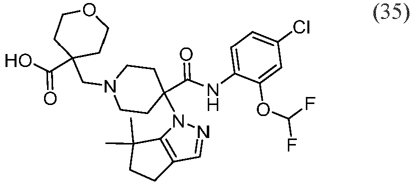 | 4-[[4-[[4-chloro-2-(difluoromethoxy)phenyl] carbamoyl]-4-(6,6-dimethyl-4,5-dihydrocyclopenta[c]pyra zol-1-yl)-1-piperidyl]methyl]tetrahyd ropyran-4-carboxylic acid | ¹H NMR (400 MHz, DMSO) δ 1.15 (s, 6H), 1.59 - 1.64 (m, 2H), 1.91 - 1.99 (m, 2H), 2.27 (t, J = 6.7 Hz, 2H), 2.54 (d, J = 6.3 Hz, 1H), 2.72 - 2.78 (m, 5H), 3.06 (s, 2H), 3.46 - 3.63 (m, 4H), 3.63 - 3.75 (m, 4H), 7.00 - 7.41 (m, 4H), 7.49 (s, 1H), 8.03 (s, 1H), 13.42 (s, 1H). |
| 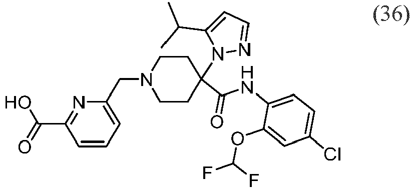 | 6-[[4-[[4-chloro-2-(difluoromethoxy)phenyl] carbamoyl]-4-(5-isopropylpyrazol-1-yl)-1-piperidyl]methyl]pyridine -2-carboxylic acid | ¹H NMR (400 MHz, DMSO) δ 7.92 (d, *J* = 9.4 Hz, 1H), 7.89 - 7.79 (m, 2H), 7.60 (d, *J* = 7.2 Hz, 1H), 7.52 (d, *J* = 1.9 Hz, 1H), 7.30 - 7.25 (m, 2H), 7.14 (t, *J* = 73.2 Hz, 1H), 6.29 (d, *J* = 1.9 Hz, 1H), 3.62 (s, 2H), 3.04 - 2.96 (m, 3H), 2.76 (q, *J =* 9.3 Hz, 2H), 2.61 - 2.52 (m, 2H), 2.47 (m, 2H), 1.11 (d, *J =* 6.7 Hz, 6H). |
| 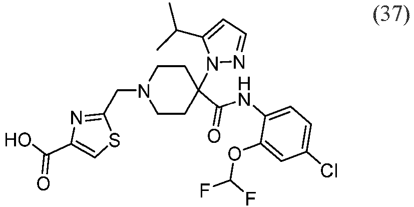 | 2-[[4-[[4-chloro-2-(difluoromethoxy)phenyl] carbamoyl]-4-(5-isopropylpyrazol-1-yl)-1-piperidyl]methyl]thiazole -4-carboxylic acid | ¹H NMR (400 MHz, DMSO) δ 8.17 (s, 1H), 7.93 - 7.88 (m, 1H), 7.52 (d, J = 1.8 Hz, 1H), 7.31 - 7.26 (m, 2H), 7.15 (t, J = 73.2 Hz, 1H), 6.29 (d, J = 1.9 Hz, 1H), 3.80 (s, 2H), 3.00 - 2.95 (m, 3H), 2.85 (dd, J = 10.5, 5.7 Hz, 2H), 2.59 (t, J = 9.8 Hz, 4H), 1.11 (d, J = 6.7 Hz, 6H). |
| 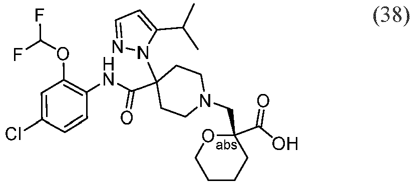 | (2*R*)-2-[[4-[[4-chloro-2-(difluoromethoxy)phenyl] carbamoyl]-4-(5-isopropylpyrazol-1-yl)-1-piperidyl]methyl]tetrahyd ropyran-2-carboxylic acid | ¹H NMR (400 MHz, DMSO) δ 8.01 (s, 1H), 7.82 (d, *J =* 9.4 Hz, 1H), 7.56 (d, *J* = 1.9 Hz, 1H), 7.44 - 7.14 (m, 2H), 7.07 (s, 1H), 6.88 (s, 0H), 6.34 (d, *J* = 1.9 Hz, 1H), 3.81 (qt, *J* = 11.8, 5.2 Hz, 2H), 3.65 - 3.19 (m, 2H), 3.17 - 3.05 (m, 2H), 2.73 - 2.60 (m, 3H), 2.48 (p, *J* = 1.9 Hz, 7H), 1.56 - 1.40 (m, 3H), 1.12 (dd, *J* = 6.6, 2.8 Hz, 6H). |
| 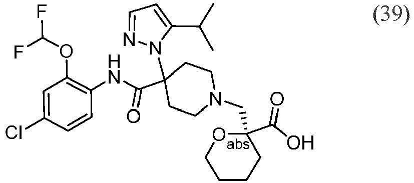 | (2*S*)-2-[[4-[[4-chloro-2-(difluoromethoxy)phenyl] carbamoyl]-4-(5-isopropylpyrazol-1-yl)-1-piperidyl]methyl]tetrahyd ropyran-2-carboxylic acid | ¹H NMR (400 MHz, DMSO) δ 8.01 (s, 1H), 7.82 (d, J = 9.4 Hz, 1H), 7.56 (d, J = 1.9 Hz, 1H), 7.44 - 7.14 (m, 2H), 7.07 (s, 1H), 6.88 (s, 0H), 6.34 (d, J = 1.9 Hz, 1H), 3.81 (qt, J = 11.8, 5.2 Hz, 2H), 3.65 - 3.19 (m, 2H), 3.17 - 3.05 (m, 2H), 2.73 - 2.60 (m, 3H), 2.48 (p, J = 1.9 Hz, 7H), 1.56 - 1.40 (m, 3H), 1.12 (dd, J = 6.6, 2.8 Hz, 6H). |
| 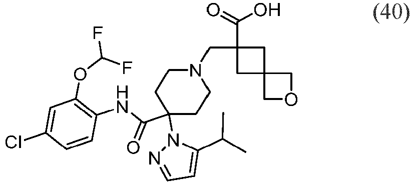 | 6-[[4-[[4-chloro-2-(difluoromethoxy)phenyl] carbamoyl]-4-(5-isopropylpyrazol-1-yl)-1-piperidyl]methyl]-2-oxaspiro[3.3]heptane-6-carboxylic acid | ¹H NMR (400 MHz, DMSO) δ 8.07 (s, 1H), 7.79 (d, *J* = 9.3 Hz, 1H), 7.68 - 7.48 (m, 3H), 7.34 - 7.23 (m, 1H), 7.08 (s, 1H), 6.35 (d, *J* = 1.9 Hz, 1H), 3.36 - 3.25 (m, 1H), 3.08 (t, *J =* 10.8 Hz, 2H), 2.93 (p, *J* = 6.7 Hz, 1H), 2.77 - 2.58 (m, 4H), 2.50 - 2.37 (m, 5H), 2.18 - 2.07 (m, 3H), 1.80 - 1.69 (m, 3H), 1.13 (d, *J =* 6.7 Hz, 6H). |
| 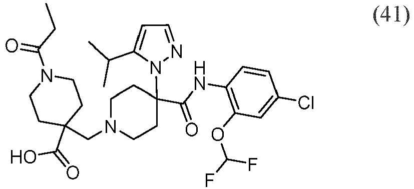 | 4-[[4-[[4-chloro-2-(difluoromethoxy)phenyl] carbamoyl]-4-(5-isopropylpyrazol-1-yl)-1-piperidyl]methyl]-1-propanoyl-piperidine-4-carboxylic acid | ¹H NMR (600 MHz, DMSO) δ 7.65 (s, 1H), 7.61 (s, 1H), 7.34 (m, 2H), 7.18 (t, J = 73.2 Hz, 1H), 6.41 (s, 1H), 3.73 (s, 1H), 3.66 - 3.02 (m, 10H), 2.87 (s, 1H), 2.68 (s, 4H), 2.31 (p, J = 6.7 Hz, 2H), 1.95 (dd, J = 45.6, 13.7 Hz, 2H), 1.55 (d, J = 43.2 Hz, 2H), 1.13 (d, J = 6.6 Hz, 6H), 0.98 (t, J = 7.4 Hz, 3H). |
| 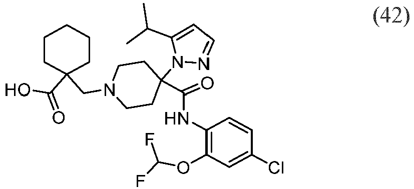 | 1-[[4-[[4-chloro-2-(difluoromethoxy)phenyl] carbamoyl]-4-(5-isopropylpyrazol-1-yl)-1-piperidyl]methyl]cyclohe xanecarboxylic acid | ¹H NMR (400 MHz, DMSO-d6) δ 8.11 (s, 1H), 7.81 (d, *J* = 9.4 Hz, 1H), 7.52 (d, *J =* 1.8 Hz, 1H), 7.31 (m, 2H), 7.15 (t, *J* = 73.2 Hz, 1H), 6.32 (d, J = 1.8 Hz, 1H), 3.17 (s, 2H), 2.91 (m, 1H), 2.71 (m, 2H), 2.38 (m, 6H), 1.89 (m, 2H), 1.49 (m, 3H), 1.33 (m, 2H), 1.22 (m, 3H), 1.09 (d, *J =* 6.7 Hz, 6H). |
| 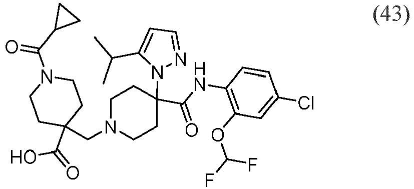 | 4-[[4-[[4-chloro-2-(difluoromethoxy)phenyl] carbamoyl]-4-(5-isopropylpyrazol-1-yl)-1-piperidyl]methyl]-1-(cyclopropanecarbonyl)pi peridine-4-carboxylic acid | ¹H NMR (400 MHz, DMSO) δ 8.01 (s, 1H), 7.82 (d, J = 9.4 Hz, 1H), 7.56 (d, J = 1.9 Hz, 1H), 7.34 - 7.27 (m, 2H), 7.07 (t, J = 73.2 Hz, 1H), 6.35 (d, J = 1.9 Hz, 1H), 3.88 (dt, J = 13.7, 4.9 Hz, 2H), 3.21 (d, J = 92.0 Hz, 8H), 2.93 (p, J = 6.7 Hz, 1H), 2.66 (d, J = 4.0 Hz, 4H), 2.01 (d, J = 13.3 Hz, 2H), 1.89 (tt, J = 7.9, 4.8 Hz, 1H), 1.53 (ddd, J = 13.5, 9.9, 3.8 Hz, 2H), 1.13 (d, J = 6.7 Hz, 6H), 0.81 - 0.61 (m, 4H). |
| 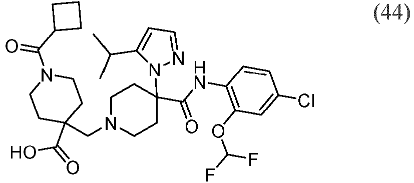 | 4-[[4-[[4-chloro-2-(difluoromethoxy)phenyl] carbamoyl]-4-(5-isopropylpyrazol-1-yl)-1-piperidyl]methyl]-1-(cyclobutanecarbonyl)pip eridine-4-carboxylic acid | ¹H NMR (400 MHz, DMSO) δ 8.01 (s, 1H), 7.82 (d, J = 9.3 Hz, 1H), 7.56 (d, J = 1.8 Hz, 1H), 7.34 - 7.27 (m, 2H), 7.25 (s, 1H), 6.34 (d, J = 1.9 Hz, 1H), 3.48 - 2.97 (m, 10H), 2.93 (p, J = 6.7 Hz, 1H), 2.73 - 2.56 (m, 4H), 2.24 - 2.04 (m, 4H), 2.02 - 1.83 (m, 3H), 1.82 - 1.68 (m, 1H), 1.48 (ddd, J = 13.7, 9.6, 4.1 Hz, 2H), 1.33 - 1.20 (m, 1H), 1.13 (d, J = 6.6 Hz, 6H). |
| 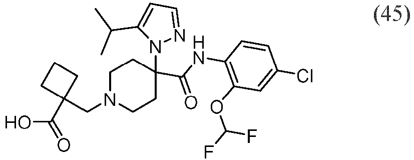 | 1-[[4-[[4-chloro-2-(difluoromethoxy)phenyl] carbamoyl]-4-(5-isopropylpyrazol-1-yl)-1-piperidyl]methyl]cyclobu tanecarboxylic acid | ¹H NMR (500 MHz, DMSO-d6) δ 8.18 (s, 1H), 7.78 (d, *J =* 9.4 Hz, 1H), 7.53 (d, *J =* 1.8 Hz, 1H), 7.32 (m, 2H), 7.15 (t, *J* = 73.2 Hz, 1H), 6.33 (d, J = 1.8 Hz, 1H), 2.92 (m, 1H), 2.70 (m, 1H), 2.45 (m, 4H), 2.35 (m, 2H), 1.90 (m, 2H), 1.81 (m, 2H), 1.09 (d, *J* = 6.7 Hz, 6H). |
| 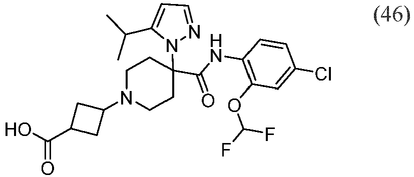 | 3-[4-[[4-chloro-2-(difluoromethoxy)phenyl] carbamoyl]-4-(5-isopropylpyrazol-1-yl)-1-piperidyl]cyclobutanecar boxylic acid | ¹H NMR (500 MHz, DMSO-d6) δ 8.21 (bs, 1H), 7.77 (d, *J* = 6.2 Hz, 1H), 7.52 (d, *J* = 1.9 Hz, 1H), 7.32 (m, 2H), 7.16 (t, *J* = 73.2 Hz, 1H), 6.33 (d, *J* = 2.1 Hz, 1H), 3.35 (m, 4H), 2.92 (m, 1H), 2.69 (m, 2H), 2.58 (m, 1H), 2.52 (m, 2H), 2.37 (m, 2H), 2.22 (m, 2H), 1.95 (m, 1H), 1.10 (d, *J* = 6.6 Hz, 6H). |
| 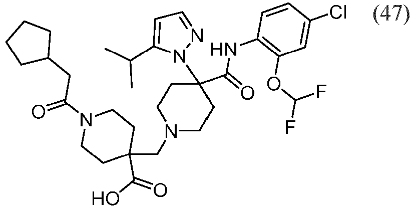 | 4-[[4-[[4-chloro-2-(difluoromethoxy)phenyl] carbamoyl]-4-(5-isopropylpyrazol-1-yl)-1-piperidyl]methyl]-1-(2-cyclopentylacetyl)piperid ine-4-carboxylic acid | ¹H NMR (400 MHz, DMSO) δ 8.11 (s, 1H), 7.80 (d, J = 9.4 Hz, 1H), 7.31 (m, 2H), 7.14 (t, J = 73.2 Hz, 1H), 6.32 (d, J = 1.9 Hz, 1H), 4.03 (d, J = 13.4 Hz, 1H), 3.68 (d, J = 13.8 Hz, 1H), 3.48 - 3.36 (m, 1H), 3.13 (t, J = 12.3 Hz, 1H), 2.91 (p, J = 6.7 Hz, 1H), 2.84 - 2.63 (m, 3H), 2.48 - 2.20 (m, 10H), 2.10 (p, J = 7.6 Hz, 1H), 1.95 (d, J = 13.5 Hz, 1H), 1.88 (d, J = 13.4 Hz, 1H), 1.81 - 1.67 (m, 2H), 1.62 - 1.19 (m, 8H), 1.09 (d, J = 6.6 Hz, 6H). |
| 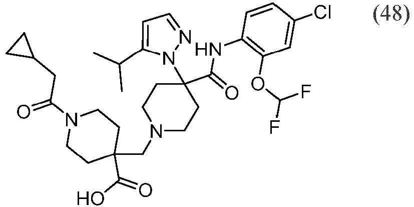 | 4-[[4-[[4-chloro-2-(difluoromethoxy)phenyl] carbamoyl]-4-(5-isopropylpyrazol-1-yl)-1-piperidyl]methyl]-1-(2-cyclopropylacetyl)piperid ine-4-carboxylic acid | ¹H NMR (600 MHz, DMSO) δ 12.62 (br s, 1H), 8.11 (s, 1H), 7.80 (d, J = 9.3 Hz, 1H), 7.52 (dd, J = 1.9, 0.6 Hz, 1H), 7.34 - 7.30 (m, 2H), 7.14 (t, J = 73.2 Hz, 1H), 6.32 (d, J = 1.8 Hz, 1H), 4.13 - 3.95 (m, 1H), 3.75 - 3.58 (m, 1H), 3.13 (ddd, J = 13.9, 10.9, 3.0 Hz, 1H), 2.91 (p, J = 6.7 Hz, 1H), 2.80 (ddd, J = 13.8, 10.9, 3.1 Hz, 1H), 2.75 - 2.67 (m, 2H), 2.52 - 2.38 (m, 6H), 2.34 (t, J = 11.1 Hz, 2H), 2.23 (d, J = 6.7 Hz, 2H), 1.92 (dd, J = 38.9, 13.4 Hz, 2H), 1.39 (td, J = 12.1, 4.1 Hz, 1H), 1.30 (ddd, J = 13.5, 11.0, 4.2 Hz, 1H), 1.09 (d, J = 6.7 Hz, 6H), 0.99 - 0.88 (m, 1H), 0.51 - 0.35 (m, 2H), 0.16 - 0.05 (m, 2H). |
| 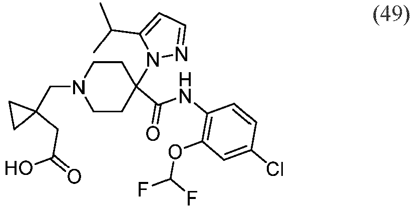 | 2-[1-[[4-[[4-chloro-2-(difluoromethoxy)phenyl] carbamoyl]-4-(5-isopropylpyrazol-1-yl)-1-piperidyl]methyl]cyclopr opyl]acetic acid | ¹H NMR (600 MHz, DMSO) δ 7.82 - 7.78 (m, 1H), 7.55 - 7.51 (m, 1H), 7.34 - 7.31 (m, 2H), 7.16 (t, *J* = 73.2 Hz, 1H), 6.33 (d, *J* = 1.8 Hz, 1H), 2.94 (p, *J =* 6.8 Hz, 1H), 2.91 - 2.85 (m, 2H), 2.56 - 2.49 (m, 4H), 2.47 - 2.41 (m, 2H), 2.35 (s, 2H), 2.28 (s, 2H), 1.10 (d, *J =* 6.7 Hz, 6H), 0.45 - 0.41 (m, 2H), 0.37 - 0.33 (m, 2H). |
| 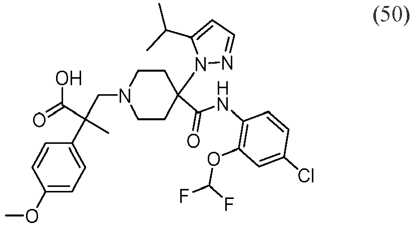 | 3-[4-[[4-chloro-2-(difluoromethoxy)phenyl] carbamoyl]-4-(5-isopropylpyrazol-1-yl)-1-piperidyl]-2-(4-methoxyphenyl)-2-methyl-propanoic acid | ¹H NMR (600 MHz, CDCl3) δ 8.23 (d, J = 8.9 Hz, 1H), 7.55 (s, 1H), 7.38 - 7.27 (m, 1H), 7.24 (d, J = 8.8 Hz, 2H), 7.17 (dd, J = 8.9, 2.3 Hz, 1H), 7.13 (s, 1H), 7.05 (d, J = 2.3 Hz, 1H), 6.86 (d, J = 8.9 Hz, 2H), 6.27 (t, J = 72.4 Hz, 1H), 6.24 (d, J = 1.9 Hz, 2H), 3.79 (s, 3H), 3.12 - 2.25 (m, 9H), 1.47 (s, 3H), 1.08 (dd, J = 9.3, 6.7 Hz, 6H). |
| 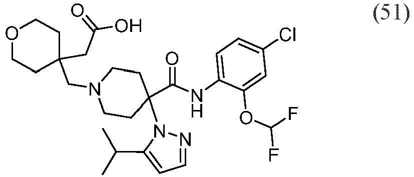 | 2-[4-[[4-[[4-chloro-2-(difluoromethoxy)phenyl] carbamoyl]-4-(5-isopropylpyrazol-1-yl)-1-piperidyl]methyl]tetrahyd ropyran-4-yl]acetic acid | ¹H NMR (400 MHz, MeOD) δ 1.22 (d, J = 6.7 Hz, 6H), 1.42 - 1.56 (m, 2H), 1.88 (t, J = 7.5 Hz, 2H), 2.02 - 2.14 (m, 2H), 2.67 - 2.81 (m, 2H), 2.81 - 2.95 (m, 2H), 2.95 - 3.07 (m, 3H), 3.07 - 3.22 (m, 2H), 3.41 - 3.54 (m, 2H), 3.54 - 3.69 (m, 2H), 3.72 - 3.88 (m, 2H), 6.42 (d, J = 2.0 Hz, 1H), 6.83 (t, J = 73.0 Hz, 1H), 7.28 (d, J = 7.8 Hz, 2H), 7.64 (d, J = 1.9 Hz, 1H), 7.99 (d, J = 8.7 Hz, 1H). |
| 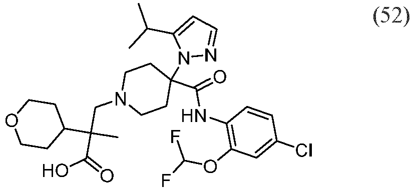 | 3-[4-[[4-chloro-2-(difluoromethoxy)phenyl] carbamoyl]-4-(5-isopropylpyrazol-1-yl)-1-piperidyl]-2-methyl-2-tetrahydropyran-4-yl-propanoic acid | ¹H NMR (400 MHz, DMSO-d6) δ 8.14 (bs, 1H), 7.80 (m, 1H), 7.52 (d, *J =* 1.8 Hz, 1H), 7.31 (m, 2H), 7.15 (t, *J =* 73.2 Hz, 1H), 6.32 (d, *J* = 2.1 Hz, 1H), 3.85 (m, 1H), 3.22 (m, 4H), 2.92 (m, 1H), 2.73 (m, 2H), 2.63, (m, 1H), 2.54 (m, 2H), 2.46 (m, 2H), 2.33 (m, 2H), 1.70 (m, 1H), 1.48 (m, 1H), 1.28 (m, 3H), 1.09 (d, *J* = 6.7 Hz, 6H), 1.05 (s, 3H). |
| 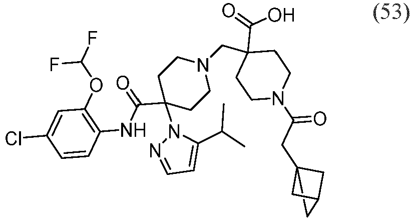 | 1-[2-(1-bicyclo[1.1.1]pentanyl)ac etyl]-4-[[4-[[4-chloro-2-(difluoromethoxy)phenyl] carbamoyl]-4-(5-isopropylpyrazol-1-yl)-1-piperidyl]methyl]piperidi ne-4-carboxylic acid | ¹H NMR (400 MHz, DMSO) δ 8.11 (s, 1H), 7.80 (d, J = 9.4 Hz, 1H), 7.52 (d, J = 1.8 Hz, 1H), 7.36 - 7.28 (m, 2H), 7.14 (t, J = 73.2 Hz, 1H), 6.32 (d, J = 1.9 Hz, 1H), 4.10 - 3.97 (m, 1H), 3.63 (d, J = 13.8 Hz, 1H), 3.41 (d, J = 6.6 Hz, 1H), 3.12 (t, J = 12.3 Hz, 1H), 2.91 (p, J = 6.7 Hz, 1H), 2.83 - 2.66 (m, 3H), 2.56 - 2.29 (m, 10H), 1.92 (dd, J = 27.0, 13.2 Hz, 2H), 1.71 (s, 6H), 1.45 - 1.22 (m, 2H), 1.09 (d, J = 6.7 Hz, 6H). |
| 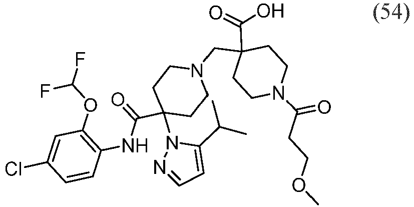 | 4-[[4-[[4-chloro-2-(difluoromethoxy)phenyl] carbamoyl]-4-(5-isopropylpyrazol-1-yl)-1-piperidyl]methyl]-1-(3-methoxypropanoyl)piperi dine-4-carboxylic acid | ¹H NMR (400 MHz, DMSO, 90 °C) δ 7.98 (s, 1H), 7.83 (d, J = 9.4 Hz, 1H), 7.55 (d, J = 1.9 Hz, 1H), 7.35 - 7.26 (m, 2H), 7.06 (t, J = 73.2 Hz, 1H), 6.33 (d, J = 1.9 Hz, 1H), 3.57 (t, J = 6.6 Hz, 2H), 3.23 (s, 3H), 3.83 - 2.89 (m, 11H), 2.61 (d, J = 14.0 Hz, 4H), 2.53 (t, J = 6.6 Hz, 2H), 1.98 (d, J = 13.6 Hz, 2H), 1.51 (t, J = 12.3 Hz, 2H), 1.12 (d, J = 6.7 Hz, 6H). |
| 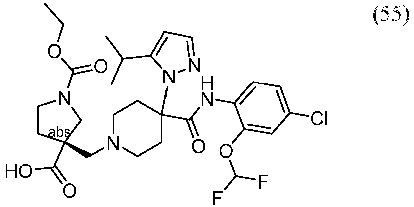 | (3*S*)-3-[[4-[[4-chloro-2-(difluoromethoxy)phenyl] carbamoyl]-4-(5-isopropylpyrazol-1-yl)-1-piperidyl]methyl]-1-ethoxycarbonyl-pyrrolidine-3-carboxylic acid | ¹H NMR (400 MHz, DMSO) δ 1.10 (d, J = 6.6 Hz, 6H), 1.13 - 1.20 (m, 3H), 1.77 - 1.93 (m, 1H), 2.08 - 2.24 (m, 1H), 2.30 - 2.49 (m, 4H), 2.53 - 2.83 (m, 5H), 2.91 (p, J = 6.7 Hz, 1H), 3.11 - 3.25 (m, 2H), 3.73 (d, J = 10.7 Hz, 1H), 3.94 - 4.08 (m, 2H), 6.33 (d, J = 1.9 Hz, 1H), 6.91 - 7.36 (m, 3H), 7.53 (d, J = 1.8 Hz, 1H), 7.77 (d, J = 9.4 Hz, 1H), 8.22 (s, 1H), 12.91 (s, 1H). |
| 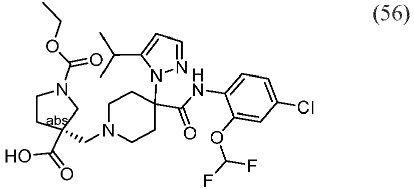 | (3*R*)-3-[[4-[[4-chloro-2-(difluoromethoxy)phenyl] carbamoyl]-4-(5-isopropylpyrazol-1-yl)-1-piperidyl]methyl]-1-ethoxycarbonyl-pyrrolidine-3-carboxylic acid | ¹H NMR (400 MHz, DMSO) δ 1.10 (d, J = 6.6 Hz, 6H), 1.13 - 1.20 (m, 3H), 1.78 - 1.93 (m, 1H), 2.12 - 2.25 (m, 1H), 2.30 - 2.49 (m, 4H), 2.53 - 2.86 (m, 5H), 2.86 - 2.97 (m, 1H), 3.14 - 3.25 (m, 2H), 3.73 (d, J = 10.7 Hz, 1H), 3.95 - 4.07 (m, 2H), 6.33 (d, J = 1.9 Hz, 1H), 6.92 - 7.37 (m, 3H), 7.53 (d, J = 1.8 Hz, 1H), 7.76 (d, J = 9.3 Hz, 1H), 8.22 (s, 1H), 12.92 (s, 1H). |
| 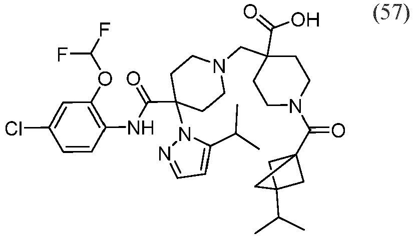 | 4-[[4-[[4-chloro-2-(difluoromethoxy)phenyl] carbamoyl]-4-(5-isopropylpyrazol-1-yl)-1-piperidyl]methyl]-1-(3-isopropylbicyclo[1.1.1]pe ntane-1-carbonyl)piperidine-4-carboxylic acid | ¹H NMR (400 MHz, DMSO, 90 °C) δ 7.91 (d, J = 9.4 Hz, 1H), 7.78 (s, 1H), 7.50 (d, J = 1.8 Hz, 1H), 7.32 - 7.25 (m, 3H), 7.04 (t, J = 73.2 Hz, 1H), 6.28 (d, J = 1.9 Hz, 1H), 3.87 (d, J = 13.4 Hz, 2H), 3.03 - 2.87 (m, 2H), 2.75 (dt, J = 10.4, 4.2 Hz, 2H), 2.59 - 2.44 (m, 6H), 2.38 (ddd, J = 13.5, 9.5, 3.6 Hz, 2H), 1.94 (d, J = 13.5 Hz, 2H), 1.82 (s, 6H), 1.65 (p, J = 6.8 Hz, 1H), 1.47 - 1.30 (m, 2H), 1.10 (d, J = 6.7 Hz, 6H), 0.80 (d, J = 6.8 Hz, 6H). |
| 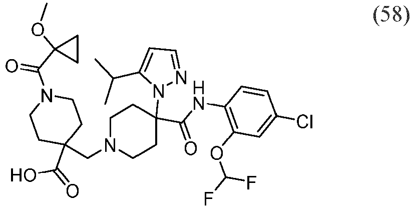 | 4-[[4-[[4-chloro-2-(difluoromethoxy)phenyl] carbamoyl]-4-(5-isopropylpyrazol-1-yl)-1-piperidyl]methyl]-1-(1-methoxycyclopropanecar bonyl)piperidine-4-carboxylic acid | ¹H NMR (400 MHz, DMSO, 90 °C) δ 7.91 (d, J = 9.4 Hz, 1H), 7.78 (s, 1H), 7.50 (d, J = 1.9 Hz, 1H), 7.36 - 7.25 (m, 2H), 7.04 (t, J = 73.2 Hz, 1H), 6.28 (d, J = 1.9 Hz, 1H), 4.01 (dd, J = 11.2, 6.8 Hz, 2H), 3.21 (s, 4H), 2.97 (p, J = 6.7 Hz, 2H), 2.76 (dt, J = 10.5, 4.4 Hz, 2H), 2.62 - 2.50 (m, 6H), 2.38 (ddd, J = 13.5, 9.5, 3.6 Hz, 2H), 1.97 (d, J = 13.6 Hz, 2H), 1.44 (ddd, J = 14.0, 10.6, 4.2 Hz, 2H), 1.10 (d, J = 6.7 Hz, 6H), 0.98 - 0.78 (m, 4H). |
| 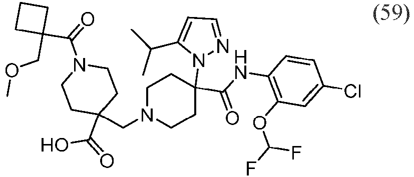 | 4-[[4-[[4-chloro-2-(difluoromethoxy)phenyl] carbamoyl]-4-(5-isopropylpyrazol-1-yl)-1-piperidyl]methyl]-1-[1-(methoxymethyl)cyclobut anecarbonyl]piperidine-4-carboxylic acid | ¹H NMR (400 MHz, DMSO, 90 °C) δ 7.91 (d, J = 9.4 Hz, 1H), 7.78 (s, 1H), 7.50 (d, J = 1.8 Hz, 1H), 7.41 - 7.24 (m, 3H), 7.04 (t, J = 73.2 Hz, 1H), 6.28 (d, J = 1.8 Hz, 1H), 3.69 (s, 2H), 3.52 (s, 2H), 3.41 (s, 1H), 3.28 (s, 3H), 3.07 - 2.89 (m, 2H), 2.74 (dd, J = 11.6, 5.9 Hz, 2H), 2.60 - 2.44 (m, 4H), 2.42 - 2.22 (m, 4H), 2.04 - 1.73 (m, 6H), 1.63 (td, J = 11.8, 8.0 Hz, 1H), 1.38 (ddd, J = 14.1, 10.4, 4.1 Hz, 2H), 1.10 (d, J = 6.7 Hz, 6H). |
| 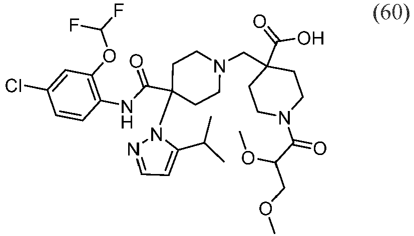 | 4-[[4-[[4-chloro-2-(difluoromethoxy)phenyl] carbamoyl]-4-(5-isopropylpyrazol-1-yl)-1-piperidyl]methyl]-1-(2,3-dimethoxypropanoyl)pipe ridine-4-carboxylic acid | ¹H NMR (400 MHz, DMSO) δ 8.16 (s, 1H), 7.78 (d, J = 9.5 Hz, 1H), 7.36 - 7.29 (m, 2H), 7.15 (t, J = 73.6 Hz, 1H), 6.33 (d, J = 1.9 Hz, 1H), 4.29 (d, J = 5.7 Hz, 1H), 4.11 - 3.92 (m, 1H), 3.89 - 3.77 (m, 1H), 3.49 (qd, J = 10.6, 5.5 Hz, 2H), 3.25 (s, 3H), 3.24 (s, 3H), 2.91 (p, J = 6.7 Hz, 2H), 2.83 - 2.73 (m, 2H), 2.64 - 2.28 (m, 10H), 2.04 - 1.85 (m, 2H), 1.53 - 1.29 (m, 2H), 1.09 (d, J = 6.7 Hz, 6H). |
| 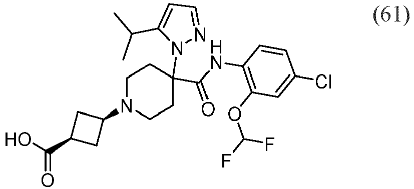 | (1*s*,3*s*)-3-[4-[[4-chloro-2-(difluoromethoxy)phenyl] carbamoyl]-4-(5-isopropylpyrazol-1-yl)-1-piperidyl]cyclobutanecar boxylic acid | ¹H NMR (400 MHz, DMSO-d6) δ 8.14 (bs, 1H), 7.80 (m, 1H), 7.52 (d, *J =* 1.8 Hz, 1H), 7.31 (m, 2H), 7.15 (t, *J =* 73.2 Hz, 1H), 6.32 (d, *J =* 2.1 Hz, 1H), 3.85 (m, 1H), 3.22 (m, 4H), 2.92 (m, 1H), 2.73 (m, 2H), 2.63, (m, 1H), 2.54 (m, 2H), 2.46 (m, 2H), 2.33 (m, 2H), 1.70 (m, 1H), 1.48 (m, 1H), 1.28 (m, 3H), 1.09 (d, *J* = 6.7 Hz, 6H), 1.05 (s, 3H). |
| 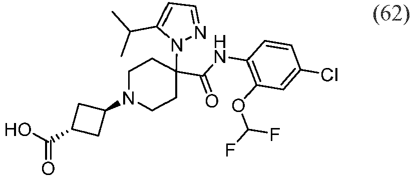 | (1*r*,3*r*)-3-[4-[[4-chloro-2-(difluoromethoxy)phenyl] carbamoyl]-4-(5-isopropylpyrazol-1-yl)-1-piperidyl]cyclobutanecar boxylic acid | ¹H NMR (500 MHz, DMSO-d6) δ 8.16 (s, 1H), 7.79 (d, *J =* 6.2 Hz, 1H), 7.51 (d, *J =* 1.9 Hz, 1H), 7.32 (m, 2H), 7.15 (t, *J* = 73.2 Hz, 1H), 6.32 (d, *J =* 2.1 Hz, 1H), 2.92 (m, 1H), 2.84 (m, 1H), 2.77 (m, 1H), 2.60 (m, 2H), 2.49 (m, 2H), 2.36 (m, 2H), 2.15 (m, 4H), 2.06 (m, 2H), 1.10 (d, *J* = 6.6 Hz, 6H). |
| 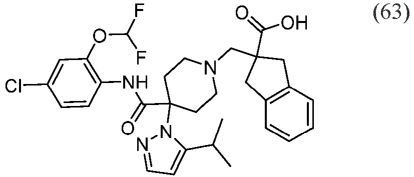 | 2-[[4-[[4-chloro-2-(difluoromethoxy)phenyl] carbamoyl]-4-(5-isopropylpyrazol-1-yl)-1-piperidyl]methyl]indane-2-carboxylic acid | ¹H NMR (600 MHz, DMSO-d6) δ 12.60 (bs, 1H), 8.13 (s, 1H), 7.80 (d, *J* = 9.4 Hz, 1H), 7.53 (d, *J* = 1.8 Hz, 1H), 7.32 (m, 2H), 7.18 (m, 2H), 7.15 (t, *J =* 73.3 Hz, 1H), 7.10 (m, 2H), 6.32 (d, J = 1.9 Hz, 1H), 3.29 (m, 2H), 2.93 (m, 3H), 2.74 (m, 2H), 2.62 (s, 2H), 2.46 (m, 3H), 2.36 (m, 2H), 1.10 (d, *J =* 6.7 Hz, 6H). |
| 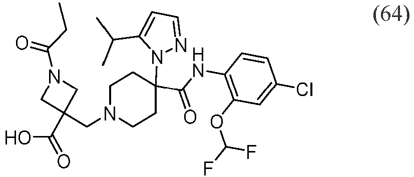 | 3-[[4-[[4-chloro-2-(difluoromethoxy)phenyl] carbamoyl]-4-(5-isopropylpyrazol-1-yl)-1-piperidyl]methyl]-1-propanoyl-azetidine-3-carboxylic acid | ¹H NMR (400 MHz, DMSO) δ 8.55 (s, 1H), 7.66 (d, J = 9.2 Hz, 1H), 7.60 (d, J = 1.9 Hz, 1H), 7.37 - 7.32 (m, 2H), 7.18 (t, J = 74.2 Hz, 1H), 6.41 (d, J = 1.9 Hz, 1H), 4.31 (d, J = 8.7 Hz, 1H), 4.06 (dd, J = 23.4, 9.3 Hz, 2H), 3.84 (d, J = 9.7 Hz, 1H), 3.56 (s, 2H), 3.20 (s, 4H), 2.97 - 2.79 (m, 1H), 2.65 (s, 4H), 2.15 - 1.96 (m, 2H), 1.14 (d, J = 6.6 Hz, 6H), 0.96 (t, J = 7.5 Hz, 3H). |
| 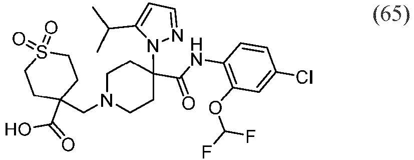 | 4-[[4-[[4-chloro-2-(difluoromethoxy)phenyl] carbamoyl]-4-(5-isopropylpyrazol-1-yl)-1-piperidyl]methyl]-1,1-dioxo-thiane-4-carboxylic acid | ¹H NMR (400 MHz, DMSO) δ 8.01 (s, 1H), 7.82 (d, J = 9.4 Hz, 1H), 7.56 (d, J = 1.9 Hz, 1H), 7.44 - 7.14 (m, 2H), 7.07 (s, 1H), 6.88 (s, 0H), 6.34 (d, J = 1.9 Hz, 1H), 3.4 (s, 2H), 3.10 (dt, J = 15.6, 3.3 Hz, 1H), 3.03 - 2.94 (m, 1H), 2.91 (t, J = 6.6 Hz, 1H), 2.74 (dt, J = 10.5, 4.5 Hz, 1H), 2.58 - 2.48 (m, 4H), 2.48 - 2.41 (m, 5H), 2.39 - 2.26 (m, 3H), 1.96 - 1.87 (m, 1H), 1.09 (d, J = 6.7 Hz, 6H). |
| 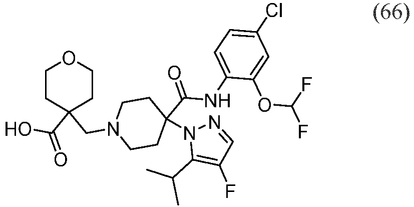 | 4-[[4-[[4-chloro-2-(difluoromethoxy)phenyl] carbamoyl]-4-(4-fluoro-5-isopropyl-pyrazol-1-yl)-1-piperidyl]methyl]tetrahyd ropyran-4-carboxylic acid | ¹H NMR (400 MHz, DMSO) δ 1.15 (d, J = 6.8 Hz, 6H), 1.41 - 1.50 (m, 2H), 1.86 (d, J = 13.3 Hz, 2H), 2.29 - 2.38 (m, 5H), 2.44 - 2.48 (m, 2H), 2.69 (dd, J = 10.5, 5.1 Hz, 3H), 2.92 - 3.03 (m, 1H), 3.38 (d, J = 11.1 Hz, 2H), 3.65 - 3.72 (m, 2H), 7.17 (t, J = 73.4 Hz, 1H), 7.33 (d, J = 7.0 Hz, 2H), 7.57 (d, J = 4.2 Hz, 1H), 7.61 (d, J = 9.2 Hz, 1H), 8.59 (s, 1H), 12.51 (s, 1H). |
| 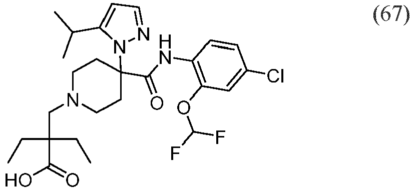 | 2-[[4-[[4-chloro-2-(difluoromethoxy)phenyl] carbamoyl]-4-(5-isopropylpyrazol-1-yl)-1-piperidyl]methyl]-2-ethyl-butanoic acid | ¹H NMR (500 MHz, DMSO-d6) δ 8.14 (s, 1H), 7.79 (d, *J =* 9.4 Hz, 1H), 7.52 (d, *J =* 1.8 Hz, 1H), 7.32 (m, 2H), 7.15 (t, *J* = 73.2 Hz, 1H), 6.32 (d, J = 1.8 Hz, 1H), 3.17 (s, 2H), 2.91 (m, 1H), 2.70 (m, 2H), 2.45 (m, 4H), 2.35 (m, 2H), 1.50 (m, 4H), 1.09 (d, *J =* 6.7 Hz, 6H), 0.74 (t, J = 7.4 Hz, 6H). |
| 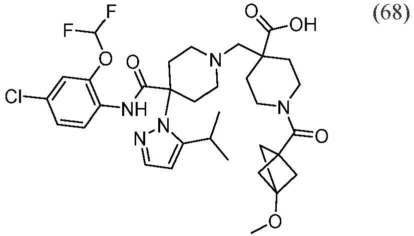 | 4-[[4-[[4-chloro-2-(difluoromethoxy)phenyl] carbamoyl]-4-(5-isopropylpyrazol-1-yl)-1-piperidyl]methyl]-1-(3-methoxybicyclo[1.1.1]pe ntane-1-carbonyl)piperidine-4-carboxylic acid | ¹H NMR (400 MHz, DMSO) δ 8.00 (s, 1H), 7.82 (d, J = 9.3 Hz, 1H), 7.56 (d, J = 1.8 Hz, 1H), 7.37 - 7.28 (m, 2H), 7.07 (t, J = 73.2 Hz, 1H), 6.34 (d, J = 1.9 Hz, 1H), 3.79 (d, J = 13.8 Hz, 2H), 3.21 (s, 3H), 3.29 - 2.86 (m, 13H), 2.13 (s, 6H), 1.98 (d, J = 13.8 Hz, 2H), 1.60 - 1.42 (m, 2H), 1.13 (d, J = 6.7 Hz, 6H). |
| 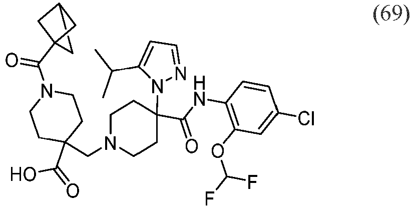 | 1-(bicyclo[1.1.1]pentane-1-carbonyl)-4-[[4-[[4-chloro-2-(difluoromethoxy)phenyl] carbamoyl]-4-(5-isopropylpyrazol-1-yl)-1-piperidyl]methyl]piperidi ne-4-carboxylic acid | ¹H NMR (400 MHz, DMSO) δ 8.01 (s, 1H), 7.82 (d, J = 9.4 Hz, 1H), 7.56 (d, J = 1.9 Hz, 1H), 7.37 - 7.28 (m, 2H), 7.07 (t, J = 73.2 Hz, 1H), 6.34 (d, J = 1.9 Hz, 1H), 3.84 - 3.79 (m, 2H), 3.26-3.23 (m, 4H), 3.08 (s, 2H), 3.01 (d, J = 10.7 Hz, 2H), 2.96 - 2.90 (m, 1H), 2.70 - 2.59 (m, 4H), 2.44 (s, 1H), 2.07 (d, J = 3.1 Hz, 6H), 1.98 (d, J = 13.9 Hz, 2H), 1.54-1.45 (m, 2H), 1.13 (d, J = 6.7 Hz, 6H). |
| 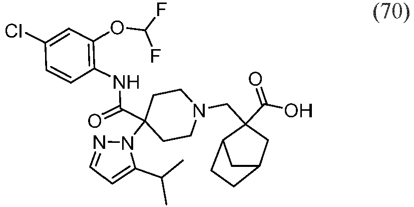 | 2-[[4-[[4-chloro-2-(difluoromethoxy)phenyl] carbamoyl]-4-(5-isopropylpyrazol-1-yl)-1-piperidyl]methyl]norborn ane-2-carboxylic acid | ¹H NMR (400 MHz, DMSO) δ 8.01 (s, 1H), 7.82 (d, J = 9.4 Hz, 1H), 7.56 (d, J = 1.9 Hz, 1H), 7.44 - 7.14 (m, 2H), 7.17 (t, J = 73.3 Hz, 1H), 6.88 (s, 0H), 6.34 (d, J = 1.9 Hz, 1H), 2.91 (hept, J = 6.8 Hz, 1H), 2.77 - 2.66 (m, 3H), 2.47 - 2.27 (m, 7H), 2.18 (d, J = 4.5 Hz, 1H), 2.10 - 2.06 (m, 1H), 1.92 - 1.85 (m, 1H), 1.56 - 1.32 (m, 4H), 1.25 - 1.06 (m, 9H). |
| 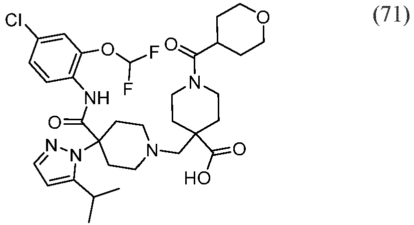 | 4-[[4-[[4-chloro-2-(difluoromethoxy)phenyl] carbamoyl]-4-(5-isopropylpyrazol-1-yl)-1-piperidyl]methyl]-1-(tetrahydropyran-4-carbonyl)piperidine-4-carboxylic acid | ¹H NMR (400 MHz, DMSO) δ 8.00 (s, 1H), 7.82 (d, J = 9.3 Hz, 1H), 7.56 (d, J = 1.8 Hz, 1H), 7.37 - 7.27 (m, 2H), 7.07 (t, J = 73.2 Hz, 1H), 6.34 (d, J = 1.9 Hz, 1H), 3.89 - 3.74 (m, 4H), 3.39 (td, J = 11.5, 2.5 Hz, 2H), 3.23 (d, J = 12.5 Hz, 4H), 3.11 - 2.97 (m, 4H), 2.93 (p, J = 6.8 Hz, 1H), 2.84 (ddd, J = 10.8, 6.8, 4.1 Hz, 1H), 2.73 - 2.54 (m, 4H), 1.99 (d, J = 13.7 Hz, 2H), 1.62 (dtd, J = 13.6, 11.2, 4.3 Hz, 2H), 1.55 - 1.42 (m, 4H), 1.13 (d, J = 6.7 Hz, 6H). |
| 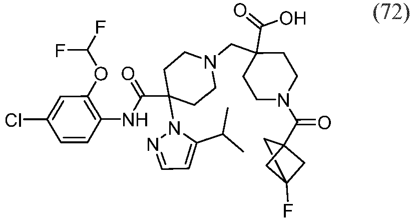 | 4-[[4-[[4-chloro-2-(difluoromethoxy)phenyl] carbamoyl]-4-(5-isopropylpyrazol-1-yl)-1-piperidyl]methyl]-1-(3-fluorobicyclo[1.1.1]penta ne-1-carbonyl)piperidine-4-carboxylic acid | ¹H NMR (400 MHz, DMSO) δ 8.43 (s, 1H), 7.80 - 7.52 (m, 2H), 7.39 - 7.31 (m, 2H), 7.18 (t, J = 73.8 Hz, 1H), 6.42 (s, 1H), 3.54 (d, J = 57.8 Hz, 5H), 2.87 (s, 1H), 2.79 - 2.61 (m, 4H), 2.38 (dd, J = 6.4, 2.7 Hz, 10H), 2.05 - 1.77 (m, 3H), 1.65 - 1.48 (m, 2H), 1.13 (d, J = 6.6 Hz, 6H). |
| 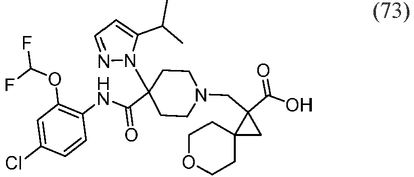 | 2-[[4-[[4-chloro-2-(difluoromethoxy)phenyl] carbamoyl]-4-(5-isopropylpyrazol-1-yl)-1-piperidyl]methyl]-6-oxaspiro[2.5]octane-2-carboxylic acid | ¹H NMR (600 MHz, CDCl3) δ 8.27 (d, J = 8.9 Hz, 1H), 7.60 (d, J = 1.9 Hz, 1H), 7.19 (dd, J = 8.9, 2.3 Hz, 2H), 7.08 (d, J = 2.4 Hz, 1H), 6.32 (t, J = 72.4 Hz, 1H), 6.28 (d, J = 1.9 Hz, 1H), 4.09 - 3.93 (m, 2H), 3.52 - 3.45 (m, 2H), 3.25 - 2.40 (m, 11H), 2.01 - 1.90 (m, 1H), 1.85 (dd, J = 13.7, 2.5 Hz, 1H), 1.75 (td, J = 12.6, 4.1 Hz, 1H), 1.64 (dd, J = 4.7, 1.5 Hz, 1H), 1.14 (d, J = 6.7 Hz, 6H), 1.10 (d, J = 12.9 Hz, 1H), 0.45 (d, J = 4.7 Hz, 1H). |
| 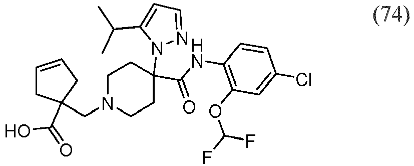 | 1-[[4-[[4-chloro-2-(difluoromethoxy)phenyl] carbamoyl]-4-(5-isopropylpyrazol-1-yl)-1-piperidyl]methyl]cyclope nt-3-ene-1-carboxylic acid | ¹H NMR (400 MHz, DMSO) δ 8.01 (s, 1H), 7.82 (d, J = 9.4 Hz, 1H), 7.56 (d, J = 1.9 Hz, 1H), 7.44 - 7.14 (m, 2H), 7.17 (t, J = 73.3 Hz, 1H), 6.88 (s, 0H), 6.34 (d, J = 1.9 Hz, 1H), 5.60 (s, 2H), 4.09 (s, 1H), 3.17 (s, 2H), 2.92 (hept, J = 6.7 Hz, 1H), 2.75 - 2.67 (m, 4H), 2.57 (s, 2H), 2.46 (d, J = 13.7 Hz, 3H), 2.35 (ddt, J = 19.9, 12.2, 3.7 Hz, 6H), 1.09 (d, J = 6.7 Hz, 6H). |
| 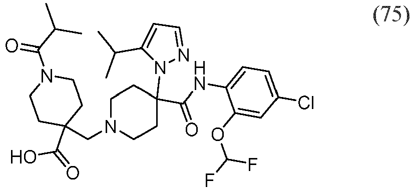 | 4-[[4-[[4-chloro-2-(difluoromethoxy)phenyl] carbamoyl]-4-(5-isopropylpyrazol-1-yl)-1-piperidyl]methyl]-1-(2-methylpropanoyl)piperidi ne-4-carboxylic acid | ¹H NMR (400 MHz, DMSO) δ 0.97 (t, J = 6.5 Hz, 6H), 1.08 (d, J = 6.6 Hz, 6H), 1.18 - 1.45 (m, 2H), 1.83 - 2.05 (m, 2H), 2.27 - 2.47 (m, 7H), 2.64 - 2.98 (m, 5H), 3.10 - 3.23 (m, 1H), 3.66 - 3.79 (m, 1H), 3.95 - 4.09 (m, 1H), 6.32 (d, J = 1.9 Hz, 1H), 6.92 - 7.40 (m, 3H), 7.52 (d, J = 1.8 Hz, 1H), 7.74 - 7.84 (m, 1H), 8.13 (s, 1H), 12.65 (s, 1H). |
| 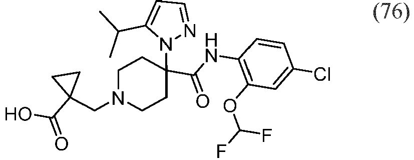 | 1-[[4-[[4-chloro-2-(difluoromethoxy)phenyl] carbamoyl]-4-(5-isopropylpyrazol-1-yl)-1-piperidyl]methyl]cyclopr opanecarboxylic acid | ¹H NMR (600 MHz, DMSO) δ 7.75 (d, J = 9.1 Hz, 1H), 7.56 - 7.52 (m, 1H), 7.36 - 7.31 (m, 2H), 7.16 (t, J = 73.2 Hz, 1H), 6.34 (d, J = 1.9 Hz, 1H), 3.03 - 2.96 (m, 2H), 2.96 - 2.90 (m, 1H), 2.67 - 2.59 (m, 6H), 2.59 - 2.52 (m, 2H), 2.49 - 2.38 (m, 1H), 1.11 (d, J = 6.7 Hz, 6H), 1.04 (q, J = 3.7 Hz, 2H), 0.67 (q, J = 3.8 Hz, 2H). |
| 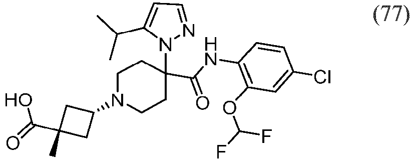 | (1*s*,3*s*)-3-[4-[[4-chloro-2-(difluoromethoxy)phenyl] carbamoyl]-4-(5-isopropylpyrazol-1-yl)-1-piperidyl]-1-methyl-cyclobutanecarboxylic acid | ¹H NMR (400 MHz, DMSO-d6) δ 8.15 (s, 1H), 7.81 (m, 1H), 7.52 (d, *J =* 1.8 Hz, 1H), 7.33 (d, *J =* 2.5 Hz, 1H), 7.31 (m, 1H), 7.15 (t, *J =* 73.3 Hz, 1H), 6.32 (d, *J =* 1.9 Hz, 1H), 2.92 (m, 1H), 2.69 (m, 1H), 2.60 (m, 2H), 2.51 (m, 3H), 2.35 (m, 2H), 2.15 (m, 3H), 1.87 (m, 2H), 1.29 (s, 3H), 1.10 (d, *J =* 6.6 Hz, 6H). |
| 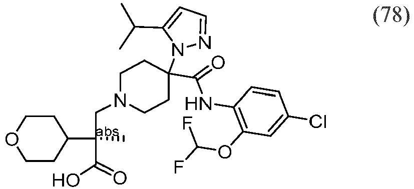 | (2*S*)-3-[4-[[4-chloro-2-(difluoromethoxy)phenyl] carbamoyl]-4-(5-isopropylpyrazol-1-yl)-1-piperidyl]-2-methyl-2-tetrahydropyran-4-yl-propanoic acid | ¹H NMR (500 MHz, DMSO) δ 12.47 (br s, 1H), 8.12 (s, 1H), 7.80 (d, J = 9.4 Hz, 1H), 7.52 (d, J = 1.8 Hz, 1H), 7.35 - 7.30 (m, 1H), 7.14 (t, J = 73.2 Hz, 1H), 6.32 (d, J = 1.8 Hz, 1H), 3.84 (dt, J = 10.4, 5.1 Hz, 2H), 3.23 (dddd, J = 11.5, 9.8, 7.7, 2.1 Hz, 2H), 2.92 (hept, J = 6.7 Hz, 1H), 2.73 (ddt, J = 16.4, 10.3, 4.7 Hz, 2H), 2.63 (d, J = 13.4 Hz, 1H), 2.56 - 2.28 (m, 8H), 1.70 (ddt, J = 11.9, 7.0, 3.4 Hz, 1H), 1.48 (d, J = 12.8 Hz, 1H), 1.29 (dddd, J = 41.4, 25.6, 12.2, 8.0 Hz, 3H), 1.09 (d, J = 6.6 Hz, 6H), 1.02 (s, 3H). |
| 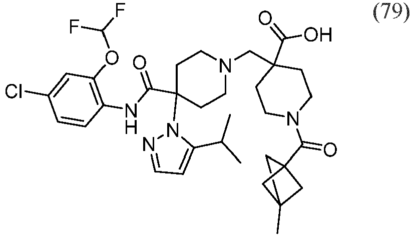 | 4-[[4-[[4-chloro-2-(difluoromethoxy)phenyl] carbamoyl]-4-(5-isopropylpyrazol-1-yl)-1-piperidyl]methyl]-1-(3-methylbicyclo[1.1.1]pent ane-1-carbonyl)piperidine-4-carboxylic acid | ¹H NMR (400 MHz, DMSO) δ 8.08 (s, 1H), 7.81 (d, J = 9.3 Hz, 1H), 7.57 (d, J = 1.9 Hz, 1H), 7.36 - 7.28 (m, 2H), 7.08 (t, J = 73.2 Hz, 1H), 6.35 (d, J = 1.9 Hz, 1H), 3.79 - 3.71 (m, 2H), 3.35 - 3.31 (m, 2H), 3.22 (s, 2H), 3.15-3.10 (m, 4H), 2.93 - 2.90 (m, 1H), 2.76 (t, J = 9.5 Hz, 4H), 1.97 (d, J = 13.6 Hz, 2H), 1.90 (d, J = 1.7 Hz, 6H), 1.61 - 1.52 (m, 2H), 1.13 (d, J = 4.0 Hz, 6H), 1.10 (s, 3H). |
| 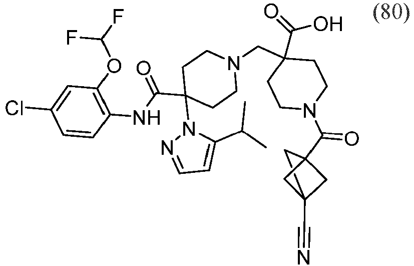 | 4-[[4-[[4-chloro-2-(difluoromethoxy)phenyl] carbamoyl]-4-(5-isopropylpyrazol-1-yl)-1-piperidyl]methyl]-1-(3-cyanobicyclo[1.1.1]penta ne-1-carbonyl)piperidine-4-carboxylic acid | ¹H NMR (400 MHz, DMSO) δ 7.99 (s, 1H), 7.82 (d, J = 9.4 Hz, 1H), 7.56 (d, J = 1.8 Hz, 1H), 7.38 - 7.26 (m, 2H), 7.07 (t, J = 73.2 Hz, 1H), 6.34 (d, J = 1.9 Hz, 1H), 3.78 (d, J = 16.1 Hz, 2H), 3.17 (s, 2H), 3.02 (s, 2H), 2.94 (s, 1H), 2.63 (s, 4H), 2.53 (s, 6H), 2.49 (s, 4H), 1.98 (d, J = 13.5 Hz, 2H), 1.56 - 1.43 (m, 2H), 1.13 (d, J = 6.7 Hz, 6H). |
| 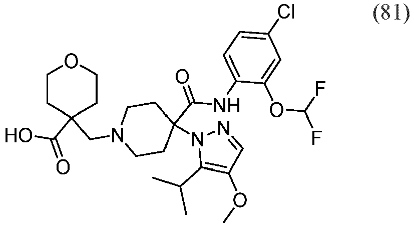 | 4-[[4-[[4-chloro-2-(difluoromethoxy)phenyl] carbamoyl]-4-(5-isopropyl-4-methoxy-pyrazol-1-yl)-1-piperidyl]methyl]tetrahyd ropyran-4-carboxylic acid | ¹H NMR (400 MHz, DMSO) δ 1.10 (d, J = 6.7 Hz, 6H), 1.45 (t, J = 10.7 Hz, 2H), 1.86 (d, J = 13.3 Hz, 2H), 2.32 (s, 6H), 2.45 (s, 2H), 2.67 - 2.74 (m, 2H), 2.83 - 2.90 (m, 1H), 3.38 (d, J = 11.1 Hz, 2H), 3.61 - 3.77 (m, 5H), 6.95 - 7.38 (m, 3H), 7.46 (s, 1H), 7.78 (d, J = 9.2 Hz, 1H), 8.15 (s, 1H), 12.53 (s, 1H). |
| 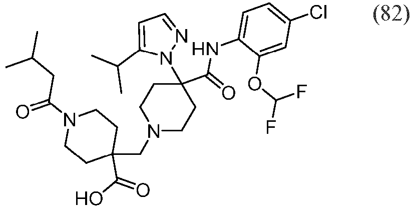 | 4-[[4-[[4-chloro-2-(difluoromethoxy)phenyl] carbamoyl]-4-(5-isopropylpyrazol-1-yl)-1-piperidyl]methyl]-1-(3-methylbutanoyl)piperidin e-4-carboxylic acid | ¹H NMR (400 MHz, DMSO) δ 0.88 (dd, J = 6.6, 1.7 Hz, 6H), 1.09 (d, J = 6.6 Hz, 6H), 1.21 - 1.33 (m, 1H), 1.33 - 1.43 (m, 1H), 1.83 - 1.91 (m, 1H), 1.90 - 2.03 (m, 2H), 2.16 (d, J = 6.9 Hz, 2H), 2.28 - 2.39 (m, 2H), 2.39 - 2.49 (m, 6H), 2.65 - 2.76 (m, 2H), 2.74 - 2.85 (m, 1H), 2.91 (p, J = 6.7 Hz, 1H), 3.13 (t, J = 12.2 Hz, 1H), 3.63 - 3.75 (m, 1H), 4.04 (d, J = 13.0 Hz, 1H), 6.32 (d, J = 1.9 Hz, 1H), 6.94 - 7.35 (m, 3H), 7.52 (d, J = 1.9 Hz, 1H), 7.79 (d, J = 9.4 Hz, 1H), 8.13 (s, 1H), 12.65 (s, 1H). |
| 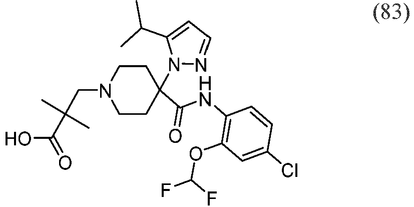 | 3-[4-[[4-chloro-2-(difluoromethoxy)phenyl] carbamoyl]-4-(5-isopropylpyrazol-1-yl)-1-piperidyl]-2,2-dimethyl-propanoic acid | ¹H NMR (400 MHz, DMSO) δ 8.10 (s, 1H), 7.85 - 7.75 (m, 1H), 7.58 (d, *J* = 1.8 Hz, 1H), 7.33 - 7.28 (m, 2H), 7.08 (t, *J =* 73.2 Hz, 1H), 6.37 (d, *J =* 1.9 Hz, 1H), 3.47 - 3.37 (m, 2H), 3.26 - 3.10 (m, 3H), 3.02 - 2.84 (m, 1H), 2.73 (t, *J =* 5.6 Hz, 4H), 1.24 (s, 6H), 1.14 (d, *J =* 6.7 Hz, 6H). |
| 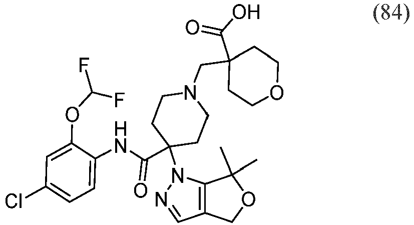 | 4-[[4-[[4-chloro-2-(difluoromethoxy)phenyl] carbamoyl]-4-(6,6-dimethyl-4*H*-furo[3,4-c]pyrazol-1-yl)-1-piperidyl]methyl]tetrahyd ropyran-4-carboxylic acid | ¹H NMR (400 MHz, DMSO-d6) δ 8.04 (s, 1H), 7.80 (m, 1H), 7.49 (s, 1H), 7.35 (m, 1H), 7.20 (t, J = 73.0 Hz, 1H), 4.69 (s, 2H), 3.70 (m, 2H), 3.38 (m, 3H), 2.77 (m, 2H), 2.49 (m, 4H), 2.36 (m, 4H), 1.88 (m, 2H), 1.46 (m, 2H), 1.38 (s, 6H). |
| 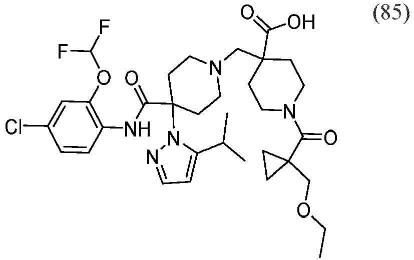 | 4-[[4-[[4-chloro-2-(difluoromethoxy)phenyl] carbamoyl]-4-(5-isopropylpyrazol-1-yl)-1-piperidyl]methyl]-1-[1-(ethoxymethyl)cycloprop anecarbonyl]piperidine-4-carboxylic acid | ¹H NMR (400 MHz, DMSO. 90 °C) δ 7.98 (s, 1H), 7.83 (d, J = 9.3 Hz, 1H), 7.56 (d, J = 1.8 Hz, 1H), 7.35 - 7.28 (m, 2H), 7.07 (t, J = 73.2 Hz, 1H), 6.34 (d, J = 1.9 Hz, 1H), 3.86 (dt, J = 13.8, 4.8 Hz, 2H), 3.47 - 3.37 (m, 4H), 3.35 - 3.11 (m, 4H), 3.04 - 2.88 (m, 4H), 2.71 - 2.53 (m, 4H), 1.98 (d, J = 13.3 Hz, 2H), 1.50 (ddd, J = 13.6, 9.5, 4.0 Hz, 2H), 1.12 (d, J = 6.7 Hz, 6H), 1.08 (t, J = 7.0 Hz, 3H), 0.84 - 0.77 (m, 3H), 0.73 - 0.67 (m, 2H). |
| 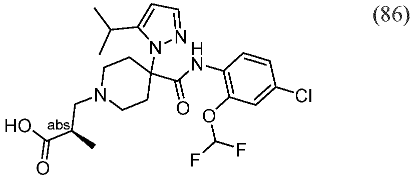 | (2*R*)-3-[4-[[4-chloro-2-(difluoromethoxy)phenyl] carbamoyl]-4-(5-isopropylpyrazol-1-yl)-1-piperidyl]-2-methyl-propanoic acid | ¹H NMR (500 MHz, DMSO) δ 8.21 (s, 1H), 7.82 - 7.73 (m, 1H), 7.53 (d, *J =* 1.8 Hz, 1H), 7.34 - 7.31 (m, 2H), 7.15 (t, *J =* 73.2 Hz, 1H), 6.33 (d, *J =* 1.9 Hz, 1H), 2.98 - 2.90 (m, 1H), 2.89 - 2.82 (m, 1H), 2.78 - 2.68 (m, 1H), 2.62 - 2.46 (m, 5H), 2.43 - 2.33 (m, 3H), 2.28 (dd, *J =* 11.8, 5.8 Hz, 1H), 1.10 (d, *J =* 6.6 Hz, 6H), 1.02 (d, *J =* 6.7 Hz, 3H). |
| 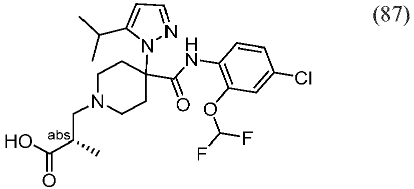 | (2*S*)-3-[4-[[4-chloro-2-(difluoromethoxy)phenyl] carbamoyl]-4-(5-isopropylpyrazol-1-yl)-1-piperidyl]-2-methyl-propanoic acid | ¹H NMR (500 MHz, DMSO) δ 8.21 (s, 1H), 7.84 - 7.74 (m, 1H), 7.53 (d, *J* = 1.8 Hz, 1H), 7.34 - 7.31 (m, 2H), 7.15 (t, *J =* 73.2 Hz, 1H), 6.33 (d, *J =* 1.9 Hz, 1H), 2.98 - 2.90 (m, 1H), 2.90 - 2.83 (m, 1H), 2.77 - 2.69 (m, 1H), 2.64 - 2.45 (m, 5H), 2.44 - 2.34 (m, 3H), 2.28 (dd, *J =* 11.8, 5.8 Hz, 1H), 1.10 (d, *J =* 6.6 Hz, 6H), 1.02 (d, *J =* 6.7 Hz, 3H). |
| 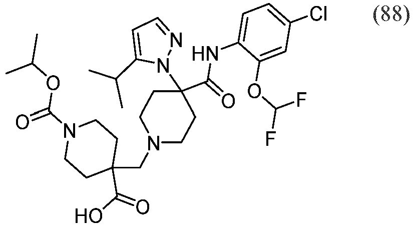 | 4-[[4-[[4-chloro-2-(difluoromethoxy)phenyl] carbamoyl]-4-(5-isopropylpyrazol-1-yl)-1-piperidyl]methyl]-1-isopropoxycarbonyl-piperidine-4-carboxylic acid | ¹H NMR (400 MHz, DMSO) δ 1.08 (d, J = 6.7 Hz, 6H), 1.16 (d, J = 6.2 Hz, 6H), 1.28 - 1.40 (m, 2H), 1.83 - 1.96 (m, 2H), 2.24 - 2.39 (m, 2H), 2.38 - 2.49 (m, 5H), 2.66 - 2.78 (m, 2H), 2.84 - 3.02 (m, 4H), 3.65 - 3.77 (m, 2H), 4.75 (p, J = 6.2 Hz, 1H), 6.32 (d, J = 1.9 Hz, 1H), 6.91 - 7.38 (m, 3H), 7.52 (d, J = 1.8 Hz, 1H), 7.76 - 7.83 (m, 1H), 8.13 (s, 1H), 12.71 (s, 1H). |
| 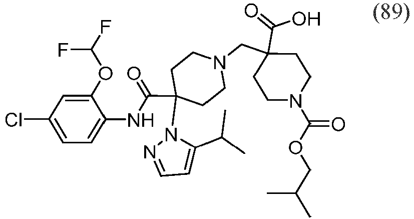 | 4-[[4-[[4-chloro-2-(difluoromethoxy)phenyl] carbamoyl]-4-(5-isopropylpyrazol-1-yl)-1-piperidyl]methyl]-1-isobutoxycarbonyl-piperidine-4-carboxylic acid | ¹H NMR (400 MHz, DMSO) δ 0.87 (d, J = 6.7 Hz, 6H), 1.08 (d, J = 6.7 Hz, 6H), 1.28 - 1.43 (m, 2H), 1.78 - 1.96 (m, 3H), 2.25 - 2.38 (m, 2H), 2.39 - 2.49 (m, 6H), 2.62 - 2.79 (m, 2H), 2.84 - 3.08 (m, 2H), 3.66 - 3.82 (m, 5H), 6.32 (d, J = 1.9 Hz, 1H), 6.92 - 7.36 (m, 3H), 7.52 (d, J = 1.8 Hz, 1H), 7.79 (d, J = 9.4 Hz, 1H), 8.13 (s, 1H), 12.68 (s, 1H). |
| 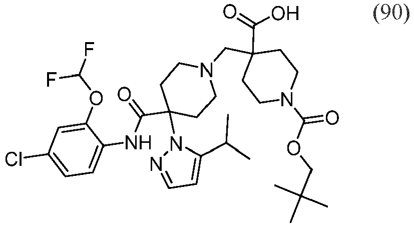 | 4-[[4-[[4-chloro-2-(difluoromethoxy)phenyl] carbamoyl]-4-(5-isopropylpyrazol-1-yl)-1-piperidyl]methyl]-1-(2,2-dimethylpropoxycarbonyl )piperidine-4-carboxylic acid | ¹H NMR (400 MHz, DMSO) δ 0.89 (s, 9H), 1.08 (d, J = 6.7 Hz, 6H), 1.29 - 1.45 (m, 2H), 1.84 - 1.97 (m, 2H), 2.29 - 2.38 (m, 2H), 2.39 - 2.49 (m, 6H), 2.64 - 2.78 (m, 2H), 2.84 - 3.10 (m, 2H), 3.68 (s, 2H), 3.74 (d, J = 13.2 Hz, 2H), 6.32 (d, J = 1.9 Hz, 1H), 6.92 - 7.38 (m, 3H), 7.52 (d, J = 1.8 Hz, 1H), 7.79 (d, J = 9.4 Hz, 1H), 8.13 (s, 1H), 12.68 (s, 1H). |
| 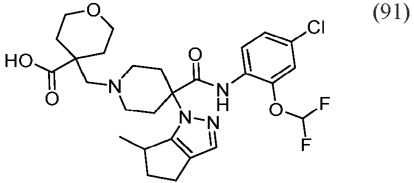 | 4-[[4-[[4-chloro-2-(difluoromethoxy)phenyl] carbamoyl]-4-(6-methyl-5,6-dihydro-4*H-*cyclopenta[c]pyrazol-1-yl)-1-piperidyl]methyl]tetrahyd ropyran-4-carboxylic acid | ¹H NMR (400 MHz, DMSO) δ 0.96 (d, J = 6.8 Hz, 3H), 1.40 - 1.49 (m, 2H), 1.86 (d, J = 13.4 Hz, 2H), 1.97 - 2.16 (m, 2H), 2.16 - 2.35 (m, 4H), 2.39 - 2.49 (m, 4H), 2.55 - 2.65 (m, 1H), 2.65 - 2.77 (m, 3H), 2.77 - 2.84 (m, 1H), 3.04 - 3.12 (m, 1H), 3.38 (d, J = 11.5 Hz, 2H), 3.65 - 3.73 (m, 1H), 7.20 (t, J = 73.0 Hz, 1H), 7.31 (dd, J = 8.8, 2.4 Hz, 1H), 7.35 (d, J = 2.4 Hz, 1H), 7.40 (s, 1H), 8.06 (s, 1H), 8.10 (d, J = 8.8 Hz, 1H), 12.52 (s, 1H). |
| 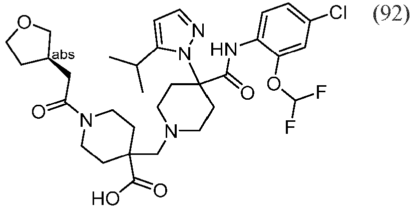 | 4-[[4-[[4-chloro-2-(difluoromethoxy)phenyl] carbamoyl]-4-(5-isopropylpyrazol-1-yl)-1-piperidyl]methyl]-1-[2-[(3*R*)-tetrahydrofuran-3-yl]acetyl]piperidine-4-carboxylic acid | ¹H NMR (400 MHz, DMSO) δ 1.09 (d, J = 6.7 Hz, 6H), 1.22 - 1.53 (m, 2H), 1.82 - 2.05 (m, 3H), 2.28 - 2.48 (m, 11H), 2.64 - 2.85 (m, 3H), 2.91 (p, J = 6.6 Hz, 1H), 3.06 - 3.25 (m, 2H), 3.54 - 3.74 (m, 3H), 3.75 - 3.85 (m, 1H), 3.97 - 4.10 (m, 1H), 6.32 (d, J = 1.9 Hz, 1H), 6.94 - 7.36 (m, 3H), 7.52 (d, J = 1.8 Hz, 1H), 7.75 - 7.84 (m, 1H), 8.13 (s, 1H). |
| 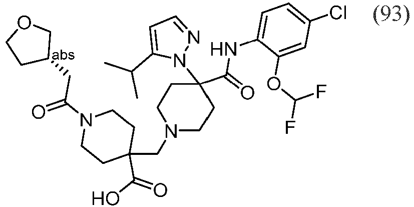 | 4-[[4-[[4-chloro-2-(difluoromethoxy)phenyl] carbamoyl]-4-(5-isopropylpyrazol-1-yl)-1-piperidyl]methyl]-1-[2-[(3*S*)-tetrahydrofuran-3-yl]acetyl]piperidine-4-carboxylic acid | ¹H NMR (400 MHz, DMSO) δ 1.09 (d, J = 6.7 Hz, 6H), 1.21 - 1.54 (m, 3H), 1.82 - 2.04 (m, 3H), 2.27 - 2.48 (m, 10H), 2.63 - 2.84 (m, 2H), 2.91 (p, J = 6.6 Hz, 1H), 3.06 - 3.26 (m, 2H), 3.54 - 3.74 (m, 3H), 3.74 - 3.84 (m, 1H), 3.95 - 4.09 (m, 1H), 6.32 (d, J = 1.9 Hz, 1H), 6.92 - 7.35 (m, 3H), 7.52 (d, J = 1.8 Hz, 1H), 7.74 - 7.83 (m, 1H), 8.13 (s, 1H). |
| 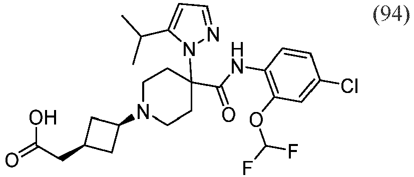 | (1*s*,3*s*)-2-[3-[4-[[4-chloro-2-(difluoromethoxy)phenyl] carbamoyl]-4-(5-isopropylpyrazol-1-yl)-1-piperidyl]cyclobutyl]aceti c acid | ¹H NMR (600 MHz, DMSO-d6) δ 8.15 (s, 1H), 7.80 (d, *J =* 6.2 Hz, 1H), 7.52 (d, *J =* 1.9 Hz, 1H), 7.32 (m, 2H), 7.15 (t, *J* = 73.2 Hz, 1H), 6.32 (d, *J =* 2.1 Hz, 1H), 3.32 (m, 4H), 2.93 (m, 1H), 2.61 (bs, 1H), 2.49 (bs, 1H), 2.36 (bs, 1H), 2.29 (m, 2H), 2.16 (m, 4H), 1.45 (bs, 2H), 1.10 (d, *J =* 6.6 Hz, 6H). |
| 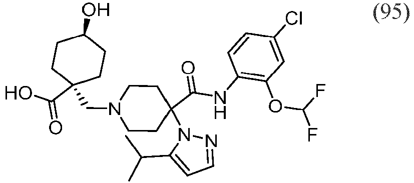 | (1*s*,4*s*)-1-[[4-[[4-chloro-2-(difluoromethoxy)phenyl] carbamoyl]-4-(5-isopropylpyrazol-1-yl)-1-piperidyl]methyl]-4-hydroxy-cyclohexanecarboxylic acid | ¹H NMR (400 MHz, DMSO-d6) δ 8.10 (s, 1H), 7.81 (d, *J =* 9.4 Hz, 1H), 7.52 (d, *J =* 1.8 Hz, 1H), 7.32 (m, 2H), 7.14 (t, *J* = 73.2 Hz, 1H), 6.32 (d, J = 1.8 Hz, 1H), 4.42 (d, J = 4.5 Hz, 1H), 2.91 (m, 1H), 2.70 (m, 2H), 2.39 (m, 7H), 2.00 (m, 2H), 1.66 (m, 2H), 1.17 (m, 4H), 1.09 (d, *J =* 6.7 Hz, 6H). |
| 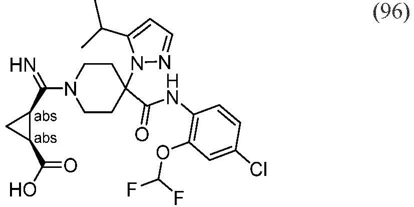 | (1*S*,2*R*)-2-[4-[[4-chloro-2-(difluoromethoxy)phenyl] carbamoyl]-4-(5-isopropylpyrazol-1-yl)piperidine-1-carboximidoyl]cycloprop anecarboxylic acid | ¹H NMR (600 MHz, DMSO) δ 8.35 (s, 1H), 7.73 (d, *J =* 9.2 Hz, 1H), 7.54 (s, 1H), 7.43 (s, 1H), 7.35 - 7.31 (m, 2H), 7.15 (t, *J =* 73.2 Hz, 1H), 6.80 (s, 1H), 6.35 (s, 1H), 4.04-3.87 (m, 2H), 3.59 (t, *J =* 11.3 Hz, 1H), 3.15 (t, *J =* 12.1 Hz, 1H), 2.96 - 2.90 (m, 1H), 2.46 - 2.38 (m, 2H), 2.32 - 2.25 (m, 1H), 2.23 - 2.15 (m, 1H), 2.06 - 1.99 (m, 1H), 1.98 - 1.89 (m, 1H), 1.32 - 1.22 (m, 1H), 1.12 (m, 6H), 1.01 - 0.94 (m, 1H). |
| 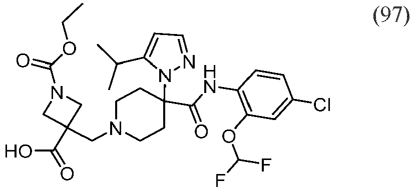 | 3-[[4-[[4-chloro-2-(difluoromethoxy)phenyl] carbamoyl]-4-(5-isopropylpyrazol-1-yl)-1-piperidyl]methyl]-1-ethoxycarbonyl-azetidine-3-carboxylic acid | ¹H NMR (400 MHz, DMSO) δ 1.10 (d, J = 6.6 Hz, 6H), 1.15 (t, J = 7.1 Hz, 3H), 2.22 - 2.39 (m, 2H), 2.42 - 2.48 (m, 2H), 2.61 - 2.76 (m, 2H), 2.83 (s, 2H), 2.87 - 3.02 (m, 1H), 3.34 - 3.43 (m, 2H), 3.76 (s, 2H), 3.99 (q, J = 7.1 Hz, 2H), 4.02 - 4.11 (m, 2H), 6.33 (d, J = 1.9 Hz, 1H), 6.93 - 7.43 (m, 3H), 7.53 (d, J = 1.8 Hz, 1H), 7.76 (d, J = 9.4 Hz, 1H), 8.22 (s, 1H), 13.50 (s, 1H). |
| 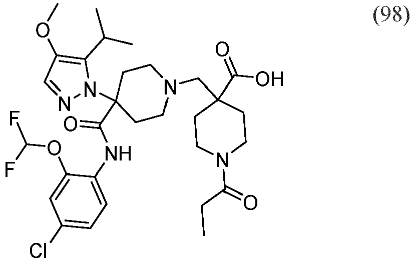 | 4-[[4-[[4-chloro-2-(difluoromethoxy)phenyl] carbamoyl]-4-(5-isopropyl-4-methoxy-pyrazol-1-yl)-1-piperidyl]methyl]-1-propanoyl-piperidine-4-carboxylic acid | ¹H NMR (400 MHz, DMSO) δ 0.96 (t, J = 7.4 Hz, 3H), 1.10 (d, J = 6.9 Hz, 6H), 1.25 (d, J = 15.1 Hz, 1H), 1.33 - 1.43 (m, 1H), 1.84 - 1.99 (m, 2H), 2.23 - 2.38 (m, 4H), 2.43 - 2.48 (m, 4H), 2.69 (q, J = 4.8 Hz, 2H), 2.74 - 2.84 (m, 1H), 2.82 - 2.94 (m, 2H), 3.12 (t, J = 12.1 Hz, 1H), 3.38 (q, J = 7.1 Hz, 1H), 3.59 - 3.70 (m, 1H), 3.71 (s, 3H), 4.03 (d, J = 13.2 Hz, 1H), 6.96 - 7.35 (m, 3H), 7.46 (s, 1H), 7.78 (d, J = 9.3 Hz, 1H), 8.15 (s, 1H), 12.62 (s, 1H). |
| 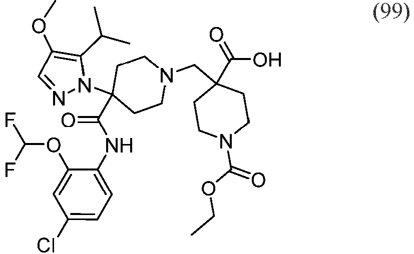 | 4-[[4-[[4-chloro-2-(difluoromethoxy)phenyl] carbamoyl]-4-(5-isopropyl-4-methoxy-pyrazol-1-yl)-1-piperidyl]methyl]-1-ethoxycarbonyl-piperidine-4-carboxylic acid | ¹H NMR (400 MHz, DMSO) δ 0.96 (t, J = 7.4 Hz, 3H), 1.08 - 1.13 (m, 6H), 1.20 - 1.45 (m, 3H), 1.84 - 1.99 (m, 2H), 2.20 - 2.36 (m, 6H), 2.43 - 2.48 (m, 3H), 2.63 - 2.73 (m, 2H), 2.73 - 2.92 (m, 2H), 3.12 (t, J = 12.3 Hz, 1H), 3.65 (d, J = 14.2 Hz, 1H), 3.71 (s, 3H), 4.03 (d, J = 13.0 Hz, 1H), 6.97 - 7.36 (m, 3H), 7.46 (s, 1H), 7.78 (d, J = 9.3 Hz, 1H), 8.15 (s, 1H), 12.62 (s, 1H). |
| 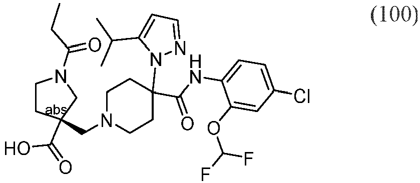 | (3*S*)-3-[[4-[[4-chloro-2-(difluoromethoxy)phenyl] carbamoyl]-4-(5-isopropylpyrazol-1-yl)-1-piperidyl]methyl]-1-propanoyl-pyrrolidine-3-carboxylic acid | ¹H NMR (400 MHz, DMSO) δ 0.96 (q, J = 7.1 Hz, 3H), 1.09 (dd, J = 7.0, 5.2 Hz, 6H), 1.74 - 1.97 (m, 1H), 2.07 - 2.30 (m, 4H), 2.37 (d, J = 11.0 Hz, 1H), 2.46 (s, 1H), 2.52 - 2.81 (m, 5H), 2.91 (p, J = 6.7 Hz, 1H), 3.12 - 3.24 (m, 1H), 3.32 - 3.43 (m, 3H), 3.44 - 3.54 (m, 1H), 3.79 (dd, J = 24.5, 11.1 Hz, 1H), 6.33 (d, J = 1.8 Hz, 1H), 6.96 - 7.35 (m, 3H), 7.53 (d, J = 1.8 Hz, 1H), 7.77 (d, J = 9.3 Hz, 1H), 8.20 (s, 1H), 12.97 (s, 1H). |
| 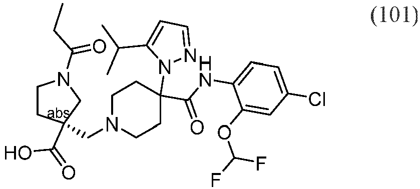 | (3*R*)-3-[[4-[[4-chloro-2-(difluoromethoxy)phenyl] carbamoyl]-4-(5-isopropylpyrazol-1-yl)-1-piperidyl]methyl]-1-propanoyl-pyrrolidine-3-carboxylic acid | ¹H NMR (400 MHz, DMSO) δ 0.97 (td, J = 7.4, 4.5 Hz, 3H), 1.11 (d, J = 6.6 Hz, 6H), 1.77 - 2.02 (m, 2H), 2.08 - 2.31 (m, 4H), 2.38 - 2.48 (m, 2H), 2.56 (dd, J = 10.9, 4.8 Hz, 2H), 2.82 - 2.97 (m, 2H), 3.17 - 3.44 (m, 4H), 3.47 - 3.56 (m, 1H), 3.79 (dd, J = 29.7, 11.2 Hz, 1H), 6.35 (s, 1H), 6.92 - 7.38 (m, 3H), 7.55 (s, 1H), 7.73 (s, 1H), 8.26 (s, 1H), 13.10 (s, 1H). |
| 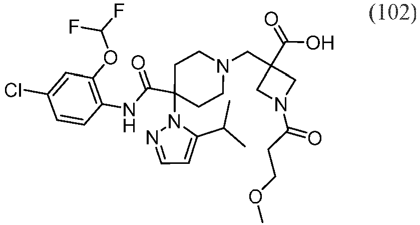 | 3-[[4-[[4-chloro-2-(difluoromethoxy)phenyl] carbamoyl]-4-(5-isopropylpyrazol-1-yl)-1-piperidyl]methyl]-1-(3-methoxypropanoyl)azetid ine-3-carboxylic acid | ¹H NMR (400 MHz, DMSO) δ 1.10 (d, J = 6.6 Hz, 6H), 2.26 (t, J = 6.4 Hz, 2H), 2.34 (q, J = 6.1 Hz, 2H), 2.47 - 2.43 (m, 4H), 2.66 - 2.75 (m, 2H), 2.83 (d, J = 2.7 Hz, 2H), 2.91 (p, J = 6.7 Hz, 1H), 3.19 (s, 3H), 3.49 (t, J = 6.4 Hz, 2H), 3.68 (d, J = 9.7 Hz, 1H), 3.98 (dd, J = 9.0, 5.3 Hz, 2H), 4.31 (d, J = 8.5 Hz, 1H), 6.33 (d, J = 1.9 Hz, 1H), 6.95 - 7.42 (m, 3H), 7.53 (d, J = 1.8 Hz, 1H), 7.76 (d, J = 9.3 Hz, 1H), 8.22 (s, 1H). |
| 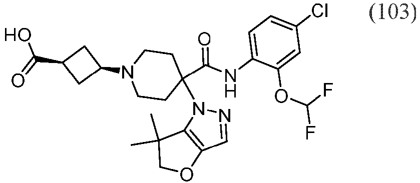 | (1*s*,3*s*)-3-[4-[[4-chloro-2-(difluoromethoxy)phenyl] carbamoyl]-4-(6,6-dimethyl-5H-furo[3,2-c]pyrazol-1-yl)-1-piperidyl]cyclobutanecar boxylic acid | ¹H NMR (400 MHz, DMSO) δ 1.21 (s, 6H), 1.84 - 2.05 (m, 4H), 2.13 - 2.25 (m, 2H), 2.25 - 2.39 (m, 2H), 2.45 (d, J = 9.5 Hz, 2H), 2.52 - 2.60 (m, 1H), 2.61 - 2.75 (m, 3H), 4.53 (s, 2H), 7.21 (t, J = 73.1 Hz, 1H), 7.22 (s, 1H), 7.28 - 7.34 (m, 1H), 7.35 (d, J = 2.3 Hz, 1H), 7.83 (s, 1H), 8.02 (d, J = 8.8 Hz, 1H). |
| 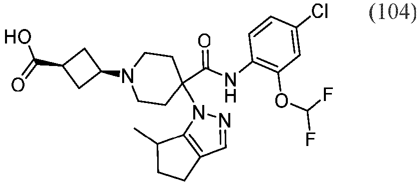 | (1*s*,3*s*)-3-[4-[[4-chloro-2-(difluoromethoxy)phenyl] carbamoyl]-4-(6-methyl-5,6-dihydro-4H-cyclopenta[c]pyrazol-1-yl)-1-piperidyl]cyclobutanecar boxylic acid | ¹H NMR (400 MHz, MeOD) δ 1.10 (d, J = 6.9 Hz, 3H), 2.09 - 2.25 (m, 1H), 2.29 - 2.47 (m, 3H), 2.47 - 2.64 (m, 5H), 2.64 - 3.00 (m, 6H), 3.10 - 3.23 (m, 2H), 3.21 - 3.32 (m, 1H), 3.44 (s, 1H), 6.88 (t, J = 72.8 Hz, 1H), 7.25 (d, J = 7.6 Hz, 2H), 7.48 (s, 1H), 8.14 - 8.28 (m, 1H). |
| 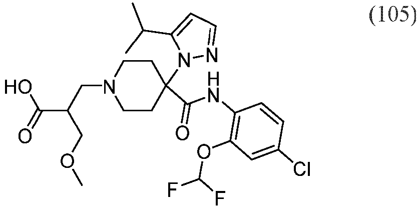 | 2-[[4-[[4-chloro-2-(difluoromethoxy)phenyl] carbamoyl]-4-(5-isopropylpyrazol-1-yl)-1-piperidyl]methyl]-3-methoxy-propanoic acid | ¹H NMR (400 MHz, DMSO-d6) δ 8.21 (s, 1H), 7.78 (d, *J =* 9.4 Hz, 1H), 7.53 (d, *J =* 1.8 Hz, 1H), 7.32 (m, 2H), 7.15 (t, *J* = 73.2 Hz, 1H), 6.33 (d, J = 1.8 Hz, 1H), 3.48 (m, 4H), 3.21 (s, 3H), 2.93 (m, 1H), 2.83 (m, 1H), 2.75 (m, 2H), 2.49 (m, 3H), 2.38 (m, 3H), 1.10 (d, *J =* 6.7 Hz, 6H). |
| 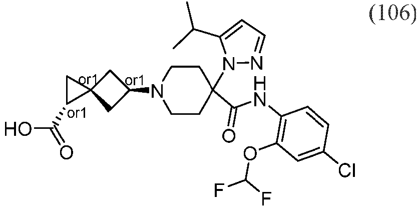 | *rel-*(1*R*,3*r*,5*S*)*-*5-[4-[[4-chloro-2-(difluoromethoxy)phenyl] carbamoyl]-4-(5-isopropylpyrazol-1-yl)-1-piperidyl]spiro[2.3]hexan e-2-carboxylic acid | ¹H NMR (400 MHz, DMSO) δ 8.01 (s, 1H), 7.82 (d, J = 9.4 Hz, 1H), 7.56 (d, J = 1.9 Hz, 1H), 7.44 - 7.14 (m, 2H), 7.17 (t, J = 73.3 Hz, 1H), 6.88 (s, 0H), 6.34 (d, J = 1.9 Hz, 1H), 2.99 (d, J = 6.7 Hz, 2H), 2.97 (s, 2H), 2.86 (q, J = 7.4 Hz, 2H), 2.70 - 2.51 (m, 2H), 2.41 (t, J = 11.8 Hz, 1H), 2.25 (t, J = 10.2 Hz, 1H), 2.19 - 1.95 (m, 2H), 1.46 (dd, J = 8.2, 5.5 Hz, 1H), 1.25 (s, 1H), 1.11 (d, J = 6.7 Hz, 6H), 1.05 - 0.82 (m, 3H). |
| 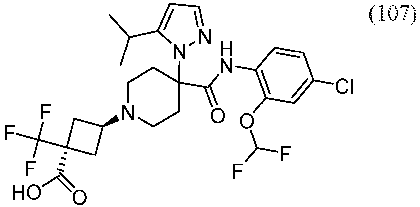 | (1*s*,3*s*)-3-[4-[[4-chloro-2-(difluoromethoxy)phenyl] carbamoyl]-4-(5-isopropylpyrazol-1-yl)-1-piperidyl]-1-(trifluoromethyl)cyclobut anecarboxylic acid | ¹H NMR (500 MHz, DMSO) δ 8.22 (s, 1H), 7.80 - 7.75 (m, 1H), 7.51 (d, J = 1.8 Hz, 1H), 7.35 - 7.28 (m, 2H), 7.15 (t, J = 73.3 Hz, 1H), 6.32 (d, J = 1.8 Hz, 1H), 2.93 (hept, J = 6.6 Hz, 1H), 2.84 (p, J = 8.0 Hz, 1H), 2.69 - 2.62 (m, 3H), 2.48 (dd, J = 7.3, 2.7 Hz, 3H), 2.39 (td, J = 10.8, 5.9 Hz, 3H), 2.28 - 2.18 (m, 4H), 1.10 (d, J = 6.7 Hz, 6H). |
| 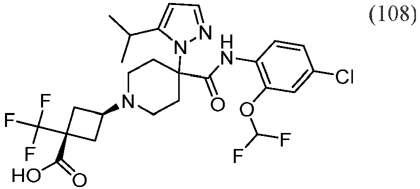 | *(*1*r*,3*r*)-3-[4-[[4-chloro-2-(difluoromethoxy)phenyl] carbamoyl]-4-(5-isopropylpyrazol-1-yl)-1-piperidyl]-1-(trifluoromethyl)cyclobut anecarboxylic acid | ¹H NMR (500 MHz, DMSO) δ 8.42 (s, 1H), 8.22 (s, 1H), 7.80 - 7.75 (m, 1H), 7.51 (d, J = 1.8 Hz, 1H), 7.35 - 7.28 (m, 2H), 7.15 (t, J = 73.3 Hz, 1H), 6.32 (d, J = 1.8 Hz, 1H), 3.76 - 3.59 (m, 2H), 3.58 - 3.43 (m, 2H), 3.20 - 2.87 (m, 4H), 2.77 - 2.53 (m, 6H), 1.11 (d, J = 6.6 Hz, 6H). |
| 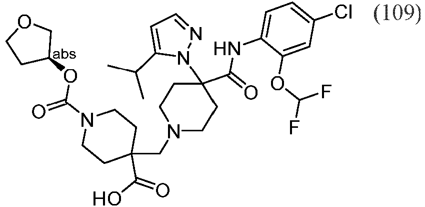 | 4-[[4-[[4-chloro-2-(difluoromethoxy)phenyl] carbamoyl]-4-(5-isopropylpyrazol-1-yl)-1-piperidyl]methyl]-1-[(3*S*)-tetrahydrofuran-3-yl]oxycarbonyl-piperidine-4-carboxylic acid | ¹H NMR (400 MHz, DMSO) δ 1.08 (d, J = 6.7 Hz, 6H), 1.29 - 1.42 (m, 2H), 1.81 - 1.96 (m, 3H), 2.01 - 2.15 (m, 1H), 2.28 - 2.47 (m, 7H), 2.65 - 2.77 (m, 2H), 2.85 - 3.04 (m, 3H), 3.60 - 3.81 (m, 6H), 5.04 - 5.15 (m, 1H), 6.32 (d, J = 1.9 Hz, 1H), 6.91 - 7.36 (m, 3H), 7.52 (d, J = 1.8 Hz, 1H), 7.79 (d, J = 9.4 Hz, 1H), 8.13 (s, 1H), 12.63 (s, 1H). |
| 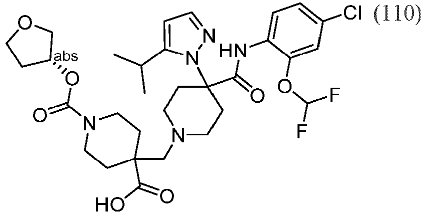 | 4-[[4-[[4-chloro-2-(difluoromethoxy)phenyl] carbamoyl]-4-(5-isopropylpyrazol-1-yl)-1-piperidyl]methyl]-1-[(3*R*)-tetrahydrofuran-3-yl]oxycarbonyl-piperidine-4-carboxylic acid | ¹H NMR (400 MHz, DMSO) δ 1.10 (d, J = 6.6 Hz, 6H), 1.36 - 1.52 (m, 2H), 1.83 - 1.97 (m, 4H), 2.03 - 2.15 (m, 1H), 2.60 - 2.52 (m, 2H), 2.76 - 3.16 (m, 8H), 3.59 - 3.82 (m, 6H), 5.04 - 5.17 (m, 1H), 6.36 (d, J = 1.9 Hz, 1H), 6.92 - 7.40 (m, 3H), 7.55 (d, J = 1.9 Hz, 1H), 7.73 (d, J = 8.8 Hz, 1H), 8.30 (s, 1H). |
| 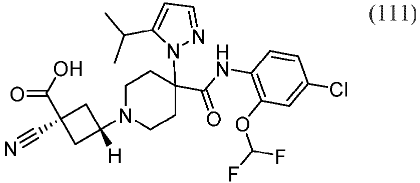 | *(*1*r*,3*r*)-3-[4-[[4-chloro-2-(difluoromethoxy)phenyl] carbamoyl]-4-(5-isopropylpyrazol-1-yl)-1-piperidyl]-1-cyano-cyclobutanecarboxylic acid | ¹H NMR (500 MHz, PYRIDINE) δ 8.45 (s, 1H), 8.35 (d, J = 8.8 Hz, 1H), 7.76 (d, J = 1.8 Hz, 1H), 7.48 (d, J = 2.3 Hz, 1H), 7.31 - 7.25 (m, 2H), 7.05 (s, 1H), 6.40 (d, J = 1.9 Hz, 1H), 3.22 - 3.09 (m, 3H), 2.95 (td, J = 9.3, 2.6 Hz, 3H), 2.92 - 2.72 (m, 5H), 2.43 (s, 3H), 1.17 (d, J = 6.7 Hz, 6H). |
| 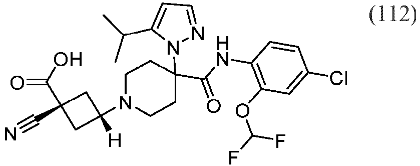 | (1*s*,3*s*)-3-[4-[[4-chloro-2-(difluoromethoxy)phenyl] carbamoyl]-4-(5-isopropylpyrazol-1-yl)-1-piperidyl]-1-cyano-cyclobutanecarboxylic acid | ¹H NMR (500 MHz, PYRIDINE) δ 8.41 - 8.35 (m, 1H), 7.74 (d, *J =* 1.9 Hz, 1H), 7.48 (d, *J =* 2.3 Hz, 1H), 7.31 - 7.24 (m, 2H), 6.88 (s, 2H), 6.38 (d, *J* = 1.8 Hz, 1H), 3.20 (dp, *J* = 42.2, 7.2 Hz, 3H), 3.02 - 2.94 (m, 2H), 2.87 (d, *J =* 13.6 Hz, 2H), 2.76 (td, *J* = 7.7, 4.2 Hz, 4H), 2.73 - 2.65 (m, 1H), 2.34 (s, 1H), 1.31 (d, *J =* 22.7 Hz, 1H), 1.16 (d, *J =* 6.7 Hz, 6H). |
| 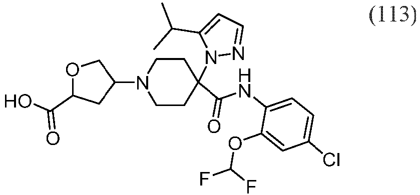 | 4-[4-[[4-chloro-2-(difluoromethoxy)phenyl] carbamoyl]-4-(5-isopropylpyrazol-1-yl)-1-piperidyl]tetrahydrofuran -2-carboxylic acid | ¹H NMR (400 MHz, DMSO) δ 7.91 (d, J = 9.4 Hz, 1H), 7.51 (d, J = 1.8 Hz, 1H), 7.30 - 7.25 (m, 2H), 7.14 (t, J = 73.2 Hz, 1H), 6.28 (d, J = 1.9 Hz, 1H), 4.34 - 4.26 (m, 1H), 3.97 - 3.88 (m, 1H), 3.59 (dd, J = 8.4, 6.0 Hz, 1H), 2.97 (q, J = 6.8 Hz, 4H), 2.75 (m, 1H), 2.62 (m, 1H), 2.53 (d, J = 6.0 Hz, 2H), 2.45 - 2.34 (m, 4H), 2.07 - 1.99 (m, 2H), 1.11 (d, J = 6.7 Hz, 6H). |
| 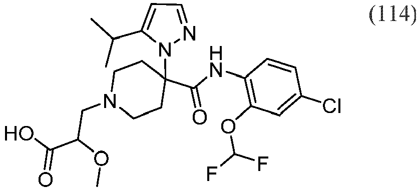 | 3-[4-[[4-chloro-2-(difluoromethoxy)phenyl] carbamoyl]-4-(5-isopropylpyrazol-1-yl)-1-piperidyl]-2-methoxy-propanoic acid | ¹H NMR (500 MHz, DMSO-d6) δ 8.18 (s, 1H), 7.79 (d, *J =* 9.4 Hz, 1H), 7.53 (d, *J =* 1.8 Hz, 1H), 7.32 (m, 2H), 7.15 (t, *J* = 73.2 Hz, 1H), 6.32 (d, J = 1.8 Hz, 1H), 3.86 (m, 1H), 3.27 (s, 3H), 2.90 (m, 1H), 2.85 (m, 1H), 2.78 (m, 1H), 2.60 (m, 3H), 2.50 (m, 3H), 2.37 (m, 2H), 1.10 (d, *J* = 6.7 Hz, 6H). |
| 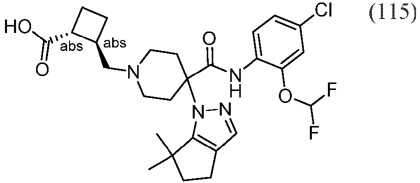 | (1R,2R)-2-[[4-[[4-chloro-2-(difluoromethoxy)phenyl] carbamoyl]-4-(6,6-dimethyl-4,5-dihydrocyclopenta[c]pyra zol-1-yl)-1-piperidyl]methyl]cyclobu tanecarboxylic acid | ¹H NMR (400 MHz, DMSO) δ 8.06 (d, *J =* 8.7 Hz, 1H), 7.45 (s, 1H), 7.35 (s, 1H), 7.27 (dt, *J =* 5.4, 2.7 Hz, 2H), 7.16 (t, *J =* 67.6 Hz, 1H), 2.88 - 2.76 (m, 3H), 2.72 (q, *J =* 8.6 Hz, 1H), 2.63 (m, 1H), 2.58 (d, *J =* 13.6 Hz, 2H), 2.52 (d, *J =* 6.7 Hz, 1H), 2.45 - 2.28 (m, 5H), 2.29 - 2.20 (m, 3H), 2.03 - 1.92 (m, 2H), 1.90 (m, 1H), 1.61 - 1.51 (m, 1H), 1.18 (s, 6H). |
| 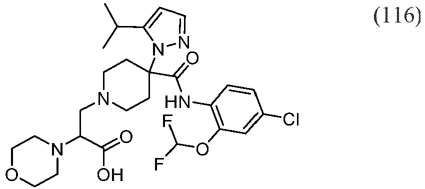 | 3-[4-[[4-chloro-2-(difluoromethoxy)phenyl] carbamoyl]-4-(5-isopropylpyrazol-1-yl)-1-piperidyl]-2-morpholino-propanoic acid | ¹H NMR (600 MHz, DMSO-d6) δ 8.19 (s, 1H), 7.81 (d, *J =* 9.4 Hz, 1H), 7.53 (d, *J =* 1.8 Hz, 1H), 7.32 (m, 2H), 7.15 (t, *J* = 73.2 Hz, 1H), 6.33 (d, J = 1.8 Hz, 1H), 3.53 (m, 4H), 3.30 (m, 1H), 2.90 (m, 2H), 2.74 (m, 2H), 2.59 (m, 4H), 2.47 (m, 4H), 2.37 (m, 3H), 1.10 (d, *J =* 6.7 Hz, 6H). |
| 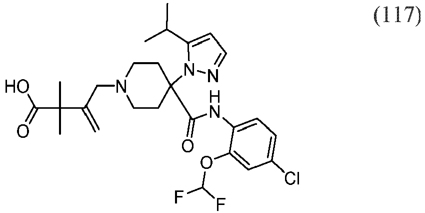 | 3-[[4-[[4-chloro-2-(difluoromethoxy)phenyl] carbamoyl]-4-(5-isopropylpyrazol-1-yl)-1-piperidyl]methyl]-2,2-dimethyl-but-3-enoic acid | ¹H NMR (400 MHz, MeOD) δ 8.00 (d, *J =* 8.5 Hz, 1H), 7.64 (d, *J =* 1.9 Hz, 1H), 7.30 - 7.24 (m, 2H), 6.83 (t, *J =* 73.0 Hz, 1H), 6.42 (d, *J =* 2.0 Hz, 1H), 5.52 (s, 1H), 5.33 (s, 1H), 3.65 (s, 2H), 3.57 - 3.47 (m, 2H), 3.27 - 3.06 (m, 2H), 2.99 (p, *J =* 6.7 Hz, 1H), 2.94 - 2.82 (m, 2H), 2.81 - 2.68 (m, 2H), 1.41 (s, 6H), 1.21 (d, *J* = 6.7 Hz, 6H). |
| 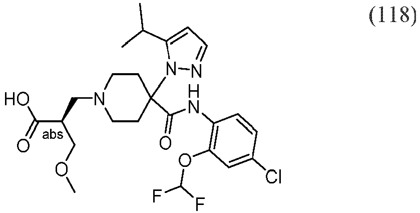 | (2*S*)-2-[[4-[[4-chloro-2-(difluoromethoxy)phenyl] carbamoyl]-4-(5-isopropylpyrazol-1-yl)-1-piperidyl]methyl]-3-methoxy-propanoic acid | ¹H NMR (400 MHz, DMSO-d6) δ 8.21 (s, 1H), 7.78 (d, *J =* 9.4 Hz, 1H), 7.53 (d, *J =* 1.8 Hz, 1H), 7.32 (m, 2H), 7.15 (t, *J* = 73.2 Hz, 1H), 6.33 (d, J = 1.8 Hz, 1H), 3.48 (m, 4H), 3.21 (s, 3H), 2.93 (m, 1H), 2.83 (m, 1H), 2.75 (m, 2H), 2.49 (m, 3H), 2.38 (m, 3H), 1.10 (d, *J =* 6.7 Hz, 6H). |
| 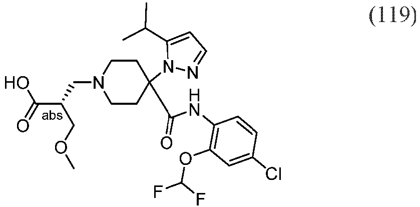 | (2R)-2-[[4-[[4-chloro-2-(difluoromethoxy)phenyl] carbamoyl]-4-(5-isopropylpyrazol-1-yl)-1-piperidyl]methyl]-3-methoxy-propanoic acid | ¹H NMR (400 MHz, DMSO-d6) δ 8.21 (s, 1H), 7.78 (d, *J =* 9.4 Hz, 1H), 7.53 (d, *J =* 1.8 Hz, 1H), 7.32 (m, 2H), 7.15 (t, *J* = 73.2 Hz, 1H), 6.33 (d, J = 1.8 Hz, 1H), 3.48 (m, 4H), 3.21 (s, 3H), 2.93 (m, 1H), 2.83 (m, 1H), 2.75 (m, 2H), 2.49 (m, 3H), 2.38 (m, 3H), 1.10 (d, *J =* 6.7 Hz, 6H). |
| 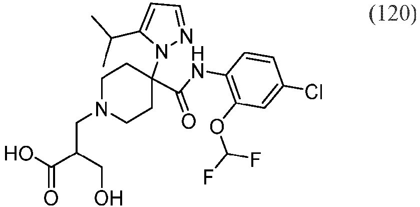 | 2-[[4-[[4-chloro-2-(difluoromethoxy)phenyl] carbamoyl]-4-(5-isopropylpyrazol-1-yl)-1-piperidyl]methyl]-3-hydroxy-propanoic acid | ¹H NMR (400 MHz, DMSO-d6) δ 8.21 (s, 1H), 7.78 (d, *J =* 9.4 Hz, 1H), 7.53 (d, *J =* 1.8 Hz, 1H), 7.32 (m, 2H), 7.15 (t, *J* = 73.2 Hz, 1H), 6.33 (d, J = 1.8 Hz, 1H), 3.53 (m, 4H), 2.93 (m, 1H), 2.83 (m, 1H), 2.72 (m, 1H), 2.61 (m, 1H), 2.50 (m, 3H), 2.40 (m, 4H), 1.10 (d, *J =* 6.7 Hz, 6H). |
| 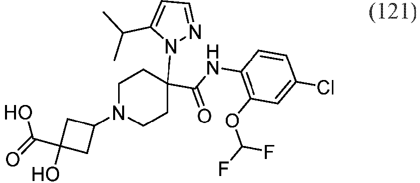 | 3-[4-[[4-chloro-2-(difluoromethoxy)phenyl] carbamoyl]-4-(5-isopropylpyrazol-1-yl)-1-piperidyl]-1-hydroxy-cyclobutanecarboxylic acid | ¹H NMR (400 MHz, DMSO) δ 7.99 (s, 1H), 7.93 - 7.82 (m, 1H), 7.56 - 7.49 (m, 1H), 7.33 - 7.22 (m, 2H), 7.06 (t, J = 73.0 Hz, 1H), 6.32 (d, J = 1.9 Hz, 1H), 3.15 - 2.90 (m, 4H), 2.70 - 2.50 (m, 4H), 2.47 (d, J = 1.9 Hz, 4H), 2.43 - 2.35 (m, 2H), 2.28 - 2.19 (m, 2H), 1.13 (d, J = 6.8 Hz, 6H). |
| 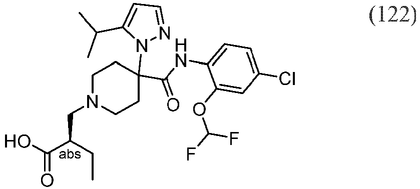 | (2R)-2-[[4-[[4-chloro-2-(difluoromethoxy)phenyl] carbamoyl]-4-(5-isopropylpyrazol-1-yl)-1-piperidyl]methyl]butanoi c acid | ¹H NMR (400 MHz, METHANOL-d4) δ ppm 0.89 - 1.02 (m, 3 H) 1.14 - 1.26 (m, 6 H) 1.51 - 1.63 (m, 1 H) 1.64 - 1.77 (m, 1 H) 2.49 - 2.61 (m, 1 H) 2.64 - 2.79 (m, 2 H) 2.79 - 2.90 (m, 2 H) 2.90 - 3.02 (m, 2 H) 3.03 - 3.16 (m, 2 H) 3.16 - 3.28 (m, 1 H) 3.38 - 3.59 (m, 2 H) 6.34 - 6.45 (m, 1 H) 6.59 - 7.02 (m, 1 H) 7.17 - 7.34 (m, 2 H) 7.54 - 7.67 (m, 1 H) 7.89 - 8.05 (m, 1 H). |
| 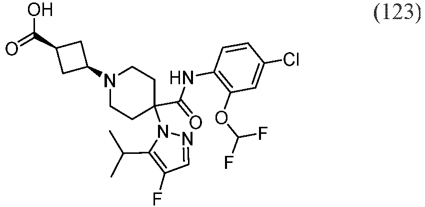 | (1*s*,3*s*)-3-[4-[[4-chloro-2-(difluoromethoxy)phenyl] carbamoyl]-4-(4-fluoro-5-isopropyl-pyrazol-1-yl)-1-piperidyl]cyclobutanecar boxylic acid | ¹H NMR (500 MHz, DMSO) δ 8.62 (s, 1H), 7.67 - 7.58 (m, 1H), 7.56 (d, J = 4.2 Hz, 1H), 7.34 (d, J = 2.3 Hz, 2H), 7.10 (d, J = 73.3 Hz, 1H), 2.99 (p, J = 6.8 Hz, 1H), 2.67 (tt, J = 9.8, 8.2 Hz, 1H), 2.57 (ddd, J = 15.3, 7.3, 3.8 Hz, 3H), 2.36 (d, J = 9.9 Hz, 4H), 2.25 - 2.13 (m, 4H), 1.95 - 1.83 (m, 2H), 1.16 (d, J = 6.8 Hz, 6H). |
| 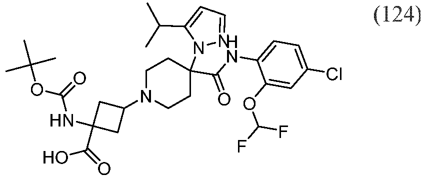 | 1-(*tert-*butoxycarbonylamino)-3-[4-[[4-chloro-2-(difluoromethoxy)phenyl] carbamoyl]-4-(5-isopropylpyrazol-1-yl)-1-piperidyl]cyclobutanecar boxylic acid | ¹H NMR (400 MHz, DMSO) δ 1.10 (d, J = 6.6 Hz, 6H), 1.36 (s, 9H), 2.14 - 2.45 (m, 8H), 2.59 - 2.76 (m, 2H), 2.87 - 3.01 (m, 2H), 6.32 (d, J = 1.9 Hz, 1H), 7.05 (s, 1H), 6.95 - 7.37 (m, 3H), 7.52 (d, J = 1.8 Hz, 1H), 7.79 (d, J = 9.1 Hz, 1H), 8.22 (s, 1H). |
| 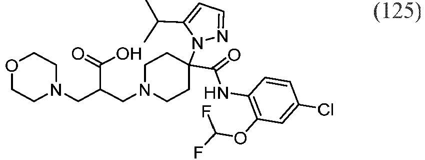 | 2-[[4-[[4-chloro-2-(difluoromethoxy)phenyl] carbamoyl]-4-(5-isopropylpyrazol-1-yl)-1-piperidyl]methyl]-3-morpholino-propanoic acid | ¹H NMR (600 MHz, DMSO-d6) δ 8.19 (s, 1H), 7.78 (d, *J =* 9.4 Hz, 1H), 7.52 (d, *J =* 1.8 Hz, 1H), 7.32 (m, 2H), 7.15 (t, *J* = 73.2 Hz, 1H), 6.33 (d, J = 1.8 Hz, 1H), 3.53 (m, 4H), 2.93 (m, 1H), 2.84 (m, 1H), 2.78 (m, 1H), 2.67 (m, 1H), 2.49 (m, 4H), 2.41 (m, 2H), 2.35 (m, 8H), 1.10 (d, *J* = 6.7 Hz, 6H). |
| 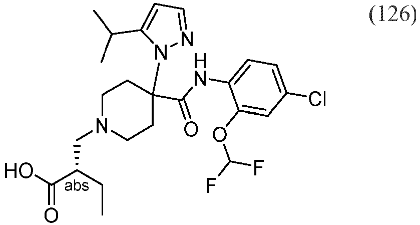 | (2*S*)-2-[[4-[[4-chloro-2-(difluoromethoxy)phenyl] carbamoyl]-4-(5-isopropylpyrazol-1-yl)-1-piperidyl]methyl]butanoi c acid | ¹H NMR (400 MHz, METHANOL-d4) δ ppm 0.89 - 1.02 (m, 3 H) 1.14 - 1.26 (m, 6 H) 1.51 - 1.63 (m, 1 H) 1.64 - 1.77 (m, 1 H) 2.49 - 2.61 (m, 1 H) 2.64 - 2.79 (m, 2 H) 2.79 - 2.90 (m, 2 H) 2.90 - 3.02 (m, 2 H) 3.03 - 3.16 (m, 2 H) 3.16 - 3.28 (m, 1 H) 3.38 - 3.59 (m, 2 H) 6.34 - 6.45 (m, 1 H) 6.59 - 7.02 (m, 1 H) 7.17 - 7.34 (m, 2 H) 7.54 - 7.67 (m, 1 H) 7.89 - 8.05 (m, 1 H). |
| 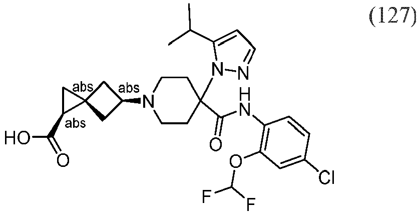 | (1*S*,3*r*,5*R*)-5-[4-[[4-chloro-2-(difluoromethoxy)phenyl] carbamoyl]-4-(5-isopropylpyrazol-1-yl)-1-piperidyl]spiro[2.3]hexan e-2-carboxylic acid | ¹H NMR (400 MHz, DMSO) δ 8.01 (s, 1H), 7.82 (d, J = 9.4 Hz, 1H), 7.56 (d, J = 1.9 Hz, 1H), 7.44 - 7.14 (m, 2H), 7.17 (t, J = 73.3 Hz, 1H), 6.88 (s, 0H), 6.34 (d, J = 1.9 Hz, 1H), 2.99 (d, J = 6.7 Hz, 2H), 2.97 (s, 2H), 2.86 (q, J = 7.4 Hz, 2H), 2.70 - 2.51 (m, 2H), 2.41 (t, J = 11.8 Hz, 1H), 2.25 (t, J = 10.2 Hz, 1H), 2.19 - 1.95 (m, 2H), 1.46 (dd, J = 8.2, 5.5 Hz, 1H), 1.25 (s, 1H), 1.11 (d, J = 6.7 Hz, 6H), 1.05 - 0.82 (m, 3H). |
| 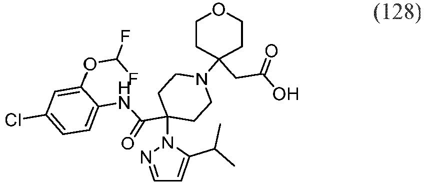 | 2-[4-[4-[[4-chloro-2-(difluoromethoxy)phenyl] carbamoyl]-4-(5-isopropylpyrazol-1-yl)-1-piperidyl]tetrahydropyran -4-yl]acetic acid | ¹H NMR (400 MHz, DMSO-d6) δ 1.10 (d, J = 6.6 Hz, 6H), 1.48 - 1.65 (m, 2H), 1.71 - 1.88 (m, 2H), 2.24 - 2.45 (m, 5H), 2.57 - 2.72 (m, 3H), 2.92 (dd, J = 15.1, 8.4 Hz, 3H), 3.49 (d, J = 6.8 Hz, 2H), 3.71 (t, J = 9.0 Hz, 2H), 6.33 (d, J = 1.9 Hz, 1H), 7.16 (t, J = 73.1 Hz, 1H), 7.30 - 7.34 (m, 2H), 7.53 (d, J = 1.8 Hz, 1H), 7.78 (dd, J = 13.0, 9.3 Hz, 1H), 8.25 (s, 1H), 13.39 (s, 1H). |
| 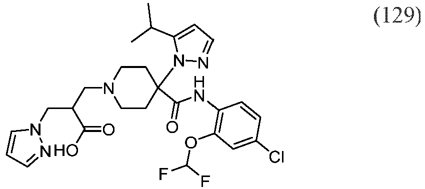 | 2-[[4-[[4-chloro-2-(difluoromethoxy)phenyl] carbamoyl]-4-(5-isopropylpyrazol-1-yl)-1-piperidyl]methyl]-3-pyrazol-1-yl-propanoic acid | ¹H NMR (400 MHz, DMSO-d6) δ 8.21 (s, 1H), 7.78 (d, *J =* 9.4 Hz, 1H), 7.65 (m, 1H), 7.52 (d, *J =* 1.8 Hz, 1H), 7.41 (m, 1H), 7.35 (m, 2H), 7.15 (t, *J* = 73.2 Hz, 1H), 6.32 (d, J = 1.8 Hz, 1H), 6.20 (m, 1H), 4.30 (m, 2H), 3.08 (m, 1H), 2.92 (m, 1H), 2.80 (m, 1H), 2.71 (m, 1H), 2.52 (m, 4H), 2.38 (m, 2H), 2.30 (m, 2H), 1.10 (d, *J =* 6.7 Hz, 6H). |
| 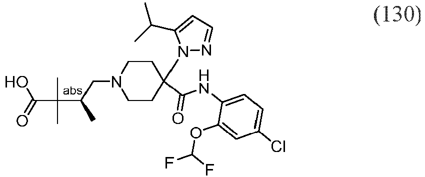 | (3*S*)-4-[4-[[4-chloro-2-(difluoromethoxy)phenyl] carbamoyl]-4-(5-isopropylpyrazol-1-yl)-1-piperidyl]-2,2,3-trimethyl-butanoic acid | ¹H NMR (400 MHz, METHANOL-d4) δ ppm 1.02 (d, J=7.13 Hz, 3 H) 1.07 - 1.13 (m, 3 H) 1.15 - 1.23 (m, 9 H) 2.09 - 2.22 (m, 1 H) 2.62 - 2.88 (m, 6 H) 2.91 - 3.12 (m, 3 H) 3.33 - 3.47 (m, 2 H) 6.34 - 6.42 (m, 1 H) 6.59 - 7.01 (m, 1 H) 7.19 - 7.30 (m, 2 H) 7.57 - 7.64 (m, 1 H) 7.95 - 8.05 (m, 1 H). |
| 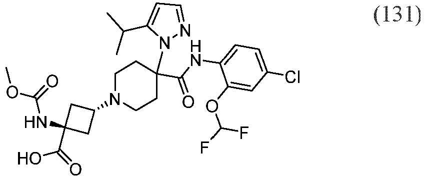 | *(*1*r*,3*r*)-3-[4-[[4-chloro-2-(difluoromethoxy)phenyl] carbamoyl]-4-(5-isopropylpyrazol-1-yl)-1-piperidyl]-1-(methoxycarbonylamino) cyclobutanecarboxylic acid | ¹H NMR (400 MHz, DMSO) δ 1.12 (d, J = 6.7 Hz, 6H), 2.40 - 2.47 (m, 2H), 2.52 - 2.71 (m, 6H), 2.99 - 2.70 (m, 2H), 3.05 - 3.26 (m, 3H), 3.53 (s, 3H), 6.38 (d, J = 1.9 Hz, 1H), 7.18 (t, J = 73.3 Hz, 1H), 7.30 - 7.35 (m, 2H), 7.56 (d, J = 1.9 Hz, 1H), 7.67 (d, J = 8.7 Hz, 1H), 7.83 (s, 1H), 8.48 (s, 1H). |
| 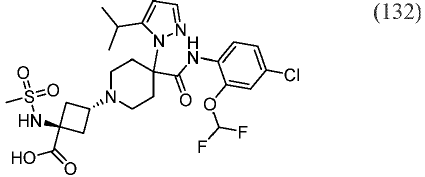 | *(1r,3r)-3-[4-[[4-chloro-2-*(difluoromethoxy)phenyl] carbamoyl]-4-(5-isopropylpyrazol-1-yl)-1-piperidyl]-1-(methanesulfonamido)cyc lobutanecarboxylic acid | ¹H NMR (500 MHz, DMSO) δ 1.12 (d, J = 6.6 Hz, 6H), 2.56 - 2.52 (m, 1H), 2.71 - 2.57 (m, 4H), 2.92 - 2.87 (m, 1H), 2.98 (s, 3H), 6.39 (s, 1H), 7.01 - 7.37 (m, 1H), 7.34 (d, J = 8.1 Hz, 2H), 7.58 (s, 1H), 7.67 (d, J = 8.3 Hz, 1H), 7.88 (s, 1H). |
| 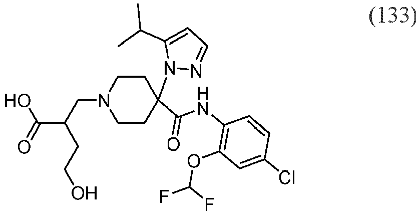 | 2-[[4-[[4-chloro-2-(difluoromethoxy)phenyl] carbamoyl]-4-(5-isopropylpyrazol-1-yl)-1-piperidyl]methyl]-4-hydroxy-butanoic acid | ¹H NMR (600 MHz, DMSO-d6) δ 8.20 (s, 1H), 7.79 (d, *J =* 7.1 Hz, 1H), 7.53 (m, 1H), 7.31 (m, 2H), 7.16 (t, *J =* 73.3 Hz, 1H), 6.33 (d, *J =* 2.1 Hz, 1H), 3.99 (m, 4H), 2.93 (m, 1H), 2.86 (m, 1H), 2.72 (m, 1H), 2.62 (m, 1H), 2.55 (m, 1H), 2.36 (m, 4H), 1.68 (m, 1H), 1.53 (m, 1H), 1.10 (d, *J =* 6.7 Hz, 6H). |
| 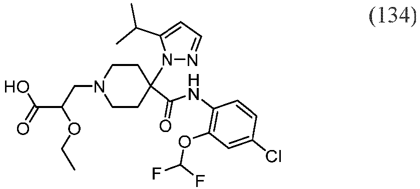 | 3-[4-[[4-chloro-2-(difluoromethoxy)phenyl] carbamoyl]-4-(5-isopropylpyrazol-1-yl)-1-piperidyl]-2-ethoxy-propanoic acid | ¹H NMR (500 MHz, DMSO) δ 8.21 (s, 1H), 7.80 - 7.75 (m, 1H), 7.59 (d, *J =* 1.9 Hz, 1H), 7.34 - 7.28 (m, 2H), 7.10 (t, *J =* 73.2 Hz, 1H), 6.37 (d, *J =* 1.9 Hz, 1H), 4.31 (dd, *J =* 9.3, 3.4 Hz, 1H), 3.75 - 3.64 (m, 1H), 3.59 - 3.49 (m, 2H), 3.46 - 3.40 (m, 1H), 3.36 - 3.20 (m, 3H), 2.98 - 2.88 (m, 1H), 2.83 - 2.64 (m, 4H), 1.19 - 1.12 (m, 9H). |
| 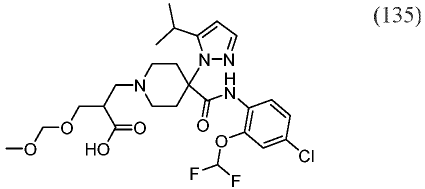 | 2-[[4-[[4-chloro-2-(difluoromethoxy)phenyl] carbamoyl]-4-(5-isopropylpyrazol-1-yl)-1-piperidyl]methyl]-3-(methoxymethoxy)propan oic acid | ¹H NMR (400 MHz, DMSO-d6) δ 8.21 (s, 1H), 7.78 (d, *J =* 9.4 Hz, 1H), 7.53 (d, *J =* 1.8 Hz, 1H), 7.32 (m, 2H), 7.15 (t, *J* = 73.2 Hz, 1H), 6.33 (d, J = 1.8 Hz, 1H), 4.52 (m, 2H), 3.60 (m, 4H), 3.23 (s, 3H), 2.93 (m, 1H), 2.78 (m, 2H), 2.53 (m, 3H), 2.39 (m, 4H), 1.10 (d, *J =* 6.7 Hz, 6H). |
| 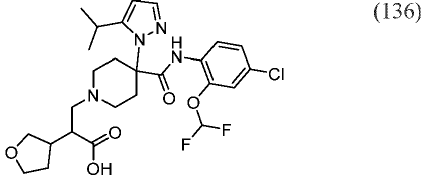 | 3-[4-[[4-chloro-2-(difluoromethoxy)phenyl] carbamoyl]-4-(5-isopropylpyrazol-1-yl)-1-piperidyl]-2-tetrahydrofuran-3-yl-propanoic acid | ¹H NMR (400 MHz, DMSO) δ 7.91 (dd, J = 9.3, 1.0 Hz, 1H), 7.81 (s, 1H), 7.51 (t, J = 1.8 Hz, 1H), 7.31 - 7.21 (m, 2H), 7.15 (t, J = 73.3 Hz, 1H), 6.28 (t, J = 1.7 Hz, 1H), 3.82 - 3.65 (m, 2H), 3.59 (q, J = 7.7 Hz, 1H), 3.36 (dt, J = 12.8, 8.1 Hz, 1H), 2.98 (hept, J = 6.9 Hz, 1H), 2.83 (dd, J = 16.8, 10.9 Hz, 1H), 2.69 (t, J = 7.0 Hz, 1H), 2.61 (dt, J = 12.2, 3.0 Hz, 1H), 2.54 (d, J = 14.7 Hz, 3H), 2.45 - 2.34 (m, 4H), 2.28 (ddt, J = 12.6, 7.7, 3.9 Hz, 2H), 1.94 (ddtd, J = 17.0, 12.3, 7.6, 4.5 Hz, 1H), 1.81 - 1.70 (m, 1H), 1.60 (ddq, J = 17.9, 12.1, 8.0 Hz, 1H), 1.11 (dt, J = 6.7, 1.2 Hz, 6H). |
| 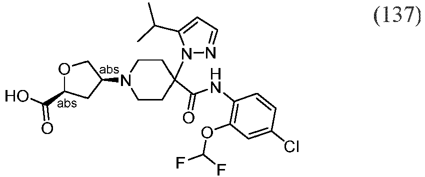 | (2*S*,4*S*)-4-[4-[[4-chloro-2-(difluoromethoxy)phenyl] carbamoyl]-4-(5-isopropylpyrazol-1-yl)-1-piperidyl]tetrahydrofuran -2-carboxylic acid | ¹H NMR (400 MHz, DMSO) δ 8.18 (s, 1H), 7.87 - 7.72 (m, 1H), 7.53 (d, *J* = 1.9 Hz, 1H), 7.36 - 6.94 (m, 3H), 6.33 (d, *J =* 1.9 Hz, 1H), 4.31 (t, *J =* 7.8 Hz, 1H), 3.86 (dd, *J =* 8.5, 6.3 Hz, 1H), 3.65 (t, *J =* 7.9 Hz, 1H), 2.97 - 2.73 (m, 3H), 2.71 - 2.59 (m, 1H), 2.49 - 2.31 (m, 7H), 1.84 - 1.73 (m, 1H), 1.10 (d, *J =* 6.6 Hz, 6H). |
| 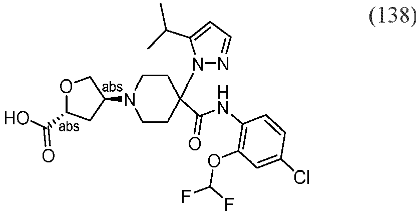 | (2*R*,4*S*)-4-[4-[[4-chloro-2-(difluoromethoxy)phenyl] carbamoyl]-4-(5-isopropylpyrazol-1-yl)-1-piperidyl]tetrahydrofuran -2-carboxylic acid | ¹H NMR (400 MHz, DMSO) δ 8.26 (s, 1H), 7.82 - 7.70 (m, 1H), 7.54 (d, *J =* 1.9 Hz, 1H), 7.36 - 6.95 (m, 3H), 6.34 (d, *J =* 1.9 Hz, 1H), 4.45 (t, *J =* 6.6 Hz, 1H), 3.96 (dd, *J =* 8.9, 6.6 Hz, 1H), 3.75 - 3.64 (m, 1H), 2.99 - 2.83 (m, 2H), 2.81 - 2.67 (m, 1H), 2.50 (p, *J =* 1.8 Hz, 7H), 2.14 (s, 2H), 1.10 (d, *J =* 6.6 Hz, 6H). |
| 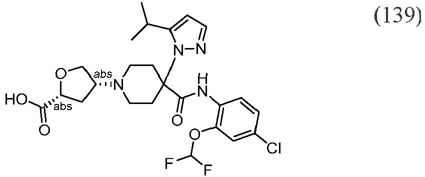 | (2R,4R)-4-[4-[[4-chloro-2-(difluoromethoxy)phenyl] carbamoyl]-4-(5-isopropylpyrazol-1-yl)-1-piperidyl]tetrahydrofuran -2-carboxylic acid | ¹H NMR (500 MHz, DMSO) δ 8.18 (s, 1H), 7.83 - 7.72 (m, 1H), 7.53 (d, *J* = 1.9 Hz, 1H), 7.34 - 7.31 (m, 2H), 7.15 (t, *J* = 73.2 Hz, 1H), 6.33 (d, *J =* 1.8 Hz, 1H), 4.30 (t, *J* = 7.8 Hz, 1H), 3.86 (dd, *J =* 8.5, 6.3 Hz, 1H), 3.65 (dd, *J =* 8.5, 7.4 Hz, 1H), 2.92 (p, *J =* 6.7 Hz, 1H), 2.81 (dt, *J =* 23.3, 7.6 Hz, 2H), 2.65 (q, *J* = 5.7 Hz, 1H), 2.50 - 2.31 (m, 4H), 1.85 - 1.67 (m, 1H), 1.24 (t, *J=* 7.2 Hz, 1H), 1.10 (d, *J =* 6.6 Hz, 6H). |
| 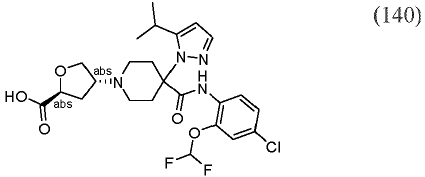 | (2S,4R)-4-[4-[[4-chloro-2-(difluoromethoxy)phenyl] carbamoyl]-4-(5-isopropylpyrazol-1-yl)-1-piperidyl]tetrahydrofuran -2-carboxylic acid | ¹H NMR (500 MHz, DMSO) δ 8.20 (s, 1H), 7.80 - 7.74 (m, 1H), 7.53 (d, *J* = 1.9 Hz, 1H), 7.38 - 6.94 (m, 3H), 6.33 (d, *J = 1.9* Hz, 1H), 4.42 (t, *J =* 6.6 Hz, 1H), 3.94 (dd, *J =* 8.6, 6.6 Hz, 1H), 3.71 - 3.59 (m, 1H), 2.97 - 2.88 (m, 1H), 2.77 (m, 1H), 2.69 - 2.47 (m, 5H), 2.45 - 2.31 (m, 3H), 2.10 - 2.07 (m, 2H), 1.10 (d, *J =* 6.6 Hz, 6H). |
| 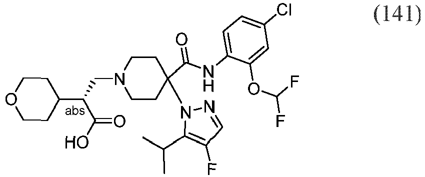 | (2R)-3-[4-[[4-chloro-2-(difluoromethoxy)phenyl] carbamoyl]-4-(4-fluoro-5-isopropyl-pyrazol-1-yl)-1-piperidyl]-2-tetrahydropyran-4-yl-propanoic acid | ¹H NMR (500 MHz, DMSO) δ 1.17 (dd, J = 6.9, 3.6 Hz, 6H), 1.22 - 1.40 (m, 3H), 1.36 - 1.47 (m, 1H), 1.49 - 1.59 (m, 1H), 1.70 - 1.86 (m, 1H), 2.39 - 2.49 (m, 2H), 2.51 - 2.62 (m, 2H), 2.66 - 3.16 (m, 6H), 3.07 - 3.29 (m, 3H), 3.75 - 3.88 (m, 2H), 7.19 (t, J = 73.4 Hz, 1H), 7.34 (d, J = 7.4 Hz, 2H), 7.57 (d, J = 9.1 Hz, 1H), 7.62 (d, J = 4.1 Hz, 1H), 8.80 (s, 1H), 12.09 (s, 1H). |
| 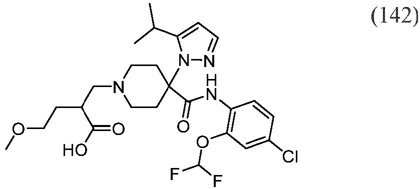 | 2-[[4-[[4-chloro-2-(difluoromethoxy)phenyl] carbamoyl]-4-(5-isopropylpyrazol-1-yl)-1-piperidyl]methyl]-4-methoxy-butanoic acid | ¹H NMR (400 MHz, DMSO-d6) δ 8.18 (s, 1H), 7.79 (d, *J =* 9.4 Hz, 1H), 7.53 (d, *J =* 1.8 Hz, 1H), 7.32 (m, 2H), 7.15 (t, *J* = 73.2 Hz, 1H), 6.33 (d, J = 1.8 Hz, 1H), 3.32 (m, 4H), 3.20 (s, 3H), 2.93 (m, 1H), 2.83 (m, 1H), 2.69 (m, 1H), 2.58 (m, 2H), 2.48 (m, 2H), 2.35 (m, 3H), 1.72 (m, 1H), 1.62 (m, 1H), 1.10 (d, *J =* 6.7 Hz, 6H). |
| 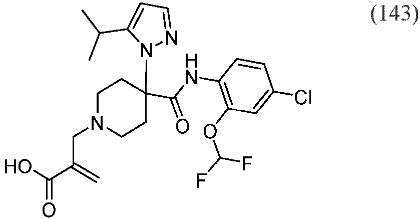 | 2-[[4-[[4-chloro-2-(difluoromethoxy)phenyl] carbamoyl]-4-(5-isopropylpyrazol-1-yl)-1-piperidyl]methyl]prop-2-enoic acid | ¹H NMR (600 MHz, DMSO-d6) δ 8.22 (s, 1H), 7.79 (d, *J =* 9.4 Hz, 1H), 7.53 (d, *J =* 1.8 Hz, 1H), 7.32 (m, 2H), 7.15 (t, *J* = 73.2 Hz, 1H), 6.33 (d, J = 1.8 Hz, 1H), 6.09 (m, 1H), 5.68 (m, 1H), 3.16 (m, 2H), 2.93 (m, 1H), 2.77 (m, 2H), 2.51 (m, 2H), 2.43 (m, 4H), 1.10 (d, *J =* 6.7 Hz, 6H). |
| 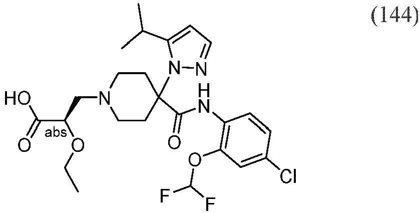 | (2R)-3-[4-[[4-chloro-2-(difluoromethoxy)phenyl] carbamoyl]-4-(5-isopropylpyrazol-1-yl)-1-piperidyl]-2-ethoxy-propanoic acid | ¹H NMR (400 MHz, DMSO) δ 8.16 (s, 1H), 7.84 - 7.77 (m, 1H), 7.53 (d, *J =* 1.8 Hz, 1H), 7.37 - 6.94 (m, 3H), 6.32 (d, *J =* 1.9 Hz, 1H), 3.94 (t, *J* = 5.7 Hz, 1H), 3.60 - 3.48 (m, 1H), 3.43 - 3.30 (m, 1H), 2.99 - 2.89 (m, 1H), 2.87 - 2.74 (m, 2H), 2.59 (d, *J =* 5.7 Hz, 2H), 2.50 - 2.42 (m, 4H), 2.41 - 2.27 (m, 2H), 1.16 - 1.02 (m, 9H). |
| 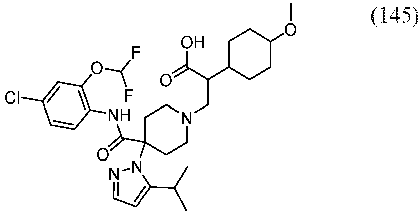 | 3-[4-[[4-chloro-2-(difluoromethoxy)phenyl] carbamoyl]-4-(5-isopropylpyrazol-1-yl)-1-piperidyl]-2-(4-methoxycyclohexyl)prop anoic acid | ¹H NMR (400 MHz, DMSO) δ 7.91 (dd, *J =* 9.3, 1.0 Hz, 1H), 7.81 (s, 1H), 7.51 (t, *J =* 1.8 Hz, 1H), 7.31 - 7.21 (m, 2H), 7.15 (t, *J =* 73.3 Hz, 1H), 6.28 (t, *J* = 1.7 Hz, 1H), 3.19 (d, J = 9.9 Hz, 3H), 2.98 (dq, J = 13.4, 6.2 Hz, 2H), 2.90 - 2.80 (m, 1H), 2.74 - 2.62 (m, 1H), 2.59 - 2.46 (m, 6H), 2.43 - 2.30 (m, 6H), 1.97 (d, J = 8.8 Hz, 1H), 1.77 (d, J = 12.8 Hz, 1H), 1.67 - 1.28 (m, 4H), 1.11 (dd, J = 6.7, 1.5 Hz, 6H), 1.08 - 0.96 (m, 1H). |
| 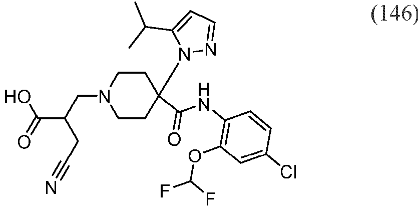 | 2-[[4-[[4-chloro-2-(difluoromethoxy)phenyl] carbamoyl]-4-(5-isopropylpyrazol-1-yl)-1-piperidyl]methyl]-3-cyano-propanoic acid | ¹H NMR (600 MHz, DMSO-d6) δ 8.23 (s, 1H), 7.78 (d, *J =* 9.4 Hz, 1H), 7.53 (d, *J =* 1.8 Hz, 1H), 7.32 (m, 2H), 7.15 (t, *J* = 73.2 Hz, 1H), 6.33 (d, J = 1.8 Hz, 1H), 3.17 (s, 1H), 2.93 (m, 2H), 2.80 (m, 2H), 2.69 (m, 2H), 2.58 (m, 2H), 2.50 (m, 4H), 2.41 (m, 2H), 1.10 (d, *J =* 6.7 Hz, 6H). |
| 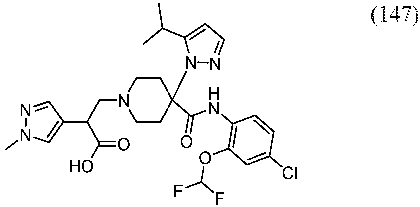 | 3-[4-[[4-chloro-2-(difluoromethoxy)phenyl] carbamoyl]-4-(5-isopropylpyrazol-1-yl)-1-piperidyl]-2-(1-methylpyrazol-4-yl)propanoic acid | ¹H NMR (500 MHz, DMSO) δ 8.18 (s, 1H), 7.83 - 7.76 (m, 1H), 7.55 (s, 1H), 7.52 (d, *J =* 1.9 Hz, 1H), 7.34 - 6.99 (m, 4H), 6.32 (d, *J* = 1.9 Hz, 1H), 3.77 (s, 3H), 3.75 - 3.66 (m, 1H), 3.00 - 2.79 (m, 3H), 2.75 - 2.64 (m, 1H), 2.50 (m, 3H), 2.43 - 2.29 (m, 4H), 1.10 (dd, *J* = 6.7, 2.0 Hz, 6H). |
| 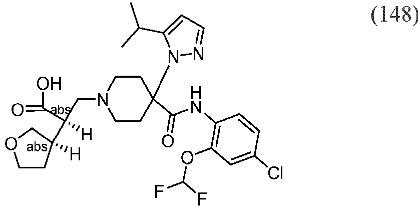 | (2R)-3-[4-[[4-chloro-2-(difluoromethoxy)phenyl] carbamoyl]-4-(5-isopropylpyrazol-1-yl)-1-piperidyl]-2-[(3R)-tetrahydrofuran-3-yl]propanoic acid | ¹H NMR (400 MHz, DMSO) δ 7.91 (dd, *J = 9.3,* 1.0 Hz, 1H), 7.81 (s, 1H), 7.51 (t, *J =* 1.8 Hz, 1H), 7.31 - 7.21 (m, 2H), 7.15 (t, *J =* 73.3 Hz, 1H), 6.28 (t, *J* = 1.7 Hz, 1H), 3.79 (dd, *J* = 8.5, 7.4 Hz, 1H), 3.69 (td, *J* = 8.3, 4.6 Hz, 1H), 3.59 (q, *J =* 7.7 Hz, 1H), 3.42 - 3.29 (m, 1H), 2.98 (hept, *J =* 6.7 Hz, 1H), 2.83 (td, *J =* 10.6, 5.1 Hz, 1H), 2.69 (dd, *J =* 10.6, 5.7 Hz, 1H), 2.62 - 2.50 (m, 5H), 2.43 - 2.33 (m, 4H), 2.33 - 2.23 (m, 2H), 1.92 (dtd, *J =* 12.3, 7.7, 4.7 Hz, 1H), 1.68 - 1.58 (m, 1H), 1.11 (dd, *J =* 6.7, 1.6 Hz, 6H). |
| 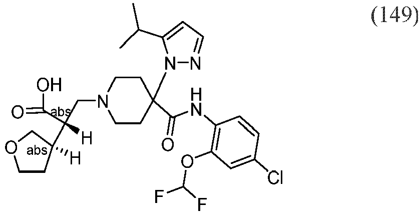 | (2*S*)-3-[4-[[4-chloro-2-(difluoromethoxy)phenyl] carbamoyl]-4-(5-isopropylpyrazol-1-yl)-1-piperidyl]-2-[(3R)-tetrahydrofuran-3-yl]propanoic acid | ¹H NMR (400 MHz, DMSO) δ 7.91 (dd, *J= 9.3,* 1.0 Hz, 1H), 7.81 (s, 1H), 7.51 (t, *J=* 1.8 Hz, 1H), 7.31 - 7.21 (m, 2H), 7.15 (t, *J=* 73.3 Hz, 1H), 6.28 (t, *J* = 1.7 Hz, 1H), 3.80 - 3.66 (m, 2H), 3.59 (td, *J* = 8.1, 7.1 Hz, 1H), 3.37 (dd, *J=* 8.5, 7.5 Hz, 1H), 2.99 (h, *J* = 6.7 Hz, 1H), 2.88 (d, *J =* 6.1 Hz, 1H), 2.86 (s, 1H), 2.71 (dd, *J =* 10.6, 5.4 Hz, 1H), 2.67 - 2.52 (m, 3H), 2.45 - 2.36 (m, 4H), 2.29 (dt, *J =* 16.0, 8.0 Hz, 1H), 2.05 - 1.83 (m, 2H), 1.57 (dq, *J =* 12.2, 8.1 Hz, 2H), 1.11 (dd, *J =* 6.7, 1.5 Hz, 6H). |
| 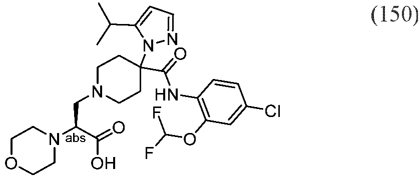 | (2*S*)-3-[4-[[4-chloro-2-(difluoromethoxy)phenyl] carbamoyl]-4-(5-isopropylpyrazol-1-yl)-1-piperidyl]-2-morpholino-propanoic acid | ¹H NMR (400 MHz, DMSO) δ 8.19 (s, 1H), 7.82 - 7.75 (m, 1H), 7.53 (d, *J =* 1.8 Hz, 1H), 7.37 - 6.92 (m, 3H), 6.33 (d, *J =* 1.8 Hz, 1H), 3.57 - 3.49 (m, 4H), 3.30 (dd, *J =* 9.0, 5.5 Hz, 1H), 2.99 - 2.85 (m, 2H), 2.79 - 2.69 (m, 2H), 2.62 - 2.56 (m, 4H), 2.49 - 2.31 (m, 8H), 1.10 (d, *J* = 6.6 Hz, 6H). |
| 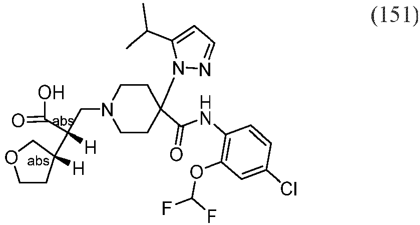 | (2*S*)-3-[4-[[4-chloro-2-(difluoromethoxy)phenyl] carbamoyl]-4-(5-isopropylpyrazol-1-yl)-1-piperidyl]-2-[(3*S*)-tetrahydrofuran-3-yl]propanoic acid | ¹H NMR (400 MHz, DMSO) δ 7.91 (dd, *J =* 9.3, 1.0 Hz, 1H), 7.81 (s, 1H), 7.51 (t, *J =* 1.8 Hz, 1H), 7.31 - 7.21 (m, 2H), 7.15 (t, *J =* 73.3 Hz, 1H), 6.28 (t, *J* = 1.7 Hz, 1H), 3.80 - 3.66 (m, 2H), 3.59 (td, *J* = 8.1, 7.1 Hz, 1H), 3.37 (dd, *J =* 8.5, 7.5 Hz, 1H), 2.99 (h, *J* = 6.7 Hz, 1H), 2.88 (d, *J =* 6.1 Hz, 1H), 2.86 (s, 1H), 2.71 (dd, *J =* 10.6, 5.4 Hz, 1H), 2.67 - 2.52 (m, 3H), 2.45 - 2.36 (m, 4H), 2.29 (dt, *J =* 16.0, 8.0 Hz, 1H), 2.05 - 1.83 (m, 2H), 1.57 (dq, *J =* 12.2, 8.1 Hz, 2H), 1.11 (dd, *J =* 6.7, 1.5 Hz, 6H). |
| 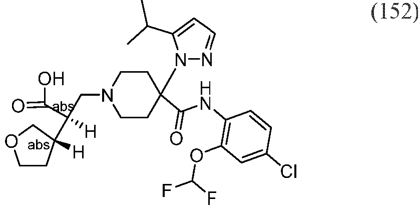 | (2R)-3-[4-[[4-chloro-2-(difluoromethoxy)phenyl] carbamoyl]-4-(5-isopropylpyrazol-1-yl)-1-piperidyl]-2-[(3*S*)-tetrahydrofuran-3-yl]propanoic acid | ¹H NMR (400 MHz, DMSO) δ 7.91 (dd, *J = 9.3,* 1.0 Hz, 1H), 7.81 (s, 1H), 7.51 (t, *J =* 1.8 Hz, 1H), 7.31 - 7.21 (m, 2H), 7.15 (t, *J =* 73.3 Hz, 1H), 6.28 (t, *J* = 1.7 Hz, 1H), 3.79 (dd, *J* = 8.5, 7.4 Hz, 1H), 3.69 (td, *J* = 8.3, 4.6 Hz, 1H), 3.59 (q, *J =* 7.7 Hz, 1H), 3.42 - 3.29 (m, 1H), 2.98 (hept, *J =* 6.7 Hz, 1H), 2.83 (td, *J =* 10.6, 5.1 Hz, 1H), 2.69 (dd, *J =* 10.6, 5.7 Hz, 1H), 2.62 - 2.50 (m, 5H), 2.43 - 2.33 (m, 4H), 2.33 - 2.23 (m, 2H), 1.92 (dtd, *J =* 12.3, 7.7, 4.7 Hz, 1H), 1.68 - 1.58 (m, 1H), 1.11 (dd, *J =* 6.7, 1.6 Hz, 6H). |
| 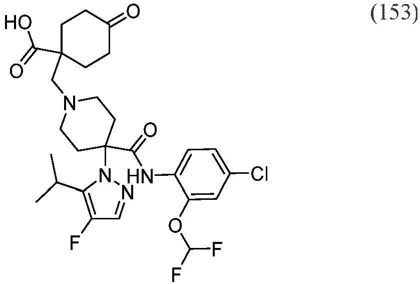 | 1-[[4-[[4-chloro-2-(difluoromethoxy)phenyl] carbamoyl]-4-(4-fluoro-5-isopropyl-pyrazol-1-yl)-1-piperidyl]methyl]-4-oxo-cyclohexanecarboxylic acid | ¹H NMR (500 MHz, DMSO) δ 1.20 (d, J = 6.8 Hz, 6H), 1.87 - 2.03 (m, 2H), 2.20 - 2.29 (m, 2H), 2.32 - 2.43 (m, 4H), 2.56 - 3.02 (m, 4H), 3.06 - 3.26 (m, 1H), 3.66 - 3.39 (m, 6H), 7.02 - 7.43 (m, 3H), 7.53 (d, J = 8.5 Hz, 1H), 7.70 (s, 1H), 8.87 (s, 1H), 13.52 (s, 1H). |
| 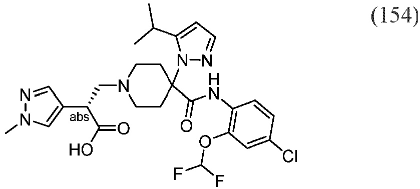 | (2R)-3-[4-[[4-chloro-2-(difluoromethoxy)phenyl] carbamoyl]-4-(5-isopropylpyrazol-1-yl)-1-piperidyl]-2-(1-methylpyrazol-4-yl)propanoic acid | ¹H NMR (500 MHz, DMSO) δ 8.18 (s, 1H), 7.83 - 7.77 (m, 1H), 7.55 (s, 1H), 7.52 (d, *J =* 1.9 Hz, 1H), 7.34 - 6.97 (m, 4H), 6.32 (d, *J =* 1.8 Hz, 1H), 3.77 (s, 3H), 3.70 (dd, *J =* 10.4, 5.2 Hz, 1H), 2.98 - 2.82 (m, 3H), 2.77 - 2.66 (m, 1H), 2.61 - 2.44 (m, *J* = 3.2 Hz, 3H), 2.42 - 2.31 (m, 4H), 1.10 (dd, *J* = 6.7, 2.0 Hz, 6H). |
| 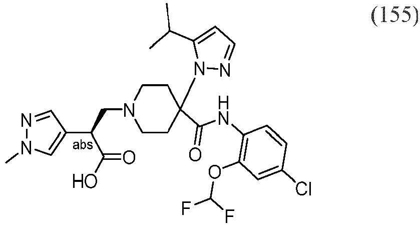 | (2*S*)-3-[4-[[4-chloro-2-(difluoromethoxy)phenyl] carbamoyl]-4-(5-isopropylpyrazol-1-yl)-1-piperidyl]-2-(1-methylpyrazol-4-yl)propanoic acid | ¹H NMR (400 MHz, DMSO) δ 8.18 (s, 1H), 7.79 (m, 1H), 7.55 (s, 1H), 7.52 (d, *J =* 1.9 Hz, 1H), 7.40 - 6.94 (m, 4H), 6.32 (d, *J =* 1.9 Hz, 1H), 3.77 (s, 3H), 3.70 (dd, *J =* 10.4, 5.3 Hz, 1H), 3.01 - 2.80 (m, 3H), 2.77 - 2.68 (m, 1H), 2.58 - 2.44 (m, 3H), 2.44 - 2.28 (m, 4H), 1.10 (dd, *J* = 6.7, 1.6 Hz, 6H). |
| 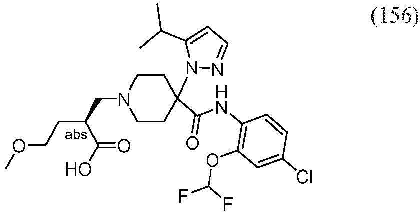 | (2*S*)-2-[[4-[[4-chloro-2-(difluoromethoxy)phenyl] carbamoyl]-4-(5-isopropylpyrazol-1-yl)-1-piperidyl]methyl]-4-methoxy-butanoic acid | ¹H NMR (400 MHz, DMSO-d6) δ 8.18 (s, 1H), 7.79 (d, *J =* 9.4 Hz, 1H), 7.53 (d, *J =* 1.8 Hz, 1H), 7.32 (m, 2H), 7.15 (t, *J* = 73.2 Hz, 1H), 6.33 (d, J = 1.8 Hz, 1H), 3.32 (m, 4H), 3.20 (s, 3H), 2.93 (m, 1H), 2.83 (m, 1H), 2.69 (m, 1H), 2.58 (m, 2H), 2.48 (m, 2H), 2.35 (m, 3H), 1.72 (m, 1H), 1.62 (m, 1H), 1.10 (d, *J =* 6.7 Hz, 6H). |
| 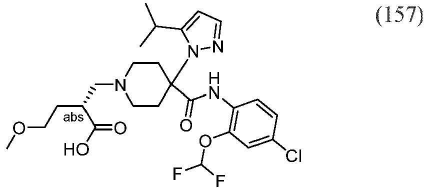 | (2R)-2-[[4-[[4-chloro-2-(difluoromethoxy)phenyl] carbamoyl]-4-(5-isopropylpyrazol-1-yl)-1-piperidyl]methyl]-4-methoxy-butanoic acid | ¹H NMR (400 MHz, DMSO-d6) δ 8.18 (s, 1H), 7.79 (d, *J =* 9.4 Hz, 1H), 7.53 (d, *J =* 1.8 Hz, 1H), 7.32 (m, 2H), 7.15 (t, *J* = 73.2 Hz, 1H), 6.33 (d, J = 1.8 Hz, 1H), 3.32 (m, 4H), 3.20 (s, 3H), 2.93 (m, 1H), 2.83 (m, 1H), 2.69 (m, 1H), 2.58 (m, 2H), 2.48 (m, 2H), 2.35 (m, 3H), 1.72 (m, 1H), 1.62 (m, 1H), 1.10 (d, *J =* 6.7 Hz, 6H). |
| 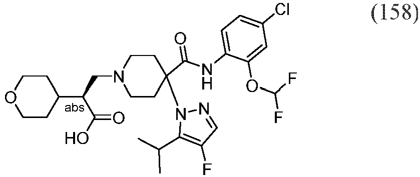 | (2*S*)-3-[4-[[4-chloro-2-(difluoromethoxy)phenyl] carbamoyl]-4-(4-fluoro-5-isopropyl-pyrazol-1-yl)-1-piperidyl]-2-tetrahydropyran-4-yl-propanoic acid | ¹H NMR (500 MHz, DMSO) δ 1.13 - 1.18 (m, 6H), 1.17 - 1.37 (m, 3H), 1.38 - 1.45 (m, 1H), 1.53 - 1.60 (m, 1H), 1.63 - 1.76 (m, 1H), 2.20 - 2.41 (m, 7H), 2.50 - 2.65 (m, 2H), 2.74 - 2.87 (m, 1H), 2.98 (p, J = 6.9 Hz, 1H), 3.23 (t, J = 11.6 Hz, 2H), 3.74 - 3.86 (m, 2H), 7.01 - 7.36 (m, 3H), 7.56 (d, J = 4.1 Hz, 1H), 7.58 - 7.63 (m, 1H), 8.61 (s, 1H), 12.08 (s, 1H). |
| 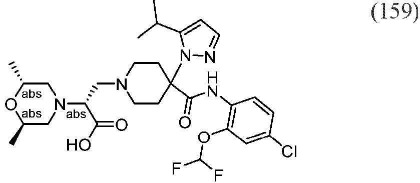 | (2*R*)-3-[4-[[4-chloro-2-(difluoromethoxy)phenyl] carbamoyl]-4-(5-isopropylpyrazol-1-yl)-1-piperidyl]-2-[(2*R*,6*R*)-2,6-dimethylmorpholin-4-yl]propanoic acid | ¹H NMR (500 MHz, DMSO) δ 8.18 (s, 1H), 7.81 - 7.77 (m, 1H), 7.53 (d, *J =* 1.8 Hz, 1H), 7.34 - 7.31 (m, 2H), 7.15 (t, *J =* 73.3 Hz, 1H), 6.32 (d, *J =* 1.9 Hz, 1H), 3.87 - 3.77 (m, 2H), 3.24 (dd, *J =* 8.4, 6.1 Hz, 1H), 2.98 - 2.84 (m, 2H), 2.72 (m, 2H), 2.64 (dd, *J =* 11.1, 3.2 Hz, 2H), 2.61 - 2.32 (m, 7H), 2.27 (dd, *J =* 11.1, 5.7 Hz, 2H), 1.14 - 1.04 (m, 12H). |
| 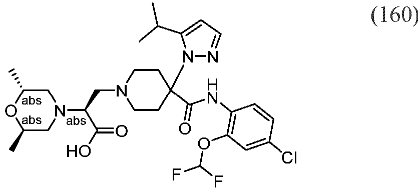 | (2*S*)-3-[4-[[4-chloro-2-(difluoromethoxy)phenyl] carbamoyl]-4-(5-isopropylpyrazol-1-yl)-1-piperidyl]-2-[(2*R*,6*R*)-2,6-dimethylmorpholin-4-yl]propanoic acid | ¹H NMR (400 MHz, DMSO) δ 8.19 (s, 1H), 7.83 - 7.75 (m, 1H), 7.53 (d, *J =* 1.8 Hz, 1H), 7.40 - 6.88 (m, 3H), 6.33 (d, *J =* 1.9 Hz, 1H), 3.93 - 3.77 (m, 2H), 3.28 (dd, *J =* 8.8, 5.7 Hz, 1H), 3.01 - 2.82 (m, 2H), 2.78 - 2.65 (m, 2H), 2.60 (dd, *J =* 11.2, 3.2 Hz, 2H), 2.56 - 2.33 (m, 7H), 2.30 (dd, *J =* 11.2, 5.8 Hz, 2H), 1.13 - 1.06 (m, 12H). |
| 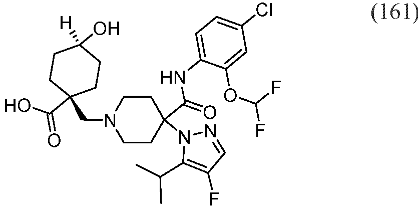 | (1*r*,4*r*)*-*1-[[4-[[4-chloro-2-(difluoromethoxy)phenyl] carbamoyl]-4-(4-fluoro-5-isopropyl-pyrazol-1-yl)-1-piperidyl]methyl]-4-hydroxy-cyclohexanecarboxylic acid | ¹H NMR (500 MHz, DMSO) δ 1.15 (d, J = 6.8 Hz, 6H), 1.29 - 1.45 (m, 2H), 1.48 - 1.67 (m, 6H), 2.26 - 2.40 (m, 4H), 2.43 - 2.50 (m, 4H), 2.65 - 2.75 (m, 2H), 2.98 (p, J = 7.0 Hz, 1H), 3.55 (s, 1H), 4.34 (s, 1H), 7.00 - 7.36 (m, 3H), 7.56 (d, J = 4.1 Hz, 1H), 7.62 (d, J = 9.2 Hz, 1H), 8.58 (s, 1H), 12.34 (s, 1H). |
| 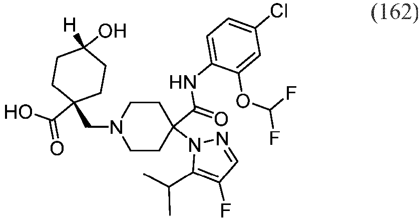 | (1*s*,4*s*)-1-[[4-[[4-chloro-2-(difluoromethoxy)phenyl] carbamoyl]-4-(4-fluoro-5-isopropyl-pyrazol-1-yl)-1-piperidyl]methyl]-4-hydroxy-cyclohexanecarboxylic acid | ¹H NMR (500 MHz, DMSO) δ 1.07 - 1.28 (m, 10H), 1.61 - 1.70 (m, 2H), 1.95 - 2.02 (m, 2H), 2.26 - 2.38 (m, 6H), 2.41 - 2.48 (m, 2H), 2.63 - 2.72 (m, 2H), 2.93 - 3.01 (m, 1H), 3.27 - 3.36 (m, 1H), 4.41 (s, 1H), 6.98 - 7.36 (m, 3H), 7.56 (d, J = 4.2 Hz, 1H), 7.59 - 7.64 (m, 1H), 8.56 (s, 1H). |
| 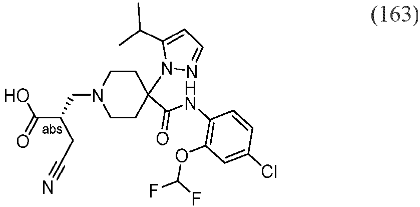 | (2*S*)-2-[[4-[[4-chloro-2-(difluoromethoxy)phenyl] carbamoyl]-4-(5-isopropylpyrazol-1-yl)-1-piperidyl]methyl]-3-cyano-propanoic acid | ¹H NMR (600 MHz, DMSO-d6) δ 8.23 (s, 1H), 7.78 (d, *J =* 9.4 Hz, 1H), 7.53 (d, *J =* 1.8 Hz, 1H), 7.32 (m, 2H), 7.15 (t, *J* = 73.2 Hz, 1H), 6.33 (d, J = 1.8 Hz, 1H), 3.17 (s, 1H), 2.93 (m, 2H), 2.80 (m, 2H), 2.69 (m, 2H), 2.58 (m, 2H), 2.50 (m, 4H), 2.41 (m, 2H), 1.10 (d, *J =* 6.7 Hz, 6H). |
| 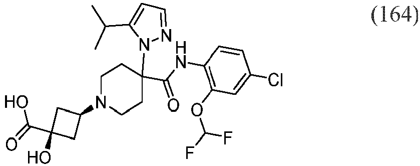 | (1*s*,3*s*)-3-[4-[[4-chloro-2-(difluoromethoxy)phenyl] carbamoyl]-4-(5-isopropylpyrazol-1-yl)-1-piperidyl]-1-hydroxy-cyclobutanecarboxylic acid | ¹H NMR (400 MHz, DMSO) δ 7.99 (s, 1H), 7.93 - 7.82 (m, 1H), 7.56 - 7.49 (m, 1H), 7.33 - 7.22 (m, 2H), 7.06 (t, *J =* 73.0 Hz, 1H), 6.32 (d, *J =* 1.9 Hz, 1H), 3.15 - 2.90 (m, 4H), 2.70 - 2.50 (m, 4H), 2.47 (d, *J =* 1.9 Hz, 4H), 2.43 - 2.35 (m, 2H), 2.28 - 2.19 (m, 2H), 1.13 (d, *J =* 6.8 Hz, 6H). |
| 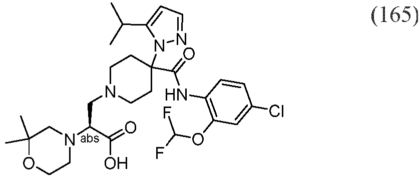 | (2*S*)-3-[4-[[4-chloro-2-(difluoromethoxy)phenyl] carbamoyl]-4-(5-isopropylpyrazol-1-yl)-1-piperidyl]-2-(2,2-dimethylmorpholin-4-yl)propanoic acid | ¹H NMR (500 MHz, DMSO) δ 8.19 (s, 1H), 7.84 - 7.76 (m, 1H), 7.53 (d, *J =* 1.8 Hz, 1H), 7.34 - 7.31 (m, 2H), 7.15 (t, *J =* 73.2 Hz, 1H), 6.33 (d, *J =* 1.9 Hz, 1H), 3.59 - 3.47 (m, 2H), 3.27 (dd, *J =* 8.6, 6.0 Hz, 1H), 2.98 - 2.82 (m, 2H), 2.79 - 2.66 (m, 2H), 2.61 - 2.25 (m, 11H), 1.14 - 1.07 (m, 12H). |
| 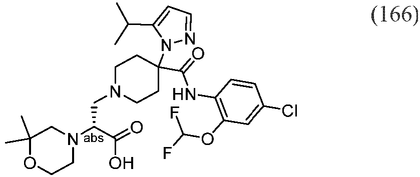 | (2*R*)-3-[4-[[4-chloro-2-(difluoromethoxy)phenyl] carbamoyl]-4-(5-isopropylpyrazol-1-yl)-1-piperidyl]-2-(2,2-dimethylmorpholin-4-yl)propanoic acid | ¹H NMR (400 MHz, DMSO) δ 8.18 (s, 1H), 7.87 - 7.76 (m, 1H), 7.53 (d, *J =* 1.8 Hz, 1H), 7.38 - 6.91 (m, 3H), 6.33 (d, *J =* 1.9 Hz, 1H), 3.61 - 3.47 (m, 2H), 3.28 (dd, *J =* 8.6, 6.0 Hz, 1H), 2.98 - 2.83 (m, 2H), 2.80 - 2.65 (m, 2H), 2.61 - 2.24 (m, 11H), 1.16 - 1.05 (m, 12H). |
| 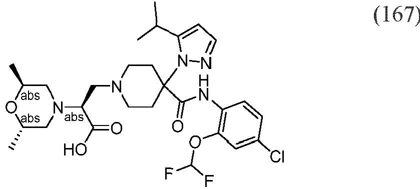 | (2*S*)-3-[4-[[4-chloro-2-(difluoromethoxy)phenyl] carbamoyl]-4-(5-isopropylpyrazol-1-yl)-1-piperidyl]-2-[(2*S*,6*S*)-2,6-dimethylmorpholin-4-yl]propanoic acid | ¹H NMR (500 MHz, DMSO) δ 8.19 (s, 1H), 7.81 - 7.76 (m, 1H), 7.52 (d, *J =* 1.8 Hz, 1H), 7.35 - 7.31 (m, 2H), 7.15 (t, *J =* 73.3 Hz, 1H), 6.33 (d, *J =* 1.8 Hz, 1H), 3.90 - 3.80 (m, 2H), 3.27 (dd, *J =* 8.8, 5.8 Hz, 1H), 2.99 - 2.81 (m, 2H), 2.78 - 2.65 (m, 2H), 2.60 (dd, *J =* 11.0, 3.2 Hz, 2H), 2.49 - 2.44 (m, 1H), 2.43 - 2.33 (m, 6H), 2.29 (dd, *J =* 11.2, 5.8 Hz, 2H), 1.12 - 1.06 (m, 12H). |
| 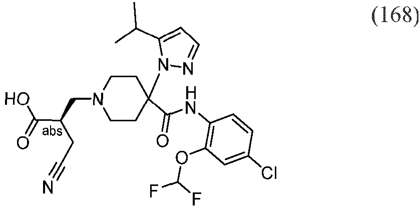 | (2*R*)-2-[[4-[[4-chloro-2-(difluoromethoxy)phenyl] carbamoyl]-4-(5-isopropylpyrazol-1-yl)-1-piperidyl]methyl]-3-cyano-propanoic acid | ¹H NMR (600 MHz, DMSO-d6) δ 8.23 (s, 1H), 7.78 (d, *J =* 9.4 Hz, 1H), 7.53 (d, *J =* 1.8 Hz, 1H), 7.32 (m, 2H), 7.15 (t, *J* = 73.2 Hz, 1H), 6.33 (d, J = 1.8 Hz, 1H), 3.17 (s, 1H), 2.93 (m, 2H), 2.80 (m, 2H), 2.69 (m, 2H), 2.58 (m, 2H), 2.50 (m, 4H), 2.41 (m, 2H), 1.10 (d, *J =* 6.7 Hz, 6H). |
| 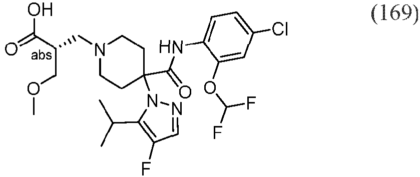 | (2*R*)-2-[[4-[[4-chloro-2-(difluoromethoxy)phenyl] carbamoyl]-4-(4-fluoro-5-isopropyl-pyrazol-1-yl)-1-piperidyl]methyl]-3-methoxy-propanoic acid | ¹H NMR (400 MHz, DMSO-d6) δ 8.64 (s, 1H), 7.62 (m, 1H), 7.57 (d, *J =* 4.1 Hz, 1H), 7.34 (m, 1H), 7.33 (m, 1H), 7.17 (t, *J =* 73.4 Hz, 1H), 3.46 (m, 2H), 3.20 (s, 3H), 2.99 (m, 1H), 2.78 (m, 2H), 2.69 (m, 1H), 2.53 (m, 2H), 2.39 (m, 6H), 1.16 (d, *J =* 6.8 Hz, 6H). |
| 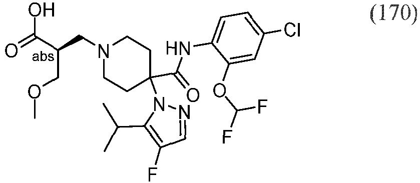 | (2*S*)-2-[[4-[[4-chloro-2-(difluoromethoxy)phenyl] carbamoyl]-4-(4-fluoro-5-isopropyl-pyrazol-1-yl)-1-piperidyl]methyl]-3-methoxy-propanoic acid | ¹H NMR (400 MHz, DMSO-d6) δ 8.64 (s, 1H), 7.62 (m, 1H), 7.57 (d, *J =* 4.1 Hz, 1H), 7.34 (m, 1H), 7.33 (m, 1H), 7.17 (t, *J=* 73.4 Hz, 1H), 3.46 (m, 2H), 3.20 (s, 3H), 2.99 (m, 1H), 2.78 (m, 2H), 2.69 (m, 1H), 2.53 (m, 2H), 2.39 (m, 6H), 1.16 (d, *J =* 6.8 Hz, 6H). |
| 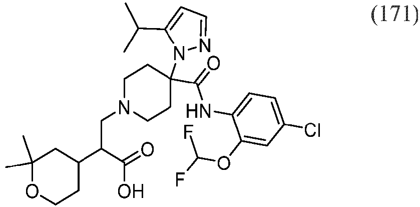 | 3-[4-[[4-chloro-2-(difluoromethoxy)phenyl] carbamoyl]-4-(5-isopropylpyrazol-1-yl)-1-piperidyl]-2-(2,2-dimethyltetrahydropyran-4-yl)propanoic acid | ¹H NMR (500 MHz, DMSO) δ 1.00 - 1.09 (m, 3H), 1.08 - 1.14 (m, 12H), 1.17 - 1.25 (m, 1H), 1.45 - 1.35 (m, 1H), 1.51 (d, J = 12.8 Hz, 1H), 1.88 - 1.98 (m, 1H), 2.38 - 2.48 (m, 3H), 2.60 - 2.53 (m, 2H), 2.82 - 3.02 (m, 1H), 3.54 - 3.45 (m, 1H), 3.67 - 3.55 (m, 1H), 6.35 (s, 1H), 7.17 (t, J = 73.2 Hz, 1H), 7.37 - 7.29 (m, 2H), 7.55 (s, 1H), 7.74 (d, J = 8.7 Hz, 1H), 8.29 (s, 1H). |
| 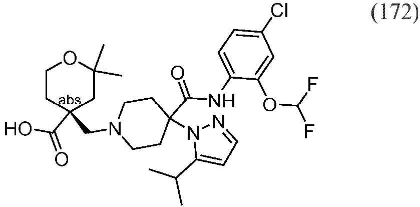 | (4*R*)-4-[[4-[[4-chloro-2-(difluoromethoxy)phenyl] carbamoyl]-4-(5-isopropylpyrazol-1-yl)-1-piperidyl]methyl]-2,2-dimethyl-tetrahydropyran-4-carboxylic acid | ¹H NMR (500 MHz, DMSO) δ 0.98 - 1.16 (m, 12H), 1.21 - 1.28 (m, 2H), 1.84 (dd, J = 13.5, 2.0 Hz, 1H), 2.05 (d, J = 13.1 Hz, 1H), 2.20 (d, J = 13.5 Hz, 1H), 2.37 - 2.29 (m, 2H), 2.48 - 2.38 (m, 4H), 2.70 (q, J = 9.1 Hz, 2H), 2.91 (p, J = 6.7 Hz, 1H), 3.38 (q, J = 7.0 Hz, 1H), 3.48 - 3.59 (m, 1H), 3.69 (td, J = 12.1, 2.2 Hz, 1H), 6.32 (d, J = 1.9 Hz, 1H), 7.07 (d, J = 73.2 Hz, 2H), 7.28 - 7.35 (m, 2H), 7.52 (d, J = 1.9 Hz, 1H), 7.76 - 7.82 (m, 1H), 8.11 (s, 1H), 12.42 (s, 1H). |
| 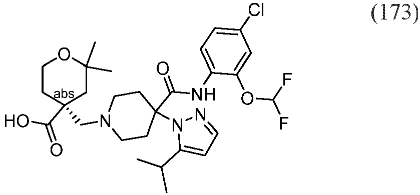 | (4*S*)-4-[[4-[[4-chloro-2-(difluoromethoxy)phenyl] carbamoyl]-4-(5-isopropylpyrazol-1-yl)-1-piperidyl]methyl]-2,2-dimethyl-tetrahydropyran-4-carboxylic acid | ¹H NMR (500 MHz, DMSO) δ 0.98 - 1.16 (m, 12H), 1.21 - 1.28 (m, 2H), 1.84 (dd, J = 13.5, 2.0 Hz, 1H), 2.05 (d, J = 13.1 Hz, 1H), 2.20 (d, J = 13.5 Hz, 1H), 2.37 - 2.27 (m, 2H), 2.48 - 2.38 (m, 4H), 2.74 - 2.64 (m, 2H)), 2.91 (p, J = 6.7 Hz, 1H), 3.38 (q, J = 7.0 Hz, 1H), 3.48 - 3.59 (m, 1H), 3.69 (td, J = 12.1, 2.2 Hz, 1H), 6.32 (d, J = 1.9 Hz, 1H), 7.07 (d, J = 73.2 Hz, 2H), 7.28 - 7.35 (m, 2H), 7.52 (d, J = 1.9 Hz, 1H), 7.76 - 7.82 (m, 1H), 8.11 (s, 1H), 12.42 (s, 1H). |
| 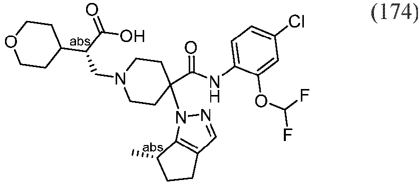 | (2*S*)-3-[4-[[4-chloro-2-(difluoromethoxy)phenyl] carbamoyl]-4-[(6*S*)-6-methyl-5,6-dihydro-4*H-*cyclopenta[c]pyrazol-1-yl]-1-piperidyl]-2-tetrahydropyran-4-yl-propanoic acid | ¹H NMR (500 MHz, DMSO) δ 0.96 (d, J = 6.8 Hz, 3H), 1.14 - 1.34 (m, 3H), 1.41 (d, J = 12.9 Hz, 1H), 1.55 (d, J = 13.1 Hz, 1H), 1.68 (d, J = 9.6 Hz, 1H), 1.89 (s, 1H), 1.99 - 2.19 (m, 3H), 2.25 - 2.39 (m, 3H), 2.40 - 2.49 (m, 3H), 2.55 - 2.80 (m, 4H), 2.99 (d, J = 11.2 Hz, 1H), 3.10 (t, J = 7.3 Hz, 1H), 3.20 - 3.26 (m, 2H), 3.76 - 3.85 (m, 2H), 7.05 - 7.36 (m, 3H), 7.40 (s, 1H), 8.14 - 8.05 (m, 2H). |
| 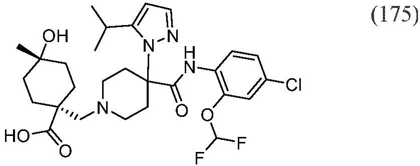 | (1*r*,4*r*)-1-[[4-[[4-chloro-2-(difluoromethoxy)phenyl] carbamoyl]-4-(5-isopropylpyrazol-1-yl)-1-piperidyl]methyl]-4-hydroxy-4-methyl-cyclohexanecarboxylic acid | ¹H NMR (500 MHz, DMSO-d6) δ 7.81 (d, *J =* 9.4 Hz, 1H), 7.52 (d, *J =* 1.8 Hz, 1H), 7.31 (m, 2H), 7.14 (t, *J =* 73.2 Hz, 1H), 6.32 (d, J = 1.8 Hz, 1H), 2.91 (m, 1H), 2.75 (m, 2H), 2.54 (m, 2H), 2.46 (m, 4H), 2.35 (m, 2H), 1.91 (m, 2H), 1.38 (m, 4H), 1.31 (m, 2H), 1.08 (d, *J =* 6.7 Hz, 6H), 1.06 (s, 3H). |
| 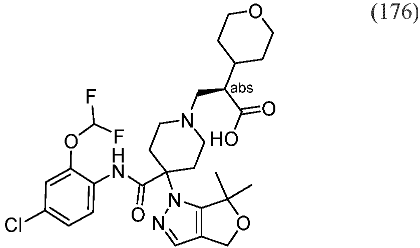 | (2*R*)-3-[4-[[4-chloro-2-(difluoromethoxy)phenyl] carbamoyl]-4-(6,6-dimethyl-4*H*-furo[3,4-c]pyrazol-1-yl)-1-piperidyl]-2-tetrahydropyran-4-yl-propanoic acid | ¹H NMR (500 MHz, DMSO) δ 1.06 - 1.48 (m, 10H), 1.56 (d, J = 13.0 Hz, 1H), 1.64 - 1.74 (m, 1H), 2.09 (s, 1H), 2.20 - 2.41 (m, 5H), 2.51 - 2.56 (m, 2H), 2.70 (s, 1H), 2.92 (s, 1H), 3.23 (t, J = 11.5 Hz, 2H), 3.81 (d, J = 11.3 Hz, 2H), 4.68 (s, 2H), 7.05 - 7.33 (m, 2H), 7.36 (d, J = 2.3 Hz, 1H), 7.48 (s, 1H), 7.82 (s, 1H), 8.00 (t, J = 9.5 Hz, 1H), 12.32 (s, 1H). |
| 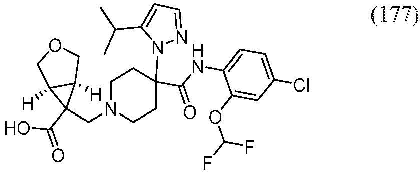 | (1*S*,5*R*)-6-[[4-[[4-chloro-2-(difluoromethoxy)phenyl] carbamoyl]-4-(5-isopropylpyrazol-1-yl)-1-piperidyl]methyl]-3-oxabicyclo[3.1.0]hexane-6-carboxylic acid | ¹H NMR (500 MHz, MeOD) δ 1.18 (d, J = 6.7 Hz, 6H), 1.75 - 1.83 (m, 2H), 2.85 - 2.71 (m, 4H), 2.91 - 3.09 (m, 5H), 3.61 - 3.50 (m, 2H), 3.73 - 3.66 (m, 2H), 4.13 (d, J = 8.4 Hz, 2H), 6.37 (d, J = 2.0 Hz, 1H), 6.80 (t, J = 73.0 Hz, 2H), 7.20 - 7.27 (m, 2H), 7.59 (d, J = 1.9 Hz, 1H), 7.97 (d, J = 8.5 Hz, 1H). |
| 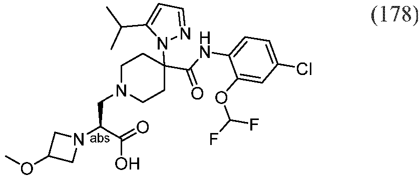 | (2*S*)-3-[4-[[4-chloro-2-(difluoromethoxy)phenyl] carbamoyl]-4-(5-isopropylpyrazol-1-yl)-1-piperidyl]-2-(3-methoxyazetidin-1-yl)propanoic acid | ¹H NMR (500 MHz, DMSO) δ 1.03 - 1.15 (m, 6H), 2.33 - 2.48 (m, 7H), 2.52 - 2.55 (m, 1H), 2.69 - 2.76 (m, 1H), 2.77 - 2.84 (m, 1H), 2.89 - 2.97 (m, 1H), 3.17 (s, 3H), 3.30 - 3.36 (m, 1H), 3.37 - 3.44 (m, 1H), 3.44 - 3.52 (m, 1H), 3.78 - 3.86 (m, 1H), 3.88 - 3.97 (m, 1H), 4.01 - 4.09 (m, 1H), 6.29 - 6.35 (m, 1H), 6.97 - 7.35 (m, 3H), 7.49 - 7.54 (m, 1H), 7.78 (d, J = 9.3 Hz, 1H), 8.18 (s, 1H). |
| 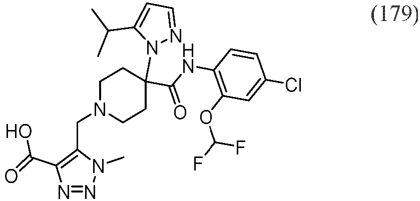 | 5-[[4-[[4-chloro-2-(difluoromethoxy)phenyl] carbamoyl]-4-(5-isopropylpyrazol-1-yl)-1-piperidyl]methyl]-1-methyl-triazole-4-carboxylic acid | ¹H NMR (400 MHz, DMSO) δ 7.90 (d, *J = 9.4* Hz, 1H), 7.53 (d, *J =* 1.8 Hz, 1H), 7.33 - 7.26 (m, 2H), 7.14 (t, *J =* 73.2 Hz, 1H), 6.30 (d, *J =* 1.9 Hz, 1H), 4.03 (s, 3H), 3.94 (s, 2H), 2.76 (m, 3H), 2.55 (m, *J =* 10.4 Hz, 4H), 2.42 (m, 2H), 1.10 (d, *J =* 6.7 Hz, 6H). |
| 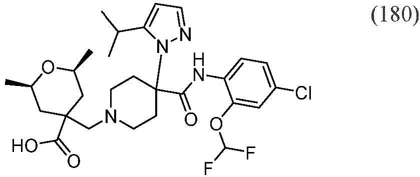 | (2*R*,6*S*)-4-[[4-[[4-chloro-2-(difluoromethoxy)phenyl] carbamoyl]-4-(5-isopropylpyrazol-1-yl)-1-piperidyl]methyl]-2,6-dimethyl-tetrahydropyran-4-carboxylic acid | ¹H NMR (500 MHz, MeOD) δ 1.04 (t, J = 12.0 Hz, 2H), 1.13 - 1.20 (m, 12H), 2.06 (d, J = 12.9 Hz, 2H), 2.61 - 2.81 (m, 4H), 2.83 (s, 2H), 2.96 (p, J = 6.7 Hz, 1H), 3.09 (t, J = 11.0 Hz, 2H), 3.30 - 3.27 (m, 2H), 3.74 (dt, J = 12.3, 6.1 Hz, 2H), 6.38 (d, J = 1.9 Hz, 1H), 6.79 (t, J = 72.9 Hz, 1H), 7.21 - 7.28 (m, 2H), 7.60 (d, J = 1.9 Hz, 1H), 7.99 (d, J = 8.5 Hz, 1H). |
| 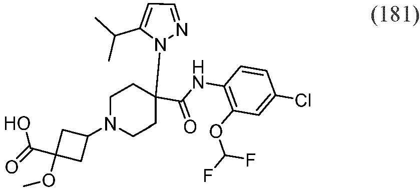 | 3-[4-[[4-chloro-2-(difluoromethoxy)phenyl] carbamoyl]-4-(5-isopropylpyrazol-1-yl)-1-piperidyl]-1-methoxy-cyclobutanecarboxylic acid | ¹H NMR (400 MHz, DMSO-d6) δ 7.90 (dd, *J =* 9.4, 5.8 Hz, 1H), 7.84 (d, *J* = 14.4 Hz, 1H), 7.51 (dd, *J* = 3.3, 1.9 Hz, 1H), 7.32 - 7.22 (m, 2H), 7.17 (t, *J* = 73.4 Hz, 1H), 6.29 (d, *J =* 1.9 Hz, 1H), 3.14 (s, 3H), 2.98 (p, *J =* 6.6 Hz, 1H), 2.87 (p, *J =* 7.0 Hz, 1H), 2.73 (dd, *J =* 11.7, 5.4 Hz, 2H), 2.65 (dd, *J* = 11.2, 5.0 Hz, 1H), 2.58 (dd, *J =* 14.6, 6.8 Hz, 3H), 2.47 - 2.38 (m, 2H), 2.38 - 2.18 (m, 3H), 1.98 - 1.89 (m, 1H), 1.11 (dd, *J =* 6.7, 1.3 Hz, 6H). |
| 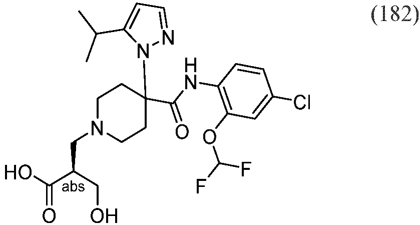 | (2*S*)-2-[[4-[[4-chloro-2-(difluoromethoxy)phenyl] carbamoyl]-4-(5-isopropylpyrazol-1-yl)-1-piperidyl]methyl]-3-hydroxy-propanoic acid | ¹H NMR (400 MHz, DMSO-d6) δ 7.90 (dd, *J =* 9.4, 5.8 Hz, 1H), 7.84 (d, *J* = 14.4 Hz, 1H), 7.51 (dd, *J* = 3.3, 1.9 Hz, 1H), 7.32 - 7.22 (m, 2H), 7.17 (t, *J =* 73.4 Hz, 1H), 6.29 (d, *J =* 1.9 Hz, 1H), 3.58 (qd, *J =* 10.5, 5.7 Hz, 3H), 2.97 (h, *J* = 6.7 Hz, 1H), 2.89 - 2.72 (m, 2H), 2.66 - 2.52 (m, 5H), 2.42 (dtd, *J* = 11.6, 6.2, 3.7 Hz, 4H), 1.22 - 1.08 (m, 6H). |
| 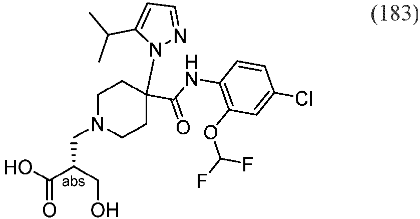 | (2*R*)-2-[[4-[[4-chloro-2-(difluoromethoxy)phenyl] carbamoyl]-4-(5-isopropylpyrazol-1-yl)-1-piperidyl]methyl]-3-hydroxy-propanoic acid | ¹H NMR (400 MHz, DMSO-d6) δ 7.90 (dd, *J =* 9.4, 5.8 Hz, 1H), 7.84 (d, *J* = 14.4 Hz, 1H), 7.51 (dd, *J* = 3.3, 1.9 Hz, 1H), 7.32 - 7.22 (m, 2H), 7.17 (t, *J =* 73.4 Hz, 1H), 6.29 (d, *J =* 1.9 Hz, 1H), 3.58 (qd, *J =* 10.5, 5.7 Hz, 3H), 2.97 (h, *J* = 6.7 Hz, 1H), 2.89 - 2.72 (m, 2H), 2.66 - 2.52 (m, 5H), 2.42 (dtd, *J* = 11.6, 6.2, 3.7 Hz, 4H), 1.22 - 1.08 (m, 6H). |
| 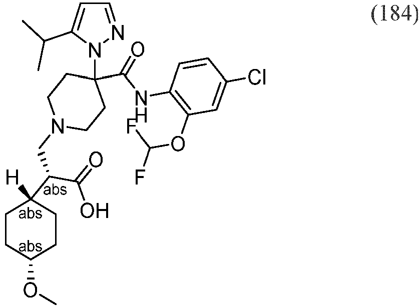 | (2*R*)-3-[4-[[4-chloro-2-(difluoromethoxy)phenyl] carbamoyl]-4-(5-isopropylpyrazol-1-yl)-1-piperidyl]-2-((1*s*,4*S*)-4-methoxycyclohexyl)prop anoic acid | ¹H NMR (400 MHz, DMSO-d6) δ 7.90 (dd, *J =* 9.4, 5.8 Hz, 1H), 7.84 (d, *J* = 14.4 Hz, 1H), 7.51 (dd, *J* = 3.3, 1.9 Hz, 1H), 7.32 - 7.22 (m, 2H), 7.17 (t, *J =* 73.4 Hz, 1H), 6.29 (d, *J =* 1.9 Hz, 1H), 3.32 (dt, *J =* 4.5, 2.3 Hz, 1H), 3.18 (s, 3H), 2.98 (p, *J =* 6.7 Hz, 2H), 2.83 (dd, *J* = 11.7, 5.5 Hz, 1H), 2.65 (dd, *J =* 9.2, 4.9 Hz, 1H), 2.50 (s, 3H), 2.44 - 2.27 (m, 5H), 1.77 (d, *J =* 11.8 Hz, 2H), 1.55 (s, 1H), 1.47 - 1.28 (m, 6H), 1.11 (dd, *J =* 6.7, 1.7 Hz, 6H). |
| 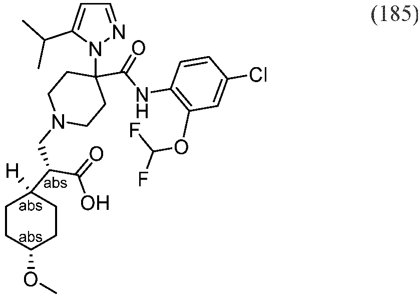 | (2*R*)-3-[4-[[4-chloro-2-(difluoromethoxy)phenyl] carbamoyl]-4-(5-isopropylpyrazol-1-yl)-1-piperidyl]-2-((1*r*,4*R*)-4-methoxycyclohexyl)prop anoic acid | ¹H NMR (400 MHz, DMSO-d6) δ 7.90 (dd, *J =* 9.4, 5.8 Hz, 1H), 7.84 (d, *J* = 14.4 Hz, 1H), 7.51 (dd, *J* = 3.3, 1.9 Hz, 1H), 7.32 - 7.22 (m, 2H), 7.17 (t, *J* = 73.4 Hz, 1H), 6.29 (d, *J =* 1.9 Hz, 1H), 3.32 (dt, *J =* 4.5, 2.3 Hz, 1H), 3.18 (s, 3H), 2.98 (p, *J =* 6.7 Hz, 2H), 2.83 (dd, *J* = 11.7, 5.5 Hz, 1H), 2.65 (dd, *J =* 9.2, 4.9 Hz, 1H), 2.50 (s, 3H), 2.44 - 2.27 (m, 5H), 1.77 (d, *J =* 11.8 Hz, 2H), 1.55 (s, 1H), 1.47 - 1.28 (m, 6H), 1.11 (dd, *J =* 6.7, 1.7 Hz, 6H). |
| 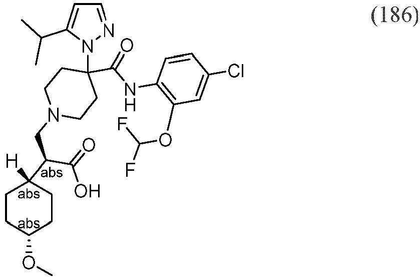 | (2*S*)-3-[4-[[4-chloro-2-(difluoromethoxy)phenyl] carbamoyl]-4-(5-isopropylpyrazol-1-yl)-1-piperidyl]-2-((1*s*,4*R*)-4-methoxycyclohexyl)prop anoic acid | ¹H NMR (400 MHz, DMSO-d6) δ 7.90 (dd, *J =* 9.4, 5.8 Hz, 1H), 7.84 (d, *J* = 14.4 Hz, 1H), 7.51 (dd, *J* = 3.3, 1.9 Hz, 1H), 7.32 - 7.22 (m, 2H), 7.17 (t, *J =* 73.4 Hz, 1H), 6.29 (d, *J =* 1.9 Hz, 1H), 3.32 (dt, *J =* 4.5, 2.3 Hz, 1H), 3.18 (s, 3H), 2.98 (p, *J =* 6.7 Hz, 2H), 2.83 (dd, *J* = 11.7, 5.5 Hz, 1H), 2.65 (dd, *J =* 9.2, 4.9 Hz, 1H), 2.50 (s, 3H), 2.44 - 2.27 (m, 5H), 1.77 (d, *J =* 11.8 Hz, 2H), 1.55 (s, 1H), 1.47 - 1.28 (m, 6H), 1.11 (dd, *J =* 6.7, 1.7 Hz, 6H). |
| 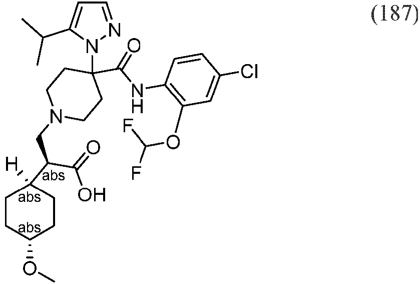 | (2*S*)-3-[4-[[4-chloro-2-(difluoromethoxy)phenyl] carbamoyl]-4-(5-isopropylpyrazol-1-yl)-1-piperidyl]-2-((1*r*,4*S*)-4-methoxycyclohexyl)prop anoic acid | ¹H NMR (400 MHz, DMSO-d6) δ 7.90 (dd, *J =* 9.4, 5.8 Hz, 1H), 7.84 (d, *J* = 14.4 Hz, 1H), 7.51 (dd, *J* = 3.3, 1.9 Hz, 1H), 7.32 - 7.22 (m, 2H), 7.17 (t, *J =* 73.4 Hz, 1H), 6.29 (d, *J =* 1.9 Hz, 1H), 3.32 (dt, *J =* 4.5, 2.3 Hz, 1H), 3.18 (s, 3H), 2.98 (p, *J =* 6.7 Hz, 2H), 2.83 (dd, *J* = 11.7, 5.5 Hz, 1H), 2.65 (dd, *J =* 9.2, 4.9 Hz, 1H), 2.50 (s, 3H), 2.44 - 2.27 (m, 5H), 1.77 (d, *J =* 11.8 Hz, 2H), 1.55 (s, 1H), 1.47 - 1.28 (m, 6H), 1.11 (dd, *J =* 6.7, 1.7 Hz, 6H). |
| 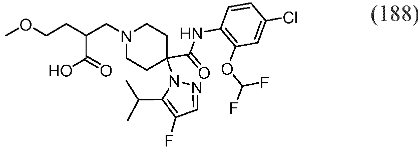 | 2-[[4-[[4-chloro-2-(difluoromethoxy)phenyl] carbamoyl]-4-(4-fluoro-5-isopropyl-pyrazol-1-yl)-1-piperidyl]methyl]-4-methoxy-butanoic acid | ¹H NMR (400 MHz, DMSO-d6) δ 8.63 (s, 1H), 7.60 (m, 1H), 7.57 (d, *J =* 4.1 Hz, 1H), 7.34 (m, 2H), 7.17 (t, *J =* 73.3 Hz, 1H), 3.32 (m, 4H), 3.20 (s, 3H), 2.99 (m, 1H), 2.79 (m, 1H), 2.65 (m, 1H), 2.55 (m, 2H), 2.48 (m, 1H), 2.35 (m, 3H), 2.30 (m, 1H), 1.72 (m, 1H), 1.62 (m, 1H), 1.10 (d, *J =* 6.7 Hz, 6H). |
| 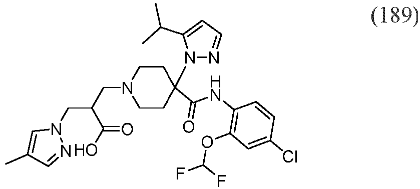 | 2-[[4-[[4-chloro-2-(difluoromethoxy)phenyl] carbamoyl]-4-(5-isopropylpyrazol-1-yl)-1-piperidyl]methyl]-3-(4-methylpyrazol-1-yl)propanoic acid | ¹H NMR (400 MHz, DMSO-d6) δ 7.90 (dd, *J =* 9.4, 5.8 Hz, 1H), 7.84 (d, *J* = 14.4 Hz, 1H), 7.51 (dd, *J* = 3.3, 1.9 Hz, 1H), 7.35 (t, *J =* 0.9 Hz, 1H),7.32 - 7.22 (m, 2H), 7.17 (s, 1H), 7.05 (t, *J =* 73.4 Hz, 1H), 6.29 (d, *J =* 1.9 Hz, 1H), 4.27 (dd, *J =* 13.7, 7.1 Hz, 1H), 4.18 (dd, *J =* 13.7, 5.8 Hz, 1H), 3.07 - 3.00 (m, 1H), 3.00 - 2.91 (m, 1H), 2.84 - 2.76 (m, 1H), 2.72 (d, *J =* 6.0 Hz, 1H), 2.59 - 2.51 (m, 3H), 2.46 - 2.35 (m, 5H), 1.98 (d, *J =* 0.7 Hz, 3H), 1.11 (d, *J = 6.7* Hz, 6H). |
| 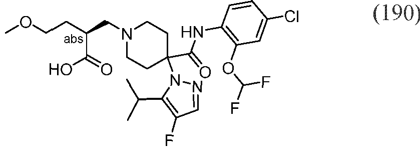 | (2*S*)-2-[[4-[[4-chloro-2-(difluoromethoxy)phenyl] carbamoyl]-4-(4-fluoro-5-isopropyl-pyrazol-1-yl)-1-piperidyl]methyl]-4-methoxy-butanoic acid | ¹H NMR (400 MHz, DMSO-d6) δ 8.14 (bs, 1H), 7.62 (m, 1H), 7.57 (d, *J =* 4.1 Hz, 1H), 7.34 (m, 1H), 7.33 (m, 1H), 7.17 (t, *J =* 73.4 Hz, 1H), 3.35 (tt, *J =* 6.6, 3.2 Hz, 3H), 3.21 (s, 3H), 3.04 (p, *J =* 6.9 Hz, 2H), 2.80 (t, *J =* 5.8 Hz, 1H), 2.69 (dt, *J =* 10.6, 4.7 Hz, 1H), 2.60 - 2.49 (m, 3H), 2.43 - 2.30 (m, 4H), 1.83 - 1.59 (m, 2H), 1.17 (d, *J =* 6.9 Hz, 6H). |
| 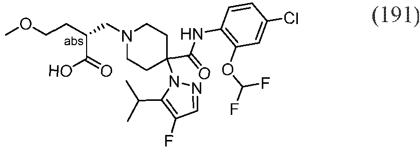 | (2*R*)-2-[[4-[[4-chloro-2-(difluoromethoxy)phenyl] carbamoyl]-4-(4-fluoro-5-isopropyl-pyrazol-1-yl)-1-piperidyl]methyl]-4-methoxy-butanoic acid | ¹H NMR (400 MHz, DMSO-d6) δ 8.14 (bs, 1H), 7.62 (m, 1H), 7.57 (d, *J =* 4.1 Hz, 1H), 7.34 (m, 1H), 7.33 (m, 1H), 7.17 (t, *J =* 73.4 Hz, 1H), 3.35 (tt, *J =* 6.6, 3.2 Hz, 3H), 3.21 (s, 3H), 3.04 (p, *J =* 6.9 Hz, 2H), 2.80 (t, *J =* 5.8 Hz, 1H), 2.69 (dt, *J =* 10.6, 4.7 Hz, 1H), 2.60 - 2.49 (m, 3H), 2.43 - 2.30 (m, 4H), 1.83 - 1.59 (m, 2H), 1.17 (d, *J =* 6.9 Hz, 6H). |
| 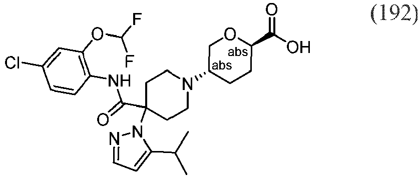 | (2*R*,5*S*)-5-[4-[[4-chloro-2-(difluoromethoxy)phenyl] carbamoyl]-4-(5-isopropylpyrazol-1-yl)-1-piperidyl]tetrahydropyran -2-carboxylic acid | ¹H NMR (600 MHz, DMSO) δ 8.13 (s, 1H), 7.84 - 7.76 (m, 1H), 7.52 (d, *J =* 1.9 Hz, 1H), 7.34 - 7.29 (m, 2H), 7.15 (t, *J =* 73.2 Hz, 1H), 6.31 (d, *J =* 1.9 Hz, 1H), 3.99 - 3.91 (m, 1H), 3.71 - 3.65 (m, 1H), 3.20 (t, *J =* 10.6 Hz, 1H), 2.98 - 2.88 (m, 1H), 2.81 - 2.74 (m, 2H), 2.61 - 2.43 (m, 4H), 2.40 - 2.25 (m, 3H), 1.98 - 1.88 (m, 2H), 1.45 - 1.37 (m, 2H), 1.09 (d, *J =* 6.7 Hz, 6H). |
| 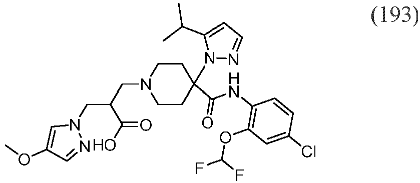 | 2-[[4-[[4-chloro-2-(difluoromethoxy)phenyl] carbamoyl]-4-(5-isopropylpyrazol-1-yl)-1-piperidyl]methyl]-3-(4-methoxypyrazol-1-yl)propanoic acid | ¹H NMR (400 MHz, DMSO-d6) δ 7.90 (dd, *J =* 9.4, 5.8 Hz, 1H), 7.84 (d, *J* = 14.4 Hz, 1H), 7.51 (dd, *J* = 3.3, 1.9 Hz, 1H), 7.35 (d, *J =* 0.9 Hz, 1H),7.32 - 7.22 (m, 2H), 7.17 (d, J = 0.9 Hz, 1H), 7.05 (t, *J* = 73.4 Hz, 1H), 6.29 (d, *J =* 1.9 Hz, 1H), 4.27 (dd, *J =* 13.7, 7.1 Hz, 1H), 4.18 (dd, *J =* 13.7, 5.8 Hz, 1H), 3.07 - 3.00 (m, 1H), 3.00 - 2.91 (m, 1H), 2.84 - 2.76 (m, 1H), 2.72 (d, *J =* 6.0 Hz, 1H), 2.59 - 2.51 (m, 3H), 2.46 - 2.35 (m, 5H), 1.98 (d, *J =* 0.7 Hz, 3H), 1.11 (d, *J =* 6.7 Hz, 6H). |
| 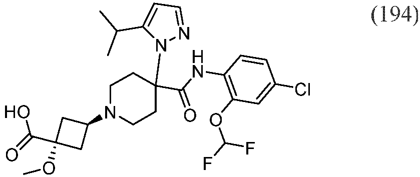 | (1*r*,3*r*)-3-[4-[[4-chloro-2-(difluoromethoxy)phenyl] carbamoyl]-4-(5-isopropylpyrazol-1-yl)-1-piperidyl]-1-methoxy-cyclobutanecarboxylic acid | ¹H NMR (600 MHz, DMSO) δ 8.31 (s, 1H), 7.76 (d, *J =* 8.9 Hz, 1H), 7.53 (d, *J =* 1.9 Hz, 1H), 7.32 (d, *J* = 7.7 Hz, 2H), 7.15 (t, *J =* 73.2 Hz, 1H), 6.33 (d, *J =* 1.9 Hz, 1H), 3.17 (s, 1H), 2.96 - 2.91 (m, 1H), 2.91 - 2.86 (m, 1H), 2.82 (s, 2H), 2.54 (s, 2H), 2.48 - 2.35 (m, 6H), 2.33 - 2.26 (m, 2H), 2.27 - 2.18 (m, 2H), 1.10 (d, *J =* 6.7 Hz, 6H). |
| 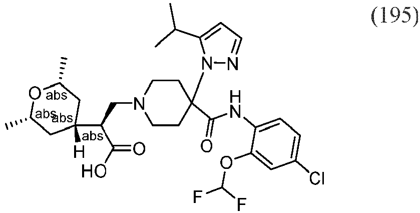 | (2*S*)-3-[4-[[4-chloro-2-(difluoromethoxy)phenyl] carbamoyl]-4-(5-isopropylpyrazol-1-yl)-1-piperidyl]-2-[(2*R*,4*r*,6*S*)-2,6-dimethyltetrahydropyran-4-yl]propanoic acid | ¹H NMR (400 MHz, DMSO) δ 0.73 - 0.83 (m, 1H), 0.88 (q, J = 12.3 Hz, 1H), 1.05 (d, J = 6.1 Hz, 6H), 1.07 - 1.13 (m, 6H), 1.45 (d, J = 12.7 Hz, 1H), 1.56 - 1.66 (m, 1H), 1.76 (s, 1H), 2.18 - 2.28 (m, 1H), 2.27 - 2.40 (m, 4H), 2.41 - 2.53 (m, 2H), 2.56 - 2.69 (m, 1H), 2.80 - 2.88 (m, 1H), 2.92 (p, J = 6.6 Hz, 1H), 3.30 - 3.44 (m, 4H), 6.32 (d, J = 1.9 Hz, 1H), 6.94 - 7.36 (m, 1H), 7.32 (dd, J = 6.9, 3.2 Hz, 2H), 7.52 (d, J = 1.9 Hz, 1H), 7.79 (d, J = 9.4 Hz, 1H), 8.16 (s, 1H). |
| 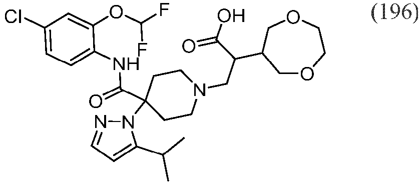 | 3-[4-[[4-chloro-2-(difluoromethoxy)phenyl] carbamoyl]-4-(5-isopropylpyrazol-1-yl)-1-piperidyl]-2-(1,4-dioxepan-6-yl)propanoic acid | ¹H NMR (400 MHz, DMSO) δ 1.10 (dd, J = 6.7, 2.9 Hz, 6H), 2.21 - 2.40 (m, 3H), 2.57 (d, J = 6.0 Hz, 2H), 2.75 - 2.62 (m, 1H), 2.99 - 2.83 (m, 2H), 3.49 - 3.67 (m, 7H), 3.69 - 3.83 (m, 2H), 6.33 (d, J = 1.9 Hz, 1H), 7.15 (t, J = 72.9 Hz, 1H), 7.27 - 7.36 (m, 2H), 7.52 (d, J = 1.8 Hz, 1H), 7.78 (d, J = 9.3 Hz, 1H), 8.20 (s, 1H). |
| 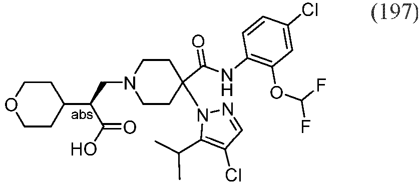 | (2*S*)-3-[4-[[4-chloro-2-(difluoromethoxy)phenyl] carbamoyl]-4-(4-chloro-5-isopropyl-pyrazol-1-yl)-1-piperidyl]-2-tetrahydropyran-4-yl-propanoic acid | ¹H NMR (499 MHz, DMSO) δ 8.81 (s, 1H), 7.61 (s, 1H), 7.58 - 7.52 (m, 1H), 7.36 - 7.30 (m, 1H), 7.32 (s, 1H), 7.17 ( s, 1H), 3.85 - 3.78 (m, 2H), 3.28 - 3.19 (m, 2H), 3.06 (h, J = 7.0 Hz, 1H), 2.86 (s, 1H), 2.66 - 2.56 (m, 2H), 2.43 - 2.34 (m, 8H), 1.71 (dtt, J = 11.2, 7.2, 3.6 Hz, 1H), 1.56 (ddt, J = 11.1, 4.2, 2.2 Hz, 1H), 1.42 (ddd, J = 13.1, 4.1, 2.1 Hz, 1H), 1.38 - 1.17 (m, 9H). |
| 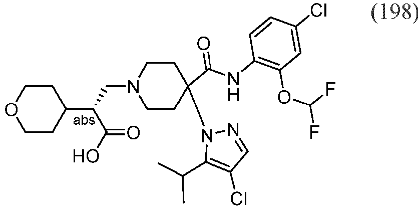 | (2R)-3-[4-[[4-chloro-2-(difluoromethoxy)phenyl] carbamoyl]-4-(4-chloro-5-isopropyl-pyrazol-1-yl)-1-piperidyl]-2-tetrahydropyran-4-yl-propanoic acid | ¹H NMR (499 MHz, DMSO) δ 8.81 (s, 1H), 7.61 (s, 1H), 7.58 - 7.52 (m, 1H), 7.36 - 7.30 (m, 1H), 7.32 (s, 1H), 7.17(s, 1H), 3.85 - 3.78 (m, 2H), 3.24 (td, J = 11.9, 2.0 Hz, 2H), 3.06 (h, J = 7.0 Hz, 1H), 2.86 (s, 1H), 2.66 - 2.57 (m, 2H), 2.42 - 2.34 (m, 8H), 1.72 (tdt, J = 11.2, 7.1, 3.5 Hz, 1H), 1.56 (ddd, J = 13.2, 4.0, 2.2 Hz, 1H), 1.42 (ddd, J = 13.0, 4.0, 2.1 Hz, 1H), 1.38 - 1.17 (m, 9H). |
| 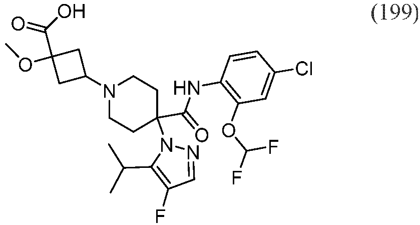 | 3-[4-[[4-chloro-2-(difluoromethoxy)phenyl] carbamoyl]-4-(4-fluoro-5-isopropyl-pyrazol-1-yl)-1-piperidyl]-1-methoxy-cyclobutanecarboxylic acid | ¹H NMR (400 MHz, DMSO-d6) δ 8.72 (s, 1H), 7.59 (m, 2H), 7.34 (m, 2H), 7.18 (t, *J =* 73.2 Hz, 1H), 3.15 (s, 3H), 2.99 (m, 1H), 2.85 (m, 1H), 2.73 (m, 2H), 2.40 (m, 6H), 2.23 (m, 4H), 1.17 (d, *J =* 6.7 Hz, 6H). |
| 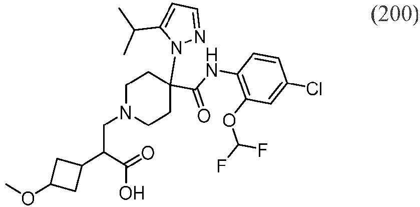 | 3-[4-[[4-chloro-2-(difluoromethoxy)phenyl] carbamoyl]-4-(5-isopropylpyrazol-1-yl)-1-piperidyl]-2-(3-methoxycyclobutyl)propa noic acid | ¹H NMR (400 MHz, DMSO) δ 7.90 (d, *J = 9.4* Hz, 1H), 7.81 (s, 1H), 7.51 (d, *J =* 1.8 Hz, 1H), 7.31 - 7.25 (m, 2H), 7.05 (t, *J =* 73.2 Hz, 1H), 6.28 (d, *J =* 1.9 Hz, 1H), 3.71 - 3.56 (m, 1H), 3.11 (s, 1H), 3.10 (s, 3H), 2.98 (p, *J =* 6.7 Hz, 1H), 2.81 (d, *J =* 11.0 Hz, 1H), 2.68 (dd, *J =* 10.5, 5.7 Hz, 1H), 2.57 - 2.50 (m, 4H), 2.47 - 2.21 (m, 5H), 1.99 (q, *J =* 6.6 Hz, 1H), 1.87 (q, *J =* 8.8 Hz, 1H), 1.67 - 1.59 (m, 1H), 1.57 - 1.49 (m, 1H), 1.11 (dd, *J =* 6.7, 1.7 Hz, 6H). |
| 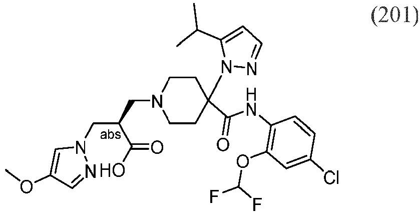 | (2*R*)-2-[[4-[[4-chloro-2-(difluoromethoxy)phenyl] carbamoyl]-4-(5-isopropylpyrazol-1-yl)-1-piperidyl]methyl]-3-(4-methoxypyrazol-1-yl)propanoic acid | ¹H NMR (400 MHz, DMSO-d6) δ 7.90 (dd, *J =* 9.4, 5.8 Hz, 1H), 7.84 (d, *J* = 14.4 Hz, 1H), 7.51 (dd, *J* = 3.3, 1.9 Hz, 1H), 7.35 (d, *J =* 0.9 Hz, 1H),7.32 - 7.22 (m, 2H), 7.17 (d, J = 0.9 Hz, 1H), 7.05 (t, *J* = 73.4 Hz, 1H), 6.29 (d, *J =* 1.9 Hz, 1H), 4.27 (dd, *J =* 13.7, 7.1 Hz, 1H), 4.18 (dd, *J =* 13.7, 5.8 Hz, 1H), 3.07 - 3.00 (m, 1H), 3.00 - 2.91 (m, 1H), 2.84 - 2.76 (m, 1H), 2.72 (d, *J =* 6.0 Hz, 1H), 2.59 - 2.51 (m, 3H), 2.46 - 2.35 (m, 5H), 1.98 (d, *J =* 0.7 Hz, 3H), 1.11 (d, *J =* 6.7 Hz, 6H). |
| 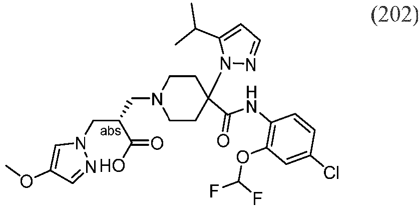 | (2*S*)-2-[[4-[[4-chloro-2-(difluoromethoxy)phenyl] carbamoyl]-4-(5-isopropylpyrazol-1-yl)-1-piperidyl]methyl]-3-(4-methoxypyrazol-1-yl)propanoic acid | ¹H NMR (400 MHz, DMSO-d6) δ 7.90 (dd, *J =* 9.4, 5.8 Hz, 1H), 7.84 (d, *J* = 14.4 Hz, 1H), 7.51 (dd, *J* = 3.3, 1.9 Hz, 1H), 7.35 (d, *J =* 0.9 Hz, 1H),7.32 - 7.22 (m, 2H), 7.17 (d, J = 0.9 Hz, 1H), 7.05 (t, *J* = 73.4 Hz, 1H), 6.29 (d, *J = 1.9* Hz, 1H), 4.27 (dd, *J =* 13.7, 7.1 Hz, 1H), 4.18 (dd, *J =* 13.7, 5.8 Hz, 1H), 3.07 - 3.00 (m, 1H), 3.00 - 2.91 (m, 1H), 2.84 - 2.76 (m, 1H), 2.72 (d, *J =* 6.0 Hz, 1H), 2.59 - 2.51 (m, 3H), 2.46 - 2.35 (m, 5H), 1.98 (d, *J =* 0.7 Hz, 3H), 1.11 (d, *J =* 6.7 Hz, 6H). |
| 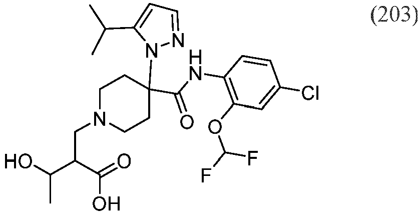 | 2-[[4-[[4-chloro-2-(difluoromethoxy)phenyl] carbamoyl]-4-(5-isopropylpyrazol-1-yl)-1-piperidyl]methyl]-3-hydroxy-butanoic acid | ¹H NMR (400 MHz, DMSO-d6) δ 8.14 (s, 1H), 7.80 (m, 1H), 7.52 (d, *J =* 1.8 Hz, 1H), 7.31 (m, 2H), 7.15 (t, *J =* 73.2 Hz, 1H), 6.32 (m, 1H), 3.79 (m, 1H), 2.92 (m, 1H), 2.74 (m, 1H), 2.69 (m, 1H), 2.54 (m, 1H), 2.48 (m, 4H), 2.36 (m, 4H), 1.10 (d, *J =* 6.7 Hz, 6H), 1.05 (m, 3H). |
| 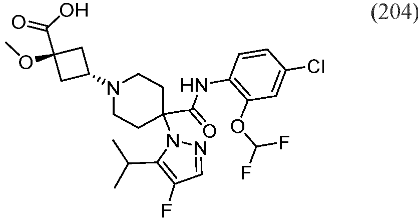 | (1*r*,3*r*)-3-[4-[[4-chloro*-2-*(difluoromethoxy)phenyl] carbamoyl]-4-(4-fluoro-5-isopropyl-pyrazol-1-yl)-1-piperidyl]-1-methoxy-cyclobutanecarboxylic acid | ¹H NMR (500 MHz, DMSO-d6) δ 8.73 (s, 1H), 7.59 (m, 2H), 7.33 (m, 2H), 7.17 (t, *J =* 73.2 Hz, 1H), 3.15 (s, 3H), 2.99 (m, 1H), 2.85 (m, 1H), 2.73 (m, 2H), 2.40 (m, 6H), 2.23 (m, 4H), 1.17 (d, *J =* 6.8 Hz, 6H). |
| 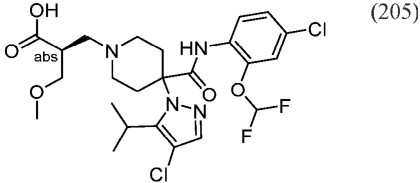 | (2*S*)-2-[[4-[[4-chloro-2-(difluoromethoxy)phenyl] carbamoyl]-4-(4-chloro-5-isopropyl-pyrazol-1-yl)-1-piperidyl]methyl]-3-methoxy-propanoic acid | ¹H NMR (499 MHz, DMSO) δ 8.83 (s, 1H), 7.61 (s, 1H), 7.58 - 7.52 (m, 1H), 7.36 - 7.30 (m, 2H), 7.17 (s, 1H), 3.45 (qd, J = 9.3, 6.1 Hz, 2H), 3.21 (s, 3H), 3.07 (p, J = 6.9 Hz, 2H), 2.82 - 2.65 (m, 4H), 2.56-2.51 (m, 1H), 2.38 (q, J = 5.1 Hz, 6H), 1.28 - 1.17 (m, 6H). |
| 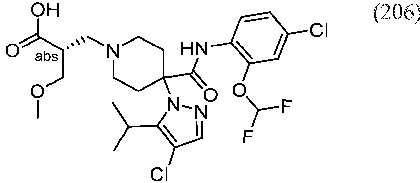 | (2*R*)-2-[[4-[[4-chloro-2-(difluoromethoxy)phenyl] carbamoyl]-4-(4-chloro-5-isopropyl-pyrazol-1-yl)-1-piperidyl]methyl]-3-methoxy-propanoic acid | ¹H NMR (600 MHz, DMSO) δ 8.84 (s, 1H), 7.61 (s, 1H), 7.54 (d, J = 8.4 Hz, 1H), 7.36 - 7.31 (m, 2H), 7.17 (s, 1H), 3.46 (qd, J = 9.3, 6.1 Hz, 2H), 3.21 (s, 3H), 3.07 (p, J = 7.0 Hz, 2H), 2.83 - 2.65 (m, 4H), 2.56-2.51 (m, 1H), 2.41 - 2.36 (m, 6H), 1.23 (dd, J = 6.9, 1.2 Hz, 6H). |
| 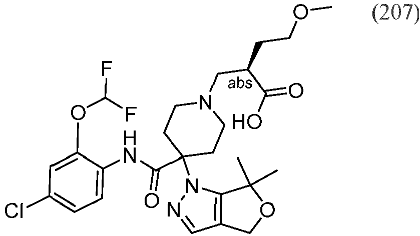 | (2R)-2-[[4-[[4-chloro-2-(difluoromethoxy)phenyl] carbamoyl]-4-(6,6-dimethyl-4*H*-furo[3,4-c]pyrazol-1-yl)-1-piperidyl]methyl]-4-methoxy-butanoic acid | ¹H NMR (400 MHz, DMSO) δ 1.38 (s, 6H), 1.56 - 1.76 (m, 2H), 2.23 - 2.08 (m, 1H)), 2.42 - 2.23 (m, 4H), 2.52 - 2.64 (m, 2H), 2.83 - 2.70 (m, 1H), 2.98 - 2.85 (m, 1H), 3.35 - 3.30 (m, 4H), 4.69 (s, 2H), 7.21 (t, J = 73.0 Hz, 1H), 7.31 (dd, J = 8.8, 2.3 Hz, 1H), 7.36 (d, J = 2.4 Hz, 1H), 7.48 (s, 1H), 7.85 (s, 1H), 7.98 (d, J = 8.8 Hz, 1H). |
| 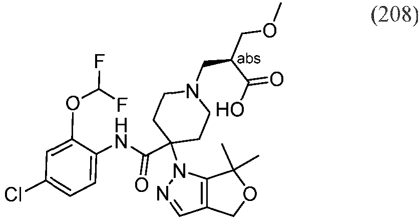 | (2*S*)-2-[[4-[[4-chloro-2-(difluoromethoxy)phenyl] carbamoyl]-4-(6,6-dimethyl-4*H*-furo[3,4-c]pyrazol-1-yl)-1-piperidyl]methyl]-3-methoxy-propanoic acid | ¹H NMR (400 MHz, DMSO) δ 1.38 (s, 6H), 2.10 - 2.41 (m, 5H), 2.79 (d, J = 8.8 Hz, 2H), 2.90 (d, J = 11.6 Hz, 1H), 3.21 (s, 3H), 3.31 - 3.54 (m, 5H), 4.69 (s, 2H), 7.21 (t, J = 73.0 Hz, 1H), 7.31 (dd, J = 8.8, 2.3 Hz, 1H), 7.36 (d, J = 2.3 Hz, 1H), 7.48 (s, 1H), 7.82 - 7.90 (m, 1H), 7.98 (d, J = 8.8 Hz, 1H), 12.65 (s, 1H). |
| 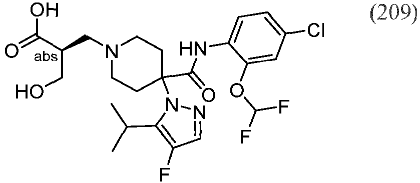 | (2*S*)-2-[[4-[[4-chloro-2-(difluoromethoxy)phenyl] carbamoyl]-4-(4-fluoro-5-isopropyl-pyrazol-1-yl)-1-piperidyl]methyl]-3-hydroxy-propanoic acid | ¹H NMR (500 MHz, DMSO) δ 1.16 (d, J = 6.8 Hz, 6H), 2.22 - 2.46 (m, 6H), 2.52 - 2.77 (m, 3H), 2.81 (d, J = 7.3 Hz, 1H), 2.94 - 3.04 (m, 1H), 3.48 - 3.59 (m, 2H), 7.00 - 7.37 (m, 3H), 7.58 (t, J = 7.4 Hz, 2H), 8.68 (s, 1H). |
| 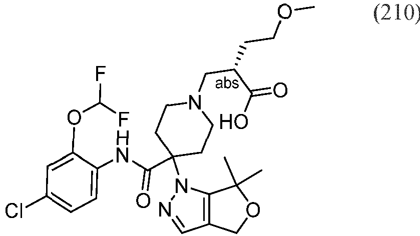 | (2*S*)-2-[[4-[[4-chloro-2-(difluoromethoxy)phenyl] carbamoyl]-4-(6,6-dimethyl-4*H*-furo[3,4-c]pyrazol-1-yl)-1-piperidyl]methyl]-4-methoxy-butanoic acid | ¹H NMR (400 MHz, DMSO) δ 1.38 (d, J = 1.8 Hz, 6H), 1.54 - 1.79 (m, 2H), 2.16 (s, 1H), 2.28 (dd, J = 11.7, 5.7 Hz, 4H), 2.52 - 2.65 (m, 2H), 2.74 (s, 1H), 2.90 (d, J = 11.1 Hz, 1H), 3.32 (dd, J = 7.7, 4.6 Hz, 4H), 4.69 (s, 2H), 7.21 (t, J = 73.0 Hz, 1H), 7.31 (dd, J = 8.8, 2.3 Hz, 1H), 7.36 (d, J = 2.4 Hz, 1H), 7.48 (s, 1H), 7.85 (s, 1H), 7.98 (d, J = 8.8 Hz, 1H). |
| 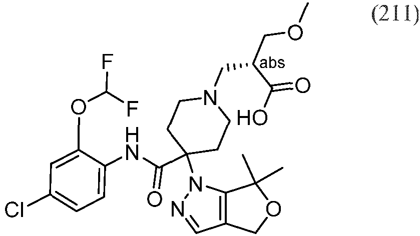 | (2*R*)-2-[[4-[[4-chloro-2-(difluoromethoxy)phenyl] carbamoyl]-4-(6,6-dimethyl-4*H*-furo[3,4-c]pyrazol-1-yl)-1-piperidyl]methyl]-3-methoxy-propanoic acid | ¹H NMR (400 MHz, DMSO) δ 1.38 (s, 6H), 2.07 - 2.44 (m, 5H), 2.79 (d, J = 9.2 Hz, 2H), 2.86 - 2.96 (m, 1H), 3.21 (s, 3H), 3.33 - 3.52 (m, 5H), 4.69 (s, 2H), 7.21 (t, J = 73.0 Hz, 1H), 7.31 (dd, J = 8.8, 2.3 Hz, 1H), 7.36 (d, J = 2.4 Hz, 1H), 7.48 (s, 1H), 7.87 (s, 1H), 7.98 (d, J = 8.8 Hz, 1H). |
| 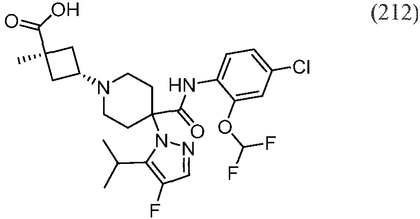 | (1*r*,3*r*)-3*-*[4-[[4-chloro-2-(difluoromethoxy)phenyl] carbamoyl]-4-(4-fluoro-5-isopropyl-pyrazol-1-yl)-1-piperidyl]-1-methyl-cyclobutanecarboxylic acid | ¹H NMR (400 MHz, DMSO) δ 1.15 (d, J = 6.8 Hz, 6H), 1.29 (s, 3H), 1.87 (t, J = 9.3 Hz, 2H), 2.07 - 2.26 (m, 4H), 2.37 (s, 4H), 2.56 (dd, J = 10.9, 5.3 Hz, 2H), 2.63 - 2.74 (m, 1H), 2.99 (p, J = 6.8 Hz, 1H), 7.17 (t, J = 73.4 Hz, 1H), 7.31 - 7.35 (m, 2H), 7.56 (d, J = 4.1 Hz, 1H), 7.61 (d, J = 9.2 Hz, 1H), 8.63 (s, 1H). |
| 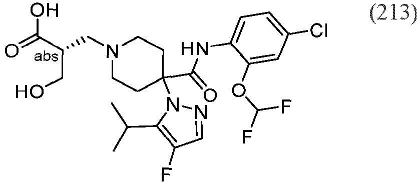 | (2*R*)-2-[[4-[[4-chloro-2-(difluoromethoxy)phenyl] carbamoyl]-4-(4-fluoro-5-isopropyl-pyrazol-1-yl)-1-piperidyl]methyl]-3-hydroxy-propanoic acid | ¹H NMR (500 MHz, DMSO) δ 1.16 (d, J = 6.8 Hz, 6H), 2.32 - 2.44 (m, 6H), 2.57 - 2.66 (m, 2H), 2.65 - 2.74 (m, 1H), 2.74 - 2.84 (m, 1H), 2.99 (p, J = 6.7 Hz, 1H), 3.48 - 3.58 (m, 2H), 6.98 - 7.36 (m, 3H), 7.55 - 7.62 (m, 2H), 8.68 (s, 1H). |
| 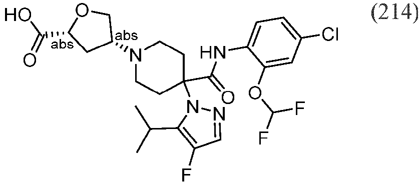 | (2*R*,4*R*)-4-[4-[[4-chloro-2-(difluoromethoxy)phenyl] carbamoyl]-4-(4-fluoro-5-isopropyl-pyrazol-1-yl)-1-piperidyl]tetrahydrofuran -2-carboxylic acid | ¹H NMR (400 MHz, DMSO) δ 1.19 (d, J = 6.8 Hz, 6H), 2.04 - 2.14 (m, 1H), 2.69 - 2.56 (m, 5H), 2.88 - 3.02 (m, 2H), 3.97 - 3.88 (m, 2H), 4.06 - 3.96 (m, 1H), 4.39 (t, J = 8.0 Hz, 1H), 7.21 (t, J = 73.3 Hz, 1H), 7.38 - 7.28 (m, 3H), 7.52 (d, J = 8.6 Hz, 1H), 7.68 (s, 1H), 8.94 (s, 1H). |
| 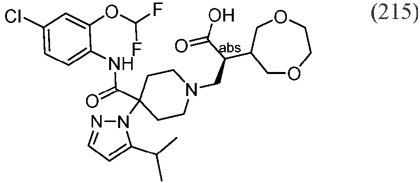 | (2*S*)-3-[4-[[4-chloro-2-(difluoromethoxy)phenyl] carbamoyl]-4-(5-isopropylpyrazol-1-yl)-1-piperidyl]-2-(1,4-dioxepan-6-yl)propanoic acid | ¹H NMR (400 MHz, DMSO) δ 8.20 (s, 1H), 7.81 - 7.76 (m, 1H), 7.53 (d, *J =* 1.9 Hz, 1H), 7.37 - 6.93 (m, 3H), 6.33 (d, *J =* 1.9 Hz, 1H), 3.75 (td, *J=* 12.0, 5.2 Hz, 2H), 3.66 - 3.48 (m, 6H), 2.98 - 2.82 (m, 2H), 2.75 - 2.67 (m, 1H), 2.65 - 2.15 (m, 10H), 1.10 (dd, *J=* 6.7, 2.9 Hz, 6H). |
| 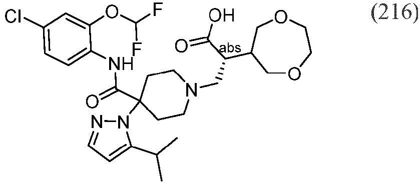 | (2*R*)-3-[4-[[4-chloro-2-(difluoromethoxy)phenyl] carbamoyl]-4-(5-isopropylpyrazol-1-yl)-1-piperidyl]-2-(1,4-dioxepan-6-yl)propanoic acid | ¹H NMR (400 MHz, DMSO) δ 8.18 (s, 1H), 7.84 - 7.75 (m, 1H), 7.53 (d, *J =* 1.8 Hz, 1H), 7.38 - 6.93 (m, 3H), 6.33 (d, *J =* 1.9 Hz, 1H), 3.84 - 3.68 (m, 2H), 3.68 - 3.48 (m, 6H), 2.98 - 2.85 (m, 2H), 2.72 - 2.64 (m, 1H), 2.62 - 2.19 (m, 10H), 1.10 (dd, *J =* 6.7, 2.9 Hz, 6H). |
| 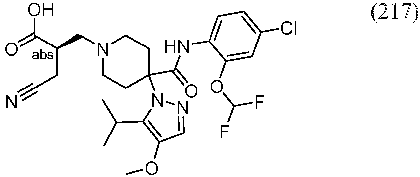 | (2*R*)-2-[[4-[[4-chloro-2-(difluoromethoxy)phenyl] carbamoyl]-4-(5-isopropyl-4-methoxy-pyrazol-1-yl)-1-piperidyl]methyl]-3-cyano-propanoic acid | ¹H NMR (400 MHz, DMSO) δ 1.06 - 1.14 (m, 6H), 2.28-2.42 (m, 7H), 2.56-2.69 (m, 3H), 2.75 (q, J = 7.2 Hz, 3H), 2.85 - 2.94 (m, 1H), 3.72 (s, 3H), 7.17 (t, J = 73.3 Hz, 1H), 7.30 - 7.34 (m, 2H), 7.46 (s, 1H), 7.79 (d, J = 9.3 Hz, 1H), 8.17 (s, 1H). |
| 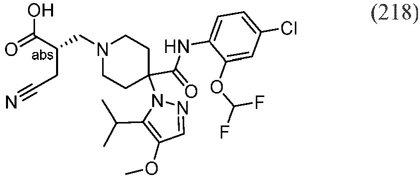 | (2*S*)-2-[[4-[[4-chloro-2-(difluoromethoxy)phenyl] carbamoyl]-4-(5-isopropyl-4-methoxy-pyrazol-1-yl)-1-piperidyl]methyl]-3-cyano-propanoic acid | ¹H NMR (400 MHz, DMSO) δ 1.06 - 1.14 (m, 6H), 2.29-2.44 (m, 7H), 2.56-2.69 (m, 2H), 2.74 (q, J = 7.2 Hz, 3H), 2.84 - 2.94 (m, 1H), 3.72 (s, 3H), 7.17 (t, J = 73.3 Hz, 1H), 7.30 - 7.34 (m, 2H), 7.46 (s, 1H), 7.79 (d, J = 9.2 Hz, 1H), 8.17 (s, 1H). |
| 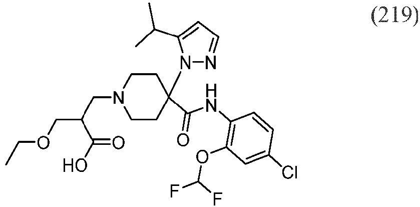 | 2-[[4-[[4-chloro-2-(difluoromethoxy)phenyl] carbamoyl]-4-(5-isopropylpyrazol-1-yl)-1-piperidyl]methyl]-3-ethoxy-propanoic acid | ¹H NMR (500 MHz, DMSO-d6) δ 8.21 (s, 1H), 7.78 (d, *J =* 9.4 Hz, 1H), 7.53 (d, *J =* 1.8 Hz, 1H), 7.32 (m, 2H), 7.15 (t, *J* = 73.2 Hz, 1H), 6.33 (d, J = 1.8 Hz, 1H), 3.49 (m, 4H), 3.39 (m, 4H), 2.93 (m, 1H), 2.84 (m, 1H), 2.74 (m, 2H), 2.52 (m, 2H), 2.37 (m, 2H), 1.09 (d, *J =* 6.7 Hz, 6H), 1.07 (t, J = 6.9 Hz, 3H). |
| 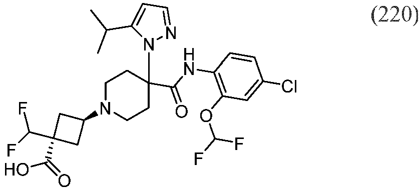 | (1*s*,3*s*)-3-[4-[[4-chloro-2-(difluoromethoxy)phenyl] carbamoyl]-4-(5-isopropylpyrazol-1-yl)-1-piperidyl]-1-(difluoromethyl)cyclobut anecarboxylic acid | ¹H NMR (400 MHz, DMSO) δ 7.91 (d, *J =* 9.4 Hz, 1H), 7.83 (s, 1H), 7.50 (d, *J =* 1.9 Hz, 1H), 7.31 - 7.25 (m, 2H), 7.05 (t, *J =* 73.2 Hz, 1H), 6.28 (d, *J =* 1.9 Hz, 1H), 6.11 (t, *J =* 57.0 Hz, 1H), 2.98 (p, *J =* 6.7 Hz, 1H), 2.87 (dq, *J =* 11.0, 7.7 Hz, 2H), 2.64 (dt, *J =* 10.1, 5.1 Hz, 2H), 2.59 - 2.51 (m, 2H), 2.44 - 2.36 (m, 2H), 2.26 (qd, *J =* 11.1, 5.1 Hz, 3H), 2.12 - 2.05 (m, 2H), 1.11 (d, *J =* 6.7 Hz, 6H). |
| 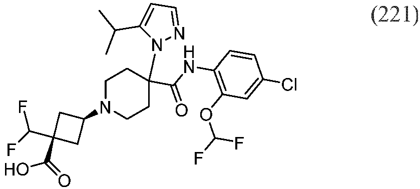 | (1*r*,3*r*)-3-[4-[[4-chloro-2-(difluoromethoxy)phenyl] carbamoyl]-4-(5-isopropylpyrazol-1-yl)-1-piperidyl]-1-(difluoromethyl)cyclobut anecarboxylic acid | ¹H NMR (400 MHz, DMSO) δ 7.90 (d, *J =* 9.4 Hz, 1H), 7.85 (s, 1H), 7.50 (d, *J =* 1.8 Hz, 1H), 7.30 - 7.25 (m, 2H), 7.05 (t, *J =* 73.2 Hz, 1H), 6.28 (d, *J =* 1.9 Hz, 1H), 6.23 (t, *J =* 56.5 Hz, 1H), 2.98 (p, *J* = 6.7 Hz, 1H), 2.77 (t, *J =* 7.5 Hz, 1H), 2.70 (dt, *J =* 10.4, 4.4 Hz, 2H), 2.62 - 2.53 (m, 2H), 2.41 (ddd, *J =* 13.6, 9.6, 3.5 Hz, 2H), 2.37 - 2.24 (m, 6H), 1.11 (d, *J* = 6.7 Hz, 6H). |
| 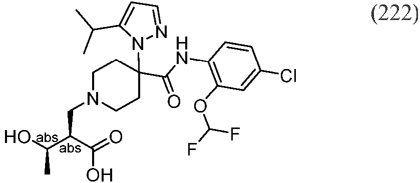 | (2*R*,3*R*)-2-[[4-[[4-chloro-2-(difluoromethoxy)phenyl] carbamoyl]-4-(5-isopropylpyrazol-1-yl)-1-piperidyl]methyl]-3-hydroxy-butanoic acid | ¹H NMR (400 MHz, DMSO-d6) δ 8.19 (s, 1H), 7.79 (d, *J =* 9.4 Hz, 1H), 7.53 (d, *J =* 1.8 Hz, 1H), 7.32 (m, 2H), 7.15 (t, *J* = 73.2 Hz, 1H), 6.33 (d, J = 1.8 Hz, 1H), 3.80 (m, 2H), 2.91 (m, 2H), 2.69 (m, 1H), 2.54 (m, 2H), 2.48 (m, 3H), 2.33 (m, 4H), 1.10 (d, *J =* 6.7 Hz, 6H), 1.05 (d, J = 6.2 Hz, 3H). |
| 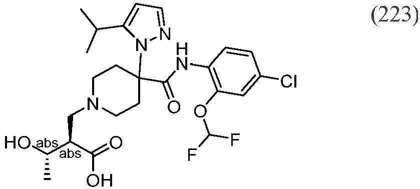 | (2*R*,3*S*)-2-[[4-[[4-chloro-2-(difluoromethoxy)phenyl] carbamoyl]-4-(5-isopropylpyrazol-1-yl)-1-piperidyl]methyl]-3-hydroxy-butanoic acid | ¹H NMR (400 MHz, DMSO-d6) δ 8.19 (s, 1H), 7.79 (d, *J =* 9.4 Hz, 1H), 7.53 (d, *J =* 1.8 Hz, 1H), 7.32 (m, 2H), 7.15 (t, *J* = 73.2 Hz, 1H), 6.33 (d, J = 1.8 Hz, 1H), 3.80 (m, 2H), 2.91 (m, 2H), 2.69 (m, 1H), 2.54 (m, 2H), 2.48 (m, 3H), 2.33 (m, 4H), 1.10 (d, *J =* 6.7 Hz, 6H), 1.05 (d, J = 6.2 Hz, 3H). |
| 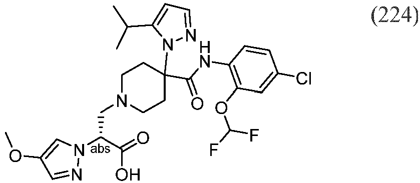 | (2*R*)-3-[4-[[4-chloro-2-(difluoromethoxy)phenyl] carbamoyl]-4-(5-isopropylpyrazol-1-yl)-1-piperidyl]-2-(4-methoxypyrazol-1-yl)propanoic acid | ¹H NMR (499 MHz, DMSO) δ 8.21 (s, 1H), 7.82 - 7.74 (m, 1H), 7.58 (d, *J =* 2.4 Hz, 1H), 7.52 (d, *J* = 1.8 Hz, 1H), 7.33 - 7.30 (m, 2H), 7.15 (t, *J =* 73.3 Hz, 1H), 6.32 (d, *J =* 1.9 Hz, 1H), 5.66 (d, *J =* 2.4 Hz, 1H), 4.93 (t, *J =* 7.2 Hz, 1H), 3.72 (s, 3H), 2.99 (d, *J* = 7.3 Hz, 2H), 2.96 - 2.88 (m, 1H), 2.86 - 2.70 (m, 2H), 2.61 - 2.40 (m, 4H), 2.39 - 2.27 (m, 2H), 1.09 (d, *J =* 6.7 Hz, 6H). |
| 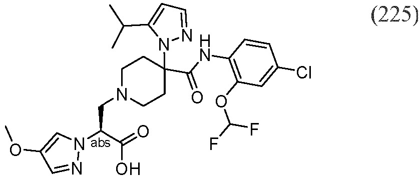 | (2*S*)-3-[4-[[4-chloro-2-(difluoromethoxy)phenyl] carbamoyl]-4-(5-isopropylpyrazol-1-yl)-1-piperidyl]-2-(4-methoxypyrazol-1-yl)propanoic acid | ¹H NMR (499 MHz, DMSO) δ 8.21 (s, 1H), 7.82 - 7.73 (m, 1H), 7.58 (d, *J =* 2.4 Hz, 1H), 7.52 (d, *J* = 1.9 Hz, 1H), 7.33 - 7.30 (m, 2H), 7.15 (t, *J =* 73.2 Hz, 1H), 6.32 (d, *J =* 1.8 Hz, 1H), 5.65 (d, *J =* 2.4 Hz, 1H), 4.93 (t, *J =* 7.3 Hz, 1H), 3.72 (s, 3H), 2.99 (d, *J* = 7.3 Hz, 2H), 2.95 - 2.88 (m, 1H), 2.86 - 2.72 (m, 2H), 2.61 - 2.42 (m, 4H), 2.40 - 2.26 (m, 2H), 1.09 (d, *J =* 6.6 Hz, 6H). |
| 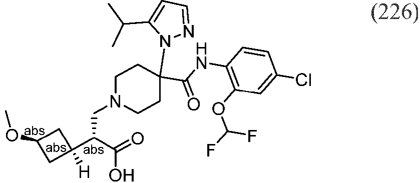 | (2*R*)-3-[4-[[4-chloro-2-(difluoromethoxy)phenyl] carbamoyl]-4-(5-isopropylpyrazol-1-yl)-1-piperidyl]-2-((1*s*,3*S*)-3-methoxycyclobutyl)propa noic acid | ¹H NMR (400 MHz, DMSO) δ 7.90 (d, *J = 9.4* Hz, 1H), 7.85 (s, 1H), 7.50 (d, *J =* 1.8 Hz, 1H), 7.30 - 7.25 (m, 2H), 7.05 (t, *J =* 73.2 Hz, 1H), 6.28 (d, *J =* 1.9 Hz, 1H), 3.72 - 3.59 (m, 1H), 3.10 (s, 3H), 2.98 (p, *J =* 6.7 Hz, 1H), 2.81 (d, *J =* 10.8 Hz, 1H), 2.68 (dd, *J =* 10.5, 5.7 Hz, 1H), 2.58 - 2.49 (m, 5H), 2.46 - 2.20 (m, 6H), 1.86 (p, *J =* 8.1 Hz, 1H), 1.68 - 1.58 (m, 1H), 1.53 (td, *J=* 10.1, 7.6 Hz, 1H), 1.11 (dd, *J =* 6.7, 1.6 Hz, 6H). |
| 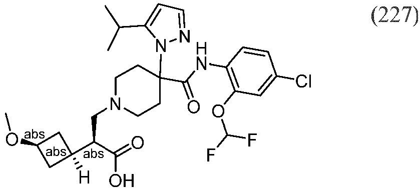 | (2*S*)-3-[4-[[4-chloro-2-(difluoromethoxy)phenyl] carbamoyl]-4-(5-isopropylpyrazol-1-yl)-1-piperidyl]-2-((1*s*,3*R*)-3-methoxycyclobutyl)propa noic acid | ¹H NMR (400 MHz, DMSO) δ 7.90 (d, *J = 9.4* Hz, 1H), 7.85 (s, 1H), 7.50 (d, *J =* 1.8 Hz, 1H), 7.30 - 7.25 (m, 2H), 7.05 (t, *J =* 73.2 Hz, 1H), 6.28 (d, *J =* 1.9 Hz, 1H), 3.72 - 3.59 (m, 1H), 3.10 (s, 3H), 2.98 (p, *J =* 6.7 Hz, 1H), 2.81 (d, *J =* 10.8 Hz, 1H), 2.68 (dd, *J =* 10.5, 5.7 Hz, 1H), 2.58 - 2.49 (m, 5H), 2.46 - 2.20 (m, 6H), 1.86 (p, *J =* 8.1 Hz, 1H), 1.68 - 1.58 (m, 1H), 1.53 (td, *J =* 10.1, 7.6 Hz, 1H), 1.11 (dd, *J =* 6.7, 1.6 Hz, 6H). |
| 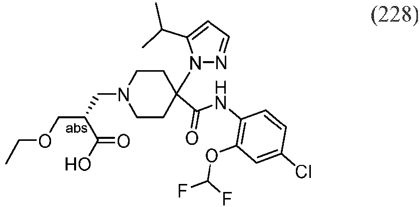 | (2*S*)-2-[[4-[[4-chloro-2-(difluoromethoxy)phenyl] carbamoyl]-4-(5-isopropylpyrazol-1-yl)-1-piperidyl]methyl]-3-ethoxy-propanoic acid | ¹H NMR (500 MHz, DMSO-d6) δ 8.21 (s, 1H), 7.78 (d, *J =* 9.4 Hz, 1H), 7.53 (d, *J =* 1.8 Hz, 1H), 7.32 (m, 2H), 7.15 (t, *J* = 73.2 Hz, 1H), 6.33 (d, J = 1.8 Hz, 1H), 3.49 (m, 4H), 3.39 (m, 4H), 2.93 (m, 1H), 2.84 (m, 1H), 2.74 (m, 2H), 2.52 (m, 2H), 2.37 (m, 2H), 1.09 (d, *J =* 6.7 Hz, 6H), 1.07 (t, J = 6.9 Hz, 3H). |
| 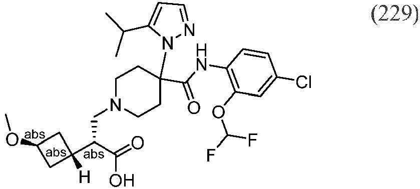 | (2R)-3-[4-[[4-chloro-2-(difluoromethoxy)phenyl] carbamoyl]-4-(5-isopropylpyrazol-1-yl)-1-piperidyl]-2-((1*r*,3*R*)-3-methoxycyclobutyl)propa noic acid | ¹H NMR (400 MHz, DMSO) δ 7.91 (d, *J =* 9.4 Hz, 1H), 7.81 (s, 1H), 7.51 (d, *J =* 1.8 Hz, 1H), 7.32 - 7.25 (m, 2H), 7.05 (t, *J =* 73.2 Hz, 1H), 6.28 (d, *J =* 1.9 Hz, 1H), 3.88 (p, *J =* 5.9 Hz, 1H), 3.11 (s, 3H), 2.98 (p, *J =* 6.7 Hz, 1H), 2.83 (d, *J =* 10.7 Hz, 1H), 2.68 (dt, *J =* 9.3, 5.4 Hz, 1H), 2.58 - 2.50 (m, 4H), 2.51 - 2.47 (m, 2H), 2.43 - 2.29 (m, 3H), 2.29 - 2.20 (m, 1H), 2.08 - 1.91 (m, 4H), 1.11 (dd, *J =* 6.7, 1.9 Hz, 6H). |
| 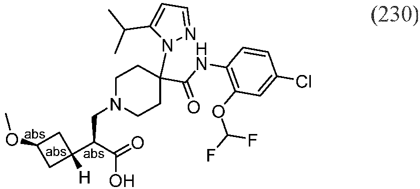 | (2*S*)-3-[4-[[4-chloro-2-(difluoromethoxy)phenyl] carbamoyl]-4-(5-isopropylpyrazol-1-yl)-1-piperidyl]-2-((1*r*,3*S*)-3-methoxycyclobutyl)propa noic acid | ¹H NMR (400 MHz, DMSO) δ 7.91 (d, *J = 9.4* Hz, 1H), 7.81 (s, 1H), 7.51 (d, *J =* 1.8 Hz, 1H), 7.32 - 7.25 (m, 2H), 7.05 (t, *J =* 73.2 Hz, 1H), 6.28 (d, *J =* 1.9 Hz, 1H), 3.88 (p, *J =* 5.9 Hz, 1H), 3.11 (s, 3H), 2.98 (p, *J =* 6.7 Hz, 1H), 2.83 (d, *J =* 10.7 Hz, 1H), 2.68 (dt, *J =* 9.3, 5.4 Hz, 1H), 2.58 - 2.50 (m, 4H), 2.51 - 2.47 (m, 2H), 2.43 - 2.29 (m, 3H), 2.29 - 2.20 (m, 1H), 2.08 - 1.91 (m, 4H), 1.11 (dd, *J =* 6.7, 1.9 Hz, 6H). |
| 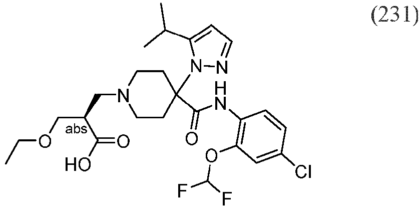 | (2R)-2-[[4-[[4-chloro-2-(difluoromethoxy)phenyl] carbamoyl]-4-(5-isopropylpyrazol-1-yl)-1-piperidyl]methyl]-3-ethoxy-propanoic acid | ¹H NMR (500 MHz, DMSO-d6) δ 8.21 (s, 1H), 7.78 (d, *J =* 9.4 Hz, 1H), 7.53 (d, *J =* 1.8 Hz, 1H), 7.32 (m, 2H), 7.15 (t, *J* = 73.2 Hz, 1H), 6.33 (d, J = 1.8 Hz, 1H), 3.49 (m, 4H), 3.39 (m, 4H), 2.93 (m, 1H), 2.84 (m, 1H), 2.74 (m, 2H), 2.52 (m, 2H), 2.37 (m, 2H), 1.09 (d, *J =* 6.7 Hz, 6H), 1.07 (t, J = 6.9 Hz, 3H). |
| 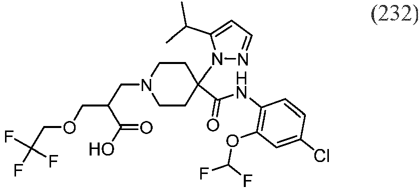 | 2-[[4-[[4-chloro-2-(difluoromethoxy)phenyl] carbamoyl]-4-(5-isopropylpyrazol-1-yl)-1-piperidyl]methyl]-3-(2,2,2-trifluoroethoxy)propanoic acid | ¹H NMR (600 MHz, DMSO-d6) δ 8.22 (s, 1H), 7.78 (d, *J =* 9.4 Hz, 1H), 7.53 (d, *J =* 1.9 Hz, 1H), 7.32 (m, 2H), 7.15 (t, J = 73.2Hz, 1H), 6.33 (d, J = 1.8 Hz, 1H), 4.04 (m, 2H), 3.75 (m, 2H), 2.93 (m, 1H), 2.83 (m, 2H), 2.75 (m, 1H), 2.49 (m, 4H), 2.42 (m, 4H), 1.09 (d, *J =* 6.7 Hz, 6H). |
| 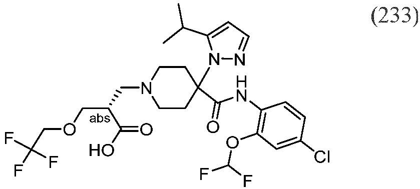 | (2*S*)-2-[[4-[[4-chloro-2-(difluoromethoxy)phenyl] carbamoyl]-4-(5-isopropylpyrazol-1-yl)-1-piperidyl]methyl]-3-(2,2,2-trifluoroethoxy)propanoic acid | ¹H NMR (600 MHz, DMSO-d6) δ 8.22 (s, 1H), 7.77 (d, *J =* 9.4 Hz, 1H), 7.53 (d, *J =* 1.9 Hz, 1H), 7.32 (m, 2H), 7.15 (t, J = 73.2Hz, 1H), 6.33 (d, J = 1.8 Hz, 1H), 4.04 (m, 2H), 3.75 (m, 2H), 2.93 (m, 1H), 2.81 (m, 2H), 2.74 (m, 1H), 2.51 (m, 4H), 2.40 (m, 4H), 1.09 (d, *J =* 6.7 Hz, 6H). |
| 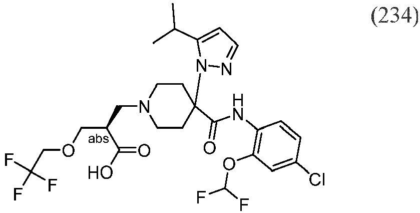 | (2*R*)-2-[[4-[[4-chloro-2-(difluoromethoxy)phenyl] carbamoyl]-4-(5-isopropylpyrazol-1-yl)-1-piperidyl]methyl]-3-(2,2,2-trifluoroethoxy)propanoic acid | ¹H NMR (500 MHz, DMSO-d6) δ 8.22 (s, 1H), 7.77 (d, *J =* 9.4 Hz, 1H), 7.53 (t, *J =* 1.9 Hz, 1H), 7.32 (m, 2H), 7.15 (t, J = 73.2Hz, 1H), 6.33 (d, J = 1.8 Hz, 1H), 4.04 (m, 2H), 3.75 (m, 2H), 2.93 (m, 1H), 2.81 (m, 2H), 2.74 (m, 1H), 2.51 (m, 4H), 2.40 (m, 4H), 1.11 (d, *J =* 6.7 Hz, 6H). |
| 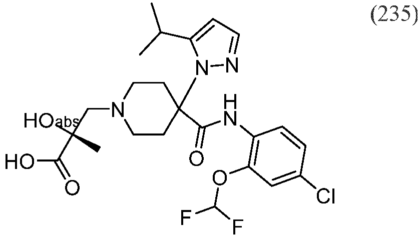 | (2*R*)-3-[4-[[4-chloro-2-(difluoromethoxy)phenyl] carbamoyl]-4-(5-isopropylpyrazol-1-yl)-1-piperidyl]-2-hydroxy-2-methyl-propanoic acid | ¹H NMR (499 MHz, DMSO) δ 8.18 (s, 1H), 7.84 - 7.75 (m, 1H), 7.53 (d, *J =* 1.8 Hz, 1H), 7.34 - 7.31 (m, 2H), 7.15 (t, *J =* 73.2 Hz, 1H), 6.33 (d, *J =* 1.9 Hz, 1H), 3.07 - 2.98 (m, 1H), 2.97 - 2.80 (m, 2H), 2.71 - 2.62 (m, 2H), 2.60 - 2.35 (m, 6H), 1.20 (s, 3H), 1.10 (dd, *J =* 6.7, 1.7 Hz, 6H). |
| 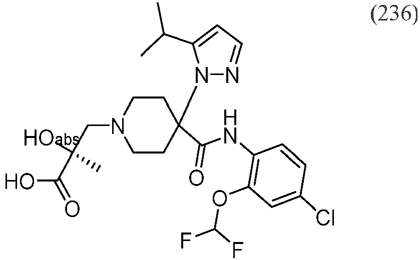 | (2*S*)-3-[4-[[4-chloro-2-(difluoromethoxy)phenyl] carbamoyl]-4-(5-isopropylpyrazol-1-yl)-1-piperidyl]-2-hydroxy-2-methyl-propanoic acid | ¹H NMR (499 MHz, DMSO) δ 8.18 (s, 1H), 7.82 - 7.77 (m, 1H), 7.53 (d, *J =* 1.9 Hz, 1H), 7.34 - 7.31 (m, 2H), 7.15 (t, *J =* 73.2 Hz, 1H), 6.33 (d, *J =* 1.9 Hz, 1H), 3.09 - 2.99 (m, 1H), 2.97 - 2.82 (m, 2H), 2.73 - 2.62 (m, 2H), 2.61 - 2.33 (m, 6H), 1.20 (s, 3H), 1.10 (dd, *J =* 6.7, 1.7 Hz, 6H). |
| 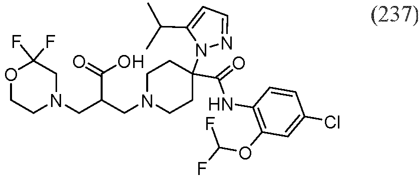 | 2-[[4-[[4-chloro-2-(difluoromethoxy)phenyl] carbamoyl]-4-(5-isopropylpyrazol-1-yl)-1-piperidyl]methyl]-3-(2,2-difluoromorpholin-4-yl)propanoic acid | ¹H NMR (500 MHz, DMSO-d6) δ 8.20 (s, 1H), 7.78 (d, *J =* 9.4 Hz, 1H), 7.53 (d, *J =* 1.8 Hz, 1H), 7.32 (m, 2H), 7.15 (t, *J* = 73.2 Hz, 1H), 6.33 (d, J = 1.8 Hz, 1H), 3.95 (m, 2H), 2.93 (m, 1H), 2.88 (m, 1H), 2.81 (m, 2H), 2.71 (m, 2H), 2.63 (m, 1H), 2.56 (m, 1H), 2.49 (m, 3H), 2.36 (m, 3H), 1.10 (d, *J =* 6.7 Hz, 6H). |
| 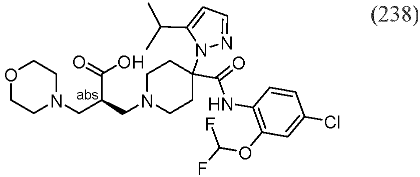 | (2*S*)-2-[[4-[[4-chloro-2-(difluoromethoxy)phenyl] carbamoyl]-4-(5-isopropylpyrazol-1-yl)-1-piperidyl]methyl]-3-morpholino-propanoic acid | ¹H NMR (400 MHz, DMSO-d6) δ 8.20 (s, 1H), 7.78 (d, *J =* 9.4 Hz, 1H), 7.53 (d, *J =* 1.8 Hz, 1H), 7.32 (m, 2H), 7.15 (t, *J* = 73.2 Hz, 1H), 6.33 (d, J = 1.8 Hz, 1H), 3.54 (m, 4H), 2.92 (m, 1H), 2.85 (m, 1H), 2.79 (m, 1H), 2.68 (m, 1H), 2.49 (m, 3H), 2.41 (m, 2H), 2.35 (m, 8H), 1.10 (m, 6H). |
| 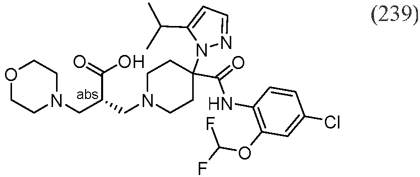 | (2*R*)-2-[[4-[[4-chloro-2-(difluoromethoxy)phenyl] carbamoyl]-4-(5-isopropylpyrazol-1-yl)-1-piperidyl]methyl]-3-morpholino-propanoic acid | ¹H NMR (400 MHz, DMSO-d6) δ 8.18 (s, 1H), 7.79 (d, *J =* 9.4 Hz, 1H), 7.53 (d, *J =* 1.8 Hz, 1H), 7.32 (m, 2H), 7.15 (t, *J* = 73.2 Hz, 1H), 6.33 (d, J = 1.8 Hz, 1H), 3.53 (m, 4H), 2.93 (m, 1H), 2.81 (m, 3H), 2.68 (m, 2H), 2.49 (m, 2H), 2.41 (m, 2H), 2.35 (m, 8H), 1.10 (d, *J =* 6.7 Hz, 6H). |
| 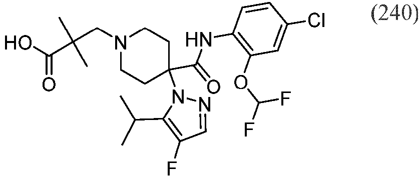 | 3-[4-[[4-chloro-2-(difluoromethoxy)phenyl] carbamoyl]-4-(4-fluoro-5-isopropyl-pyrazol-1-yl)-1-piperidyl]-2,2-dimethyl-propanoic acid | ¹H NMR (400 MHz, DMSO) δ 8.08 (s, 1H), 7.78 (d, *J = 9.3 Hz,* 1H), 7.51 (d, *J =* 4.2 Hz, 1H), 7.32 - 7.27 (m, 2H), 7.07 (t, *J =* 73.3 Hz, 1H), 3.06 - 2.96 (m, 1H), 2.76 (dt, *J =* 10.5, 4.7 Hz, 2H), 2.56 (ddd, *J =* 11.9, 8.0, 4.3 Hz, 2H), 2.44 (s, 2H), 2.43 - 2.32 (m, 4H), 1.16 (dd, *J =* 6.9, 1.0 Hz, 6H), 1.09 (s, 6H). |

The compounds described herein may be LPAR1 antagonists. By antagonizing LPAR1 the compounds described herein may treat or prevent diseases and disorders associated with LPAR1 signaling (e.g., fibrosis).

In some embodiments, the compounds described herein may be used to treat systemic sclerosis (SSc). Lysophosphatidic acid (LPA) signaling is known to be associated with various forms of tissue fibrosis. Without being bound by a particular theory, studies suggest that LPAR inhibition can be used to treat fibrotic disease such as SSc.

In certain embodiments, Ring A is an optionally substituted 4-membered carbocyclylene.

In some embodiments, Ring A is a 4-membered carbocyclylene, optionally substituted with 1-3 substituents each independently selected from the group consisting of halo, C₁₋₆alkyl, C₁₋₆haloalkyl, -O(C₁₋₆alkyl), and -O(C₁₋₆haloalkyl).

In certain embodiments, X is an optionally substituted 3-7-membered carbocyclylene, 3-7-membered heterocyclylene, -CH(3-7-membered carbocyclyl)-, -CH(3-7-membered heterocyclyl)-, -CH(C₁₋₆alkyl), -CH(C₁₋₆haloalkyl), or -CH(C₁₋₆alkoxyC₁₋₆alkyl);

In some embodiments, X is an optionally substituted 3-7-membered heterocyclylene, - CH(3-7-membered heterocyclyl)-, or -CH(C₁₋₆alkoxyC₁₋₆alkyl).

In certain embodiments, each R^{a} is independently selected from the group consisting of -Cl and -OCHF₂, and n is 2.

In some embodiments, provided herein is a compound or a pharmaceutically acceptable salt thereof, wherein the compound is Compound 96.

In certain embodiments, the compound of Formula (I) has the structure of Formula (Ib): 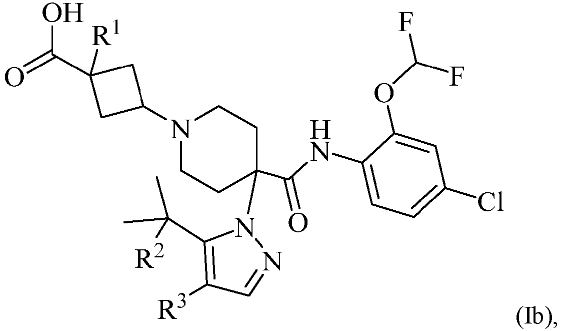 or a pharmaceutically acceptable salt thereof,
wherein:
R¹ is hydrogen or C₁₋₆alkyl; and
R² and R³ are each independently hydrogen, halo, optionally substituted (C₁₋₃alkyl), or optionally substituted alkoxy(C₁₋₃alkyl); or
R² and R³ taken together with the atoms to which they are attached, form a fused optionally substituted 4-6 membered carbocyclyl or fused optionally substituted 4-6 membered heterocyclyl.

In some embodiments, R¹ is hydrogen or methyl.

In certain embodiments, R² and R³ are each hydrogen.

In some embodiments, the compound is selected from the following table:

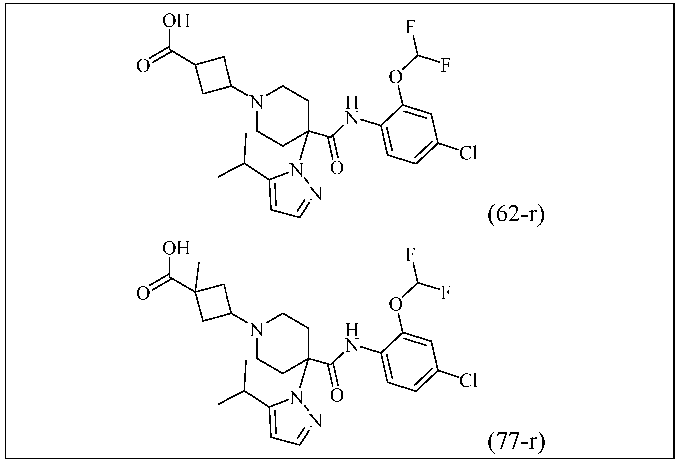

In some embodiments, the compound is selected from the following table:

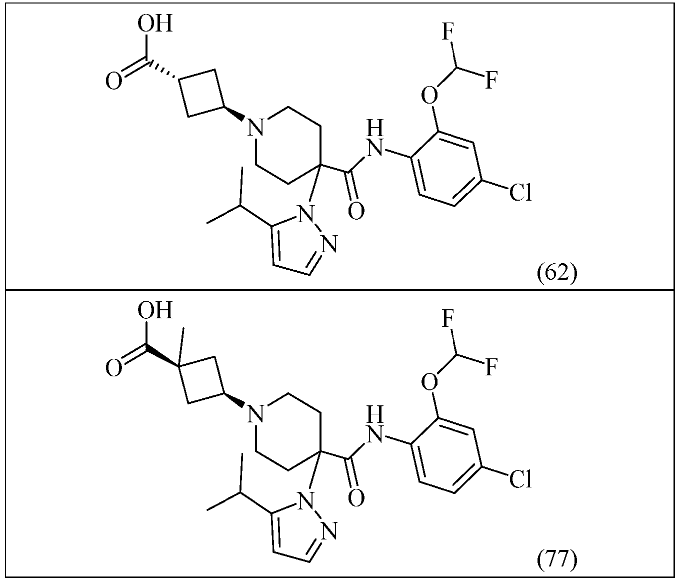

In certain embodiments, the compound of Formula (II) the structure of Formula (IIb): 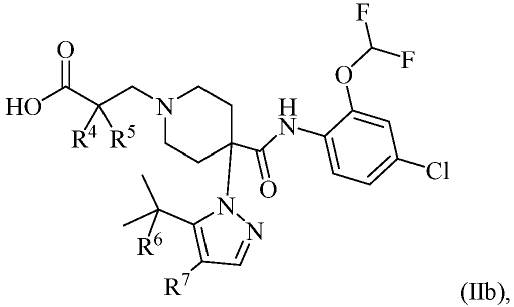 or a pharmaceutically acceptable salt thereof,
wherein:
R⁴ is hydrogen and R⁵ is an optionally substituted 3-7-membered carbocyclyl, 3-7-membered heterocyclyl, C₁₋₆alkyl, C₁₋₆haloalkyl, or C₁₋₆alkoxyC₁₋₆alkyl; or
R⁴ and R⁵ taken together with the atom to which they are attached form an optionally substituted 3-7-membered carbocyclylene or an optionally susbstituted 3-7-membered heterocyclylene; and
R⁶ and R⁷ are each independently hydrogen, halo, optionally substituted (C₁₋₃alkyl), or optionally substituted alkoxy(C₁₋₃alkyl); or
R⁶ and R⁷ taken together with the atoms to which they are attached, form a fused optionally substituted 4-6 membered carbocyclyl or fused optionally substituted 4-6 membered heterocyclyl.

In some embodiments, R⁴ is hydrogen and R⁵ is tetrahydropyranyl, or -CH₂OCH₃; or R⁴ and R⁵ taken together with the atom to which they are attached form a tetrahydropyran ring.

In certain embodiments, R⁶ and R⁷ are each independently hydrogen or fluoro; or R⁶ and R⁷ taken together with the atoms to which they are attached form a fused 5-membered heterocyclyl.

In some embodiments, the compound is selected from the following table:

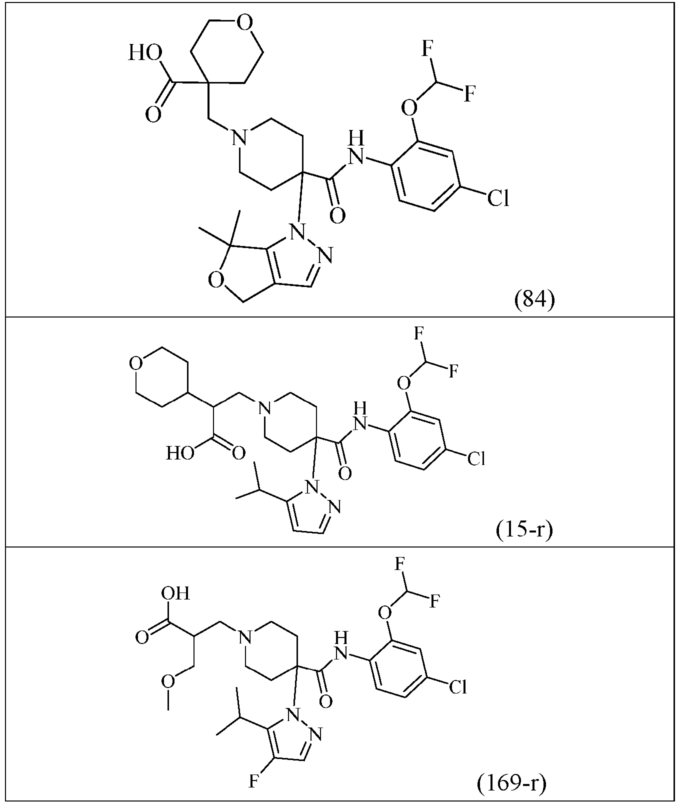

In some embodiments, the compound is selected from the following table:

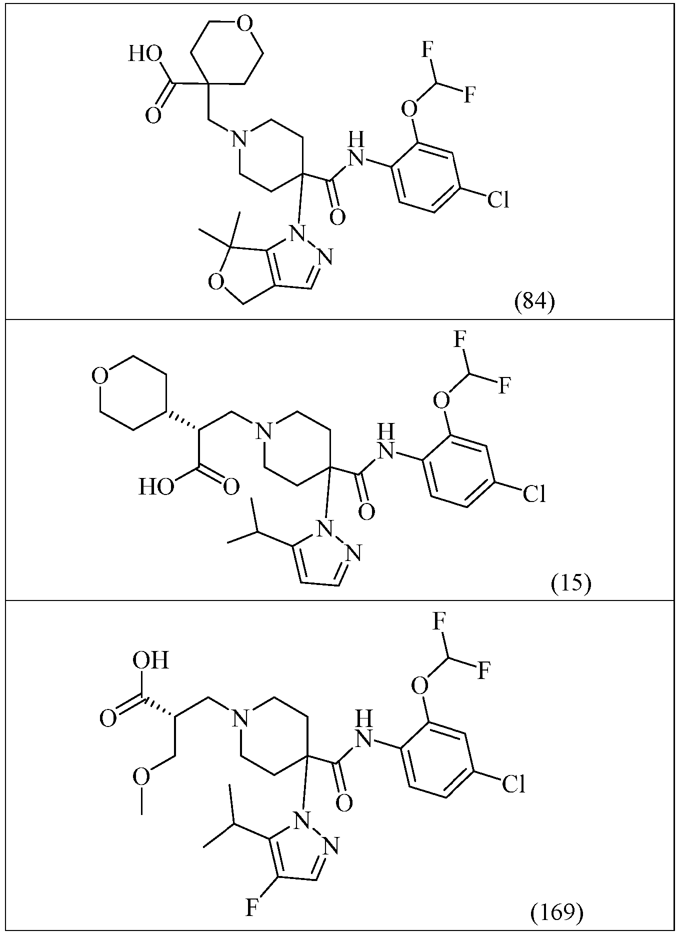

### Pharmaceutical Compositions and Methods of Use

In certain aspects, provided herein is a pharmaceutical composition, comprising a compound of Formula (I) or a compound of Formula (II) and a pharmaceutically acceptable carrier.

In some aspects, provided herein is a method of treating or preventing a disease or condition comprising administering to a subject in need thereof a therapeutically effective amount of a compound of Formula (I) or a compound of Formula (II), wherein the disease is treated by inhibiting LPAR1. In some embodiments, the disease or condition is systemic sclerosis.

In certain aspects, provided herein is a method of inhibiting LPAR1 comprising administering to a subject an effective amount of a compound of Formula (I) or a compound of Formula (II).

In certain aspects, provided herein is a method of treating systemic sclerosis (SSc) in a subject in need thereof comprising administering to the subject an effective amount of a compound of the present disclosure.

### Preparative Methods

The compounds described herein may be prepared according to any suitable method known to a person of ordinary skill in the art. The syntheses of compounds of the present disclosure are described in the below examples.

### EXAMPLES

In order that this disclosure may be more fully understood, the following Examples are set forth. It should be understood that these Examples are for illustrative purposes only and are not to be construed as limiting this disclosure in any manner.

Common abbreviations well known to those skilled in the art which are used throughout include those in Table A.

**Table A. Abbreviations**

| **Abbreviation** | **Definition** |
|---|---|
| NMR | nuclear magnetic resonance |
| s | singlet |
| br s | broad singlet |
| d | doublet |
| br d | broad doublet |
| t | triplet |
| br t | broad triplet |
| q | quartet |
| m | multiplet |
| br m | broad multiplet |
| dd | doublet of doublet |
| br dd | broad doublet of doublet |
| ddd | doublet of doublet of doublet |
| dddd | doublet of doublet of doublet of doublet |
| dt | doublet of triplet |
| dtd | doublet of triplet of doublet |
| tt | triplet of triplet |
| min | minute |
| h | hour |
| mL | milliliter |
| µL | microliter |
| L | liter |
| g | gram |
| mg | milligram |
| mmol | millimoles |
| M | molarity (moles/liter) |
| ppm | parts per million |
| MS | mass spectrometry |
| Boc | tert-butoxycarboxyl |
| ESI | Electrospray ionization |
| APCI | Atmospheric pressure chemical ionization |
| MHz | megahertz |
| m/z | ratio of mass to charge |

### Synthetic Intermediates

### Preparation #1: INT-1

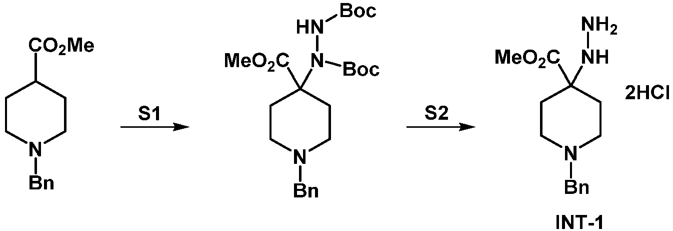

**Step 1: di-tert-butyl 1-(1-benzyl-4-(methoxycarbonyl)piperidin-4-yl)hydrazine-1,2-dicarboxylate.** To a solution of diisopropylamine (38 ml, 267 mmol) in Tetrahydrofuran (THF) (400 ml) under nitrogen at 0 °C was added butyllithium 2.5 M in hexanes (100 ml, 250 mmol) dropwise over 10 minutes. After a further 25 min the mixture was cooled to -60 °C and a solution of methyl 1-benzylpiperidine-4-carboxylate (48 g, 206 mmol) in Tetrahydrofuran (THF) (50 mL) was added dropwise over 15 min. After 30 minutes a solution of (E)-di-tert-butyl diazene-1,2-dicarboxylate (66 g, 287 mmol) in Tetrahydrofuran (THF) (200 mL) was added over 25 min. The resulting orange solution was stirred at -60 °C for 3 h. After this time the cooling bath was removed and the reaction stirred for 5 minutes and then was poured into a mixture of saturated aqueous ammonium chloride (800 mL) and ice (300 mL) and stirred vigorously for 60 minutes. The mixture was diluted with ethyl acetate (1000 mL) and the aqueous layer extracted with TBME (2 x 200 mL) and then organic layers combined and washed with further saturated aqueous ammonium chloride (5 x 200 mL), dried over sodium sulfate, filtered and concentrated under reduced pressure to afford crude product di-tert-butyl 1-(1-benzyl-4-(methoxycarbonyl)piperidin-4-yl)hydrazine-1,2-dicarboxylate (123 g, 212 mmol, 103 % yield) as an orange oil used directly in the next step. ¹H NMR (400 MHz, Methanol-d₄) δ 1.42 - 1.58 (m, 18H), 1.92 - 1.96 (m, 1H), 2.02 - 2.13 (m, 1H), 2.21 - 2.33 (m, 1H), 2.35 - 2.48 (m, 2H), 2.51 - 2.60 (m, 1H), 2.60 - 2.70 (m, 1H), 2.72 - 2.83 (m, 1H), 3.46 - 3.58 (m, 2H), 3.68 - 3.73 (m, 3H), 7.24 - 7.41 (m, 5H); MS (ESI) *m*/*z* 464 (M+H)⁺.

**Step 2: INT-1.** To a solution of di-tert-butyl 1-(1-benzyl-4-(methoxycarbonyl)piperidin-4-yl)hydrazine-1,2-dicarboxylate (42 g, 91 mmol) in methanol (180 ml) was added a solution of 4 N aqueous hydrochloric acid (227 ml, 908 mmol) and the resulting solution stirred at ambient temperature for 20 h. The mixture was then concentrated under reduced pressure and then added approx. 300 mL of cyclopentylmethyl ether solvent which caused the desired product, methyl 1-benzyl-4-hydrazinylpiperidine-4-carboxylate dihydrochloric acid (INT-1) to precipitate out of solution, which was collected and dried under vacuum (34.4 g, 99% yield). ¹H NMR (400 MHz, DMSO-d₆) δ 2.00 - 2.18 (m, 2H), 2.28 - 2.46 (m, 2H), 2.94 - 3.25 (m, 4H), 3.73 (s, 3H), 4.30 (d, J = 4.9 Hz, 2H), 6.10 (s, 1H), 7.38 - 7.50 (m, 3H), 7.59 - 7.73 (m, 2H), 9.58 (s, 2H), 11.43 (s, 1H).

### Preparation #2: INT-2

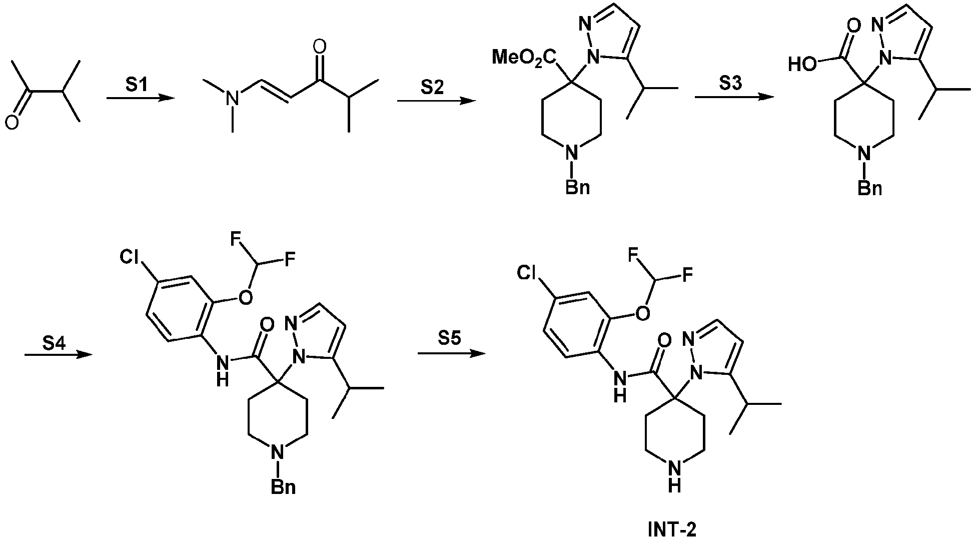

**Step 1: (E)-1-(dimethylamino)-4-methylpent-1-en-3-one.** A mixture of 3-methylbutan-2-one (25 g, 290 mmol) and 1,1-dimethoxy-N,N-dimethylmethanamine (51.9 g, 435 mmol) was stirred at 100°C for 4 days. TLC showed the starting material was consumed and a new spot was detected. The reaction mixture was then concentrated under reduced pressure to afford (E)-1-(dimethylamino)-4-methylpent-1-en-3-one (13.3 g, 29.3% yield) as brown oil. ¹H NMR (400 MHz, DMSO-d₆) δ 0.96 (d, J = 6.8 Hz, 6H), 2.66-3.11 (m, 6H), 3.17 (d, J = 5.3 Hz, 1H), 4.98 (d, J = 12.6 Hz, 1H), 7.46 (d, J = 12.8 Hz, 1H).

**Step 2: methyl 1-benzyl-4-(5-isopropyl-1H-pyrazol-1-yl)piperidine-4-carboxylate.** To a solution of **INT-1** (64.5 g, 196 mmol) in Methanol (1L) was added (E)-1-(dimethylamino)-4-methylpent-1-en-3-one (29 g, 205 mmol) and the resulting solution was heated at 65 °C for 20 hours. The reaction was then cooled to ambient temperature. The mixture was concentrated under reduced pressure and diluted with ice-water (600 mL), then carefully basified with 2 N aqueous NaOH to pH 8 (~ 300 mL) and extracted with TBME (4 x 200 mL) and 2-methyl THF (200 mL), the combined organic solutions were washed with water (100 ml), dried over sodium sulfate and concentrated under reduced pressure to afford an orange oil. This crude product was purified by silica gel chromatography using a gradient of methyl tert-butyl ether in hexanes to afford the titled product (48 g, 134 mmol, 64.8 % yield) as a yellow oil. ¹H NMR (400 MHz, DMSO-d6) δ 1.11 (d, *J=* 6.7 Hz, 6H), 2.30 (ddd, *J=* 13.1, 8.2, 3.9 Hz, 2H), 2.34 - 2.48 (m, 4H), 2.50 - 2.58 (m, 2H), 2.72 - 2.80 (m, 1H), 3.42 (s, 2H), 3.65 (s, 3H), 6.22 (d, *J=* 1.9 Hz, 1H), 7.20 - 7.26 (m, 1H), 7.27 - 7.33 (m, 4H), 7.36 (d, *J =* 1.9 Hz, 1H).

**Step 3: 1-benzyl-4-(5-isopropyl-1H-pyrazol-1-yl)piperidine-4-carboxylic acid.** To a solution of methyl 1-benzyl-4-(5-isopropyl-1H-pyrazol-1-yl)piperidine-4-carboxylate (41.47 g, 121 mmol) in MeOH (486 ml) was added sodium hydroxide 2 M (182 ml, 364 mmol). This mixture was stirred at 75°C for 4h, then cooled to ambient temperature, and concentrated to remove the methanol. Then 800 mL ethyl acetate was added to the mixture, and the solution was adjusted to a pH of 1 by the addition of 66 mL of a 6 M HCl solution. The resulting solid was filtered, then washed with 20 mL of water followed by 100 mL of ethyl acetate to provide 1-benzyl-4-(5-isopropyl-1H-pyrazol-1-yl)piperidine-4-carboxylic acid. The aqueous filtrate layer was separated and then extracted with ethyl acetate (3 x 300 mL). The combined organic extracted were then dried over Na₂SO₄, concentrated to provide additional desired product. The total 1-benzyl-4-(5-isopropyl-1H-pyrazol-1-yl)piperidine-4-carboxylic acid material obtained was (37.0 g, 111 mmol, 92% yield) as a colorless solid. MS (ESI) *m*/*z* 328 (M+H)⁺.

**Step 4: 1-benzyl-N-(4-chloro-2-(difluoromethoxy)phenyl)-4-(5-isopropyl-1H-pyrazol-1-yl)piperidine-4-carboxamide.** 1-benzyl-4-(5-isopropyl-1H-pyrazol-1-yl)piperidine-4-carboxylic acid (1.69 g, 5.17 mmol) was dissolved in dry DCM (50 ml) and 1-(Chloro-1-Pyrrolidinylmethylene)pyrrolidinium hexafluorophosphate (1.89 g, 5.68 mmol) was added followed by the addition of Hunig's Base (N,N-diisopropylethylamine) (2.71 ml, 15.50 mmol) and the mixture stirred at ambient temperature for 30 min. Then 4-Chloro-2-(difluroromethoxy)aniline (1.0 g, 5.17 mmol) was added and the mixture stirred at ambient temperature for 18 h. Then added half saturated aqueous ammonium chloride solution (20 mL) and extracted this mixture with DCM (100 mL), separated and dried the organic layer, concentrated and columned using 0-100% ethyl acetate in heptanes to afford 1-benzyl-N-(4-chloro-2-(difluoromethoxy)phenyl)-4-(5-isopropyl-1H-pyrazol-1-yl)piperidine-4-carboxamide (1.95 g, 3.88 mmol, 75% yield). MS (ESI) *m*/*z* 503.4 (M+H)⁺.

**Step 5: INT-2.** 1-benzyl-N-(4-chloro-2-(difluoromethoxy)phenyl)-4-(5-isopropyl-1H-pyrazol-1-yl)piperidine-4-carboxamide (1.95 g, 3.88 mmol) was dissolved in dichloromethane (40 ml) then 1-chloroethyl chloroformate (0.610 g, 4.26 mmol) was added and the mixture was heated at 80°C for 1 h then allowed to cool to ambient temperature and the solvent removed under vacuum. The residue was then dissolved methanol (40 mL) and the solution heated at 80°C for 18 h. The solution was then concentrated under vacuum and the residue columned using 0-40% methanol in dichloromethane to provide N-(4-chloro-2-(difluoromethoxy)phenyl)-4-(5-isopropyl-1H-pyrazol-1-yl)piperidine-4-carboxamide (**INT-2**) (1.56 g, 3.78 mmol, 97% yield). MS (ESI) *m*/*z* 413.3 (M+H)⁺.

### Synthetic Examples

### Example #1: Compounds 14 and 15: (2S)-3-[4-[[4-chloro-2-(difluoromethoxy)phenyl]carbamoyl]-4-(5-isopropylpyrazol-1-yl)-1-piperidyl]-2-tetrahydropyran-4-yl-propanoic acid and (2R)-3-[4-[[4-chloro-2-(difluoromethoxy)phenyl]carbamoyl]-4-(5-isopropylpyrazol-1-yl)-1-piperidyl]-2-tetrahydropyran-4-yl-propanoic acid

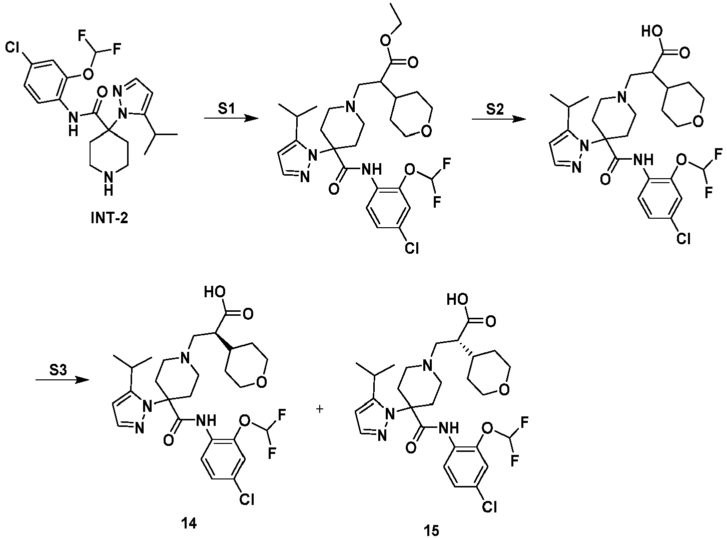

**Step 1: (ethyl 3-(4-((4-chloro-2-(difluoromethoxy)phenyl)carbamoyl)-4-(5-isopropyl-1H-pyrazol-1-yl)piperidin-1-yl)-2-(tetrahydro-2H-pyran-4-yl)propanoate.** To a solution of **INT-2** (100 mg, 0.242 mmol) dissolved in DCM (3 ml) was added ethyl 3-oxo-2-(tetrahydro-2H-pyran-4-yl)propanoate (73 mg, 0.363 mmol), and sodium triacetoxyborohydride (77 mg, 0.363 mmol). The resulting mixture was then stirred at 25 °C for 18 h. The resultant mixture was diluted with water (10 mL) and extracted with DCM (2 × 10 mL). The combined organic phase was dried over MgSO₄, filtered and the filtrate was concentrated under reduced pressure to provide a residue, which was purified by silica gel column chromatography using a gradient of 0-100% EA in heptanes to afford the title compound (120 mg, yield 83%). MS *m*/*z:* 597.4 [M+H]+.

**Step 2: *rac*-3-[4-[[4-chloro-2-(difluoromethoxy)phenyl]carbamoyl]-4-(5-isopropylpyrazol-1-yl)-1-piperidyl]-2-tetrahydropyran-4-yl-propanoic acid.** Ethyl 3-(4-((4-chloro-2-(difluoromethoxy)phenyl)carbamoyl)-4-(5-isopropyl-1*H*-pyrazol-1-yl)piperidin-1-yl)-2-(tetrahydro-2*H*-pyran-4-yl)propanoate (120 mg, 0.201 mmol) was dissolved in a mixture of tetrahydrofuran (3 mL), methanol (3 mL) and water (1 mL). Lithium hydroxide monohydrate (84 mg, 2.01 mmol) was added, and the mixture was stirred at 50°C for 18 hours. The resultant mixture was diluted with dichloromethane (10 mL) and extracted with half saturated aqueous ammonium chloride (2 × 5 mL). The combined organic phase was dried over MgSO₄, filtered and the filtrate was concentrated under reduced pressure to provide a residue, which was purified by silica gel column chromatography using a gradient of 0-20% methanol in dichloromethane to provide the racemic material (78 mg, yield 68%). ¹H NMR (600 MHz, dimethyl sulfoxide-*d*₆) δ ppm 8.14 (s, 1H), 7.80 (d, *J =* 9.4 Hz, 1H), 7.52 (t*, J* = 1.9 Hz, 1H), 7.32 (m, 2H), 7.15 (t, *J =* 73.1 Hz, 1H), 6.32 (s, 1H), 3.81 (m, 2H), 3.24 (m, 2H), 2.93 (m, 1H), 2.85 (m, 1H), 2.63 (m, 1H), 2.55 (m, 1H), 2.47 (m, 3H), 2.36 (m, 3H), 2.26 (m, 1H), 1.71 (m, 1H), 1.56 (m, 1H), 1.41 (m, 1H), 1.28 (m, 3H), 1.10 (d, *J =* 6.7 Hz, 6H). MS (APCI+) *m*/*z* 569.4 [M+H]⁺.

**Step 3: Compounds 14 and 15.** *rac*-3-[4-[[4-Chloro-2-(difluoromethoxy)phenyl]carbamoyl]-4-(5-isopropylpyrazol-1-yl)-1-piperidyl]-2-tetrahydropyran-4-yl-propanoic acid (75 mg) was subjected to SFC chiral separation employing a Whelk-O^{®} (S,S) column using a mixture of 7:3 acetontirile:methanol with 0.1% diethylamine added as the eluent to provide the title **Compound 14** (35 mg) as the first peak (material) to elute from the column. ¹H NMR (400 MHz, dimethyl sulfoxide-*d*₆) δ ppm 8.14 (s, 1H), 7.80 (d, *J =* 9.4 Hz, 1H), 7.52 (t, *J =* 1.9 Hz, 1H), 7.32 (m, 2H), 7.15 (t, *J =* 73.1 Hz, 1H), 6.32 (s, 1H), 3.81 (m, 2H), 3.24 (m, 2H), 2.91 (m, 1H), 2.86 (m, 1H), 2.63 (m, 1H), 2.51 (m, 4H), 2.34 (m, 4H), 1.72 (m, 1H), 1.56 (m, 1H), 1.41 (m, 1H), 1.29 (m, 3H), 1.10 (d, *J =* 6.7 Hz, 6H). MS (APCI+) *m*/*z* 569.4 [M+H]⁺. The second peak (material) to elute from the column provided the other title **Compound 15** (36 mg). ¹H NMR (400 MHz, dimethyl sulfoxide-*d*₆) δ ppm 8.14 (s, 1H), 7.81 (d, *J* = 9.4 Hz, 1H), 7.53 (t, *J* = 1.9 Hz, 1H), 7.32 (m, 2H), 7.15 (t, *J* = 73.1 Hz, 1H), 6.32 (s, 1H), 3.82 (m, 2H), 3.23 (m, 2H), 2.93 (m, 1H), 2.86 (m, 1H), 2.63 (m, 1H), 2.51 (m, 4H), 2.35 (m, 4H), 1.72 (m, 1H), 1.57 (m, 1H), 1.41 (m, 1H), 1.29 (m, 3H), 1.10 (d, *J =* 6.7 Hz, 6H); ¹³C NMR (101 MHz, dimethyl sulfoxide-*d*₆) δ ppm 175.57, 171.67, 151.56, 143.16, 138.88, 129.44, 128.18, 125.86, 125.06, 119.21, 119.05, 116.62, 114.03, 105.42, 67.41, 66.44, 57.70, 50.10, 49.19, 48.83, 36.09, 33.95, 33.87, 30.77, 25.87, 24.07; ¹⁹F NMR (565 MHz, dimethyl sulfoxide-*d*₆) δ ppm -82.13, -82.26. MS (APCI+) *m*/*z* 569.4 [M+H]⁺.

### Example #2: Compounds 61 and 62: (1s,3s)-3-[4-[[4-chloro-2-(difluoromethoxy)phenyl]carbamoyl]-4-(5-isopropylpyrazol-1-yl)-1-piperidyl]cyclobutanecarboxylic acid and (1r,3r)-3-[4-[[4-chloro-2-(difluoromethoxy)phenyl]carbamoyl]-4-(5-isopropylpyrazol-1-yl)-1-piperidyl]cyclobutanecarboxylic acid

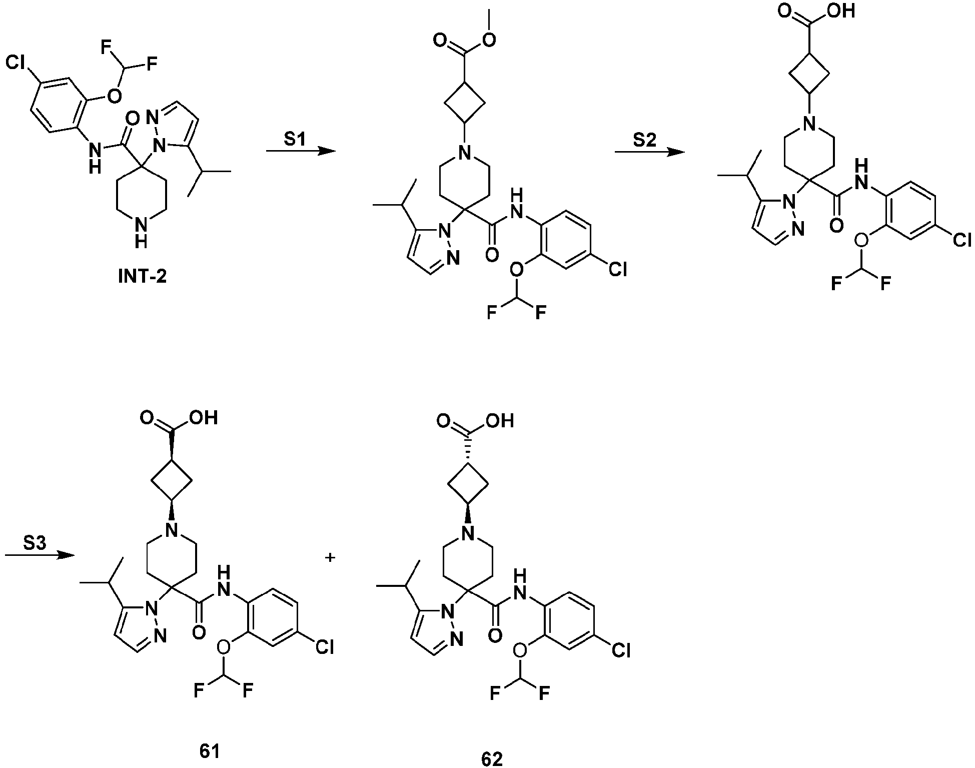

**Step 1: methyl 3-(4-((4-chloro-2-(difluoromethoxy)phenyl)carbamoyl)-4-(5-isopropyl-1H-pyrazol-1-yl)piperidin-1-yl)cyclobutane-1-carboxylate.** To a solution of **INT-2** (200 mg, 0.484 mmol) dissolved in DCE (4.5 ml) was then added methyl 3-oxocyclobutane-1-carboxylate (124 mg, 0.969 mmol), and sodium triacetoxyborohydride (154 mg, 0.727 mmol). The resulting mixture was then stirred at 25 °C for 18 h. The resultant mixture was diluted with water (10 mL), extracted with DCM (2 × 10 mL), the combined organic phase was dried over MgSO₄, filtered and the filtrate was concentrated under reduced pressure to provide the crude desired product as a residue, which was used directly in the next step (254 mg, yield 100%). MS *m*/*z:* 525.2 [M+H]+.

**Step 2: 3-(4-((4-chloro-2-(difluoromethoxy)phenyl)carbamoyl)-4-(5-isopropyl-1*H*-pyrazol-1-yl)piperidin-1-yl)cyclobutane-1-carboxylic acid.** To a solution of methyl 3-(4-((4-chloro-2-(difluoromethoxy)phenyl)carbamoyl)-4-(5-isopropyl-1*H*-pyrazol-1-yl)piperidin-1-yl)cyclobutane-1-carboxylate (254 mg, 0.484 mmol) dissolved in a mixture of tetrahydrofuran (3 mL), methanol (3 mL) and water (1 mL) was added lithium hydroxide monohydrate (204 mg, 4.84 mmol) and the mixture was stirred at ambient temperature for 18 hours. The resultant mixture was diluted with dichloromethane (10 mL) and extracted with half saturated aqueous ammonium chloride (2 × 5 mL). The combined organic phase was dried over MgSO₄, filtered and the filtrate was concentrated under reduced pressure to provide a residue, which was purified by silica gel column chromatography using a gradient of 0-20% methanol in dichloromethane to provide the title compound (130 mg, yield 53%). ¹H NMR (500 MHz, dimethyl sulfoxide-*d*₆) δ ppm 8.21 (bs, 1H), 7.77 (d, *J =* 6.2 Hz, 1H), 7.52 (d, *J =* 1.9 Hz, 1H), 7.32 (m, 2H), 7.16 (t, *J =* 73.2 Hz, 1H), 6.33 (d, *J =* 2.1 Hz, 1H), 3.35 (m, 4H), 2.92 (m, 1H), 2.69 (m, 2H), 2.58 (m, 1H), 2.52 (m, 2H), 2.37 (m, 2H), 2.22 (m, 2H), 1.95 (m, 1H), 1.10 (d, *J =* 6.6 Hz, 6H). MS (APCI+) *m*/*z*: 511.2 [M+H]⁺.

**Step 3: Compound 61 and 62.** 3-(4-((4-Chloro-2-(difluoromethoxy)phenyl)carbamoyl)-4-(5-isopropyl-1*H*-pyrazol-1-yl)piperidin-1-yl)cyclobutane-1-carboxylic acid (129 mg) was subjected to SFC separation employing a CHIRALPAK^{®} AD-H column using a mixture of 7:3 isopropanol:water with 0.1% diethylamine added as the eluent to provide the title **Compound 62** (9 mg, 6% yield). ¹H NMR (500 MHz, dimethyl sulfoxide-*d*₆) δ ppm 8.16 (s, 1H), 7.79 (d, *J =* 6.2 Hz, 1H), 7.51 (d, *J =* 1.9 Hz, 1H), 7.32 (m, 2H), 7.15 (t, *J =* 73.2 Hz, 1H), 6.32 (d, *J =* 2.1 Hz, 1H), 2.92 (m, 1H), 2.84 (m, 1H), 2.77 (m, 1H), 2.60 (m, 2H), 2.49 (m, 2H), 2.36 (m, 2H), 2.15 (m, 4H), 2.06 (m, 2H), 1.10 (d, *J =* 6.6 Hz, 6H); ¹³C NMR (101 MHz, dimethyl sulfoxide-*d*₆) δ ppm 170.81, 151.61, 143.63, 139.17, 129.83, 127.87, 125.80, 119.20, 119.00, 116.61, 114.02, 105.62, 65.30, 57.63, 45.57, 31.99, 28.62, 25.76, 24.07. ¹⁹F NMR (565 MHz, dimethyl sulfoxide-*d*₆) δ ppm -82.15, -82.29. MS *m*/*z:* (APCI+) 511.2 [M+H]⁺. The second peak to elute provided title **Compound 61** (79 mg, 61% yield) as the second peak (material) to elute from the column. ¹H NMR (400 MHz, dimethyl sulfoxide-*d*₆) δ ppm 8.15 (s, 1H), 7.80 (d, *J =* 6.2 Hz, 1H), 7.51 (d, *J =* 1.9 Hz, 1H), 7.32 (m, 2H), 7.15 (t, *J =* 73.2 Hz, 1H), 6.32 (d, *J =* 2.1 Hz, 1H), 2.93 (m, 1H), 2.67 (m, 1H), 2.58 (m, 4H), 2.49 (m, 1H), 2.36 (m, 2H), 2.20 (m, 4H), 1.90 (m, 2H), 1.10 (d, *J =* 6.6 Hz, 6H). MS (APCI+) *m*/*z:* 511.2 [M+H]⁺.

### Example #3: Compound 77: (1s,3s)-3-[4-[[4-chloro-2-(difluoromethoxy)phenyl]carbamoyl]-4-(5-isopropylpyrazol-1-yl)-1-piperidyl]-1-methyl-cyclobutanecarboxylic acid

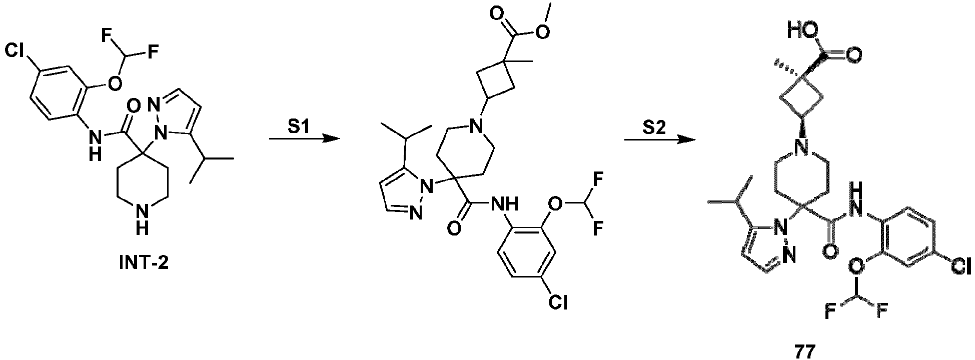

**Step 1: methyl 3-(4-((4-chloro-2-(difluoromethoxy)phenyl)carbamoyl)-4-(5-isopropyl-1H-pyrazol-1-yl)piperidin-1-yl)-1-methylcyclobutane-1-carboxylate.** To a solution of **INT-2** (200 mg, 0.484 mmol) dissolved in DCE (4.5 ml) was then added methyl 1-methyl-3-oxocyclobutane-1-carboxylate (103 mg, 0.727 mmol), and sodium triacetoxyborohydride (154 mg, 0.727 mmol). The resulting mixture was then stirred at 25 °C for 18 h. The resultant mixture was diluted with water (10 mL), extracted with dichloromethane (2 × 10 mL), the combined organic phase was dried over MgSO₄, filtered and the filtrate was concentrated under reduced pressure to provide the crude desired product as a residue, which was used directly in the next step (261 mg, yield 100%). MS *m*/*z*: 539.2 [M+H]+.

**Step 2: Compound 77.** To a solution of methyl 3-(4-((4-chloro-2-(difluoromethoxy)phenyl)carbamoyl)-4-(5-isopropyl-1H-pyrazol-1-yl)piperidin-1-yl)-1-methylcyclobutane-1-carboxylate (261 mg, 0.484 mmol) dissolved in a mixture of THF (3 mL), MeOH (3 mL) and water (1 mL) was then added lithium hydroxide monohydrate (203 mg, 4.84 mmol) and the solution stirred at rt for 18 h. The resultant mixture was diluted with dichloromethane (10 mL), extracted with half saturated aqueous ammonium chloride (2 × 5 mL), the combined organic phase was dried over MgSO₄, filtered and the filtrate was concentrated under reduced pressure to provide a residue, which was purified by silica gel column chromatography using a gradient of 0-20% MeOH in DCM to afford a mixture of cis and trans cyclobutane isomeric products (144 mg, yield 57%). This cis and trans isomeric mixture was then subjected to SFC chiral separation employing a ChiralPak IG column to provide 71 mg of the cis title compound as the major product. ¹H NMR (400 MHz, DMSO-d6) δ 8.15 (s, 1H), 7.81 (m, 1H), 7.52 (d, *J =* 1.8 Hz, 1H), 7.33 (d, *J =* 2.5 Hz, 1H), 7.31 (m, 1H), 7.15 (t, *J =* 73.3 Hz, 1H), 6.32 (d, *J =* 1.9 Hz, 1H), 2.92 (m, 1H), 2.69 (m, 1H), 2.60 (m, 2H), 2.51 (m, 3H), 2.35 (m, 2H), 2.15 (m, 3H), 1.87 (m, 2H), 1.29 (s, 3H), 1.10 (d, *J* = 6.6 Hz, 6H); ¹³C NMR (101 MHz, DMSO) δ 178.69, 171.71, 151.61, 143.16, 143.13, 138.89, 129.44, 128.25, 125.91, 125.06, 119.11, 116.65, 114.06, 105.43, 66.54, 54.02, 45.70, 36.45, 36.22, 33.58, 25.94, 24.19, 24.11; ¹⁹F NMR (565 MHz, DMSO) δ -82.11, -82.30; MS *m*/*z*: 525.0 [M+H]+.

### Example #4: Compound 84: 4-[[4-[[4-chloro-2-(difluoromethoxy)phenyl]carbamoyl]-4-(6,6-dimethyl-4H-furo[3,4-c]pyrazol-1-yl)-1-piperidyl]methyl]tetrahydropyran-4-carboxylic acid

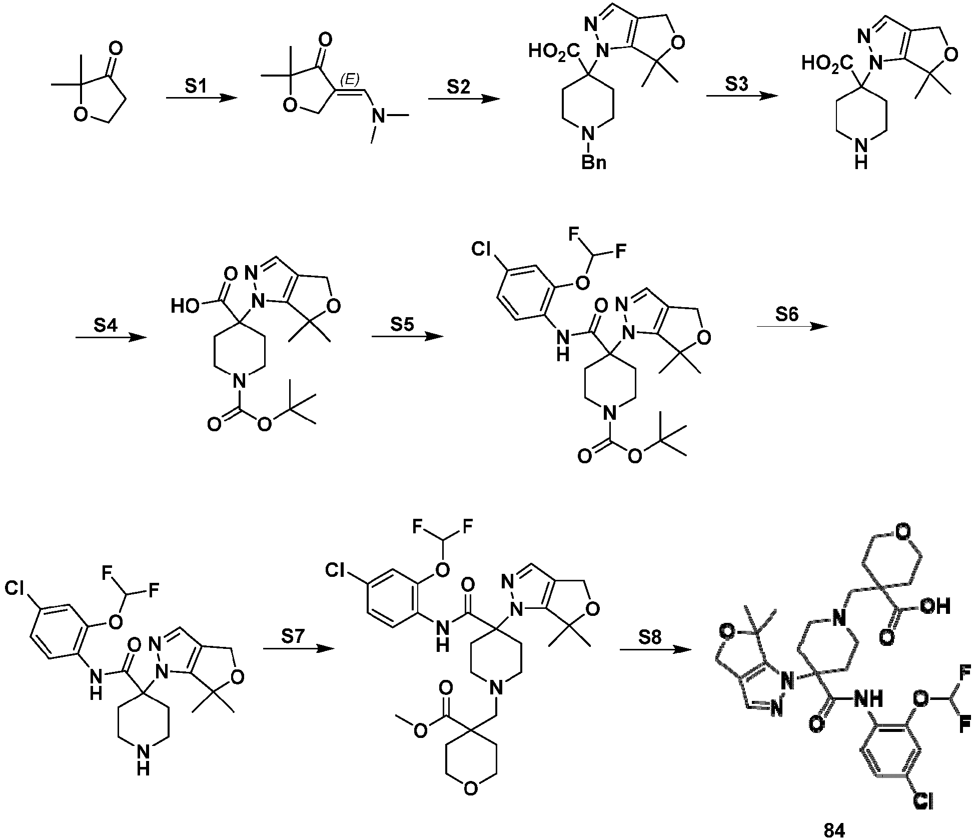

**Step 1: (E)-4-((dimethylamino)methylene)-2,2-dimethyldihydrofuran-3(2H)-one.** To mixture of 1,1-dimethoxy-N,N-dimethylmethanamine (34.1 ml, 250 mmol) and 2,2-dimethyldihydrofuran-3(2H)-one (25 g, 208 mmol) in dimethyl formamide (DMF) (416 ml) was stirred at 100°C for 18h. Then the mixture was then concentrated to give (E)-4-((dimethylamino)methylene)-2,2-dimethyldihydrofuran-3(2H)-one (37.14 g, 198 mmol, 95 % yield) as a dark brown solid.

**Step 2: 1-benzyl-4-(6,6-dimethyl-4,6-dihydro-1H-furo[3,4-c]pyrazol-1-yl)piperidine-4-carboxylic acid.** (E)-4-((dimethylamino)methylene)-2,2-dimethyldihydrofuran-3(2H)-one (34.16 g, 182 mmol), methyl 1-benzyl-4-hydazinylpiperidine-4-carboxylate dihydrochloride (Int-1, 2 HCl) (57.5 g, 151 mmol), and acetic acid (AcOH) (505 ml) was heated to 85°C for 3h. The solvent was then removed under reduced pressure. The crude product was purified by silica gel column chromatography, employing a gradient of 0-10% MeOH in DCM to afford the title product (34.19 g, 64.9% yield) as a colorless solid. ¹H NMR (500 MHz, DMSO-d6) δ 1.43 (s, 6H), 2.57 - 2.82 (m, 4H), 3.17 (m, 4H), 4.21 (s, 2H), 4.67 (s, 2H), 7.29 (s, 1H), 7.36 - 7.50 (m, 3H), 7.53 - 7.75 (m, 2H), 8.89 (s, 2H), COOH proton not observed; MS m/z: 356 [M+H]+.

**Step 3: 4-(6,6-dimethyl-4,6-dihydro-1H-furo[3,4-c]pyrazol-1-yl)piperidine-4-carboxylic acid.** 1-benzyl-4-(6,6-dimethyl-4,6-dihydro-1H-furo[3,4-c]pyrazol-1-yl)piperidine-4-carboxylic acid (34.91 g, 98 mmol), palladium on carbon (1.338 g, 12.57 mmol), and hydrogen chloride (4 M in dioxane) (24.55 ml, 98 mmol) in EtOH (1964 ml) was subjected to hydrogen gas at 5 bar and ambient temperature for 16 h. The mixture was then filtered through a glass fibre paper, and washed with methanol (6 x 50 mL). The filtrate was concentrated under reduced pressure to give crude 4-(6,6-dimethyl-4,6-dihydro-1H-furo[3,4-c]pyrazol-1-yl)piperidine-4-carboxylic acid as a hydrochloride salt (30 g, 100 mmol, 102 % yield). This material was used directly in the next reaction. MS *m*/*z:* 266 [M+H]+.

**Step 4: 1-(tert-butoxycarbonyl)-4-(6,6-dimethyl-4,6-dihydro-1H-furo[3,4-c]pyrazol-1-yl)piperidine-4-carboxylic acid.** The crude material 4-(6,6-dimethyl-4,6-dihydro-1H-furo[3,4-c]pyrazol-1-yl)piperidine-4-carboxylic acid hydrochloride salt was dissolved in DCM (492 ml), N-ethyl-N-isopropylpropan-2-amine (86 ml, 492 mmol) and di-tert-butyl dicarbonate (25.8 g, 118 mmol) were then added. The resulting reaction was stirred at ambient temperature for 18 h. The mixture was then acidified with aqueous hydrochloric acid (1M) and the two phases were separated. The organic layer was washed with brine, dried with magnesium sulfate filtered and concentrated under reduced pressure. The crude product was used directly in the next step. ¹H NMR (400 MHz, DMSO) δ 1.42 (d, *J =* 16.1 Hz, 15H), 2.20 (ddd, *J =* 13.5, 8.9, 3.9 Hz, 2H), 2.23 - 2.38 (m, 2H), 3.24 (d, *J* = 8.8 Hz, 2H), 3.52 - 3.70 (m, 2H), 4.66 (s, 2H), 7.24 (s, 1H), 13.41 (s, 1H). MS *m*/*z*: 366 [M+H]+.

**Step 5: tert-butyl 4-((4-chloro-2-(difluoromethoxy)phenyl)carbamoyl)-4-(6,6-dimethyl-4,6-dihydro-1H-furo[3,4-c]pyrazol-1-yl)piperidine-1-carboxylate.** To a solution of 1-(tert-butoxycarbonyl)-4-(6,6-dimethyl-4,6-dihydro-1H-furo[3,4-c]pyrazol-1-yl)piperidine-4-carboxylic acid (35.9 g, 98 mmol) and 4-chloro-2-(difluoromethoxy)aniline (20.02 g, 98 mmol) in Acetonitrile (491 ml) was added 1-methyl-1H-imidazole (39.2 ml, 491 mmol). After stiring at r.t for 10 min, the flask was placed into a ambient temperature water bath (due to reaction exotherm) and N-(chloro(dimethylamino)methylene)-N-methylmethanaminium hexafluorophosphate(V) (41.3 g, 147 mmol) was added in one portion. The resultant mixture was stirred at ambient temperature for 18h. The mixture was then concentrated under reduced pressure, then partitioned between a mixture of dichloromethane (600 mL) and water (400 mL). The aqueous phase was extracted with dichloromethane (2 x 400 mL), the combined organic layers were washed with brine, dried with magnesium sulfate, filtered and concentrated under reduced pressure. The resultant residue was purified by silica gel chromatography employing 0-60% methyl tert-butyl ether in heptanes to afford the title product (33.80 g, 62.5 mmol, 63.6 % yield). ¹H NMR (400 MHz, DMSO) δ 1.40 (d, *J* = 8.1 Hz, 15H), 2.22 - 2.33 (m, 2H), 2.46 (s, 2H), 3.05 (s, 2H), 3.83 (d, *J =* 13.6 Hz, 2H), 4.67 (d, *J =* 13.1 Hz, 2H), 7.01 - 7.40 (m, 3H), 7.50 (s, 1H), 7.92 (d, *J =* 8.8 Hz, 1H), 7.98 (s, 1H). MS (ESI) m/z 485 (M-tBu+H)⁺.

**Step 6: N-(4-chloro-2-(difluoromethoxy)phenyl)-4-(6,6-dimethyl-4,6-dihydro-1H-furo[3,4-c]pyrazol-1-yl)piperidine-4-carboxamide.** To a stirred solution of tert-butyl 4-((4-chloro-2-(difluoromethoxy)phenyl)carbamoyl)-4-(6,6-dimethyl-4,6-dihydro-1H-furo[3,4-c]pyrazol-1-yl)piperidine-1-carboxylate (33.8 g, 62.5 mmol) in acetonitrile (417 ml) was added Hydrochloric acid (4M in dioxane) (82 ml, 328 mmol) and the solution was stirred at ambient temperature for 18 h. The solution was then concentrated under vacuum to provide N-(4-chloro-2-(difluoromethoxy)phenyl)-4-(6,6-dimethyl-4,6-dihydro-1H-furo[3,4-c]pyrazol-1-yl)piperidine-4-carboxamide hydrochloride (29.5 g, 61.8 mmol, 99 % yield) as a light yellow solid. ¹H NMR (500 MHz, DMSO) δ 1.39 (s, 6H), 2.64 (s, 4H), 3.03 (s, 2H), 3.38 (d, *J =* 6.5 Hz, 2H), 4.70 (s, 2H), 7.05 - 7.36 (m, 2H), 7.37 (d, *J =* 2.0 Hz, 1H), 7.55 (s, 1H), 7.88 (dd, *J =* 8.6, 1.8 Hz, 1H), 8.04 (s, 1H), 9.23 (s, 2H). MS (ESI) *m*/*z:* 441 (M+H)⁺.

**Step 7: methyl 4-((4-((4-chloro-2-(difluoromethoxy)phenyl)carbamoyl)-4-(6,6-dimethyl-4,6-dihydro-1H-furo[3,4-c]|pyrazol-1-yl)piperidin-1-yl)methyl)tetrahydro-2H-pyran-4-carboxylate.** N-(4-chloro-2-(difluoromethoxy)phenyl)-4-(6,6-dimethyl-4,6-dihydro-1H-furo[3,4-c]pyrazol-1-yl)piperidine-4-carboxamide (free base) (19.87 g, 45.1 mmol) was dissolved in dichloroethane (376 ml) then added methyl 4-formyltetrahydro-2H-pyran-4-carboxylate (12.93 g, 67.6 mmol), and sodium triacetoxyborohydride (14.33 g, 67.6 mmol) then stirred the mixture at ambient temperature for 18 h. Then added an aqueous saturated sodium carbonate solution (200 mL) and extracted the solution with DCM (500 mL). The aqueous layer was extracted again with DCM (200 mL), then the combined DCM layers were washed with brine, dried over Na₂SO₄, conc and purified by silica gel column chromatography eluting with 0-60% ethyl acetate in heptanes to provide the title product (24.2 g, 90% yield) as a pink oil. ¹H NMR (400 MHz, MeOD) δ 1.48 (s, 6H), 1.61 (ddd, *J =* 13.6, 11.1, 4.5 Hz, 2H), 2.09 (dq, *J =* 12.4, 3.5 Hz, 2H), 2.47 (d, *J =* 8.8 Hz, 4H), 2.56 (s, 4H), 2.80 (dd, *J =* 7.9, 4.1 Hz, 2H), 3.50 (td, *J =* 11.5, 2.3 Hz, 2H), 3.76 (s, 3H), 3.83 (dt, *J* = 11.9, 3.9 Hz, 2H), 4.81 (s, 2H), 6.88 (t, *J =* 72.8 Hz, 1H), 7.15 - 7.28 (m, 2H), 7.49 (s, 1H), 8.14 - 8.25 (m, 1H), NH proton not seen; MS *m*/*z*: 597.5 [M+H]+.

**Step 8: Compound 84.** methyl 4-((4-((4-chloro-2-(difluoromethoxy)phenyl)carbamoyl)-4-(6,6-dimethyl-4,6-dihydro-1H-furo[3,4-c]pyrazol-1-yl)piperidin-1-yl)methyl)tetrahydro-2H-pyran-4-carboxylate (24.15 g, 40.4 mmol) was dissolved in a mixture of tetrahydrofuran (173 ml), methanol (173 ml) and water (57.8 ml) then added lithium hydroxide monohydrate (13.58 g, 324 mmol) and the mixture stirred at 50°C for 18 h. The reaction mixture was then concentrated to remove most of the organic solvent, then added DCM (1000 mL), adjusted the mixture pH to 3 by adding 2.5 N HCl (approx.. 130 mL). Then separated the two layers, and the aqueous layer was extracted with DCM (2 x 500 mL), combined organic extracts were dried over Na₂SO₄, filtered and concentrated then purified by silica gel chromatography, eluting with 0 to 10% MeOH in DCM, to afford compound 84 (4-((4-((4-chloro-2-(difluoromethoxy)phenyl)carbamoyl)-4-(6,6-dimethyl-4,6-dihydro-1H-furo[3,4-c]pyrazol-1-yl)piperidin-1-yl)methyl)tetrahydro-2H-pyran-4-carboxylic acid) (18.0 g, 30.9 mmol, 76 % yield). ¹H NMR (400 MHz, DMSO-d6) δ 8.04 (s, 1H), 7.80 (m, 1H), 7.49 (s, 1H), 7.35 (m, 1H), 7.20 (t, *J =* 73.0 Hz, 1H), 4.69 (s, 2H), 3.70 (m, 2H), 3.38 (m, 3H), 2.77 (m, 2H), 2.49 (m, 4H), 2.36 (m, 4H), 1.88 (m, 2H), 1.46 (m, 2H), 1.38 (s, 6H); ¹³C NMR (101 MHz, DMSO) δ 177.21, 170.90, 163.57, 154.02, 141.62, 141.59, 141.56, 132.74, 128.96, 128.79, 128.36, 126.07, 123.24, 119.24, 119.14, 116.54, 113.94, 80.83, 66.92, 65.76, 64.74, 63.25, 51.02, 45.74, 32.67, 32.56, 27.46; ¹⁹F NMR (565 MHz, DMSO) δ -82.80, -82.99; MS *m*/*z*: 583 [M+H]+.

### Example #5: Compound 169: (2R)-2-[[4-[[4-chloro-2-(difluoromethoxy)phenyl]carbamoyl]-4-(4-fluoro-5-isopropyl-pyrazol-1-yl)-1-piperidyl]methyl]-3-methoxy-propanoic acid

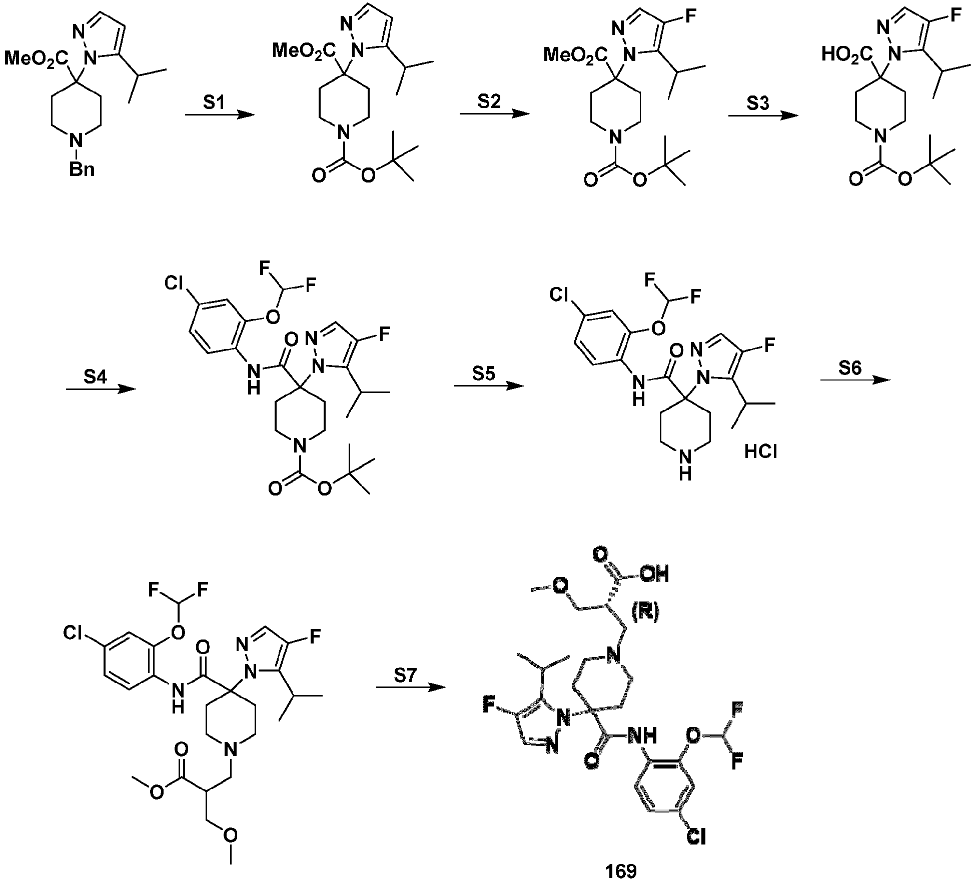

**Step 1: 1-(tert-butyl) 4-methyl 4-(5-isopropyl-1H-pyrazol-1-yl)piperidine-1,4-dicarboxylate.** A mixture of methyl 1-benzyl-4-(5-isopropyl-1H-pyrazol-1-yl)piperidine-4-carboxylate (17.9 g, 47.2 mmol) (the product of Step 2 in the preparation of INT-2 described above), MeOH (100 mL), conc hydrochloric acid (4 mL, 48.0 mmol) and palladium on carbon (JM Type 39L) (1.5 g, 1.410 mmol) was placed in a stainless steel pressure reactor and stirred under hydrogen atmosphere (5 bar) for 3 h. The mixture was filtered and the filtrate concentrated under reduced pressure. The residue was dissolved in DCM (200 mL) and di-tert-butyl dicarbonate (14.24 mL, 61.3 mmol) and DIPEA (24.72 mL, 142 mmol) were added sequentially. The mixture was stirred at ambient temperature for 18 h. The mixture was then transferred to a separatory funnel and washed with 1N hydrochloric acid (200 mL) and brine (200 mL). The organic layer was dried over magneisum sulfate, filtered and the solvent removed under reduced pressure. The residue was purified by silica gel chromatography eluting with 0-100% methyl tert-butyl ether in heptanes to afford the titled compound (16.86 g, 98 % yield) as a colorless oil which solidified on standing. MS (ESI) m/z 296.2 (M-tBu+H)⁺.

**Step 2: 1-(tert-butyl) 4-methyl 4-(4-fluoro-5-isopropyl-1H-pyrazol-1-yl)piperidine-1,4-dicarboxylate.** To a stirred mixture of 1-tert-butyl 4-methyl 4-(5-isopropyl-1H-pyrazol-1-yl)piperidine-1,4-dicarboxylate (3.02 g, 8.16 mmol) in MeCN (60 mL) was added Selectfluor^{™} (4.92 g, 13.88 mmol). The mixture was stirred at ambient temperature for 66 h. The mixture was quenched with saturated aqueous sodium bicarbonate (80 mL). The aqueous phase was extracted with ethyl acetate (3 x 50 mL), the combined organics were dried over magnesium sulfate, filtered and concentrated under reduced pressure. The crude product was purified by silica gel chromatography eluting with 0-100% methyl tert-butyl ether in heptanes, to afford 1-tert-butyl 4-methyl 4-(4-fluoro-5-isopropyl-1H-pyrazol-1-yl)piperidine-1,4-dicarboxylate (1.58 g, 45 % yield) as a pale yellow oil. MS (ESI) m/z 314.2 (M-tBu+H)+.

**Step 3: 1-(tert-butoxycarbonyl)-4-(4-fluoro-5-isopropyl-1H-pyrazol-1-yl)piperidine-4-carboxylic acid.** To a stirred solution of 1-tert-butyl 4-methyl 4-(4-fluoro-5-isopropyl-1H-pyrazol-1-yl)piperidine-1,4-dicarboxylate (1.90 g, 4.37 mmol) in MeOH (15 mL) and THF (30 mL) was added 2M aqueous lithium hydroxide (11 mL, 22.00 mmol). The reaction mixture was stirred at 35 °C for 2 h. The reaction mixture was then cooled to ambient temperature and concentrated under reduced pressure. The aqueous phase was diluted with water (80 mL) then carefully acidified with 1M HCl (aq) to a pH of 3. The aqueous phase was then extracted with ethyl acetate (3 x 80 mL). The combined organics were dried over magnesium sulfate, filtered and concentrated under reduced pressure. The crude product was purified by silica gel chromatography eluting with 0-10% ethyl acetate in heptanes to afford a pale yellow solid. The solid was purified further by trituration in ice-cold, 10% methyl tert-butyl ether:isohexane (10 mL). The solid was filtered and washed with ice-cold, 10% methyl tert-butyl ether:isohexane (2 x 5 mL) to afford the title product (1.0 g, 61 % yield) as a white solid. MS (ESI) m/z 300.2 (M-tBu+H)⁺.

**Step 4: tert-butyl 4-((4-chloro-2-(difluoromethoxy)phenyl)carbamoyl)-4-(4-fluoro-5-isopropyl-1H-pyrazol-1-yl)piperidine-1-carboxylate.** To a stirred solution of 1-(tert-butoxycarbonyl)-4-(4-fluoro-5-isopropyl-1H-pyrazol-1-yl)piperidine-4-carboxylic acid (0.620 g, 1.657 mmol) and 4-chloro-2-(difluoromethoxy)aniline (0.394 g, 1.934 mmol) in acetonitrile (6 mL) was added 1-methylimidazole (0.47 mL, 5.84 mmol). N-(chloro(dimethylamino)methylene)-N-methylmethanaminium hexafluorophosphate(V) (0.697 g, 2.486 mmol) was then added in one portion. An exotherm was observed and the solution was stirred at ambient temperature for 18 h. The mixture was partitioned between dichloromethane (20 mL) and water (50 mL). The aqueous phase was extracted with dichloromethane (2 x 20 mL), the combined organics were dried with magnesium sulfate, filtered and concentrated under reduced pressure. The crude product was purified by silica gel chromatography eluting with 0-100% methyl tert-butyl ether in heptanes to afford the title product (1.0 g, 97 % yield) as a colorless oil. MS (ESI) m/z 431.0 (M-Boc+H)⁺.

**Step 5: N-(4-chloro-2-(difluoromethoxy)phenyl)-4-(4-fluoro-5-isopropyl-1H-pyrazol-1-yl)piperidine-4-carboxamide.** To a stirred solution of tert-butyl 4-((4-chloro-2-(difluoromethoxy)phenyl)carbamoyl)-4-(4-fluoro-5-isopropyl-1H-pyrazol-1-yl)piperidine-1-carboxylate (1.0 g, 1.601 mmol) in acetonitrile (11 mL) was added 4M hydrogen chloride in dioxane (2.1 ml, 8.40 mmol) and the solution was stirred at ambient temperature for 1.5 h. The resulting suspension was cooled in an ice bath, the solid was filtered then washed with ice-cold tert-butyl methyl ether (2 x 5 mL) to yield N-(4-chloro-2-(difluoromethoxy)phenyl)-4-(4-fluoro-5-isopropyl-1H-pyrazol-1-yl)piperidine-4-carboxamide, hydrochloric acid (0.715 g, 1.454 mmol, 91 % yield) as a white solid. MS (ESI) m/z 431.0 (M+H)+.

**Step 6: methyl 3-(4-((4-chloro-2-(difluoromethoxy)phenyl)carbamoyl)-4-(4-fluoro-5-isopropyl-1H-pyrazol-1-yl)piperidin-1-yl)-2-(methoxymethyl)propanoate.** N-(4-chloro-2-(difluoromethoxy)phenyl)-4-(4-fluoro-5-isopropyl-1H-pyrazol-1-yl)piperidine-4-carboxamide, hydrochloric acid (2.0 g, 4.28 mmol) was dissolved in dichloromethane (25 ml) followed by the addition of DBU (1,8-diazabicyclo[5.4.0]undec-7-ene) (1.935 ml, 12.84 mmol) at 25 °C. Then methyl 2-(methoxymethyl)acrylate (1.466 g, 10.70 mmol) CAS [25328-81-8] was added and the mixture stirred at ambient temperature for 18 h. The solution was then diluted with saturated aqueous sodium bicarbonate (25 mL) and then extracted (3 x 25 mL) with dichloromethane, combined and dried over magnesium sulfate, filtered and concentrated. The resultant residue was subjected to a silica gel chromatography eluting with 0-75% ethyl acetane in heptanes to afford the title product as a viscous light yellow oil (2.17 g, 90 % yield). MS *m*/*z*: 561.9 [M+H]+.

**Step 7: Compound 169.** To methyl 3-(4-((4-chloro-2-(difluoromethoxy)phenyl)carbamoyl)-4-(4-fluoro-5-isopropyl-1H-pyrazol-1-yl)piperidin-1-yl)-2-(methoxymethyl)propanoate (2.17 g, 3.87 mmol) dissolved in THF (30 ml), MeOH (30.0 ml) and Water (10.00 ml) was added lithium hydroxide (0.926 g, 38.7 mmol) and the turbid mixture stirred at ambient temperature for 3h. The mixture was then diluted with saturated aqueous ammonium chloride solution (25 mL) then extracted (3 x 25 mL) with dichloromethane. The combined organic extracts were then dried over MgSO4, filtered and concentrated. The resultant residue was subjected to silica gel chromatography eluting with 0-30% MeOH in DCM to afford the recemic product as a colorless solid (1.65 g, 78 % yield). This racemic mixture was then subjected to SFC chiral separation employing a ChiralPak IC column to provide 595 mg of the chiral title compound. ¹H NMR (400 MHz, DMSO-d6) δ 8.64 (s, 1H), 7.62 (m, 1H), 7.57 (d, *J =* 4.1 Hz, 1H), 7.34 (m, 1H), 7.33 (m, 1H), 7.17 (t, *J =* 73.4 Hz, 1H), 3.46 (m, 2H), 3.20 (s, 3H), 2.99 (m, 1H), 2.78 (m, 2H), 2.69 (m, 1H), 2.53 (m, 2H), 2.39 (m, 6H), 1.16 (d, *J* = 6.8 Hz, 6H); ¹³C NMR (101 MHz, DMSO) δ 174.68, 171.12, 149.80, 147.37, 144.64, 134.16, 130.29, 127.92, 126.94, 126.14, 125.78, 119.33, 119.07, 116.75, 114.17, 71.91, 66.82, 58.63, 56.60, 49.37, 43.35, 33.61, 25.16, 21.02; ¹⁹F NMR (565 MHz, DMSO) δ -81.81, -82.00, -172.02; MS *m*/*z*: 547.2 [M+H]+.

### Example #6: Compound 36: 6-[[4-[[4-chloro-2-(difluoromethoxy)phenyl]carbamoyl]-4-(5-isopropylpyrazol-1-yl)-1-piperidyl]methyl]pyridine-2-carboxylic acid

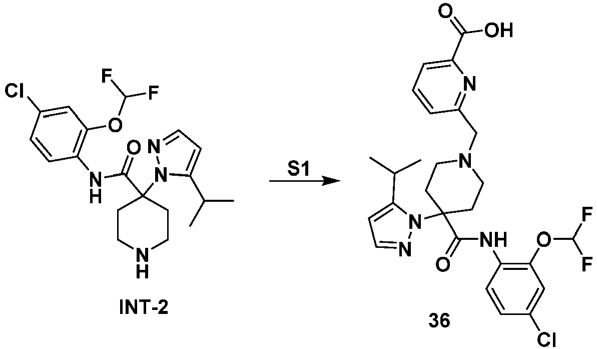

**Step 1: Compound 36.** To a 4 mL vial was added **INT-2** (1 eq., 0.07 mmol, 30 mg) in 1:1 dichloromethane/methanol (0.6 mL), followed by a solution of methyl 6-formylpicolinate (1 equivalent, 0.09 mmol, 14.5 mg) in 1:1 dichloromethane/methanol (0.6 mL), acetic acid (5 eq, 0.36 mmol, 21 µL) and MP-CNBH₃ resin (96 mg, 3 equivalents, 2.28 loading). The reaction mixture was placed to stir at room temperature overnight. Once the reaction was completed, the resin was filtered through an Isolute^{®} single fritted reservoir, and the reaction residue was dried. The dried residue was then dissolved in 1:1 dimethyl sulfoxide/methanol and purified by preparative HPLC on a Phenomenex^{®} Luna^{®} C8(2) 5 µm 100Å AXIA column (30 mm × 75 mm). A gradient of acetonitrile (A) and 10 mM ammonium acetate in water (B) was used. To the purified sample was added 1 mL of tetrahydrofuran and 1 mL of ethanol and lithium hydroxide (4 equivalents, 0.28 mmol, 280 µL) and the mixture was stirred at room temperature overnight. The reaction mixture was quenched with 1 mL of saturated aqueous NH₄Cl and extracted with 3 mL of 3:1 CHCl₃: IPA using an Isolute^{®} 6 mL phase separator to provide the title compound. ¹H NMR (400 MHz, dimethyl sulfoxide-*d₆*) δ ppm 7.92 (d, *J* = 9.4 Hz, 1H), 7.89 - 7.79 (m, 2H), 7.60 (d, *J* = 7.2 Hz, 1H), 7.52 (d, *J* = 1.9 Hz, 1H), 7.30 - 7.25 (m, 2H), 7.14 (t, *J* = 73.2 Hz, 1H), 6.29 (d, *J* = 1.9 Hz, 1H), 3.62 (s, 2H), 3.04 - 2.96 (m, 3H), 2.76 (q, *J* = 9.3 Hz, 2H), 2.61 - 2.52 (m, 2H), 2.47 (m, 2H), 1.11 (d, *J* = 6.7 Hz, 6H). MS (APCI) *m*/*z:* 548.3 [M+H]⁺.

### Example #7: Compound 37: 2-[[4-[[4-chloro-2-(difluoromethoxy)phenyl]carbamoyl]-4-(5-isopropylpyrazol-1-yl)-1-piperidyl]methyl]thiazole-4-carboxylic acid

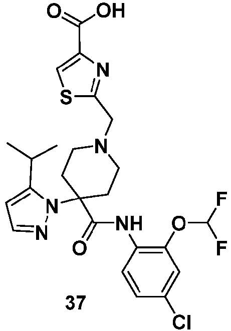

**Compound 37** was prepared according to the procedure used for the preparation of **Example #36,** substituting methyl 2-formylthiazole-4-carboxylate for methyl 6-formylpicolinate. ¹H NMR (400 MHz, dimethyl sulfoxide-*d*₆) δ ppm 8.17 (s, 1H), 7.93 - 7.88 (m, 1H), 7.52 (d, *J* = 1.8 Hz, 1H), 7.31 - 7.26 (m, 2H), 7.15 (t, *J* = 73.2 Hz, 1H), 6.29 (d, *J* = 1.9 Hz, 1H), 3.80 (s, 2H), 3.00 - 2.95 (m, 3H), 2.85 (dd, *J* = 10.5, 5.7 Hz, 2H), 2.59 (t, *J* = 9.8 Hz, 4H), 1.11 (d, *J* = 6.7 Hz, 6H). MS (APCI) *m*/*z*: 554.3 [M+H]⁺.

### Example #8: Compound 49: 2-[1-[[4-[[4-chloro-2-(difluoromethoxy)phenyl]carbamoyl]-4-(5-isopropylpyrazol-1-yl)-1-piperidyl]methyl]cyclopropyl]acetic acid

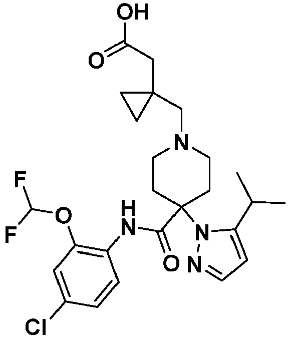

A mixture of **INT-2** (100 mg, 0.242 mmol), methyl 2-(1-(bromomethyl)cyclopropyl)acetate (75 mg, 0.363 mmol), cesium carbonate (395 mg, 1.211 mmol), and potassium iodide (8.04 mg, 0.048 mmol) in tetrahydrofuran (2 mL) was stirred at 60 °C overnight. Potassium trimethylsilanolate (93 mg, 0.727 mmol) was added and the mixture was stirred at reflux for 1 hour before concentrating the reaction under reduced pressure. The residue was purified via preparative HPLC on a Phenomenex^{®} Luna^{®} C8(2) 5 µm 100Å AXIA column (30 mm × 75 mm) using a gradient of acetonitrile and 0.1% trifluoroacetic acid in water to provide the title compound. ¹H NMR (600 MHz, dimethyl sulfoxide-*d*₆) δ ppm 7.82 - 7.78 (m, 1H), 7.55 7.51 (m, 1H), 7.34 - 7.31 (m, 2H), 7.16 (t, *J* = 73.2 Hz, 1H), 6.33 (d, *J* = 1.8 Hz, 1H), 2.94 (p, *J* = 6.8 Hz, 1H), 2.91 - 2.85 (m, 2H), 2.56 - 2.49 (m, 4H), 2.47 - 2.41 (m, 2H), 2.35 (s, 2H), 2.28 (s, 2H), 1.10 (d, *J =* 6.7 Hz, 6H), 0.45 - 0.41 (m, 2H), 0.37 - 0.33 (m, 2H). MS (ESI+) *m*/*z:* 525.0 [M+H]⁺.

### Example #9: Compound 150: (2S)-3-[4-[[4-chloro-2-(difluoromethoxy)phenyl]carbamoyl]-4-(5-isopropylpyrazol-1-yl)-1-piperidyl]-2-morpholino-propanoic acid

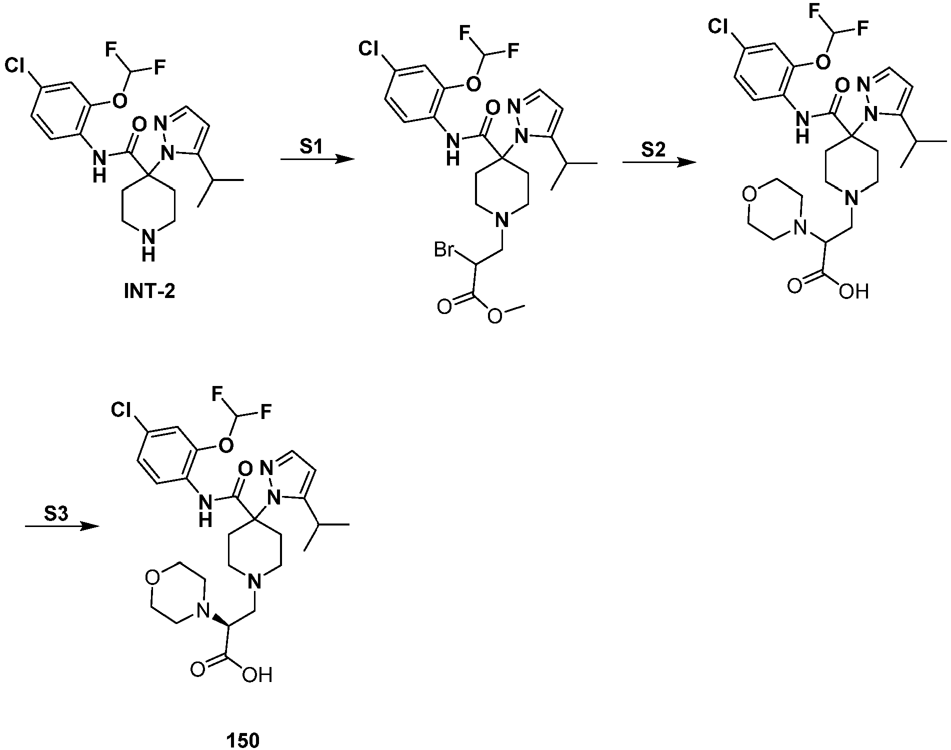

**Step 1: methyl 2-bromo-3-(4-((4-chloro-2-(difluoromethoxy)phenyl)carbamoyl)-4-(5-isopropyl-1*H*-pyrazol-1-yl)piperidin-1-yl)propanoate.** To **INT-2** (200 mg, 0.484 mmol) in dichloromethane (3 mL) was added methyl 2-bromoacrylate (120 mg, 0.727 mmol) followed by 1,8-diazabicyclo[5.4.0]undec-7-ene (118 mg, 0.775 mmol). The mixture stirred at ambient temperature for 2 hours before diluting with water (3 mL) and collecting the organic phase. The aqueous phase was washed three times with dichloromethane (3 mL) and the combined organics were dried over magnesium sulfate, filtered, and concentrated under reduced pressure. The residue was purified by flash column chromatography (ethyl acetate in heptanes) to provide the title compound. MS (APCI+) *m*/*z*: 577.0, 579.0 [M+H]⁺.

**Step 2: 3-(4-((4-chloro-2-(difluoromethoxy)phenyl)carbamoyl)-4-(5-isopropyl-1*H-*pyrazol-1-yl)piperidin-1-yl)-2-morpholinopropanoic acid.** To methyl 2-bromo-3-(4-((4-chloro-2-(difluoromethoxy)phenyl)carbamoyl)-4-(5-isopropyl-1*H*-pyrazol-1-yl)piperidin-1-yl)propanoate (80 mg, 0.138 mmol) in dry acetonitrile (2 mL) was added morpholine (0.060 mL, 0.692 mmol) and tripotassium phosphate (88 mg, 0.415 mmol). The mixture was stirred at ambient temperature for 18 hours before diluting with water (5 mL) and collecting the organic phase. The aqueous phase was then washed three times with dichloromethane (3 mL) and the combined organics were dried over magnesium sulfate, filtered, and concentrated under reduced pressure. The residue was dissolved in a mixture of tetrahydrofuran (1.5 mL), methanol (1.5 mL), and water (0.5 mL) before adding lithium hydroxide monohydrate (57.5 mg, 1.370 mmol). The mixture was stirred at ambient temperature for 60 hours before adding saturated aqueous ammonium chloride (3 mL) and collecting the organic phase. The aqueous phase was then washed three times with dichloromethane (3 mL) and the combined organics were dried over magnesium sulfate, filtered, and concentrated under reduced pressure. The residue was purified by flash column chromatography (methanol in dichloromethane) to provide the title compound. MS (APCI+) *m*/*z*: 570.8 [M+H]⁺.

**Step 3: Compound 150.** 3-(4-((4-chloro-2-(difluoromethoxy)phenyl)carbamoyl)-4-(5-isopropyl-1*H*-pyrazol-1-yl)piperidin-1-yl)-2-morpholinopropanoic acid (59.5 mg) was separated by preparative chiral supercritical fluid chromatography on CHIRALPAK^{®} IG column, (30 x 250 mm, 5 µm) at ambient temperature, using a mobile phase of 20% ethanol (0.2% triethylamine):CO₂. Fractions were collected based on UV detection at 220 nm with peaks detected at 9.2 minutes and 9.7 minutes) to provide two enantiomers. The second-eluting enantiomer was re-purified by flash column chromatography (methanol in dichloromethane) and the absolute stereochemistry was arbitrarily assigned to provide the title compound. ¹H NMR (400 MHz, dimethyl sulfoxide-*d*₆) δ ppm 8.19 (s, 1H), 7.82 - 7.75 (m, 1H), 7.53 (d, *J* = 1.8 Hz, 1H), 7.37 - 6.92 (m, 3H), 6.33 (d, *J* = 1.8 Hz, 1H), 3.57 - 3.49 (m, 4H), 3.30 (dd, *J* = 9.0, 5.5 Hz, 1H), 2.99 - 2.85 (m, 2H), 2.79 - 2.69 (m, 2H), 2.62 - 2.56 (m, 4H), 2.49 - 2.31 (m, 8H), 1.10 (d, *J =* 6.6 Hz, 6H). MS (APCI+) *m*/*z*: 570.8 [M+H]⁺.

### Example #10: Compound 179: 5-[[4-[[4-chloro-2-(difluoromethoxy)phenyl]carbamoyl]-4-(5-isopropylpyrazol-1-yl)-1-piperidyl]methyl]-1-methyl-triazole-4-carboxylic acid

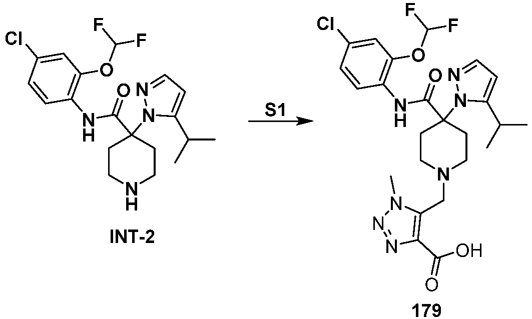

To a 4 mL vial was added **INT-2** (1 equivalent, 0.10 mmol, 42 mg) in buffer solution (0.1 M) [buffer solution (1 M): 36 g of sodium acetate, 900 mL of methanol, 88-100 mL of acetic acid, pH= 4-5] followed by the methyl 5-formyl-1-methyl-1*H*-1,2,3-triazole-4-carboxylate (1.5 eq, 0.15 mmol, 26 mg) in buffer solution (0.3 M) and MP- CNBH₃ resin (1 equivalent, 42 mg, 2.44 loading). The vial was allowed to stir at room temperature overnight. Upon completion, the reaction mixture was concentrated under reduced pressure and 75% lithium hydroxide in methanol was added. The mixture was concentrated under reduced pressure, dissolved in 1:1 dimethyl sulfoxide/methanol and purified by reverse phase HPLC. Samples were purified by preparative HPLC in a gradient of acetonitrile and 10 mM ammonium acetate in water to provide the title compound. ¹H NMR (400 MHz, dimethyl sulfoxide-*d*₆) δ ppm 7.90 (d, *J =* 9.4 Hz, 1H), 7.53 (d, *J* = 1.8 Hz, 1H), 7.33 - 7.26 (m, 2H), 7.14 (t, *J =* 73.2 Hz, 1H), 6.30 (d, *J =* 1.9 Hz, 1H), 4.03 (s, 3H), 3.94 (s, 2H), 2.76 (m, 3H), 2.55 (m, *J =* 10.4 Hz, 4H), 2.42 (m, 2H), 1.10 (d, *J =* 6.7 Hz, 6H). MS (APCI) *m*/*z*: 552.4.

### Example #11: Compound 192: (2R,5S)-5-[4-[[4-chloro-2-(difluoromethoxy)phenyl]carbamoyl]-4-(5-isopropylpyrazol-1-yl)-1-piperidyl]tetrahydropyran-2-carboxylic acid

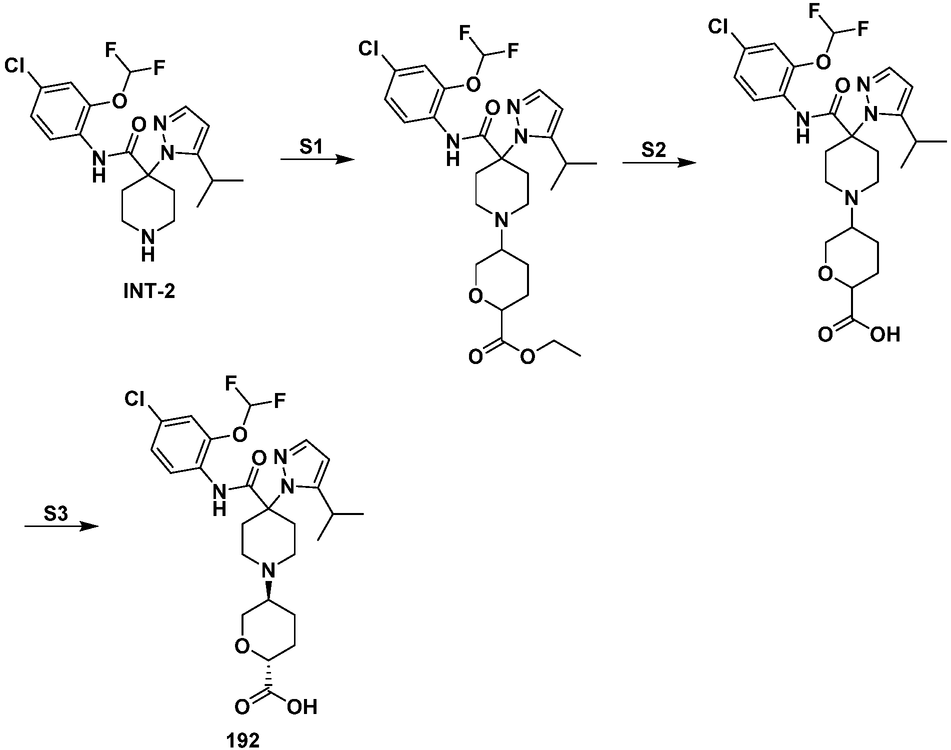

**Step 1: ethyl 5-(4-((4-chloro-2-(difluoromethoxy)phenyl)carbamoyl)-4-(5-isopropyl-1*H*-pyrazol-1-yl)piperidin-1-yl)tetrahydro-2*H*-pyran-2-carboxylate.** To **INT-2** (500 mg, 1.208 mmol) dissolved in 1,2-dichloroethane (6 mL) was added ethyl 5-oxotetrahydro-2*H*-pyran-2-carboxylate (520 mg, 3.02 mmol), and the mixture was stirred at ambient temperature for 1 hour before adding sodium triacetoxyborohydride (640 mg, 3.02 mmol). The mixture was stirred for 18 hours. The reaction mixture was then concentrated under reduced pressure to provide a residue which was purified via preparative HPLC using a gradient of acetonitrile and 10 mM ammonium acetate in water to provide the title compound. MS (ESI+) *m*/*z*: 569.2 [M+H]⁺.

**Step 2: 5-(4-((4-chloro-2-(difluoromethoxy)phenyl)carbamoyl)-4-(5-isopropyl-1*H-*pyrazol-1-yl)piperidin-1-yl)tetrahydro-2*H*-pyran-2-carboxylic acid.** To crude ethyl 5-(4-((4-chloro-2-(difluoromethoxy)phenyl)carbamoyl)-4-(5-isopropyl-1*H*-pyrazol-1-yl)piperidin-1-yl)tetrahydro-2*H*-pyran-2-carboxylate (600 mg, 1.054 mmol) in tetrahydrofuran (6 mL) was added potassium trimethylsilanolate (406 mg, 3.16 mmol) and the reaction was stirred at ambient temperature for 18 hours before concentration under reduced pressure. The residue was purified via preparative HPLC using a gradient of acetonitrile and 10 mM ammonium acetate in water to provide the title compound.

**Step 3: Compound 192.** 5-(4-((4-Chloro-2-(difluoromethoxy)phenyl)carbamoyl)-4-(5-isopropyl-1*H*-pyrazol-1-yl)piperidin-1-yl)tetrahydro-2*H*-pyran-2-carboxylic acid (453 mg) was separated by preparative chiral supercritical fluid chromatography on CHIRALPAK^{®} IC column, (30 x 250 mm, 5 µm) at ambient temperature, using a mobile phase of 40% ethanol (0.25% triethylamine):CO₂. Fractions were collected based on UV detection at 220 nm with peaks detected at 2.1 minute, 3.6 minute, 5.4 minute, and 8.9 minute) to provide four diastereomers. The third eluting diastereomer was re-purified by flash column chromatography (methanol in dichloromethane) and the diastereomer was arbitrarily assigned to provide the title compound. ¹H NMR (600 MHz, dimethyl sulfoxide-*d*₆) δ ppm 8.13 (s, 1H), 7.84 - 7.76 (m, 1H), 7.52 (d, *J* = 1.9 Hz, 1H), 7.34 - 7.29 (m, 2H), 7.15 (t, *J* = 73.2 Hz, 1H), 6.31 (d, *J* = 1.9 Hz, 1H), 3.99 - 3.91 (m, 1H), 3.71 - 3.65 (m, 1H), 3.20 (t, *J* = 10.6 Hz, 1H), 2.98 - 2.88 (m, 1H), 2.81 - 2.74 (m, 2H), 2.61 - 2.43 (m, 4H), 2.40 - 2.25 (m, 3H), 1.98 - 1.88 (m, 2H), 1.45 - 1.37 (m, 2H), 1.09 (d, *J* = 6.7 Hz, 6H). MS (ESI+) *m*/*z*: 541.0 [M+H]⁺.

### Example #12: Compound 76: 1-[[4-[[4-chloro-2-(difluoromethoxy)phenyl]carbamoyl]-4-(5-isopropylpyrazol-1-yl)-1-piperidyl]methyl]cyclopropanecarboxylic acid

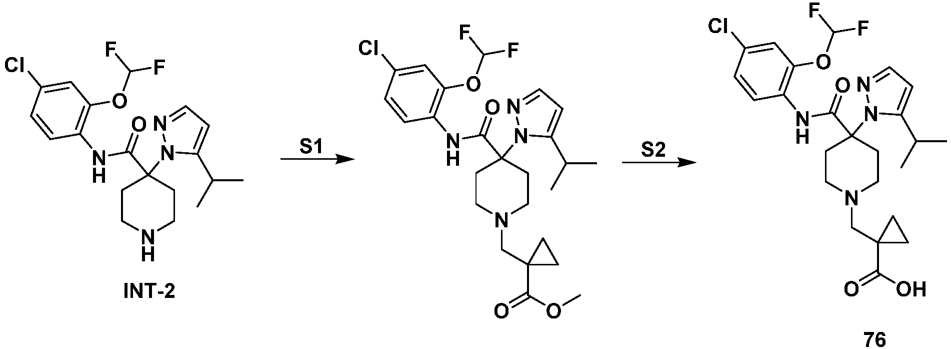

**Step 1: methyl 1-((4-((4-chloro-2-(difluoromethoxy)phenyl)carbamoyl)-4-(5-isopropyl-1*H*-pyrazol-1-yl)piperidin-1-yl)methyl)cyclopropanecarboxylate.** To a solution of **INT-2** (100 mg, 0.242 mmol) and methyl 1-formylcyclopropanecarboxylate (46.6 mg, 0.363 mmol) in 1,2-dichloroethane (2 mL) stirring at ambient temperature for 10 minutes was added sodium triacetoxyhydroborate (77 mg, 0.363 mmol) and the reaction was stirred 18 hours at room temperature. The reaction mixture was then concentrated under reduced pressure to provide a residue which was purified via preparative HPLC using a gradient of acetonitrile and 10 mM ammonium acetate in water to provide the title compound. MS (ESI+) *m*/*z:* 525.0 [M+H]⁺.

**Step 2:** Compound **76.** To a solution of methyl 1-((4-((4-chloro-2-(difluoromethoxy)phenyl)carbamoyl)-4-(5-isopropyl-1*H*-pyrazol-1-yl)piperidin-1-yl)methyl)cyclopropanecarboxylate (110 mg, 0.210 mmol) in dioxane (2 mL) was added potassium trimethylsilanolate (81 mg, 0.629 mmol) and the reaction was stirred overnight at 60 °C before concentrating under reduced pressure. The residue was purified via preparative HPLC using a gradient of acetonitrile and 10 mM ammonium acetate in water to provide the title compound. ¹H NMR (600 MHz, dimethyl sulfoxide-*d*₆) δ ppm 7.75 (d, *J* = 9.1 Hz, 1H), 7.56 - 7.52 (m, 1H), 7.36 - 7.31 (m, 2H), 7.16 (t, *J* = 73.2 Hz, 1H), 6.34 (d, *J* = 1.9 Hz, 1H), 3.03 - 2.96 (m, 2H), 2.96 - 2.90 (m, 1H), 2.67 - 2.59 (m, 6H), 2.59 - 2.52 (m, 2H), 2.49 - 2.38 (m, 1H), 1.11 (d, *J* = 6.7 Hz, 6H), 1.04 (q, *J =* 3.7 Hz, 2H), 0.67 (q, *J* = 3.8 Hz, 2H). MS (ESI+) *m*/*z*: 511.0 [M+H]⁺.

### Example #13: Compounds 137 and 140: (2S,4S)-4-[4-[[4-chloro-2-(difluoromethoxy)phenyl]carbamoyl]-4-(5-isopropylpyrazol-1-yl)-1-piperidyl]tetrahydrofuran-2-carboxylic acid and (2S,4R)-4-[4-[[4-chloro-2-(difluoromethoxy)phenyl]carbamoyl]-4-(5-isopropylpyrazol-1-yl)-1-piperidyl]tetrahydrofuran-2-carboxylic acid

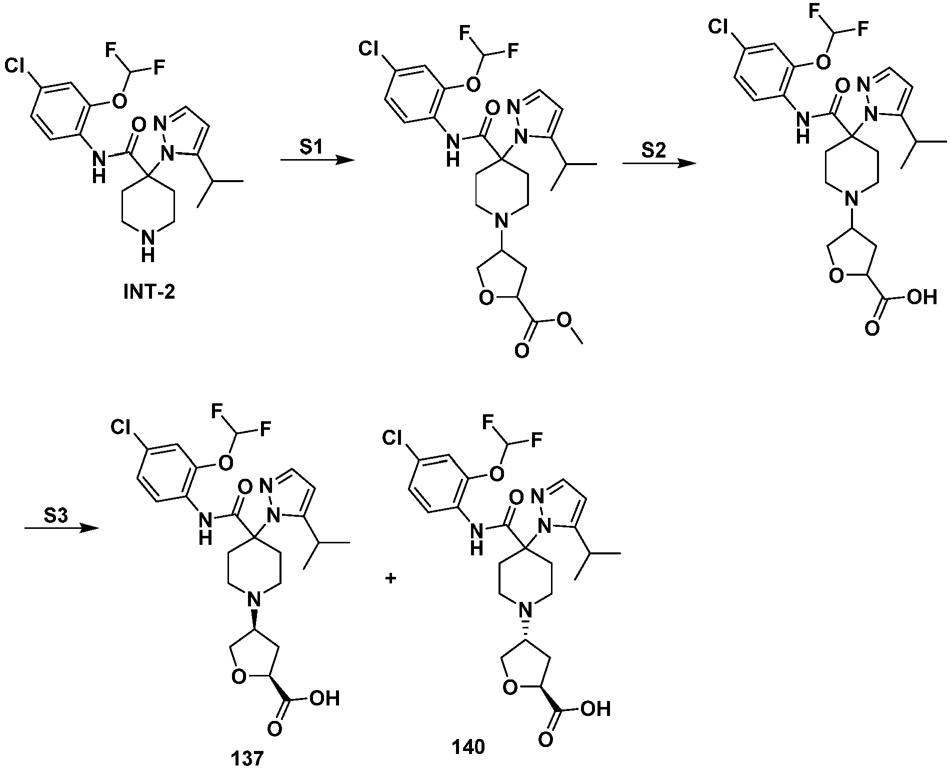

**Step 1: methyl 4-(4-((4-chloro-2-(difluoromethoxy)phenyl)carbamoyl)-4-(5-isopropyl-1*H*-pyrazol-1-yl)piperidin-1-yl)tetrahydrofuran-2-carboxylate.** To a solution of **INT-2** (500 mg, 1.211 mmol) in 1,2-dichloroethane (6 mL) was added methyl 4-oxotetrahydrofuran-2-carboxylate (0.207 mL, 1.817 mmol) and the mixture was stirred at ambient temperature for 18 hours before adding sodium triacetoxyborohydride (385 mg, 1.817 mmol). After stirring for 2 hours at ambient temperature, an additional quantity of sodium triacetoxyborohydride (385 mg, 1.817 mmol) was added and the reaction mixture was stirred for an additional 18 hours at ambient temperature. The reaction mixture was concentrated under reduced pressure and purified on a column using ethyl acetate in heptanes, to provide the title compound. MS (ESI+) *m*/*z*: 541.0 [M+H]⁺.

**Step 2: 4-(4-((4-chloro-2-(difluoromethoxy)phenyl)carbamoyl)-4-(5-isopropyl-1*H-*pyrazol-1-yl)**piperidin**-1-yl)tetrahydrofuran-2-carboxylic acid.** To crude methyl 4-(4-((4-chloro-2-(difluoromethoxy)phenyl)carbamoyl)-4-(5-isopropyl-1*H*-pyrazol-1-yl)piperidin-1-yl)tetrahydrofuran-2-carboxylate (655 mg, 1.211 mmol) in tetrahydrofuran (6 mL) was added potassium trimethylsilanolate (466 mg, 3.63 mmol) and the reaction was stirred at ambient temperature for 18 hours before concentrating under reduced pressure. The residue was purified via preparative HPLC using a gradient of acetonitrile and 10 mM ammonium acetate in water to provide title compound. MS (ESI+) *m*/*z*: 511.0 [M+H]⁺.

**Step 3: Compounds 137 and 140.** 4-(4-((4-Chloro-2-(difluoromethoxy)phenyl)carbamoyl)-4-(5-isopropyl-1*H*-pyrazol-1-yl)piperidin-1-yl)tetrahydrofuran-2-carboxylic acid (250 mg) was separated by preparative chiral supercritical fluid chromatography on CHIRALPAK^{®} IC column, (30 x 250 mm, 5 µm) at ambient temperature, using a mobile phase of 15% ethanol (0.1% triethylamine):CO₂. The sample was dissolved in a 1:1:1 ratio of acetonitrile/water/isopropanol at a concentration of 23 mg/mL and the injection volume was 0.4 mL. Fractions were collected based on UV detection at 254 nm with peaks detected at 54.1 minutes, 63.9 minutes, 73.0 minutes, and 88.3 minutes) to provide four diastereomers. The third-eluting peak (**Compound 137,** 73.0 minutes) and fourth-eluting peak (**Compound 140,** 88.3 minutes) were separated, and each was separately re-purified by flash column chromatography (methanol in dichloromethane) to provide **Compound 137** and **Compound 140.** The absolute stereochemistry of both compounds was arbitrarily assigned to provide the title compounds. **Compound 137:** ¹H NMR (400 MHz, dimethyl sulfoxide-*d*₆) δ ppm 8.18 (s, 1H), 7.87 - 7.72 (m, 1H), 7.53 (d, *J* = 1.9 Hz, 1H), 7.36 - 6.94 (m, 3H), 6.33 (d, *J* = 1.9 Hz, 1H), 4.31 (t, *J* = 7.8 Hz, 1H), 3.86 (dd, *J* = 8.5, 6.3 Hz, 1H), 3.65 (t*, J* = 7.9 Hz, 1H), 2.97 - 2.73 (m, 3H), 2.71 - 2.59 (m, 1H), 2.49 - 2.31 (m, 7H), 1.84 - 1.73 (m, 1H), 1.10 (d, *J =* 6.6 Hz, 6H). MS (ESI+) *m*/*z*: 527.0 [M+H]⁺. **Compound 140:** ¹H NMR (500 MHz, dimethyl sulfoxide-*d*₆) δ ppm 8.20 (s, 1H), 7.80 - 7.74 (m, 1H), 7.53 (d, *J* = 1.9 Hz, 1H), 7.38 - 6.94 (m, 3H), 6.33 (d, *J* = 1.9 Hz, 1H), 4.42 (t, *J* = 6.6 Hz, 1H), 3.94 (dd, *J* = 8.6, 6.6 Hz, 1H), 3.71 - 3.59 (m, 1H), 2.97 - 2.88 (m, 1H), 2.77 (m, 1H), 2.69 - 2.47 (m, 5H), 2.45 - 2.31 (m, 3H), 2.10 - 2.07 (m, 2H), 1.10 (d, *J* = 6.6 Hz, 6H). MS (ESI+) *m*/*z:* 527.2 [M+H]⁺.

### Example #14: Compound 154: (2R)-3-[4-[[4-chloro-2-(difluoromethoxy)phenyl]carbamoyl]-4-(5-isopropylpyrazol-1-yl)-1-piperidyl]-2-(1-methylpyrazol-4-yl)propanoic acid

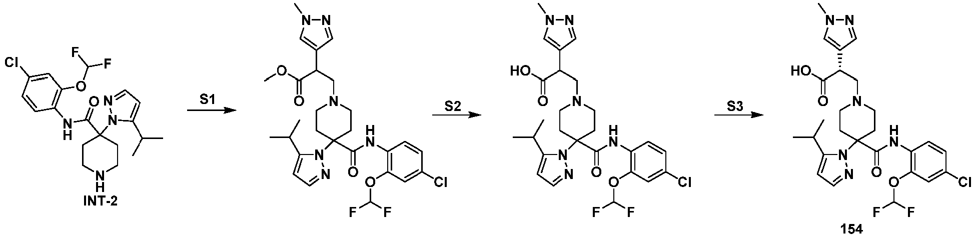

**Step 1: methyl 3-(4-((4-chloro-2-(difluoromethoxy)phenyl)carbamoyl)-4-(5-isopropyl-1*H*-pyrazol-1-yl)piperidin-1-yl)-2-(1-methyl-1*H*-pyrazol-4-yl)propanoate.** To *N*-(4-Chloro-2-(difluoromethoxy)phenyl)-4-(5-isopropyl-1*H*-pyrazol-1-yl)piperidine-4-carboxamide (**INT-2,** 313 mg, 0.758 mmol) in dichloromethane (8 mL) was added 1-methyl-2,3,4,6,7,8-hexahydro-1*H*-pyrimido[1,2-*a*]pyrimidine (186 mg, 1.213 mmol) and methyl 2-(1-methyl-1*H-*pyrazol-4-yl)acrylate (126 mg, 0.758 mmol). The reaction was stirred at ambient temperature for 60 hours before concentrating under reduced pressure. The residue was purified with flash column chromatography using ethyl acetate in heptanes, to provide the title compound. MS (ESI+) *m*/*z*: 579.0 [M+H]⁺.

**Step 2: 3-(4-((4-chloro-2-(difluoromethoxy)phenyl)carbamoyl)-4-(5-isopropyl-1*H-*pyrazol-1-yl)piperidin-1-yl)-2-(1-methyl-1*H*-pyrazol-4-yl)propanoic acid.** To methyl 3-(4-((4-chloro-2-(difluoromethoxy)phenyl)carbamoyl)-4-(5-isopropyl-1*H*-pyrazol-1-yl)piperidin-1-yl)-2-(1-methyl-1*H*-pyrazol-4-yl)propanoate (204 mg, 0.282 mmol) in tetrahydrofuran (3 mL) was added potassium trimethylsilanolate (108 mg, 0.846 mmol) and the reaction was stirred at 60 °C for 15 minutes. The mixture was concentrated, and the residue was purified with flash column chromatography (methanol in dichloromethane) to provide title compound. MS (ESI+) *m*/*z*: 565.0 [M+H]⁺.

**Step 3: Compound 154.** 3-(4-((4-Chloro-2-(difluoromethoxy)phenyl)carbamoyl)-4-(5-isopropyl-1*H*-pyrazol-1-yl)piperidin-1-yl)-2-(1-methyl-1*H*-pyrazol-4-yl)propanoic acid (133 mg) was separated by preparative chiral supercritical fluid chromatography on Regis Whelk-O^{®}1 (S,S) column, (30 x 250 mm, 5 µm) at ambient temperature, using a mobile phase of 30% ethanol (0.1% triethylamine):CO₂. Fractions were collected based on UV detection at 220 nm with peaks detected at 5.2 minutes and 7.5 minutes) to provide two enantiomers. The second-eluting enantiomer was re-purified by flash column chromatography (methanol in dichloromethane) and the absolute stereochemistry was arbitrarily assigned to provide the title compound. ¹H NMR (500 MHz, dimethyl sulfoxide-*d*₆) δ ppm 8.18 (s, 1H), 7.83 - 7.77 (m, 1H), 7.55 (s, 1H), 7.52 (d, *J* = 1.9 Hz, 1H), 7.34 - 6.97 (m, 4H), 6.32 (d, *J* = 1.8 Hz, 1H), 3.77 (s, 3H), 3.70 (dd, *J* = 10.4, 5.2 Hz, 1H), 2.98 - 2.82 (m, 3H), 2.77 - 2.66 (m, 1H), 2.61 - 2.44 (m, *J =* 3.2 Hz, 3H), 2.42 - 2.31 (m, 4H), 1.10 (dd, *J =* 6.7, 2.0 Hz, 6H). MS (ESI+) *m*/*z*: 565.0 [M+H]⁺.

### Example #15: Compound 32: (2R)-3-[4-[[4-chloro-2-(difluoromethoxy)phenyl]carbamoyl]-4-(5-isopropylpyrazol-1-yl)-1-piperidyl]-2-(3-methoxyphenyl)propanoic acid

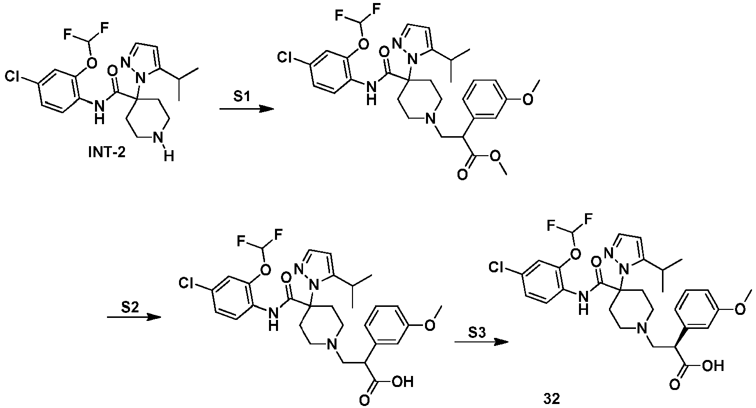

**Step 1: methyl 3-(4-((4-chloro-2-(difluoromethoxy)phenyl)carbamoyl)-4-(5-isopropyl-1*H*-pyrazol-1-yl)piperidin-1-yl)-2-(3-methoxyphenyl)propanoate.** A mixture of *N-*(4-chloro-2-(difluoromethoxy)phenyl)-4-(5-isopropyl-1*H*-pyrazol-1-yl)piperidine-4-carboxamide (**INT-2**) (0.40 g, 0.969 mmol), methyl 2-(4-methoxyphenyl)acrylate (0.363 g, 1.89 mmol) and DBU (2,3,4,6,7,8,9,10-octahydropyrimido[1,2-*a*]azepine, 0.292 mL, 1.94 mmol) were added to a 40 mL pressure vial. Dichloromethane (10 mL) was added. The mixture was stirred under nitrogen atmosphere at 25 °C for 18 hours. The reaction mixture was diluted with saturated aqueous NaHCO₃ (5 mL) and extracted with dichloromethane (2 x 5 mL). The organic layer was washed with brine (1 x 10 mL), and the combined organic extracts were dried over MgSO₄, filtered and concentrated under reduced pressure. The residue was purified by silica gel chromatography with a gradient of ethyl acetate in heptanes to provide the title compound. MS (ESI) *m*/*z:* 605 [M]⁺.

**Step 2: *rac*-3-(4-((4-chloro-2-(difluoromethoxy)phenyl)carbamoyl)-4-(5-isopropyl-1H-pyrazol-1-yl)piperidin-1-yl)-2-(3-methoxyphenyl)propanoic acid.** To methyl 3-(4-((4-chloro-2-(difluoromethoxy)phenyl)carbamoyl)-4-(5-isopropyl-1*H*-pyrazol-1-yl)piperidin-1-yl)-2-(3-methoxyphenyl)propanoate (0.476 g, 0.787 mmol) was added a mixture of tetrahydrofuran (5 mL) and methanol (5 mL) followed by the addition of lithium hydroxide (2.36 mL of 1M solution, 2.36 mmol). The reaction mixture was stirred at 25 °C for 1.5 hours. Saturated aqueous NH₄Cl solution (10 mL) was added, and the reaction mixture was extracted with dichloromethane (3 x 10 mL). The combined organic extracts were dried over MgSO₄, filtered and concentrated to give the racemic title compound. MS (APCI+) *m*/*z*: 591 [M+H]⁺.

**Step 3: Compound 32.** The resultant racemic compound from **Step 2** (0.359 g) was subjected to preparative Supercritical Fluid Chromatography (SFC) chiral separation employing a CHIRALPAK^{®} AD-H column, (30 x 250 mm, 5 µm) at 35 °C utilizing a mobile phase of 25% ethanol (containing 0.1% diethylamine): CO₂. Fractions were collected based on UV detection at 220 nm with peaks detected at 2.98 minutes and 4.67 minutes to provide the two enantiomers. The first eluting enantiomer peak (A) at 2.98 minutes was collected and concentrated. This material was re-purified on silica gel chromatography (methanol in dichloromethane) to remove traces of the diethylamine SFC additive to provide the title compound **32.** Absolute stereochemistry for the title compound was arbitrarily assigned as (*R*), >99% ee. ¹H NMR (400 MHz, dimethyl sulfoxide-*d₆*) δ ppm 7.82 (s, 1H), 7.58 - 7.42 (m, 1H), 7.33 - 7.22 (m, 3H), 7.15 (t, 73.3 Hz, 1H), 7.06 (d, *J* = 6.0 Hz, 1H), 6.87 (dd, *J* = 4.5, 2.4 Hz, 3H), 6.80 (ddd, *J* = 8.3, 2.5, 1.0 Hz, 1H), 6.28 (d, *J* = 1.9 Hz, 1H), 3.78 - 3.74 (m, 3H), 3.67 - 3.57 (m, 2H), 3.24 - 3.04 (m, 5H), 2.98 - 2.87 (m, 4H), 2.75 (d, *J* = 5.2 Hz, 1H), 1.16 - 1.09 (m, 6H). MS (APCI+) *m*/*z:* 591 [M+H]⁺.

### Example #16: Compounds 38 and 39: (2R)-2-[[4-[[4-chloro-2-(difluoromethoxy)phenyl]carbamoyl]-4-(5-isopropylpyrazol-1-yl)-1-piperidyl]methyl]tetrahydropyran-2-carboxylic acid and (2S)-2-[[4-[[4-chloro-2-(difluoromethoxy)phenyl]carbamoyl]-4-(5-isopropylpyrazol-1-yl)-1-piperidyl]methyl]tetrahydropyran-2-carboxylic acid

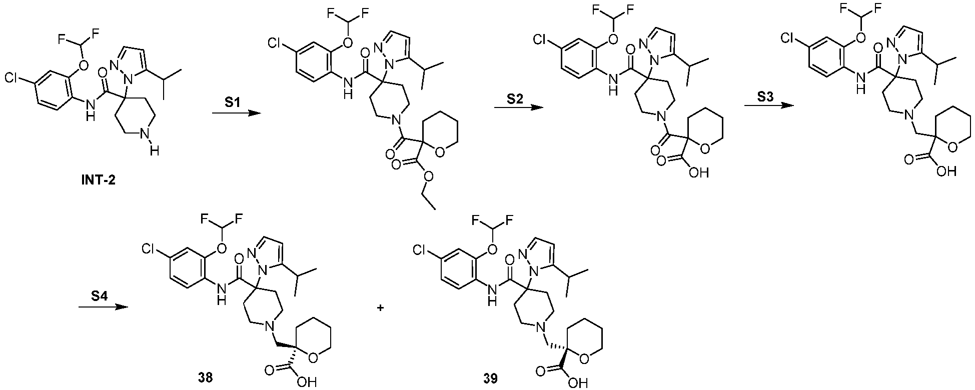

**Step 1: ethyl 2-(4-((4-chloro-2-(difluoromethoxy)phenyl)carbamoyl)-4-(5-isopropyl-1*H*-pyrazol-1-yl)piperidine-1-carbonyl)tetrahydro-2*H*-pyran-2-carboxylate.** A mixture of*N*-(4-chloro-2-(difluoromethoxy)phenyl)-4-(5-isopropyl-1*H*-pyrazol-1-yl)piperidine-4-carboxamide (**INT-2**), (01.25 g, 0.303 mmol), 2-(ethoxycarbonyl)tetrahydro-2*H*-pyran-2-carboxylic acid (0.064 g, 0.303 mmol), and HATU (2-(3*H*-[1,2,3]triazolo[4,5-*b*]pyridin-3-yl)-1,1,3,3-tetramethylisouronium hexafluorophosphate(V) (0.138 g, 0.363 mmol) were combined in a 20 mL pressure vial and charged with *N,N-*dimethylformamide (5 mL). *N*-Ethyl-*N*-isopropylpropan-2-amine (0.159 mL, 0.908 mmol) was added, and the mixture was stirred at 25 °C for 17 hours. The reaction mixture was diluted with water (15 mL) and extracted with dichloromethane (2 x 15 mL). The organics were separated and dried with MgSO₄, filtered and concentrated under reduced pressure. The residue was purified by silica gel chromatography with ethyl acetate in heptanes to give the title compound. MS (APCI+) *m*/*z*: 597 [M]⁺.

**Step 2: 2-(4-((4-chloro-2-(difluoromethoxy)phenyl)carbamoyl)-4-(5-isopropyl-1*H-*pyrazol-1-yl)piperidine-1-carbonyl)tetrahydro-2*H*-pyran-2-carboxylic acid.** To the product from **Step 1** (0.136 g, 0.228 mmol) was added a solvent mixture of tetrahydrofuran (5 mL) and methanol (5 mL) followed by the addition of lithium hydroxide (1.14 mL of 1M solution, 1.14 mmol). The reaction mixture was stirred at 50 °C for 15 hours, diluted with saturated aqueous NH₄Cl solution (10 mL) and extracted with dichloromethane (3 x 10 mL). The combined organic extracts were dried over MgSO₄, filtered and concentrated to give the title compound. MS (APCI+) *m*/*z*: 568 [M]⁺.

**Step 3: 2-((4-((4-chloro-2-(difluoromethoxy)phenyl)carbamoyl)-4-(5-isopropyl-1*H-*pyrazol-1-yl)piperidin-1-yl)methyl)tetrahydro-2*H*-pyran-2-carboxylic acid.** To a mixture of the product from **Step 2** (0.068 g, 0.120 mmol) in a 20 mL pressure vial was added Vaska's Complex^{®} (carbonylchlorobis(triphenylphosphine)iridium(I)) (0.014 g, 0.018 mmol) and 1,2-dichloroethane (3 mL). To this was added 1,1,3,3-tetramethyldisiloxane (0.161g, 1.195 mmol) with noted gas evolution, dropwise at 25 °C for 17 hours. The reaction mixture was quenched with trifluoroacetic acid (0.028 mL, 0.359 mmol) and stirred for 1 hour. The reaction mixture was concentrated under reduced pressure and the resulting residue was dissolved in dichloromethane (5 mL). Water was added (5 mL) and the biphasic aqueous mixture was made basic with 2N aqueous NaOH, to pH ~ 9-10. Saturated aqueous NH₄Cl solution (15 mL) and additional dichloromethane (10 mL) were added, and the organics were dried over MgSO₄, filtered and concentrated to give the crude product. Purification was accomplished utilizing a Reverse Phase column with a mobile phase of acetonitrile with 0.1% trifluoracetic acid to give the racemic title compound. MS (ESI+) *m*/*z*: 555 [M+ H]⁺.

**Step 4: Compounds 38 and 39** The racemic product from **Step 3** (0.055 g, 0.099 mmol) was separated by preparative chiral supercritical fluid chromatography on a CHIRAL ART Cellulose-SC (IC) column, (30 x 250 mm, 5 µm) at 35 °C, using a mobile phase of 30% ethanol (0.2% diethylamine):CO₂. Fractions were collected based on UV detection at 220 nm with peaks detected at 4.07 minutes (A) and 7.39 minutes (B) to provide the resolved enantiomer peaks, A and B. Each separate enantiomer peak was concentrated to dryness then was re-purified by silica gel chromatography to remove residual diethylamine additive from the SFC run utilizing a silica gel column, eluting with methanol in dichloromethane to provide the chiral **Compound 38** from peak A. Absolute stereochemistry was arbitrarily assigned as (*R*) with an ee > 99.4%. ¹H NMR (400 MHz, dimethyl sulfoxide-*d*₆) δ ppm 8.01 (s, 1H), 7.82 (d, *J =* 9.4 Hz, 1H), 7.56 (d, *J =* 1.9 Hz, 1H), 7.44 - 7.14 (m, 2H), 7.07 (s, 1H), 6.88 (s, 0H), 6.34 (d, *J =* 1.9 Hz, 1H), 3.81 (qt, *J =* 11.8, 5.2 Hz, 2H), 3.65 - 3.19 (m, 2H), 3.17 - 3.05 (m, 2H), 2.73 - 2.60 (m, 3H), 2.48 (p, *J =* 1.9 Hz, 7H), 1.56 - 1.40 (m, 3H), 1.12 (dd, *J =* 6.6, 2.8 Hz, 6H). MS (ESI+) *m*/*z*: 555 [M+ H]⁺. Chiral title compound **39** from peak B was purified as above to give the title **Compound 39.** Absolute stereochemistry was arbitrarily assigned as (S) with an ee > 99%. ¹H NMR (400 MHz, dimethyl sulfoxide-*d*₆) δ ppm 8.01 (s, 1H), 7.82 (d, *J* = 9.4 Hz, 1H), 7.56 (d, *J* = 1.9 Hz, 1H), 7.44 - 7.14 (m, 2H), 7.07 (t, *J* = 73.3 Hz, 1H), 6.88 (s, 1H), 6.34 (d*, J* = 1.9 Hz, 1H), 3.81 (qt*, J* = 11.8, 5.2 Hz, 2H), 3.65 - 3.19 (m, 2H), 3.17 - 3.05 (m, 2H), 2.73 - 2.60 (m, 3H), 2.48 (p, *J* = 1.9 Hz, 7H), 1.56 - 1.40 (m, 3H), 1.12 (dd, *J =* 6.6, 2.8 Hz, 6H). MS (ESI+) *m*/*z*: 555 [M+ H]⁺.

### Example #17: Compound 108: (1r,3r)-3-[4-[[4-chloro-2-(difluoromethoxy)phenyl]carbamoyl]-4-(5-isopropylpyrazol-1-yl)-1-piperidyl]-1-(trifluoromethyl)cyclobutanecarboxylic acid

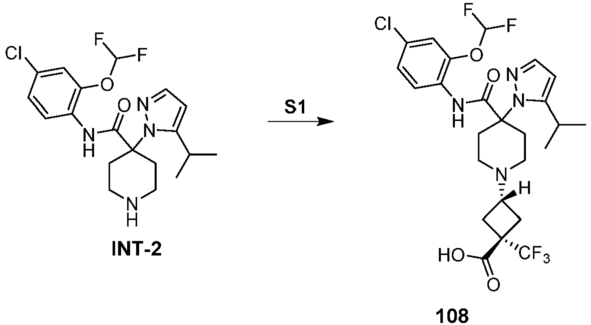

**Compound 108.** A mixture of *N*-(4-chloro-2-(difluoromethoxy)phenyl)-4-(5-isopropyl-1*H*-pyrazol-1-yl)piperidine-4-carboxamide (**INT-2**) (0.20 g, 0.484 mmol) and 3-oxo-1-(trifluoromethyl)cyclobutane-1-carboxylic acid (0.149 g, 0.775 mmol) were combined in a 20 mL pressure vial charged with 1,2-dichloroethane (5 mL) and stirred for 1 hour at 25 °C. Sodium triacetoxyborohydride (STAB) (0.164 g, 0.775 mmol) was added to the reaction and the mixture was allowed to stir for 17 hours. The mixture was quenched by the addition of water (2 mL) followed by saturated aqueous NH₄Cl (5 mL) and dichloromethane (2 x 10 mL). The organic phases were combined and dried over MgSO₄, then filtered and concentrated to give the crude title product as a mixture of cis and trans isomers (5:1 trans to cis ratio). The crude product was purified by silica gel chromatography utilizing methanol in dichloromethane which allowed for the cis and trans isomer peaks to be separated. The more polar eluting peak was concentrated and confirmed by NMR structural elucidation methodology to be the trans title compound. ¹H NMR (500 MHz, dimethyl sulfoxide-*d₆*) δ ppm 8.42 (s, 1H), 8.22 (s, 1H), 7.80 - 7.75 (m, 1H), 7.51 (d, *J =* 1.8 Hz, 1H), 7.35 - 7.28 (m, 2H), 7.15 (t, *J* = 73.3 Hz, 1H), 6.32 (d, *J =* 1.8 Hz, 1H), 3.76 - 3.59 (m, 2H), 3.58 - 3.43 (m, 2H), 3.20 - 2.87 (m, 4H), 2.77 - 2.53 (m, 6H), 1.11 (d, *J =* 6.6 Hz, 6H). MS (APCI+) *m*/*z*: 579 [M+ H]⁺.

### Example #18: Compound 164: (1s,3s)-3-[4-[[4-chloro-2-(difluoromethoxy)phenyl]carbamoyl]-4-(5-isopropylpyrazol-1-yl)-1-piperidyl]-1-hydroxy-cyclobutanecarboxylic acid

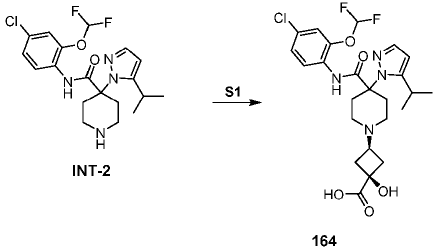

**Compound 164.** A mixture of *N*-(4-chloro-2-(difluoromethoxy)phenyl)-4-(5-isopropyl-1*H*-pyrazol-1-yl)piperidine-4-carboxamide (**INT-2**) (0.95 g, 2.30 mmol) and 1-hydroxy-3-oxocyclobutane-1-carboxylic acid (0.479 g, 3.68 mmol) were combined in a 20 mL pressure vial charged with 1,2-dichloroethane (12 mL). The mixture was stirred for 1 hour at 25 °C followed by addition of sodium triacetoxyborohydride (STAB) (0.780 g, 3.68 mmol) and the reaction was allowed to stir for 17 hours. The reaction was quenched by the addition of water (2 mL) followed by saturated aqueous NH₄Cl (5 mL) and dichloromethane (2 x10 mL). The organic phases were dried over MgSO₄, filtered and concentrated to give the crude title product. The crude product was purified utilizing silica gel chromatography eluting with methanol in dichloromethane to give the crude product. The material was then further purified on a Reverse Phase column with a mobile phase of acetonitrile with 0.1% ammonium acetate to give the title compound. ¹H NMR (400 MHz, dimethyl sulfoxide-*d*₆) δ ppm 7.99 (s, 1H), 7.93 - 7.82 (m, 1H), 7.56 - 7.49 (m, 1H), 7.33 - 7.22 (m, 2H), 7.06 (t, *J* = 73.0 Hz, 1H), 6.32 (d, *J* = 1.9 Hz, 1H), 3.15 - 2.90 (m, 4H), 2.70 - 2.50 (m, 4H), 2.47 (d, *J* = 1.9 Hz, 4H), 2.43 - 2.35 (m, 2H), 2.28 - 2.19 (m, 2H), 1.13 (d, *J* = 6.8 Hz, 6H). MS (APCI+) *m*/*z:* 527 [M+ H]⁺.

### Example #19: Compound 194: (1r,3r)-3-[4-[[4-chloro-2-(difluoromethoxy)phenyl]carbamoyl]-4-(5-isopropylpyrazol-1-yl)-1-piperidyl]-1-methoxy-cyclobutanecarboxylic acid

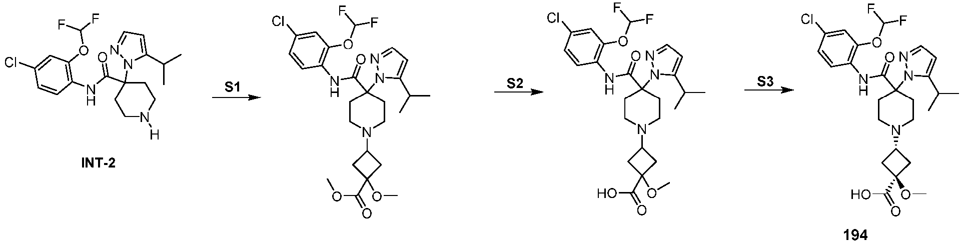

**Step 1: methyl 3-(4-((4-chloro-2-(difluoromethoxy)phenyl)carbamoyl)-4-(5-isopropyl-1H-pyrazol-1-yl)piperidin-1-yl)-1-methoxycyclobutane-1-carboxylate.** A mixture of *N*-(4-chloro-2-(difluoromethoxy)phenyl)-4-(5-isopropyl-1*H*-pyrazol-1-yl)piperidine-4-carboxamide (**INT-2**) (0.5 g, 1.21 mmol) and methyl 1-methoxy-3-oxocyclobutane-1-carboxylate (0.306 g, 1.94 mmol) were combined in a 40 mL pressure vial charged with 1,2-dichloroethane (20 mL) and stirred for 1 hour at 25 °C followed by addition of sodium triacetoxyborohydride (STAB) (0.411 g, 1.94 mmol) and the reaction was allowed to stir for 17 hours. The reaction was quenched by the addition of water (2 mL) followed by saturated aqueous NH₄Cl (5 mL). Dichloromethane was added (2 x 10 mL). The organic phase was dried over MgSO₄, filtered and concentrated to give the crude title product. The crude product was first purified utilizing silica gel chromatography eluting with ethyl acetate in heptanes to give the purified title compound. MS (APCI+) *m*/*z*: 555 [M+ H]⁺.

**Step 2: 3-(4-((4-chloro-2-(difluoromethoxy)phenyl)carbamoyl)-4-(5-isopropyl-1*H-*pyrazol-1-yl)piperidin-1-yl)-1-methoxycyclobutane-1-carboxylic acid.** To the product from **Step 1** (0.330 g, 0.594 mmol) was added a solvent mixture of tetrahydrofuran (5 mL) and methanol (5 mL) followed by the addition of lithium hydroxide (2.97 mL of 1M solution, 2.97 mmol). The reaction mixture was stirred at 25 °C for 16 hours, diluted with saturated NH₄Cl solution (10 mL) and extracted with dichloromethane (3 x 10 mL). The combined organic extracts were dried over MgSO₄, filtered and concentrated to give the title compound as a cis, trans mixture. MS (APCI+) *m*/*z*: 541 [M+ H]⁺.

**Step 3: Compound 194.** The cis, trans product mixture from **Step 2** (0.286 g) was separated by preparative chiral supercritical fluid chromatography on CHIRALPAK^{®} (IC) column, (30 x 250 mm, 5 µm) at ambient temperature, using a mobile phase of 20% ethanol (0.1% diethylamine):CO₂ Fractions were collected based on UV detection at 254 nm with peaks detected at 11.45 minutes (A) and 25.69 minutes (B) to provide the resolved isomer peaks, A and B. Peak B was concentrated to dryness then was re-purified by silica gel chromatography to remove residual diethylamine additive from the SFC run utilizing a silica gel column eluting with methanol in dichloromethane to provide the title compound from peak B (**Compound 194**) which was assigned relative cis stereochemistry by NMR structural chemistry. ¹H NMR (500 MHz, dimethyl sulfoxide-*d*₆) δ ppm 8.42 (s, 1H), 8.22 (s, 1H), 7.80 - 7.75 (m, 1H), 7.51 (d, *J =* 1.8 Hz, 1H), 7.35 - 7.28 (m, 2H), 7.15 (t, *J* = 73.3 Hz, 1H), 6.32 (d, *J* = 1.8 Hz, 1H), 3.76 - 3.59 (m, 2H), 3.58 - 3.43 (m, 2H), 3.20 - 2.87 (m, 4H), 2.77 - 2.53 (m, 6H), 1.11 (d, *J =* 6.6 Hz, 6H). MS (APCI+) *m*/*z:* 541 [M+ H]⁺.

### Example #20: Compound 40: 6-[[4-[[4-chloro-2-(difluoromethoxy)phenyl]carbamoyl]-4-(5-isopropylpyrazol-1-yl)-1-piperidyl]methyl]-2-oxaspiro[3.3]heptane-6-carboxylic

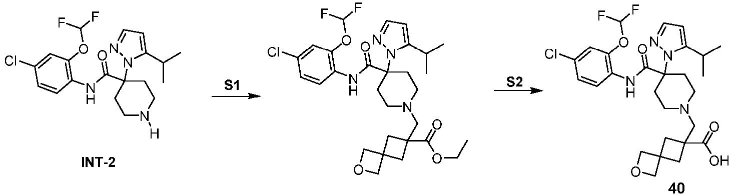

**Step 1: ethyl 6-((4-((4-chloro-2-(difluoromethoxy)phenyl)carbamoyl)-4-(5-isopropyl-1*H*-pyrazol-1-yl)piperidin-1-yl)methyl)-2-oxaspiro[3.3]heptane-6-carboxylate.** A mixture of *N*-(4-chloro-2-(difluoromethoxy)phenyl)-4-(5-isopropyl-1*H*-pyrazol-1-yl)piperidine-4-carboxamide (**INT-2**) (0.4 g, 0.969 mmol) and ethyl 6-formyl-2-oxaspiro[3.3]heptane-6-carboxylate (0.320 g, 1.45 mmol) were combined in a 40 mL pressure vial charged with 1,2-dichloroethane (20 mL) and the reaction mixture stirred for 1 hour at 25 °C. Sodium triacetoxyborohydride (STAB) (0.382 g, 1.45 mmol) was added and the reaction was allowed to stir for 17 hours. The reaction mixture was quenched by the addition of water (2 mL) followed by saturated aqueous NH₄Cl (5 mL). Dichloromethane was added (2 x 10 mL), and the organic phases were dried over MgSO₄, filtered and concentrated to give the crude title product. The crude product was purified utilizing silica gel chromatography, eluting with ethyl acetate in heptanes to give the title compound. MS (APCI+) *m*/*z*: 595 [M]⁺.

**Step 2: Compound 40.** To the product from **Step 1** (0.238 g, 0.4 mmol) was added a mixture of tetrahydrofuran (5 mL) and methanol (5 mL) followed by the addition of lithium hydroxide (2.00 mL of 1M solution, 2.00 mmol). The reaction mixture was stirred at 25 °C for 16 hours. Saturated aqueous NH₄Cl solution (10 mL) was added and the mixture was extracted with dichloromethane (3 x 10 mL). The combined organic extracts were dried over MgSO₄, filtered and concentrated to give the crude title product. The crude material was purified utilizing silica gel chromatography, eluting with methanol in dichloromethane to give the title compound. ¹H NMR (400 MHz, dimethyl sulfoxide-*d*₆) δ ppm 8.07 (s, 1H), 7.79 (d, *J =* 9.3 Hz, 1H), 7.68 - 7.48 (m, 3H), 7.34 - 7.23 (m, 1H), 7.08 (s, 1H), 6.35 (d, *J* = 1.9 Hz, 1H), 3.36 - 3.25 (m, 1H), 3.08 (t, *J* = 10.8 Hz, 2H), 2.93 (p, *J =* 6.7 Hz, 1H), 2.77 - 2.58 (m, 4H), 2.50 - 2.37 (m, 5H), 2.18 - 2.07 (m, 3H), 1.80 - 1.69 (m, 3H), 1.13 (d, *J =* 6.7 Hz, 6H). MS (ESI) *m*/*z*: 567 [M]⁺.

### Example #21: Compounds 149, 151, and 152: (2S)-3-[4-[[4-chloro-2-(difluoromethoxy)phenyl]carbamoyl]-4-(5-isopropylpyrazol-1-yl)-1-piperidyl]-2-[(3R)-tetrahydrofuran-3-yl]propanoic acid, (2S)-3-[4-[[4-chloro-2-(difluoromethoxy)phenyl]carbamoyl]-4-(5-isopropylpyrazol-1-yl)-1-piperidyl]-2-[(3S)-tetrahydrofuran-3-yl]propanoic acid, and (2R)-3-[4-[[4-chloro-2-(difluoromethoxy)phenyl]carbamoyl]-4-(5-isopropylpyrazol-1-yl)-1-piperidyl]-2-[(3S)-tetrahydrofuran-3-yl]propanoic acid

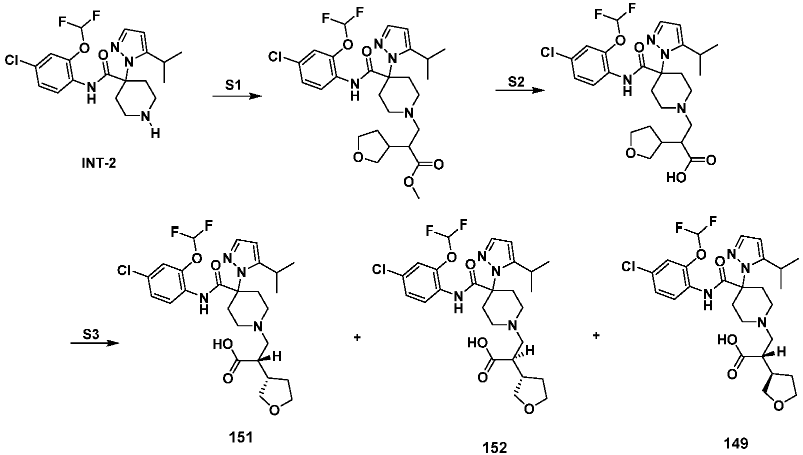

**Step 1: methyl 3-(4-((4-chloro-2-(difluoromethoxy)phenyl)carbamoyl)-4-(5-isopropyl-1*H*-pyrazol-1-yl)piperidin-1-yl)-2-(tetrahydrofuran-3-yl)propanoate.** A mixture of *N*-(4-chloro-2-(difluoromethoxy)phenyl)-4-(5-isopropyl-1*H*-pyrazol-1-yl)piperidine-4-carboxamide (**INT-2**) (0.53 g, 0.1.284 mmol) and methyl 3-oxo-2-(tetrahydrofuran-3-yl)propanoate (0.368 g, 1.926 mmol) were combined in a 40 mL pressure vial charged with 1,2-dichloroethane (20 mL). The reaction mixture was stirred for 1 hour at 25 °C. Tetramethylammonium triacetoxyborohydride (0.507 g, 1.926 mmol) was added and the reaction was allowed to stir for 17 hours. The reaction was quenched with water (5 mL) and saturated aqueous NH₄Cl (5 mL) and dichloromethane were added (2 x 10 mL). The organic phase was dried over MgSO₄, filtered and concentrated to give the crude title product. The crude product was purified utilizing silica gel chromatography eluting with ethyl acetate in heptanes to give the title compound. MS (APCI+) *m*/*z*: 569 [M]⁺.

**Step 2: 3-(4-((4-chloro-2-(difluoromethoxy)phenyl)carbamoyl)-4-(5-isopropyl-1*H-*pyrazol-1-yl)piperidin-1-yl)-2-(tetrahydrofuran-3-yl)propanoic acid.** To the product from **Step 1** (0.703 g, 1.235 mmol) was added a mixture of tetrahydrofuran (5 mL) and methanol (5 mL) followed by the addition of lithium hydroxide (6.18 mL of 1M solution, 6.18 mmol). The reaction mixture was stirred at 25 °C for 17 hours, diluted with saturated aqueous NH₄Cl solution (15 mL) and extracted with dichloromethane (3 x 10 mL). The combined organic extracts were dried over MgSO₄, filtered and concentrated to give the crude racemic title compound, which was carried on to **Step 3.** MS (APCI+) *m*/*z*: 555 [M]⁺.

**Step 3: Compounds 149, 151, and 152.** The resultant racemic compound from **Step 2** (0.551 g) was subjected to a multistep preparative Supercritical Fluid Chromatography (SFC) chiral separation utilizing three successive methods:
**Method 1:** Separation utilized a CHIRALPAK^{®} IC column, (30 x 250 mm, 5 µm) at 35 °C utilizing a mobile phase of 25% ethanol:CO₂. Fractions were collected based on UV detection at 220 nm with fraction peaks detected at 4.73 minutes (A) and 11.50 minutes (B).

**Method 2:** The first-eluting peak A at 4.73 minutes (0.275 g) was then processed with a second preparative SFC method employing a CHIRALPAK^{®} AD-H column, (30 x 250 mm, 5 µm) at 35 °C utilizing a mobile phase of 20% of 2:8 acetonitrile/isopropanol (0.1% diethylamine)/:CO₂. Fractions were collected based on UV detection at 254 nm with peaks for 2 diastereomers detected at 10.58 minutes and 14.24 minutes which were labeled fractions 1 and 2 respectively.

**Method 3:** The second-eluting peak B 11.5 minutes (0.275 g) from the first purification was subjected to a third SFC purification method to resolve the remaining two diastereomers employing a Whelk-O^{®} (S,S) column, (30 x 250 mm, 5 µm) at 35 °C utilizing a mobile phase of 25% of 1:1 acetonitrile/ethanol (0.1% diethylamine)/:CO₂. Fractions were collected based on UV detection at 254 nm with peaks for 2 additional diastereomers detected at 7.08 minutes and 9.32 minutes, labeled fractions 3 and 4 respectively. Four diastereomer peaks were isolated in order of elution by their respective methods at 10.58 minutes (1), 14.23 minutes (2), 7.08 minutes (3) and 9.32 minutes (4). Fraction Peaks 1, 3 and 4 were concentrated and each re-purified by flash chromatography (methanol in dichloromethane) to remove traces of the diethylamine SFC additive to provide (in fraction peak order 1,3 and 4) the title compounds which were arbitrarily assigned stereochemistry:
**Compound 151.** (ee >99.5%). ¹H NMR (400 MHz, dimethyl sulfoxide-*d*₆) δ ppm 7.91 (dd, *J* = 9.3, 1.0 Hz, 1H), 7.81 (s, 1H), 7.51 (t, *J* = 1.8 Hz, 1H), 7.31 - 7.21 (m, 2H), 7.15 (t, *J* = 73.3 Hz, 1H), 6.28 (t, *J =* 1.7 Hz, 1H), 3.80 - 3.66 (m, 2H), 3.59 (td, *J =* 8.1, 7.1 Hz, 1H), 3.37 (dd, *J =* 8.5, 7.5 Hz, 1H), 2.99 (h, *J =* 6.7 Hz, 1H), 2.88 (d, *J =* 6.1 Hz, 1H), 2.86 (s, 1H), 2.71 (dd, *J =* 10.6, 5.4 Hz, 1H), 2.67 - 2.52 (m, 3H), 2.45 - 2.36 (m, 4H), 2.29 (dt, *J =* 16.0, 8.0 Hz, 1H), 2.05 - 1.83 (m, 2H), 1.57 (dq, *J =* 12.2, 8.1 Hz, 2H), 1.11 (dd, *J =* 6.7, 1.5 Hz, 6H). MS (APCI+) *m*/*z*: 566 [M+ H]⁺.

**Compound 152.** (ee> 99%). ¹H NMR (400 MHz, dimethyl sulfoxide-*d*₆) δ ppm 7.91 (dd, *J* = 9.3, 1.0 Hz, 1H), 7.81 (s, 1H), 7.51 (t, *J* = 1.8 Hz, 1H), 7.31 - 7.21 (m, 2H), 7.15 (t, *J* = 73.3 Hz, 1H), 6.28 *(t, J* = 1.7 Hz, 1H), 3.79 (dd, *J =* 8.5, 7.4 Hz, 1H), 3.69 (td, *J =* 8.3, 4.6 Hz, 1H), 3.59 (q, *J* = 7.7 Hz, 1H), 3.42 - 3.29 (m, 1H), 2.98 (hept, *J =* 6.7 Hz, 1H), 2.83 (td, *J =* 10.6, 5.1 Hz, 1H), 2.69 (dd, *J =* 10.6, 5.7 Hz, 1H), 2.62 - 2.50 (m, 5H), 2.43 - 2.33 (m, 4H), 2.33 - 2.23 (m, 2H), 1.92 (dtd, *J =* 12.3, 7.7, 4.7 Hz, 1H), 1.68 - 1.58 (m, 1H), 1.11 (dd, *J* = 6.7, 1.6 Hz, 6H). MS (APCI+) *m*/*z:* 566 [M+ H]⁺.

**Compound 149.** (ee > 99.6%) ¹H NMR (400 MHz, dimethyl sulfoxide-*d*₆) δ ppm 7.91 (dd, *J* = 9.3, 1.0 Hz, 1H), 7.81 (s, 1H), 7.51 (t, *J* = 1.8 Hz, 1H), 7.31 - 7.21 (m, 2H), 7.15 (t, *J* = 73.3 Hz, 1H), 6.28 (t, *J =* 1.7 Hz, 1H), 3.80 - 3.66 (m, 2H), 3.59 (td, *J =* 8.1, 7.1 Hz, 1H), 3.37 (dd, *J =* 8.5, 7.5 Hz, 1H), 2.99 (h, *J =* 6.7 Hz, 1H), 2.88 (d, *J =* 6.1 Hz, 1H), 2.86 (s, 1H), 2.71 (dd, *J =* 10.6, 5.4 Hz, 1H), 2.67 - 2.52 (m, 3H), 2.45 - 2.36 (m, 4H), 2.29 (dt, *J =* 16.0, 8.0 Hz, 1H), 2.05 - 1.83 (m, 2H), 1.57 (dq, *J =* 12.2, 8.1 Hz, 2H), 1.11 (dd, *J =* 6.7, 1.5 Hz, 6H). MS (APCI+) *m*/*z*: 566 [M+ H]⁺.

### Example #22: Compound 170: (2S)-2-[[4-[[4-chloro-2-(difluoromethoxy)phenyl]carbamoyl]-4-(4-fluoro-5-isopropyl-pyrazol-1-yl)-1-piperidyl]methyl]-3-methoxy-propanoic acid

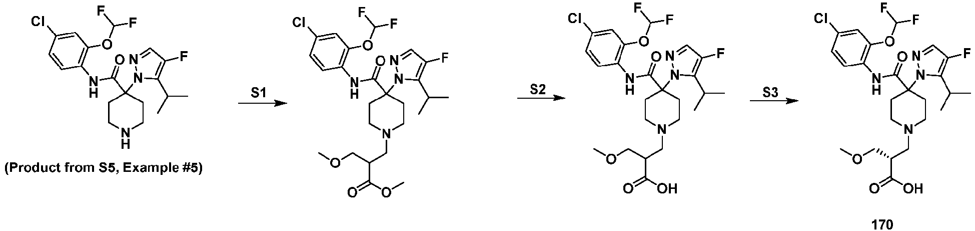

**Step 1: methyl 3-(4-((4-chloro-2-(difluoromethoxy)phenyl)carbamoyl)-4-(4-fluoro-5-isopropyl-1H-pyrazol-1-yl)piperidin-1-yl)-2-(methoxymethyl)propanoate.** A mixture of (product of **Step 5, Example #5**), *N*-(4-chloro-2-(difluoromethoxy)phenyl)-4-(4-fluoro-5-isopropyl-1*H*-pyrazol-1-yl)piperidine-4-carboxamide hydrochloride (2.0 g, 4.28 mmol) and 2,3,4,6,7,8,9,10-octahydropyrimido[1,2-*a*]azepine (1.94 mL, 12.84 mmol) were combined in a 40 mL pressure vial followed by addition of dichloromethane (25 mL) and methyl 2-(methoxymethyl)acrylate (1.466g , 10.70 mmol). The mixture was stirred at 25 °C for 17 hours, diluted with saturated aqueous NaHCO₃ (20 mL) and extracted with dichloromethane (3 x 25 mL). The organic extracts were combined, washed with brine (25 mL), dried over MgSO₄, filtered and concentrated. The crude material was purified by flash silica gel chromatography (ethyl acetate in heptanes) to give the title compound. MS (APCI+) *m*/*z:* 561 [M+ H]⁺.

**Step 2: 3-(4-((4-chloro-2-(difluoromethoxy)phenyl)carbamoyl)-4-(4-fluoro-5-isopropyl-1*H*-pyrazol-1-yl)piperidin-1-yl)-2-(methoxymethyl)propanoic acid.** To the product from **Step 1** (2.17 g, 3.87 mmol) was added a solvent mixture of tetrahydrofuran (30 mL), methanol (30 mL) and water (10 mL) followed by the addition of lithium hydroxide (0.926 g, 38.7 mmol). The reaction mixture was stirred at 25 °C for 2 hours, diluted with saturated aqueous NH₄Cl solution (35 mL) and extracted with dichloromethane (3 x 30 mL). The combined organic extracts were dried over MgSO₄, filtered and concentrated. The crude material was purified by flash chromatography (methanol in dichloromethane) to give the title compound. MS (APCI+) *m*/*z*: 547.2 [M+H]⁺.

**Step 3: Compound 170.** The material from **Step 2** (1.65 g) was separated by preparative chiral supercritical fluid chromatography on CHIRALPAK^{®} (IC) column, (30 x 250 mm, 5 µm) at 35 °C, using a mobile phase of 15% ethanol (0.1% triethylamine):CO₂. Fractions were collected based on UV detection at 254 nm with peaks detected at 7.53 minutes (A) and 10.82 minutes (B) to provide the resolved enantiomer peaks, A and B. Peak B was concentrated to dryness then was re-purified by silica gel chromatography to remove residual diethylamine additive from the SFC run utilizing a silica gel column eluting with a gradient of methanol in dichloromethane to provide the chiral title compound with stereochemistry arbitrarily assigned. ¹H NMR (400 MHz, dimethyl sulfoxide-*d*₆) δ ppm 8.64 (s, 1H), 7.62 (m, 1H), 7.57 (d, *J =* 4.1 Hz, 1H), 7.34 (m, 1H), 7.33 (m, 1H), 7.17 (t, *J =* 73.4 Hz, 1H), 3.46 (m, 2H), 3.20 (s, 3H), 2.99 (m, 1H), 2.78 (m, 2H), 2.69 (m, 1H), 2.53 (m, 2H), 2.39 (m, 6H), 1.16 (d, *J =* 6.8 Hz, 6H). MS (APCI+) *m*/*z:* 547.2 [M+H]⁺.

### Example #23: Compounds 184, 185, and 186: (2R)-3-[4-[[4-chloro-2-(difluoromethoxy)phenyl]carbamoyl]-4-(5-isopropylpyrazol-1-yl)-1-piperidyl]-2-((1s,4S)-4-methoxycyclohexyl)propanoic acid, (2R)-3-[4-[[4-chloro-2-(difluoromethoxy)phenyl]carbamoyl]-4-(5-isopropylpyrazol-1-yl)-1-piperidyl]-2-((1r,4R)-4-methoxycyclohexyl)propanoic acid, and (2S)-3-[4-[[4-chloro-2-(difluoromethoxy)phenyl]carbamoyl]-4-(5-isopropylpyrazol-1-yl)-1-piperidyl]-2-((1s,4R)-4-methoxycyclohexyl)propanoic acid

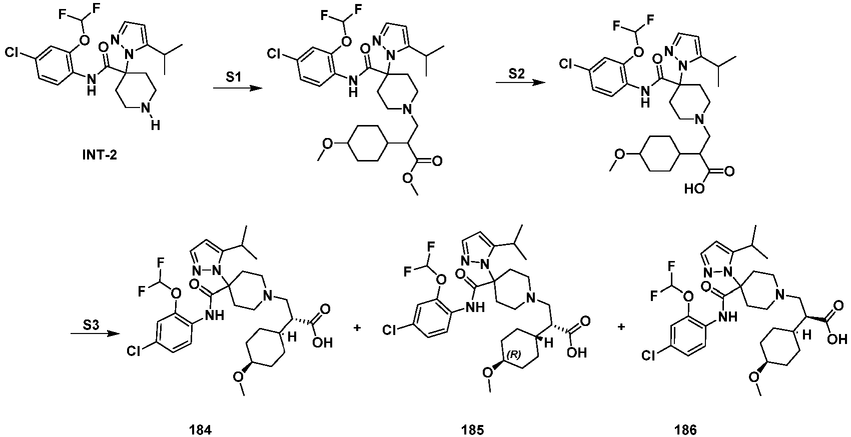

Step 1: **methyl 3-(4-((4-chloro-2-(difluoromethoxy)phenyl)carbamoyl)-4-(5-isopropyl-1H-pyrazol-1-yl)piperidin-1-yl)-2-(4-methoxycyclohexyl)propanoate.** A mixture of *N*-(4-chloro-2-(difluoromethoxy)phenyl)-4-(5-isopropyl-1*H*-pyrazol-1-yl)piperidine-4-carboxamide (**INT-2**) (0.8 g, 1.94 mmol) and methyl 2-(4-methoxycyclohexyl)-3-oxopropanoate (0.623 g, 2.91 mmol) were combined in a 40 mL pressure vial charged with 1,2-dichloroethane (20 mL). The reaction mixture was stirred for 1 hour at 25 °C followed by addition of tetramethylammonium triacetoxyborohydride (0.765 g, 2.91 mmol) and the reaction mixture was allowed to stir for 17 hours. The reaction mixture was quenched by the addition of water (5 mL) followed by saturated aqueous NH₄Cl (15 mL) and dichloromethane added (2 x 20 mL). The organic phases were dried over MgSO₄, filtered and concentrated to give the crude title product. The crude product was first purified utilizing silica gel chromatography eluting with ethyl acetate in heptanes to provide the title compound. MS (APCI+) *m*/*z*: 612 [M+H]⁺.

**Step 2: 3-(4-((4-chloro-2-(difluoromethoxy)phenyl)carbamoyl)-4-(5-isopropyl-1*H-*pyrazol-1-yl)piperidin-1-yl)-2-(4-methoxycyclohexyl)propanoic acid.** To the product from **Step 1** (1.04 g, 1.55 mmol) in a 40 mL pressure vial was added a solvent mixture of tetrahydrofuran (8 mL) and methanol (8 mL) followed by the addition of lithium hydroxide (8.51 mL of 1M solution 6.94 mmol). The reaction mixture was stirred at 60 °C for 5 hours, diluted with saturated aqueous NH₄Cl solution (20 mL) and extracted with dichloromethane (3 x 20 mL). The combined organic extracts were dried over MgSO₄, filtered and concentrated to give the crude title product. The crude material was purified utilizing silica gel chromatography eluting with methanol in dichloromethane to provide the racemic title compound. MS (APCI+) *m*/*z*: 598 [M+H]⁺.

**Step 3: Compounds 184, 185, and 186.** The resultant racemic compound from **Step 2** (0.929 g) was then subjected to a multistep preparative Supercritical Fluid Chromatography (SFC) chiral separation utilizing three successive methods:
**Method 1:** Initial separation of the compound into isomer pairs from **Step 2** (0.929 g) utilized a Regis Whelk-O^{®}1 (S,S) column, (30 x 250 mm, 5 µm) at 35 °C utilizing a mobile phase of 20% 8:2 ratio of isopropanol/acetonitrile/0.2% diethylamine:CO₂. Fractions were collected based on UV detection at 220 nm with fraction peaks detected at 10.27 minutes (A) and 13.61 minutes (B) with each peak containing a pair of isomers.

**Method 2:** The first-eluting peak A at 10.27 minutes (~0.460 g) from method 1 was then processed with a second preparative SFC method employing a CHIRALPAK^{®} AD-H column, (30 x 250 mm, 5 µm) at 35 °C utilizing a mobile phase of 20% of 4:1 isopropanol/acetonitrile (0.2% diethylamine)/:CO₂. Fractions were collected based on UV detection at 254 nm with peaks for 2 diastereomers detected at 11.82 minutes and 15.09 minutes which were labeled fractions 1 and 2 respectively.

**Method 3:** The second-eluting peak B 11.5 minutes (~0.460 g) from method 1 was subjected to a second SFC purification to resolve the remaining two diastereomers employing a CHIRALPAK^{®} IG column, (30 x 250 mm, 5 µm) at 35°C utilizing a mobile phase of 30% isopropanol (0.2% diethylamine)/:CO₂. Fractions were collected based on UV detection at 254 nm with peaks for 2 additional diastereomers detected at 4.42 minutes and 6.65 minutes, labeled fractions 3 and 4 respectively. Four diastereomer peaks were isolated in order of elution by their respective methods at 11.82 minutes (1), 15.09 minutes (2), 4.42 minutes (3) and 6.65 minutes (4). Fraction Peaks 1, 2 and 3 were concentrated and each re-purified by flash chromatography (methanol in dichloromethane) to remove traces of the diethylamine SFC additive to provide (in fraction peak order 1, 2 and 3) the title chiral compounds which were arbitrarily assigned stereochemistry:
**Compound 184.** (>99% ee). ¹H NMR (400 MHz, dimethyl sulfoxide-*d*₆) δ ppm 7.90 (dd, *J* = 9.4, 5.8 Hz, 1H), 7.84 (d, *J* = 14.4 Hz, 1H), 7.51 (dd, *J* = 3.3, 1.9 Hz, 1H), 7.32 - 7.22 (m, 2H), 7.17 (t, *J =* 73.4 Hz, 1H), 6.29 (d, *J =* 1.9 Hz, 1H), 3.32 (dt, *J =* 4.5, 2.3 Hz, 1H), 3.18 (s, 3H), 2.98 (p, *J* = 6.7 Hz, 2H), 2.83 (dd, *J* = 11.7, 5.5 Hz, 1H), 2.65 (dd, *J* = 9.2, 4.9 Hz, 1H), 2.50 (s, 3H), 2.44 - 2.27 (m, 5H), 1.77 (d, *J =* 11.8 Hz, 2H), 1.55 (s, 1H), 1.47 - 1.28 (m, 6H), 1.11 (dd, *J =* 6.7, 1.7 Hz, 6H). MS (APCI+) *m*/*z:* 598 [M+H]⁺.

**Compound 185.** (>98% ee). (>99% ee). ¹H NMR (400 MHz, dimethyl sulfoxide-*d*₆) δ ppm 7.90 (dd, *J* = 9.4, 5.8 Hz, 1H), 7.84 (d, *J* = 14.4 Hz, 1H), 7.51 (dd, *J* = 3.3, 1.9 Hz, 1H), 7.32 - 7.22 (m, 2H), 7.17 (t, *J* = 73.4 Hz, 1H), 6.29 (d, *J* = 1.9 Hz, 1H), 3.32 (dt, *J* = 4.5, 2.3 Hz, 1H), 3.18 (s, 3H), 2.98 (p, *J* = 6.7 Hz, 2H), 2.83 (dd, *J* = 11.7, 5.5 Hz, 1H), 2.65 (dd, *J* = 9.2, 4.9 Hz, 1H), 2.50 (s, 3H), 2.44 - 2.27 (m, 5H), 1.77 (d, *J =* 11.8 Hz, 2H), 1.55 (s, 1H), 1.47 - 1.28 (m, 6H), 1.11 (dd, *J* = 6.7, 1.7 Hz, 6H). MS (APCI+) *m*/*z:* 598 [M+H]⁺.

**Compound 186.** ¹H NMR (400 MHz, dimethyl sulfoxide-*d*₆) δ ppm 7.90 (dd, *J* = 9.4, 5.8 Hz, 1H), 7.84 (d, *J* = 14.4 Hz, 1H), 7.51 (dd, *J* = 3.3, 1.9 Hz, 1H), 7.32 - 7.22 (m, 2H), 7.17 (t, *J* = 73.4 Hz, 1H), 6.29 (d, *J* = 1.9 Hz, 1H), 3.32 (dt, *J* = 4.5, 2.3 Hz, 1H), 3.18 (s, 3H), 2.98 (p, *J* = 6.7 Hz, 2H), 2.83 (dd, *J =* 11.7, 5.5 Hz, 1H), 2.65 (dd, *J =* 9.2, 4.9 Hz, 1H), 2.50 (s, 3H), 2.44 - 2.27 (m, 5H), 1.77 (d, *J =* 11.8 Hz, 2H), 1.55 (s, 1H), 1.47 - 1.28 (m, 6H), 1.11 (dd, *J =* 6.7, 1.7 Hz, 6H). MS (APCI+) *m*/*z*: 598 [M+H]⁺.

### Example #24: Compound 35: 4-[[4-[[4-chloro-2-(difluoromethoxy)phenyl]carbamoyl]-4-(6,6-dimethyl-4,5-dihydrocyclopenta[c]pyrazol-1-yl)-1-piperidyl]methyl]tetrahydropyran-4-carboxylic acid

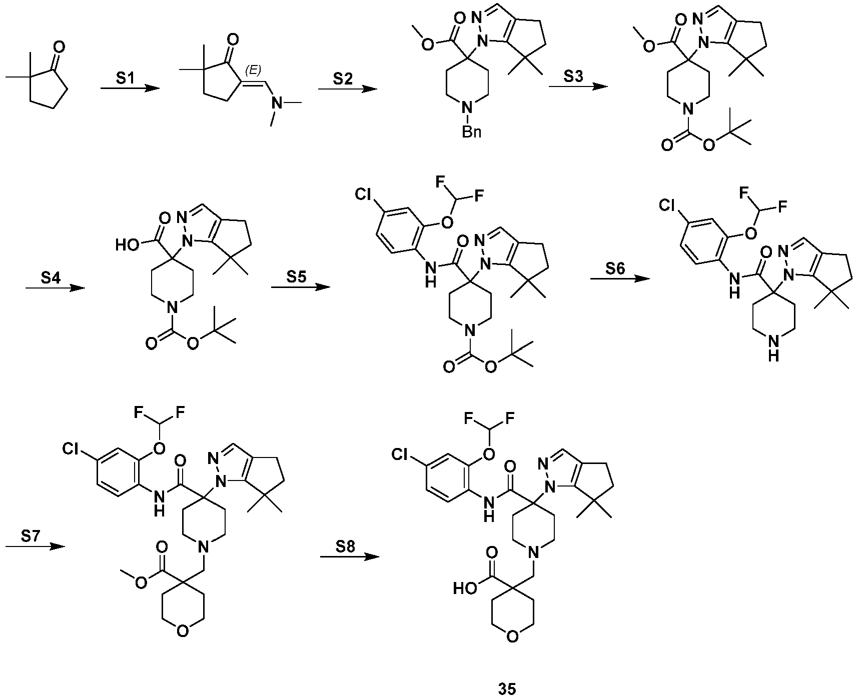

**Step 1: (E)-5-((dimethylamino)methylene)-2,2-dimethylcyclopentanone.** A mixture of *N,N-*dimethylformamide dimethyl acetal (7 mL) and 2,2-dimethylcyclopentanone (5 g) in *N,N-*dimethylformamide (90 mL) was stirred at 100 °C for 18 hours. The resulting mixture was allowed to cool to ambient temperature and then concentrated under reduced pressure to provide the title compound. MS (ESI) *m*/*z:* 168.3 (M+H)⁺.

**Step 2: methyl 1-benzyl-4-(6,6-dimethyl-5,6-dihydrocyclopenta[*c*]pyrazol-1(4*H*)-yl)piperidine-4-carboxylate.** To a solution of **INT-1** (3 g) in *n*-butanol (80 mL) was added (*E*)-5-((dimethylamino)methylene)-2,2-dimethylcyclopentanone (**Step 1**, 1.8 g) and hydrochloric acid (12 M in water) (1.5 mL) and the reaction was heated at 90 °C for 20 hours. The reaction mixture was concentrated under reduced pressure and the crude residue was partitioned between saturated aqueous sodium bicarbonate (5 mL) and ethyl acetate (5 mL). The aqueous phase was extracted with ethyl acetate (2 x 5 mL). The combined organic layers were dried over magnesium sulfate, filtered and concentrated under reduced pressure. The crude product was purified by flash column chromatography (ethyl acetate/iso-hexane) to provide the title compound. MS (ESI) *m*/*z*: 368.2 (M+H)⁺.

**Step 3: *1-tert-butyl* 4-methyl 4-(6,6-dimethyl-5,6-dihydrocyclopenta[c]pyrazol-1(4*H*)-yl)piperidine-1,4-dicarboxylate.** A mixture of methyl 1-benzyl-4-(6,6-dimethyl-5,6-dihydrocyclopenta[c]pyrazol-1(4*H*)-yl)piperidine-4-carboxylate (1.2 g), palladium, 10% on activated carbon, Type 39 (0.45 g) and hydrogen chloride (4 M in dioxane) (0.8 mL) in methanol (15 mL) was subjected to hydrogen gas at 5 bar and ambient temperature for 5 hours. The mixture was filtered through a Whatman GF/F filter paper, washing with methanol (6 x 5 mL). The filtrate was concentrated under reduced pressure and the crude material was dissolved in dichloromethane (15 mL). *N,N*-Diisopropylethylamine (3 mL) and di-*tert*-butyl dicarbonate (0.8 mL) were then added. The resulting reaction mixture was stirred at ambient temperature for 18 hours. The mixture was acidified with aqueous hydrochloric acid (1 M) and the two phases were divided. The organic layer was dried over magnesium sulfate, filtered and concentrated under reduced pressure. The crude product was purified by flash chromatography (ethyl acetate/iso-hexane) to provide the title compound. MS (ESI) *m*/*z*: 353.2 (M-*^{t}*Bu+H)⁺.

**Step 4: 1-(*tert*-butoxycarbonyl)-4-(6,6-dimethyl-5,6-dihydrocyclopenta[c]pyrazol-1(4H)-yl)piperidine-4-carboxylic acid.** To a solution of 1-*tert*-butyl 4-methyl 4-(6,6-dimethyl-5,6-dihydrocyclopenta[c]pyrazol-1(4*H*)-yl)piperidine-1,4-dicarboxylate (0.83 g) in a solvent mixture of tetrahydrofuran (15 mL) and methanol (2.5 mL) was added a 1 M aqueous solution of lithium hydroxide monohydrate (11 mL). The resulting solution was stirred at 40 °C for 18 hours. The reaction was acidified with saturated aqueous ammonium chloride (3 mL) and extracted with ethyl acetate (3 x 3 mL). The combined organic layers were dried over magnesium sulfate, filtered and concentrated under reduced pressure to provide the title compound. MS (ESI) *m*/*z:* 364.2 (M+H)⁺.

**Step 5: *tert*-butyl 4-((4-chloro-2-(difluoromethoxy)phenyl)carbamoyl)-4-(6,6-dimethyl-5,6-dihydrocyclopenta|c]pyrazol-1(4*H*)-yl)piperidine-1-carboxylate.** To a solution of 1-(tert-butoxycarbonyl)-4-(6,6-dimethyl-5,6-dihydrocyclopenta[c]pyrazol-1(4*H*)-yl)piperidine-4-carboxylic acid (510 mg) in dichloromethane (20 mL) was added 1-(chloro(pyrrolidin-1-yl)methylene)pyrrolidin-1-ium hexafluorophosphate(V) (560 mg), 4-chloro-2-(difluoromethoxy)aniline (299 mg) and *N,N-*diisopropylethylamine (0.8 mL) and the reaction mixture stirred at ambient temperature for 3 hours. The mixture was partitioned between saturated aqueous ammonium chloride (5 mL) and ethyl acetate (5 mL). The organic layer was dried over magnesium sulfate, filtered and concentrated under reduced pressure. The crude product was purified by flash chromatography (ethyl acetate/iso-hexane) to provide the title compound. MS (ESI) *m*/*z*: 483.2 (M+H)⁺.

**Step 6: *N*-(4-chloro-2-(difluoromethoxy)phenyl)-4-(6,6-dimethyl-5,6-dihydrocyclopenta[c]pyrazol-1(4H)-yl)piperidine-4-carboxamide.** To a solution of *tert*-butyl 4-((4-chloro-2-(difluoromethoxy)phenyl)carbamoyl)-4-(6,6-dimethyl-5,6-dihydrocyclopenta[*c*]pyrazol-1(4*H*)-yl)piperidine-1-carboxylate (540 mg) in 1,4-dioxane (1.2 mL), was added hydrogen chloride (4 M in 1,4-dioxane) (1.3 mL). The resulting reaction was stirred at ambient temperature for 18 hours. The reaction was concentrated under reduced pressure to provide the title compound as a hydrochloric acid salt. MS (ESI) *m*/*z*: 439.2 (M+H)⁺.

**Step 7: methyl 4-((4-((4-chloro-2-(difluoromethoxy)phenyl)carbamoyl)-4-(6,6-dimethyl-5,6-dihydrocyclopenta[*c*]pyrazol-1(4*H*)-yl)piperidin-1-yl)methyl)tetrahydro-2*H-*pyran-4-carboxylate.** To a solution of *N*-(4-chloro-2-(difluoromethoxy)phenyl)-4-(6,6-dimethyl-5,6-dihydrocyclopenta[*c*]pyrazol-1(4*H*)-yl)piperidine-4-carboxamide, hydrochloric acid salt (60 mg) and methyl 4-formyltetrahydro-2*H*-pyran-4-carboxylate (25 mg) in 1,2-dichloroethane (1.5 mL) was added tetramethylammonium triacetoxyhydroborate (47 mg) and the resulting suspension was stirred at ambient temperature for 72 hours. The reaction was quenched with saturated aqueous ammonium chloride (3 mL). The mixture was extracted with ethyl acetate (3 x 4 mL). The combined organic layers were dried over magnesium sulfate, filtered and concentrated under reduced pressure. The crude product was purified by flash chromatography (ethyl acetate/isohexane) to provide the title compound. MS (ESI) *m*/*z*: 595.2 (M+H)⁺.

**Step 8: Compound 35.** To a solution of methyl 4-((4-((4-chloro-2-(difluoromethoxy)phenyl)carbamoyl)-4-(6,6-dimethyl-5,6-dihydrocyclopenta[*c*]pyrazol-1(4*H*)-yl)piperidin-1-yl)methyl)tetrahydro-2*H*-pyran-4-carboxylate (35 mg) in tetrahydrofuran (2 mL) and methanol (0.25 mL), was added lithium hydroxide (20 mg) in water (0.5 mL). The reaction was stirred at 40 °C for 18 hours. The volatiles were removed under reduced pressure. The crude material was dissolved in ethyl acetate (5 mL) and acidified with hydrogen chloride (4 M in 1,4-dioxane) (0.15 mL). The solvent was removed under reduced pressure. The crude product was purified by flash chromatography (acetonitrile/water 0.1% formic acid) to provide the title compound. ¹H NMR (400 MHz, dimethyl sulfoxide-*d*₆) δ ppm 1.15 (s, 6H), 1.59 - 1.64 (m, 2H), 1.91 - 1.99 (m, 2H), 2.27 (t, *J =* 6.7 Hz, 2H), 2.54 (d, *J =* 6.3 Hz, 1H), 2.72 - 2.78 (m, 5H), 3.06 (s, 2H), 3.46 - 3.63 (m, 4H), 3.63 - 3.75 (m, 4H), 7.00 - 7.41 (m, 4H), 7.49 (s, 1H), 8.03 (s, 1H), 13.42 (s, 1H). MS (ESI) *m*/*z*: 582.2 (M+H)⁺.

### Example #25: Compound 66: 4-[[4-[[4-chloro-2-(difluoromethoxy)phenyl]carbamoyl]-4-(4-fluoro-5-isopropyl-pyrazol-1-yl)-1-piperidyl]methyl]tetrahydropyran-4-carboxylic acid

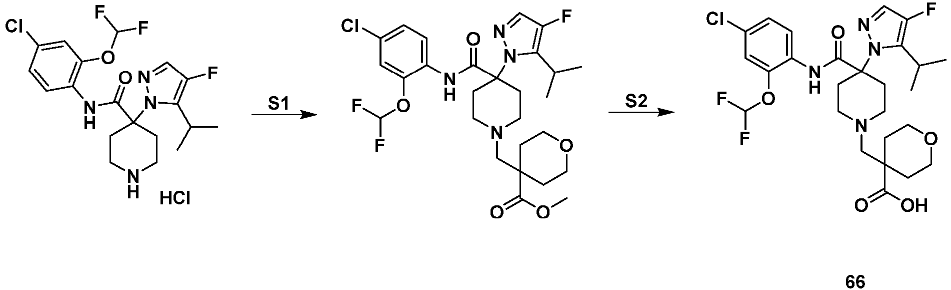

**Step 1: methyl 4-((4-((4-chloro-2-(difluoromethoxy)phenyl)carbamoyl)-4-(4-fluoro-5-isopropyl-1*H*-pyrazol-1-yl)piperidin-1-yl)methyl)tetrahydro-2*H*-pyran-4-carboxylate.** To a stirred solution of *N*-(4-chloro-2-(difluoromethoxy)phenyl)-4-(4-fluoro-5-isopropyl-1*H*-pyrazol-1-yl)piperidine-4-carboxamide hydrochloride (0.31 g) (the product of **Step 5, Example #5**), *N,N-*diisopropylethylamine (0.17 mL) and methyl 4-formyltetrahydro-2*H*-pyran-4-carboxylate (0.163 g) in dichloromethane (6 mL) was added tetramethylammonium triacetoxyhydroborate (0.249 g). The resulting suspension was stirred at ambient temperature for 18 hours. The reaction was carefully quenched with saturated aqueous sodium bicarbonate (30 mL) and extracted with dichloromethane (2 x 20 mL). The combined organics were dried over magnesium sulfate, filtered and concentrated under reduced pressure. The crude product was purified by flash chromatography (methanol/dichloromethane) to provide the title compound. MS (ESI) *m*/*z*: 588.2 (M+H)⁺.

**Step 2: Compound 66.** To a stirred solution of methyl 4-((4-((4-chloro-2-(difluoromethoxy)phenyl)carbamoyl)-4-(4-fluoro-5-isopropyl-1*H*-pyrazol-1-yl)piperidin-1-yl)methyl)tetrahydro-2*H*-pyran-4-carboxylate (0.395 g) in methanol (2 mL) and tetrahydrofuran (2 mL) was added aqueous 2 M lithium hydroxide (3 mL). The reaction mixture was stirred at 45 °C for 18 hours and then at 60 °C for 30 minutes. The reaction mixture was cooled to ambient temperature and concentrated under reduced pressure. The crude material was acidified with 4 M hydrogen chloride in 1,4-dioxane (1 mL) and concentrated under reduced pressure once more. The crude product was purified by flash chromatography (methanol/dichloromethane) to provide the title compound. ¹H NMR (400 MHz, dimethyl sulfoxide-*d*₆) δ ppm 1.15 (d, *J =* 6.8 Hz, 6H), 1.41 - 1.50 (m, 2H), 1.86 (d, *J =* 13.3 Hz, 2H), 2.29 - 2.38 (m, 5H), 2.44 - 2.48 (m, 2H), 2.69 (dd, *J =* 10.5, 5.1 Hz, 3H), 2.92 - 3.03 (m, 1H), 3.38 (d, *J* = 11.1 Hz, 2H), 3.65 - 3.72 (m, 2H), 7.17 (t, *J* = 73.4 Hz, 1H), 7.33 (d, *J =* 7.0 Hz, 2H), 7.57 (d, *J =* 4.2 Hz, 1H), 7.61 (d, *J =* 9.2 Hz, 1H), 8.59 (s, 1H), 12.51 (s, 1H). MS (ESI) *m*/*z*: 574.2 (M+H)⁺.

### Example #26: Compound 81: 4-[[4-[[4-chloro-2-(difluoromethoxy)phenyl]carbamoyl]-4-(5-isopropyl-4-methoxy-pyrazol-1-yl)-1-piperidyl]methyl]tetrahydropyran-4-carboxylic acid

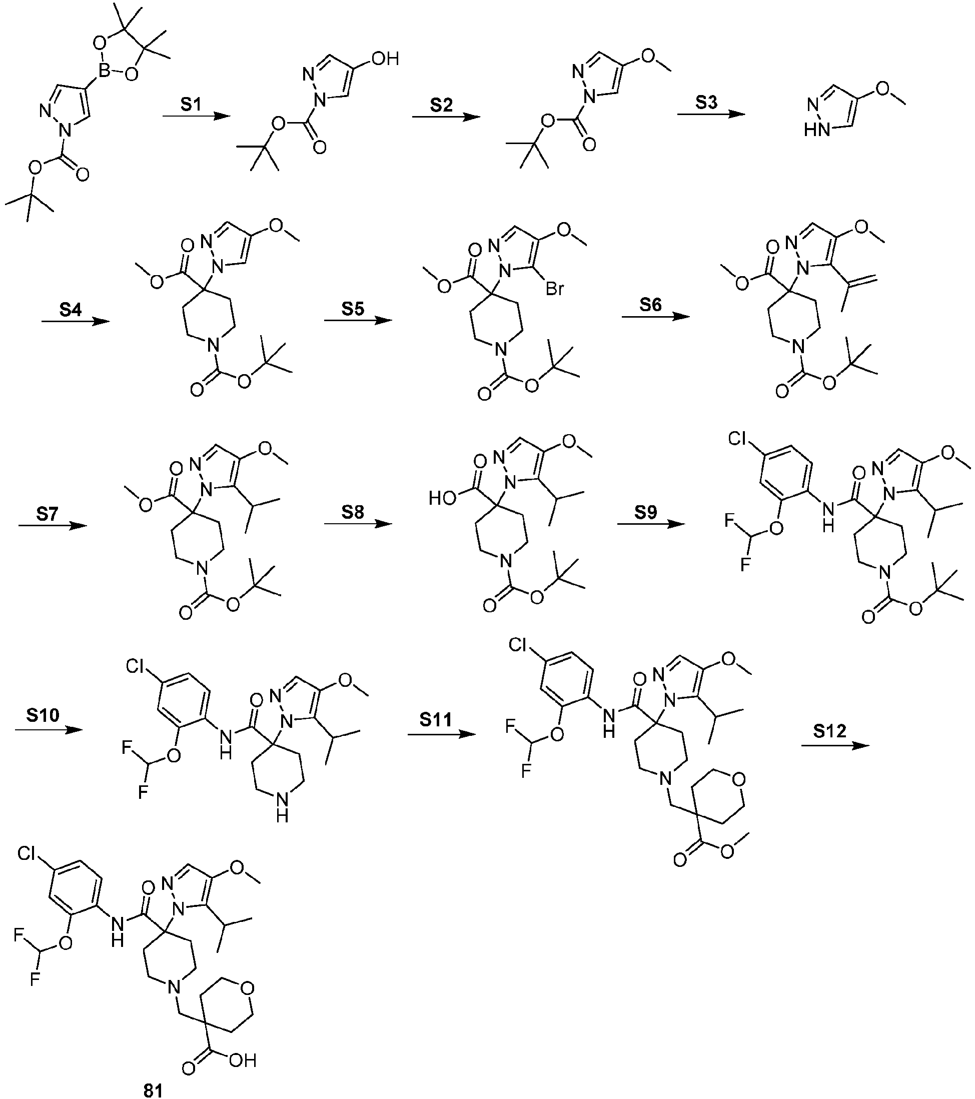

**Step 1: *tert*-butyl 4-hydroxy-1*H*-pyrazole-1-carboxylate.** To a stirred solution of *tert-*butyl 4-(4,4,5,5-tetramethyl-1,3,2-dioxaborolan-2-yl)-1*H*-pyrazole-1-carboxylate (45 g) in tetrahydrofuran (300 mL) at 0 °C was added 2 M aqueous sodium hydroxide (153 mL) and hydrogen peroxide (31 mL) sequentially. The resulting reaction mixture was stirred at 0 °C for 10 minutes before being allowed to warm to ambient temperature for a further 50 minutes. The mixture was then re-cooled to 0 °C and diluted with dichloromethane (300 mL). Aqueous hydrochloric acid (2M) was added until a pH of 2 was reached. The organics were then separated, dried over magnesium sulfate, filtered, and concentrated under reduced pressure to provide the title compound. MS (ESI) *m*/*z*: 129.1 (M-*^{t}*Bu+H)⁺.

**Step 2: *tert*-butyl 4-methoxy-1*H*-pyrazole-1-carboxylate.** To a stirred solution of *tert-*butyl 4-hydroxy-1*H*-pyrazole-1-carboxylate (36 g) and cesium carbonate (100 g) in anhydrous *N,N-*dimethylformamide (300 mL) was added methyl iodide (11.5 mL). The reaction mixture was stirred at ambient temperature for 18 hours before being quenched with water (1000 mL) and extracted with methyl *tert*-butyl ether (3 × 300 mL). The combined organics were washed with brine (500 mL), dried over magnesium sulfate, filtered, and concentrated under reduced pressure. The crude product was purified by flash column chromatography (ethyl acetate/iso-hexane) to provide the title compound. MS (ESI) *m*/*z*: 143.1 (M-*^{t}*Bu+H)⁺.

**Step 3: 4-methoxy-1*H*-pyrazole.** A mixture of *tert*-butyl 4-methoxy-1*H*-pyrazole-1-carboxylate (27 g) in 4 M hydrogen chloride in 1,4-dioxane (150 mL) was heated at 50 °C with stirring for 2 hours. After cooling to ambient temperature, the mixture was concentrated under reduced pressure. The resulting residue was triturated with diethyl ether (100 mL) and the material collected by filtration, washing with further portions of diethyl ether (2 × 25 mL), to provide the title compound as a HCl salt. ¹H NMR (400 MHz, dimethyl sulfoxide-*d*₆) δ ppm 3.68 (s, 3H), 7.48 (s, 2H), NH not observed.

**Step 4: 1-(*tert*-butyl) 4-methyl 4-(4-methoxy-1*H*-pyrazol-1-yl)piperidine-1,4-dicarboxylate.** To an ice-cooled solution of *tert*-butyl 4-oxopiperidine-1-carboxylate (22 g) and 4-methoxy-1*H*-pyrazole, hydrochloric acid salt (10 g) in tetrahydrofuran (300 mL) was added sodium hydroxide (15 g) and the mixture was stirred for 15 minutes prior to the addition of a solution of chloroform (30 mL) in tetrahydrofuran (50 mL) over 15 minutes at 0 °C. The reaction mixture was allowed to warm to ambient temperature and stirred for 18 hours. The solvent was removed under reduced pressure and the material was partitioned between aqueous 1N aqueous hydrochloric acid (200 mL) and ethyl acetate (150 mL). The aqueous layer was extracted with ethyl acetate (2 × 50 mL). The combined organics were washed with water (2 × 50 mL) and brine (50 mL), dried over magnesium sulfate, filtered, and concentrated under reduced pressure to provide the crude product which was telescoped into the next step without further purification.

Cesium carbonate (39.7 g) and methyl iodide (5.0 mL) were added sequentially to a stirred solution of crude 1-(*tert*-butoxycarbonyl)-4-(4-methoxy-1*H*-pyrazol-1-yl)piperidine-4-carboxylic acid (33 g) in *N,N*-dimethylformamide (250 mL). The reaction was stirred for 1 hour at ambient temperature. After this time, the reaction mixture was diluted with water (300 mL) and methyl *tert-*butyl ether (100 mL), and the aqueous layer was extracted with further methyl *tert*-butyl ether (2 × 100 mL). The combined organics were washed with brine (100 mL), dried over magnesium sulfate, filtered, and concentrated under reduced pressure to provide the title compound. MS (ESI) *m*/*z*: 284.1 (M-*^{t}*Bu+H)⁺.

**Step 5: 1-(*tert*-butyl) 4-methyl 4-(5-bromo-4-methoxy-1*H*-pyrazol-1-yl)piperidine-1,4-dicarboxylate.** A 1 L 3-neck round bottom flask was charged with diisopropylamine (14 mL) and tetrahydrofuran (190 mL) and cooled to 0 °C. *n*-Buyllithium (48 mL) was added dropwise, and the reaction mixture was stirred at 0 °C for 45 minutes before cooling further to -78 °C. A solution of 1-*tert*-butyl 4-methyl 4-(4-methoxy-1*H*-pyrazol-1-yl)piperidine-1,4-dicarboxylate (11.5 g) in tetrahydrofuran (190 mL) under N₂ at -78 °C was added over 30 minutes. After 10 minutes, 1,2-dibromo-1,1,2,2-tetrachloroethane (20.26 g) was added in one portion and the mixture was allowed to stir for 10 minutes before being allowed to warm to ambient temperature for 1 hour. The reaction mixture was quenched with water (100 mL) and extracted with ethyl acetate (3 × 50 mL). The combined organics were washed with brine (100 mL), dried over magnesium sulfate, filtered, and concentrated under reduced pressure. The residue was purified by flash chromatography (ethyl acetate/iso-hexane) to provide the title compound. MS (ESI) *m*/*z*: 362.0/364.0 (M+H)⁺.

**Step 6: 1-(*tert*-butyl) 4-methyl 4-(4-methoxy-5-(prop-1-en-2-yl)-1*H*-pyrazol-1-yl)piperidine-1,4-dicarboxylate.** A flask was charged with 1-*tert*-butyl 4-methyl 4-(5-bromo-4-methoxy-1*H*-pyrazol-1-yl)piperidine-1,4-dicarboxylate (5.38 g), potassium isopropenyltrifluoroborate (1.71 g), potassium carbonate (2.13 g), bis(triphenylphosphine)palladium(II) dichloride (0.27 g), 1,4-dioxane (30 mL) and water (10 mL). The mixture was degassed by bubbling with N₂ for 15 minutes before being heated to 80 °C and stirred for 18 hours. After cooling to ambient temperature, the mixture was partitioned between ethyl acetate (25 mL) and water (50 mL). The layers were separated, and the organic layer was washed with brine (50 mL), dried over magnesium sulfate, filtered, and concentrated under reduced pressure. The residue was purified by flash chromatography (ethyl acetate/iso-hexane) to provide the title compound. MS (ESI) *m*/*z*: 380.1 (M+H)⁺.

**Step 7: 1-(*tert*-butyl) 4-methyl 4-(5-isopropyl-4-methoxy-1*H*-pyrazol-1-yl)piperidine-1,4-dicarboxylate.** A glass reactor insert was charged with 1-*tert*-butyl 4-methyl 4-(4-methoxy-5-(prop-1-en-2-yl)-1*H*-pyrazol-1-yl)piperidine-1,4-dicarboxylate (2.25 g), tris(triphenylphosphine)rhodium(I) chloride (0.494 g), ethanol (15 mL) and acetic acid (1.5 mL). The mixture was placed under hydrogen atmosphere (5 bar) and stirred for 18 hours at ambient temperature. The mixture was filtered through a Whatman GF/F filter paper and the filtrate was concentrated under reduced pressure. The crude product was purified by flash chromatography on silica gel (ethyl acetate/iso-hexane) to provide the title compound. MS (ESI) *m*/*z*: 382.2 (M+H)⁺.

**Step 8: 1-(*tert*-butoxycarbonyl)-4-(5-isopropyl-4-methoxy-1H-pyrazol-1-yl)piperidine-4-carboxylic acid.** To a solution of 1-*tert*-butyl 4-methyl 4-(5-isopropyl-4-methoxy-1*H*-pyrazol-1-yl)piperidine-1,4-dicarboxylate (1.7 g) in methanol (10 mL) was added a 2 M aqueous solution of lithium hydroxide (11 mL) and the mixture allowed to stir for 18 hours at 45 °C. The reaction mixture was diluted with ethyl acetate (30 mL), acidified with 1 M aqueous hydrochloric acid to pH ~3 (~10 mL) and extracted with ethyl acetate (3 × 10 mL). The combined organics were washed with water (20 mL) and brine (20 mL), dried over magnesium sulfate, filtered, and concentrated under reduced pressure to provide the title compound. MS (ESI) *m*/*z*: 312.2 (M-*^{t}*Bu+H)⁺.

**Step 9: *tert*-butyl 4-((4-chloro-2-(difluoromethoxy)phenyl)carbamoyl)-4-(5-isopropyl-4-methoxy-1*H*-pyrazol-1-yl)piperidine-1-carboxylate.** To a solution of 1-(*tert-*butoxycarbonyl)-4-(5-isopropyl-4-methoxy-1*H*-pyrazol-1-yl)piperidine-4-carboxylic acid (1.4 g) in anhydrous dichloromethane (10 mL) was added 1-(chloro(pyrrolidin-1-yl)methylene)pyrrolidin-1-ium hexafluorophosphate(V) (1.25 g) and *N,N*-diisopropylethylamine (2 mL). 4-Chloro-2-(difluoromethoxy)aniline (0.80 g) was added and the reaction was allowed to stir at ambient temperature for 18 hours. The reaction mixture was quenched with saturated aqueous ammonium chloride (20 mL) and extracted with dichloromethane (2 × 20 mL). The combined organics were dried over magnesium sulfate, filtered, and concentrated under reduced pressure. The crude product was purified by flash chromatography on silica gel (ethyl acetate/iso-hexane) to provide the title compound. MS (ESI) *m*/*z*: 487.1 (M-*^{t}*Bu+H)⁺.

**Step 10: *N*-(4-chloro-2-(difluoromethoxy)phenyl)-4-(5-isopropyl-4-methoxy-1*H-*pyrazol-1-yl)piperidine-4-carboxamide.** Hydrogen chloride in 1,4-dioxane (4 M, 5 mL) was added to a stirred solution of *tert*-butyl 4-((4-chloro-2-(difluoromethoxy)phenyl)carbamoyl)-4-(5-isopropyl-4-methoxy-1*H*-pyrazol-1-yl)piperidine-1-carboxylate (1.2 g) in 1,4-dioxane (10 mL) and the resulting reaction mixture was allowed to stir for 18 hours at ambient temperature. The reaction mixture was concentrated under reduced pressure, azeotroping with dichloromethane (5 mL) and methyl tert-butyl ether (2 × 5 mL). A further portion of methyl tert-butyl ether (20 mL) was added, and the mixture was filtered. The material was dried under vacuum at 45 °C to provide the title compound as a hydrogen chloride salt. MS (ESI) *m*/*z*: 443.1 (M+H)⁺.

**Step 11: methyl 4-((4-((4-chloro-2-(difluoromethoxy)phenyl)carbamoyl)-4-(5-isopropyl-4-methoxy-1*H*-pyrazol-1-yl)piperidin-1-yl)methyl)tetrahydro-2H-pyran-4-carboxylate.** To a stirred solution of *N*-(4-chloro-2-(difluoromethoxy)phenyl)-4-(5-isopropyl-4-methoxy-1*H*-pyrazol-1-yl)piperidine-4-carboxamide, hydrochloric acid (0.05 g) and methyl 4-formyltetrahydro-2*H*-pyran-4-carboxylate (0.024 g) in 1,2-dichloroethane (2 mL) was added tetramethylammonium triacetoxyhydroborate (0.074 g) and the resulting suspension was stirred at 45 °C for 18 hours. The reaction was quenched by the addition of saturated aqueous sodium hydrogen carbonate (15 mL) and diluted with dichloromethane (10 mL). The aqueous layer was extracted with dichloromethane (2 × 10 mL). The combined organics were dried over magnesium sulfate, filtered, and concentrated under reduced pressure. The crude product was purified by flash chromatography (ethyl acetate/iso-hexane) to provide the title compound. MS (ESI) *m*/*z*: 599.0 (M+H)⁺.

**Step 12: Compound 81.** To a solution of methyl 4-((4-((4-chloro-2-(difluoromethoxy)phenyl)carbamoyl)-4-(5-isopropyl-4-methoxy-1*H*-pyrazol-1-yl)piperidin-1-yl)methyl)tetrahydro-2*H*-pyran-4-carboxylate (0.025 g) in methanol (0.5 mL) was added a solution of 2 M aqueous lithium hydroxide (0.1 mL) and the mixture allowed to stir for 18 hours at 45 °C before concentrating under reduced pressure. Hydrogen chloride in 1,4-dioxane (4 M, 2 mL) was then added and the reaction mixture was concentrated under reduced pressure once more. The crude product was purified by 2 g isolute cartridge, eluting with water (5 mL) and then acetonitrile (2 × 5 mL) and concentrated under reduced pressure to provide the title compound. ¹H NMR (400 MHz, dimethyl sulfoxide-*d*₆) δ ppm 1.10 (d, *J* = 6.7 Hz, 6H), 1.40 - 1.50 (m, 2H), 1.81 - 1.90 (m, 2H), 2.28 - 2.36 (m, 6H), 2.4 - 2.49 (m, 2H), 2.67 - 2.74 (m, 2H), 2.83 - 2.90 (m, 1H), 3.38 (d, *J =* 11.1 Hz, 2H), 3.61 - 3.77 (m, 5H), 6.95 - 7.38 (m, 3H), 7.46 (s, 1H), 7.78 (d, *J* = 9.2 Hz, 1H), 8.15 (s, 1H), 12.53 (s, 1H). MS (ESI) *m*/*z*: 585.1 (M+H)⁺.

### Example #27: Compound 97: 3-[[4-[[4-chloro-2-(difluoromethoxy)phenyl]carbamoyl]-4-(5-isopropylpyrazol-1-yl)-1-piperidyl]methyl]-1-ethoxycarbonyl-azetidine-3-carboxylic acid

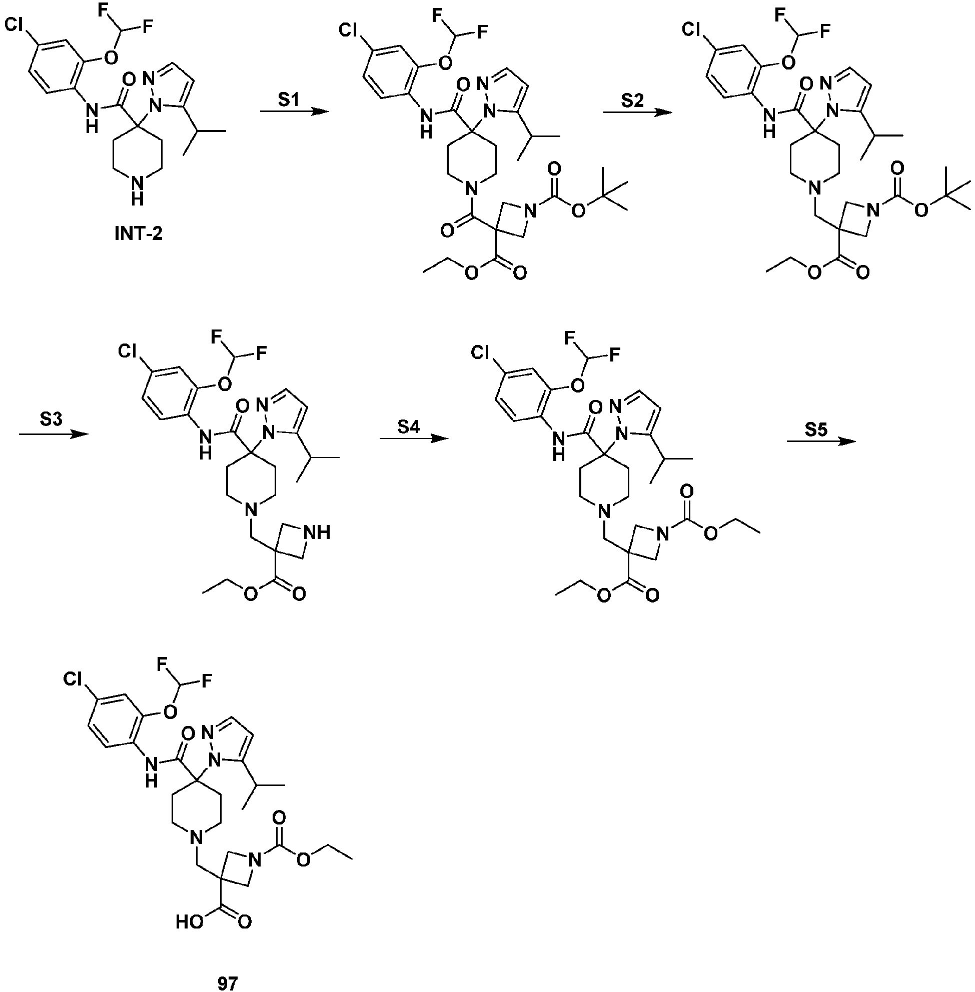

**Step 1: 1-*tert-*butyl 3-ethyl 3-(4-((4-chloro-2-(difluoromethoxy)phenyl)carbamoyl)-4-(5-isopropyl-1*H*-pyrazol-1-yl)piperidine-1-carbonyl)azetidine-1,3-dicarboxylate.** To a solution of *N*-(4-chloro-2-(difluoromethoxy)phenyl)-4-(5-isopropyl-1*H*-pyrazol-1-yl)piperidine-4-carboxamide, hydrochloric acid (**INT-2,** 396 mg) and 1-(*tert*-butoxycarbonyl)-3-(ethoxycarbonyl)azetidine-3-carboxylic acid (290 mg) in ethyl acetate (5 mL) was added triethylamine (0.3 mL) followed by propanephosphonic acid anhydride (50% in ethyl acetate) (0.8 mL). The resulting solution was stirred for 90 minutes at 60 °C. The reaction mixture was then quenched with saturated aqueous sodium hydrogen carbonate (10 mL) and the aqueous layer was extracted with ethyl acetate (3 x 10 mL). The combined organic layers were washed with brine (25 mL), dried over sodium sulfate, filtered and concentrated under reduced pressure. The crude product was purified by flash chromatography (ethyl acetate/iso-hexane) to provide the title compound. MS (ESI) *m*/*z*: 612.2 (M-*^{t}*Bu+H)⁺.

**Step 2: *1-tert-*butyl 3-ethyl 3-((4-((4-chloro-2-(difluoromethoxy)phenyl)carbamoyl)-4-(5-isopropyl-1*H*-pyrazol-1-yl)piperidin-1-yl)methyl)azetidine-1,3-dicarboxylate.** To a solution of 1-*tert*-butyl 3-ethyl 3-(4-((4-chloro-2-(difluoromethoxy)phenyl)carbamoyl)-4-(5-isopropyl-1*H*-pyrazol-1-yl)piperidine-1-carbonyl)azetidine-1,3-dicarboxylate (250 mg) and 1,1,3,3-tetramethyldisiloxane (1 mL) in dichloromethane (12 mL) was added carbonylchlorobis(triphenylphosphine)iridium (I) (40 mg) and the reaction mixture was stirred at ambient temperature for 4 hours. Trifluoroacetic acid (0.1 mL) was then added, and reaction mixture was stirred at ambient temperature for 2 hours. The reaction mixture was concentrated under reduced pressure twice from toluene (5 mL). The material was partitioned between dichloromethane (10 mL) and saturated aqueous sodium bicarbonate (10 mL). The aqueous phase was extracted with dichloromethane (2 x 10 mL) and the combined organic solution was washed sequentially with water (10 mL) and brine (10 mL). The mixture was dried over magnesium sulfate, filtered and concentrated under reduced pressure. The crude product was purified by flash chromatography (ethyl acetate (containing 2% triethylamine)/iso-hexane) to provide the title compound. MS (ESI) *m*/*z:* 654.2 (M+H)⁺.

**Step 3: ethyl 3-((4-((4-chloro-2-(difluoromethoxy)phenyl)carbamoyl)-4-(5-isopropyl-1*H*-pyrazol-1-yl)piperidin-1-yl)methyl)azetidine-3-carboxylate.** To a solution of 1-*tert-*butyl 3-ethyl 3-((4-((4-chloro-2-(difluoromethoxy)phenyl)carbamoyl)-4-(5-isopropyl-1*H-*pyrazol-1-yl)piperidin-1-yl)methyl)azetidine-1,3-dicarboxylate (102 mg) in dichloromethane (1 mL) was added dropwise trifluoroacetic acid (0.5 mL) and the resulting solution was stirred for 3 hours at ambient temperature. The reaction mixture was diluted with saturated aqueous sodium hydrogen carbonate (25 mL) and extracted with dichloromethane (3 x 25 mL). The combined organics were dried over sodium sulfate, filtered and concentrated under reduced pressure. The residue was taken up in 1 mL of 3 M hydrogen chloride in cyclopentyl methyl ether and concentrated under reduced pressure to provide the title compound. MS (ESI) *m*/*z*: 554.2 (M+H)⁺.

**Step 4: diethyl 3-((4-((4-chloro-2-(difluoromethoxy)phenyl)carbamoyl)-4-(5-isopropyl-1H-pyrazol-1-yl)piperidin-1-yl)methyl)azetidine-1,3-dicarboxylate.** To a solution of ethyl 3-((4-((4-chloro-2-(difluoromethoxy)phenyl)carbamoyl)-4-(5-isopropyl-1*H*-pyrazol-1-yl)piperidin-1-yl)methyl)azetidine-3-carboxylate (0.090 g) and ethyl carbonochloridate (20 µL) in dichloromethane (3 mL) was added *N,N*-diisopropylethylamine (60 µL) dropwise and reaction was stirred at ambient temperature for 18 hours. The reaction was quenched with methanol (0.5 mL) and concentrated under reduced pressure. The residue was purified v by flash chromatography (methanol with 0.7 M ammonia in dichloromethane) to provide the title compound. MS (ESI) *m*/*z*: 626.2 (M+H)⁺.

**Step 5: Compound** 97. To a solution of diethyl 3-((4-((4-chloro-2-(difluoromethoxy)phenyl)carbamoyl)-4-(5-isopropyl-1*H*-pyrazol-1-yl)piperidin-1-yl)methyl)azetidine-1,3-dicarboxylate (88 mg) in tetrahydrofuran (1.5 mL) was added lithium hydroxide (2 M in water) (0.70 mL) and the reaction mixture stirred for 3 hours at 45 °C. The reaction mixture was concentrated under reduced pressure. The resulting residue was dissolved in dichloromethane (2 mL) and acidified with 6 M hydrogen chloride in isopropanol (0.5 mL) before concentration under reduced pressure. The crude product was purified by preparative HPLC (acetonitrile in water) to provide the title compound. ¹H NMR (400 MHz, dimethyl sulfoxide-*d*₆) δ ppm 1.10 (d, *J* = 6.6 Hz, 6H), 1.15 (t, *J* = 7.1 Hz, 3H), 2.22 - 2.39 (m, 2H), 2.42 - 2.48 (m, 2H), 2.61 - 2.76 (m, 2H), 2.83 (s, 2H), 2.87 - 3.02 (m, 1H), 3.34 - 3.43 (m, 2H), 3.76 (s, 2H), 3.99 (q, *J* = 7.1 Hz, 2H), 4.02 - 4.11 (m, 2H), 6.33 (d, *J* = 1.9 Hz, 1H), 6.93 - 7.43 (m, 3H), 7.53 (d, *J* = 1.8 Hz, 1H), 7.76 (d, *J =* 9.4 Hz, 1H), 8.22 (s, 1H), 13.50 (s, 1H). MS (ESI) *m*/*z:* 598.5 (M+H)⁺.

### Example #28: Compound 103: (1s,3s)-3-[4-[[4-chloro-2-(difluoromethoxy)phenyl]carbamoyl]-4-(6,6-dimethyl-5H-furo[3,2-c]pyrazol-1-yl)-1-piperidyl]cyclobutanecarboxylic acid

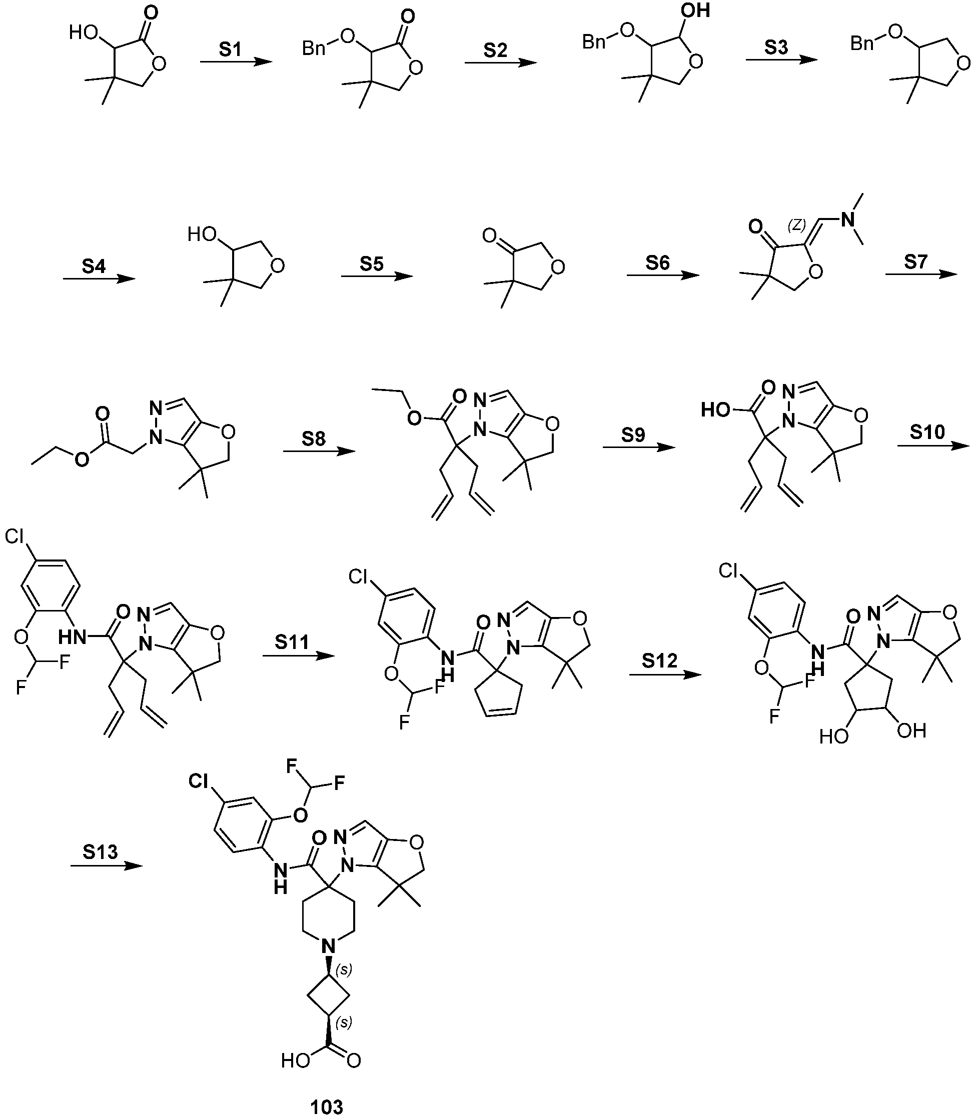

**Step 1: 3-(benzyloxy)-4,4-dimethyldihydrofuran-2(3*H*)-one.** Benzyl bromide (22 mL) was added to a suspension of cesium carbonate (100 g) and 3-hydroxy-4,4-dimethyldihydrofuran-2(3*H*)-one (20 g) in dichloromethane (500 mL). The reaction mixture was stirred at ambient temperature for 18 hours. The crude mixture was filtered, washed with dichloromethane (50 mL), and the filtrate was concentrated under reduced pressure. The residue was purified by flash column chromatography (methyl *tert*-butyl ether/iso-hexane) to provide the title compound. ¹H NMR (400 MHz, dimethyl sulfoxide-*d*₆) δ ppm 0.99 (s, 3H), 1.08 (s, 3H), 3.96 (s, 2H), 4.14 (s, 1H), 4.69 (d, *J* = 12.0 Hz, 1H), 4.91 (d, *J* = 12.0 Hz, 1H), 7.16 - 7.54 (m, 5H).

**Step 2: 3-(benzyloxy)-4,4-dimethyltetrahydrofuran-2-ol.** Diisobutylaluminum hydride (150 mL) was added dropwise via an addition funnel over 30 minutes to a stirred solution of 3-(benzyloxy)-4,4-dimethyldihydrofuran-2(3*H*)-one (26.8 g) in dichloromethane (490 mL) at -78 °C under nitrogen. The mixture was stirred at -78 °C for a further 2 hours before being quenched carefully with 1 M aqueous sulfuric acid (240 mL) and diluted with methyl *tert*-butyl ether (240 mL) and ethyl acetate (400 mL). The mixture was stirred for 15 minutes before being transferred to a separatory funnel. The layers were separated and the organic layer washed with 1 M aqueous sulfuric acid (800 mL), saturated aqueous sodium bicarbonate solution (500 mL) and brine (500 mL). The organic layer was dried over magnesium sulfate, filtered and the solvent removed under reduced pressure. The residue was purified by flash column chromatography (methyl *tert*-butyl ether/iso-hexane) to provide the title compound. ¹H NMR (400 MHz, dimethyl sulfoxide-*d*₆) δ ppm 1.00 (s, 3H), 1.04 (s, 3H), 3.41 (d, *J* = 3.0 Hz, 1H), 3.46 (d, *J* = 8.3 Hz, 1H), 3.63 (d, *J* = 8.3 Hz, 1H), 4.52 (d, *J* = 12.1 Hz, 1H), 4.63 (d, *J* = 12.2 Hz, 1H), 5.16 (dd, *J* = 5.3, 3.1 Hz, 1H), 6.36 (d, *J* = 5.2 Hz, 1H), 7.10 - 7.45 (m, 5H).

**Step 3: 4-(benzyloxy)-3,3-dimethyltetrahydrofuran.** Triethylsilane (55 mL) and boron trifluoride diethyl etherate (16 mL) were added sequentially to a stirred solution of 3-(benzyloxy)-4,4-dimethyltetrahydrofuran-2-ol (25.6 g) in anhydrous dichloromethane (450 mL) at -78 °C under nitrogen. The mixture was stirred at -78 °C for 2 hours before being allowed to warm to 0 °C and stirred for a further 1 hour. The mixture was cooled to -78 °C, sodium hydrogen carbonate (50 g) was added and the mixture was stirred at -78 °C for 15 minutes. Saturated aqueous sodium hydrogen carbonate solution (250 mL) was added, and the mixture was allowed to warm to ambient temperature over 1 hour then stirred for a further 16 hours at ambient temperature. The layers were separated, and the aqueous layer was extracted with ethyl acetate (200 mL). The combined organic layers were dried over magnesium sulfate, filtered and the solvent was removed under reduced pressure. The residue was purified by flash column chromatography (methyl tert-butyl ether/isohexane) to provide the title compound. ¹H NMR (400 MHz, dimethyl sulfoxide-*d*₆) δ ppm 0.98 (s, 3H), 1.05 (s, 3H), 3.40 (d, *J* = 7.8 Hz, 1H), 3.44 (d, *J* = 7.8 Hz, 1H), 3.61 (dd, *J* = 5.0, 2.8 Hz, 1H), 3.69 (dd, *J* = 9.7, 2.8 Hz, 1H), 3.96 (dd, *J* = 9.7, 5.0 Hz, 1H), 4.45 (d, *J* = 12.1 Hz, 1H), 4.53 (d, *J* = 12.1 Hz, 1H), 7.21 - 7.41 (m, 5H).

**Step 4: 4,4-dimethyltetrahydrofuran-3-ol.** A glass reactor insert was charged with 4-(benzyloxy)-3,3-dimethyltetrahydrofuran (22.7 g), methanol (120 mL) and palladium on carbon (JM Type 39, 2 g). The mixture was transferred to a stainless-steel pressure vessel and stirred under hydrogen atmosphere (5 bar) for 3 hours at ambient temperature. The mixture was filtered through a Whatman GF/F filter paper and the pad was washed with methanol (2 x 25 mL). The filtrate was concentrated under reduced pressure to provide the title compound. ¹H NMR (400 MHz, dimethyl sulfoxide-*d*₆) δ ppm 0.91 (s, 3H), 0.94 (s, 3H), 3.34 (d, *J =* 7.7 Hz, 1H), 3.38 - 3.47 (m, 2H), 3.67 (dd, *J =* 5.3, 3.2 Hz, 1H), 3.95 (dd, *J =* 9.1, 5.3 Hz, 1H), 4.87 (s, 1H).

**Step 5: 4,4-dimethyldihydrofuran-3(2*H*)-one.** Pyridinium chlorochromate (16.33 g) was added to a stirred solution of 4,4-dimethyltetrahydrofuran-3-ol (4.4 g) in dichloromethane (110 mL). The mixture was stirred at ambient temperature for 20 hours. The mixture was filtered through a plug of silica and eluted with ethyl acetate (500 mL). The filtrate was concentrated under reduced pressure to provide the title compound. ¹H NMR (400 MHz, dimethyl sulfoxide-*d*₆) δ ppm 1.03 (s, 6H), 3.86 (s, 2H), 3.98 (s, 2H).

**Step 6: (*Z*)-2-((dimethylamino)methylene)-4,4-dimethyldihydrofuran-3(2*H*)-one.** A mixture of *N,N*-dimethylformamide dimethyl acetal (5.2 mL) and 4,4-dimethyldihydrofuran-3(2*H*)-one (3.7 g) in *N,N*-dimethylformamide (65 mL) was stirred at 100 °C for 18 hours. After this time, the mixture was concentrated under reduced pressure. The residue was co-evaporated with toluene (2 x 50 mL). The crude product was dissolved in toluene (50 mL), filtered and the filtrate was concentrated under reduced pressure to provide the title compound. ¹H NMR (400 MHz, dimethyl sulfoxide-*d*₆) δ ppm 0.99 (s, 6H), 3.00 (s, 6H), 3.88 (s, 2H), 6.34 (s, 1H).

**Step 7: ethyl 2-(6,6-dimethyl-5,6-dihydro-1*H*-furo[3,2-*c*]pyrazol-1-yl)acetate.** Hydrochloric acid (12N, 5.5 mL) was added to a stirred suspension of ethyl 2-hydrazinylacetate, hydrochloric acid (2.02 g) and (*Z*)-2-((dimethylamino)methylene)-4,4-dimethyldihydrofuran-3(2*H*)-one (2.70 g) in ethanol (45 mL). The mixture was heated to 70 °C and stirred for 30 minutes. The mixture was allowed to cool to ambient temperature and was concentrated under reduced pressure. The residue was partitioned between ethyl acetate (25 mL) and saturated aqueous sodium hydrogen carbonate (50 mL). The layers were separated, and the organic layer was dried over magnesium sulfate, filtered and concentrated under reduced pressure. The crude product was purified by flash column chromatography (ethyl acetate/iso-hexane) to provide the title compound. MS (ESI) *m*/*z:* 225.1 (M+H)⁺.

**Step 8: ethyl 2-allyl-2-(6,6-dimethyl-5,6-dihydro-1H-furo[3,2-c]pyrazol-1-yl)pent-4-enoate.** Sodium hydride (60% dispersion in mineral oil) (0.401 g) was added in one portion to a stirred solution of ethyl 2-(6,6-dimethyl-5,6-dihydro-1*H*-furo[3,2-c]pyrazol-1-yl)acetate (0.75 g) in *N,N*-dimethylformamide (15 mL) at 0 °C under nitrogen. The mixture was stirred at 0 °C for 10 minutes before allyl bromide (1.5 mL) was added. The mixture was warmed to ambient temperature and stirred for a further 18 hours. The mixture was diluted with water (50 mL) and extracted with methyl *tert*-butyl ether (3 x 50 mL). The combined organic layers were dried over magnesium sulfate, filtered and concentrated under reduced pressure. The crude product was purified by flash column chromatography (methyl *tert*-butyl ether/iso-hexane) to provide the title compound. MS (ESI) *m*/*z*: 305.2 (M+H)⁺.

**Step 9: 2-allyl-2-(6,6-dimethyl-5,6-dihydro-1*H*-furo[3,2-*c*]pyrazol-1-yl)pent-4-enoic acid.** Lithium hydroxide monohydrate (0.520 g) was added to a stirred solution of ethyl 2-allyl-2-(6,6-dimethyl-5,6-dihydro-1*H*-furo[3,2-c]pyrazol-1-yl)pent-4-enoate (0.754 g) in methanol (10 mL). The mixture was heated to 45 °C and stirred for 18 hours. The solvent was removed under reduced pressure and the residue partitioned between ethyl acetate (20 mL) and 1N hydrochloric acid (50 mL). The layers were separated and the aqueous layer extracted with ethyl acetate (2 x 20 mL). The combined organic layers were dried over magnesium sulfate, filtered and concentrated under reduced pressure to provide the title compound. MS (ESI) *m*/*z*: 277.2 (M+H)⁺.

**Step 10: 2-allyl-N-(4-chloro-2-(difluoromethoxy)phenyl)-2-(6,6-dimethyl-5,6-dihydro-1*H*-furo[3,2-*c*]pyrazol-1-yl)pent-4-enamide.** Chloro-*N,N,N',N'-*tetramethylformamidinium hexafluorophosphate (0.833 g) was added to a stirred solution of 2-allyl-2-(6,6-dimethyl-5,6-dihydro-1*H*-furo[3,2-c]pyrazol-1-yl)pent-4-enoic acid (0.72 g), 4-chloro-2-(difluoromethoxy)aniline (0.575 g) and 1-methyl-1*H*-imidazole (0.7 mL) in acetonitrile (8 mL). The mixture was stirred at ambient temperature for 18 hours. The mixture was partitioned between ethyl acetate (10 mL) and 1N aqueous hydrochloric acid (20 mL). The layers were separated, and the aqueous layer was extracted with ethyl acetate (2 x 10 mL). The combined organic layers were washed with brine (30 mL), dried with magnesium sulfate, filtered and concentrated under reduced pressure. The residue was purified by flash column chromatography (methyl *tert*-butyl ether/isohexane) to provide the title compound. MS (ESI) *m*/*z*: 452.2 (M+H)⁺.

**Step 11: *N*-(4-chloro-2-(difluoromethoxy)phenyl)-1-(6,6-dimethyl-5,6-dihydro-1*H-*furo[3,2-*c*]pyrazol-1-yl)cyclopent-3-enecarboxamide.** Grubbs 2^{nd} generation catalyst (0.074 g) was added to a stirred solution of 2-allyl-*N*-(4-chloro-2-(difluoromethoxy)phenyl)-2-(6,6-dimethyl-5,6-dihydro-1*H*-furo[3,2-c]pyrazol-1-yl)pent-4-enamide (0.79 g) in dichloromethane (150 mL). The mixture was heated to 50 °C and stirred for 18 hours. Further Grubbs II (0.074 g, 0.087 mmol) was added and the mixture was stirred at 50 °C for a further 6 hours. After cooling to ambient temperature, the solvent was removed under reduced pressure. The residue was purified by flash column chromatography (methyl *tert*-butyl ether/iso-hexane) to provide the title compound. MS (ESI) *m*/*z*: 424.3 (M+H)⁺.

**Step 12: *N*-(4-chloro-2-(difluoromethoxy)phenyl)-1-(6,6-dimethyl-5,6-dihydro-1*H-*furo[3,2-*c*]pyrazol-1-yl)-3,4-dihydroxycyclopentanecarboxamide.** Microencapsulated osmium tetraoxide (0.3 mmol/g) (0.467 g) and 4-methylmorpholine 4-oxide (0.450 g) were added to a stirred solution of *N*-(4-chloro-2-(difluoromethoxy)phenyl)-1-(6,6-dimethyl-5,6-dihydro-1*H*-furo[3,2-*c*]pyrazol-1-yl)cyclopent-3-enecarboxamide (0.60 g) in acetone (14 mL). The mixture was heated to 45 °C and stirred for 18 hours. The mixture was filtered and the solvent removed under reduced pressure. The residue was purified by flash column chromatography (ethyl acetate/iso-hexane) to provide the title compound. MS (ESI) *m*/*z*: 458.1 (M+H)⁺.

**Step 13: Compound 103.** Sodium periodate (144 mg) was added to a stirred solution of N-(4-chloro-2-(difluoromethoxy)phenyl)-1-(6,6-dimethyl-5,6-dihydro-1*H*-furo[3,2-*c*]pyrazol-1-yl)-3,4-dihydroxycyclopentanecarboxamide (103 mg) in acetone (2 mL) and water (1 mL) at 0 °C. The mixture was allowed to warm to ambient temperature and was stirred for 1 hour. The acetone was removed under reduced pressure and the residue was partitioned between dichloromethane (10 mL) and water (10 mL). The layers were separated, and the aqueous layer was extracted with dichloromethane (2 x 10 mL). The combined organic layers were washed with brine (20 mL), dried over magnesium sulfate, filtered and concentrated under reduced pressure. The residue was taken up in methanol (3 mL) and cis-3-aminocyclobutanecarboxylic acid (25.9 mg), sodium cyanoborohydride (42.4 mg) and acetic acid (0.02 mL) were added sequentially. The mixture was stirred at ambient temperature for 72 hours. The solvent was removed under reduced pressure and the residue was purified directly by flash column chromatography (methanol/dichloromethane). The material was further purified by prep-HPLC to provide the title compound. ¹H NMR (400 MHz, dimethyl sulfoxide-*d*₆) δ ppm 1.21 (s, 6H), 1.84 - 2.05 (m, 4H), 2.13 - 2.25 (m, 2H), 2.25 - 2.39 (m, 2H), 2.45 (d, *J* = 9.5 Hz, 2H), 2.52 - 2.60 (m, 1H), 2.61 - 2.75 (m, 3H), 4.53 (s, 2H), 7.21 (t, *J* = 73.1 Hz, 1H), 7.22 (s, 1H), 7.28 - 7.34 (m, 1H), 7.35 (d, *J* = 2.3 Hz, 1H), 7.83 (s, 1H), 8.02 (d, *J =* 8.8 Hz, 1H). COOH not observed. MS (ESI) *m*/*z*: 539.1 (M+H)⁺.

### Example #29: Compound 141: (2R)-3-[4-[[4-chloro-2-(difluoromethoxy)phenyl]carbamoyl]-4-(4-fluoro-5-isopropyl-pyrazol-1-yl)-1-piperidyl]-2-tetrahydropyran-4-yl-propanoic

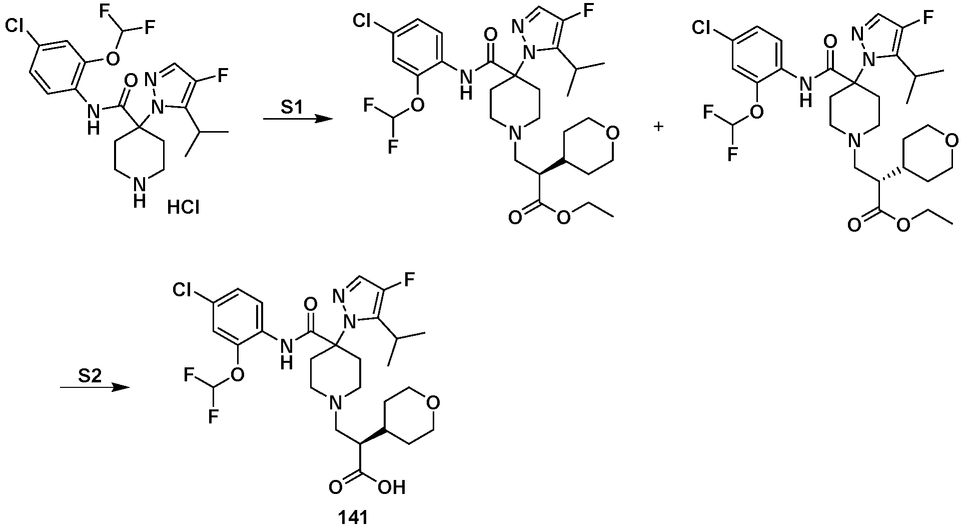

**Step** 1: **ethyl (*R*)-3-(4-((4-chloro-2-(difluoromethoxy)phenyl)carbamoyl)-4-(4-fluoro-5-isopropyl-1*H*-pyrazol-1-yl)piperidin-1-yl)-2-(tetrahydro-2*H*-pyran-4-yl)propanoate.** To a stirred solution of *N*-(4-chloro-2-(difluoromethoxy)phenyl)-4-(4-fluoro-5-isopropyl-1*H*-pyrazol-1-yl)piperidine-4-carboxamide hydrochloride (0.200 g) (the product of **Step 5, Example** *#5), N,N-*diisopropylethylamine (0.11 mL) and (*E*)-ethyl 3-hydroxy-2-(tetrahydro-2*H*-pyran-4-yl)acrylate (0.122 g) in dichloromethane (5 mL) was added tetramethylammonium triacetoxyhydroborate (0.160 g). The resulting suspension was stirred at ambient temperature for 18 hours. The reaction was carefully quenched with saturated aqueous sodium bicarbonate (30 mL) and extracted with dichloromethane (2 x 20 mL). The combined organics were dried over magnesium sulfate, filtered and concentrated under reduced pressure. The crude product was purified by flash chromatography (methanol/dichloromethane) to provide the title compound as a racemic mixture. The racemic mixture was then subjected to SFC chiral separation employing a CHIRALPAK^{®} IC 10 x 250 mm column to provide the title compound. MS (ESI) *m*/*z* 616.2 (M+H)⁺. The enantiomer ethyl (S)-3-(4-((4-chloro-2-(difluoromethoxy)phenyl)carbamoyl)-4-(4-fluoro-5-isopropyl-1*H*-pyrazol-1-yl)piperidin-1-yl)-2-(tetrahydro-2*H*-pyran-4-yl)propanoate was also isolated. MS (ESI) *m*/*z 616.2* (M+H)⁺.

Step 2: **Compound 141.** To a stirred solution of ethyl (*R*)-3-(4-((4-chloro-2-(difluoromethoxy)phenyl)carbamoyl)-4-(4-fluoro-5-isopropyl-1*H*-pyrazol-1-yl)piperidin-1-yl)-2-(tetrahydro-2*H*-pyran-4-yl)propanoate (0.100 g) in methanol (2 mL) and tetrahydrofuran (2 mL) was added a 2 M aqueous solution of lithium hydroxide (0.78 mL). The reaction mixture was stirred at 45 °C for 18 hours and at ambient temperature for 24 hours. The reaction mixture was concentrated under reduced pressure and the crude material was acidified with 4 N hydrogen chloride in 1,4-dioxane (1 mL) and concentrated under reduced pressure once more. The crude product was purified by flash chromatography (methanol/dichloromethane) to provide the title compound. ¹H NMR (500 MHz, dimethyl sulfoxide-*d*₆) δ ppm 1.17 (dd, *J =* 6.9, 3.6 Hz, 6H), 1.22 - 1.40 (m, 3H), 1.36 - 1.47 (m, 1H), 1.49 - 1.59 (m, 1H), 1.70 - 1.86 (m, 1H), 2.39 - 2.49 (m, 2H), 2.51 - 2.62 (m, 2H), 2.66 - 3.16 (m, 6H), 3.07 - 3.29 (m, 3H), 3.75 - 3.88 (m, 2H), 7.19 (t, *J* = 73.4 Hz, 1H), 7.34 (d, *J* = 7.4 Hz, 2H), 7.57 (d, *J* = 9.1 Hz, 1H), 7.62 (d, *J* = 4.1 Hz, 1H), 8.80 (s, 1H), 12.09 (s, 1H). MS (ESI) *m*/*z:* 587.2 (M+H)⁺.

### Example #30: Compound 158: (2S)-3-[4-[[4-chloro-2-(difluoromethoxy)phenyl]carbamoyl]-4-(4-fluoro-5-isopropyl-pyrazol-1-yl)-1-piperidyl]-2-tetrahydropyran-4-yl-propanoic acid

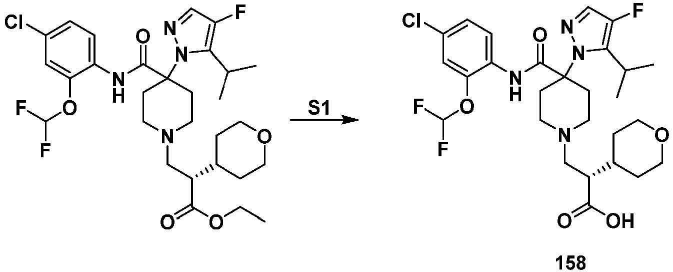

**Compound 158.** To a stirred solution of (*S*)-ethyl 3-(4-((4-chloro-2-(difluoromethoxy)phenyl)carbamoyl)-4-(4-fluoro-5-isopropyl-1*H*-pyrazol-1-yl)piperidin-1-yl)-2-(tetrahydro-2*H*-pyran-4-yl)propanoate (0.100 g) (isolated during **Step 1, Example #29**), in methanol (2 mL) and tetrahydrofuran (2 mL) was added a 2 M solution of lithium hydroxide (0.78 mL). The reaction mixture was stirred at 45 °C for 18 hours then at ambient temperature for 24 hours. The reaction mixture was concentrated under reduced pressure. The crude material was acidified with 4 N hydrogen chloride in 1,4-dioxane (1 mL) and concentrated under reduced pressure once more. The crude product was purified by flash chromatography (methanol/dichloromethane) to provide the title compound. ¹H NMR (500 MHz, dimethyl sulfoxide-*d*₆) δ ppm 1.13 - 1.18 (m, 6H), 1.17 - 1.37 (m, 3H), 1.38 - 1.45 (m, 1H), 1.53 - 1.60 (m, 1H), 1.63 - 1.76 (m, 1H), 2.20 - 2.41 (m, 7H), 2.50 - 2.65 (m, 2H), 2.74 - 2.87 (m, 1H), 2.98 (p, *J* = 6.9 Hz, 1H), 3.23 (t, *J* = 11.6 Hz, 2H), 3.74 - 3.86 (m, 2H), 7.01 - 7.36 (m, 3H), 7.56 (d, *J* = 4.1 Hz, 1H), 7.58 - 7.63 (m, 1H), 8.61 (s, 1H), 12.08 (s, 1H). MS (ESI) *m*/*z*: 589.2 (M+H)⁺.

### Example #31: Compounds 161 and 162: (1r,4r)-1-[[4-[[4-chloro-2-(difluoromethoxy)phenyl]carbamoyl]-4-(4-fluoro-5-isopropyl-pyrazol-1-yl)-1-piperidyl]methyl]-4-hydroxy-cyclohexanecarboxylic acid and (1s,4s)-1-[[4-[[4-chloro-2-(difluoromethoxy)phenyl]carbamoyl]-4-(4-fluoro-5-isopropyl-pyrazol-1-yl)-1-piperidyl]methyl]-4-hydroxy-cyclohexanecarboxylic acid

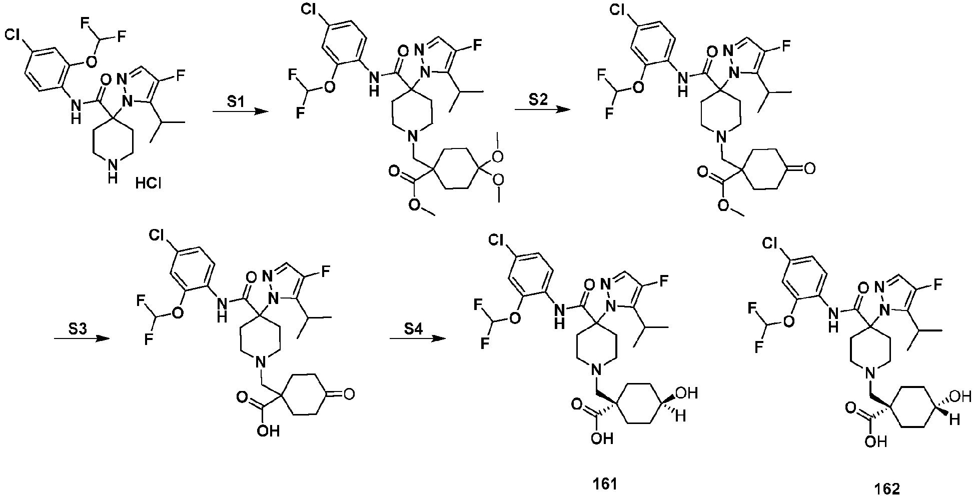

**Step 1: methyl 1-((4-((4-chloro-2-(difluoromethoxy)phenyl)carbamoyl)-4-(4-fluoro-5-isopropyl-1*H*-pyrazol-1-yl)piperidin-1-yl)methyl)-4,4-dimethoxycyclohexane-1-carboxylate.** To a stirred solution of *N*-(4-chloro-2-(difluoromethoxy)phenyl)-4-(4-fluoro-5-isopropyl-1*H*-pyrazol-1-yl)piperidine-4-carboxamide hydrochloride (0.150 g) (the product of **Step 5, Example #5**)*, N,N-diisopropylethylamine* (0.077 mL) and methyl 1-formyl-4,4-dimethoxycyclohexanecarboxylate (0.102 g) in dichloromethane (5 mL) was added tetramethylammonium triacetoxyhydroborate (0.117 g). The resulting suspension was stirred at ambient temperature for 18 hours. The reaction was carefully quenched with saturated aqueous sodium bicarbonate (30 mL) and extracted with dichloromethane (2 x 20 mL). The combined organics were dried over magnesium sulfate, filtered and concentrated under reduced pressure. The crude product was purified by flash chromatography (methanol/dichloromethane) to provide the title compound. MS (ESI) *m*/*z*: 646.2 (M+H)⁺.

**Step 2: methyl 1-((4-((4-chloro-2-(difluoromethoxy)phenyl)carbamoyl)-4-(4-fluoro-5-isopropyl-1*H*-pyrazol-1-yl)piperidin-1-yl)methyl)-4-oxocyclohexane-1-carboxylate.** To a stirred solution of methyl 1-((4-((4-chloro-2-(difluoromethoxy)phenyl)carbamoyl)-4-(4-fluoro-5-isopropyl-1*H*-pyrazol-1-yl)piperidin-1-yl)methyl)-4,4-dimethoxycyclohexanecarboxylate (0.245 g) in tetrahydrofuran (2 mL) was added 1 M hydrochloric acid (2 mL) and the solution was stirred at 50 °C for 3 hours. The resulting mixture was cooled to ambient temperature and basified with saturated aqueous sodium hydrogen carbonate solution (20 mL). The mixture was extracted with dichloromethane (3 x 20 mL) and the combined organics were dried over magnesium sulfate, filtered and concentrated under reduced pressure. The crude product was purified by flash chromatography (methanol/dichloromethane) to provide the title compound. MS (ESI) *m*/*z:* 600.2 (M+H)⁺.

**Step 3: 1-((4-((4-chloro-2-(difluoromethoxy)phenyl)carbamoyl)-4-(4-fluoro-5-isopropyl-1*H*-pyrazol-1-yl)piperidin-1-yl)methyl)-4-oxocyclohexane-1-carboxylic acid.** To a stirred solution of methyl 1-((4-((4-chloro-2-(difluoromethoxy)phenyl)carbamoyl)-4-(4-fluoro-5-isopropyl-1*H*-pyrazol-1-yl)piperidin-1-yl)methyl)-4-oxocyclohexanecarboxylate (0.220 g) in methanol (4 mL) and tetrahydrofuran (2 mL) was added a 2 M aqueous solution of lithium hydroxide (1.4 mL). The reaction mixture was stirred at 35 °C for 18 hours. The reaction mixture was concentrated under reduced pressure. The crude material was acidified with 4 N hydrogen chloride in 1,4-dioxane (1 mL) and concentrated under reduced pressure once more. The crude product was purified by flash chromatography (methanol/dichloromethane) to provide the title compound. MS (ESI) *m*/*z*: 586.2 (M+H)⁺.

**Step 4: Compound 161 and 162.** To a stirred solution of 1-((4-((4-chloro-2-(difluoromethoxy)phenyl)carbamoyl)-4-(4-fluoro-5-isopropyl-1*H*-pyrazol-1-yl)piperidin-1-yl)methyl)-4-oxocyclohexanecarboxylic acid, hydrochloric acid (60 mg) in methanol (2 mL) was added sodium borohydride (15 mg) and the solution was stirred at ambient temperature for 18 hours. Brine (20 mL) was added, and the mixture was extracted with dichloromethane (3 x 20 mL). The combined organics were dried over magnesium sulfate, filtered and concentrated under reduced pressure. The crude product was purified by flash chromatography (methanol/dichloromethane) to provide 1-((4-((4-chloro-2-(difluoromethoxy)phenyl)carbamoyl)-4-(4-fluoro-5-isopropyl-1*H-*pyrazol-1-yl)piperidin-1-yl)methyl)-4-hydroxycyclohexanecarboxylic acid as a mixture of cis and trans isomeric products. The cis and trans isomeric mixture was then subjected to achiral separation employing a Waters XBridge BEH C18 ODB column to provide the title compounds. **Compound 161.** ¹H NMR (500 MHz, dimethyl sulfoxide-*d*₆) δ ppm 1.15 (d, *J* = 6.8 Hz, 6H), 1.29 - 1.45 (m, 2H), 1.48 - 1.67 (m, 6H), 2.26 - 2.40 (m, 4H), 2.43 - 2.50 (m, 4H), 2.65 - 2.75 (m, 2H), 2.98 (p, *J* = 7.0 Hz, 1H), 3.55 (s, 1H), 4.34 (s, 1H), 7.00 - 7.36 (m, 3H), 7.56 (d, *J =* 4.1 Hz, 1H), 7.62 (d, *J =* 9.2 Hz, 1H), 8.58 (s, 1H), 12.34 (s, 1H). MS (ESI) *m*/*z*: 589.2 (M+H)⁺. **Compound 162.** ¹H NMR (500 MHz, dimethyl sulfoxide-*d*₆) δ ppm 1.07 - 1.28 (m, 10H), 1.61 - 1.70 (m, 2H), 1.95 - 2.02 (m, 2H), 2.26 - 2.38 (m, 6H), 2.41 - 2.48 (m, 2H), 2.63 - 2.72 (m, 2H), 2.93 - 3.01 (m, 1H), 3.27 - 3.36 (m, 1H), 4.41 (s, 1H), 6.98 - 7.36 (m, 3H), 7.56 (d, *J* = 4.2 Hz, 1H), 7.59 - 7.64 (m, 1H), 8.56 (s, 1H). COOH not observed. MS (ESI) *m*/*z*: 589.2 (M+H)⁺.

### Example #32: Compound 176: (2R)-3-[4-[[4-chloro-2-(difluoromethoxy)phenyl]carbamoyl]-4-(6,6-dimethyl-4H-furo[3,4-c]pyrazol-1-yl)-1-piperidyl]-2-tetrahydropyran-4-yl-propanoic acid

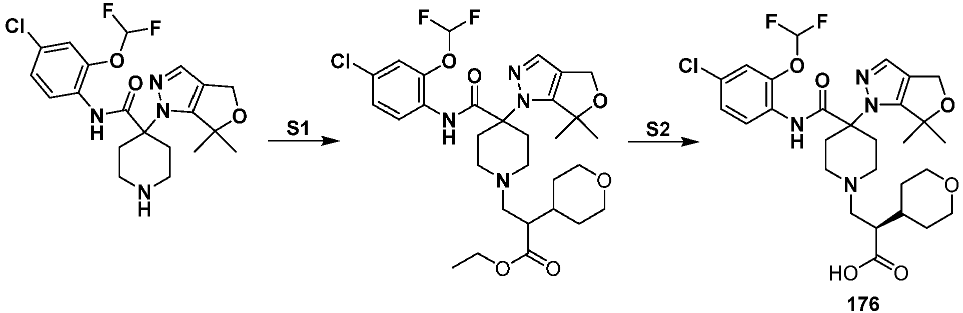

**Step 1: ethyl 3-(4-((4-chloro-2-(difluoromethoxy)phenyl)carbamoyl)-4-(6,6-dimethyl-4,6-dihydro-1*H*-furo[3,4-c]pyrazol-1-yl)piperidin-1-yl)-2-(tetrahydro-2*H*-pyran-4-yl)propanoate.** To a stirred solution of *N*-(4-chloro-2-(difluoromethoxy)phenyl)-4-(6,6-dimethyl-4,6-dihydro-1*H*-furo[3,4-*c*]pyrazol-1-yl)piperidine-4-carboxamide, hydrochloric acid (200 mg) (the product of **Step 6, Example 4**) and ethyl 3-oxo-2-(tetrahydro-2*H*-pyran-4-yl)propanoate (128 mg) in anhydrous dichloromethane (10.0 mL) was added *N,N*-diisopropylethylamine (0.14 mL) dropwise followed by tetramethylammonium triacetoxyhydroborate (232 mg). The reaction mixture was stirred at ambient temperature for 16 hours. Additional tetramethylammonium triacetoxyhydroborate (232 mg,) and *N,N-*diisopropylethylamine (0.14 mL) was added to the reaction mixture and the mixture was stirred at ambient temperature for 48 hours. After completion, the reaction mixture was cooled to 0 °C and quenched with saturated aqueous sodium bicarbonate solution (20 mL). The aqueous layer was extracted with dichloromethane (2 x 20 mL). The combined organic layer was washed with brine (20 mL), dried over anhydrous sodium sulfate, filtered and concentrated under reduced pressure. The crude material was purified by flash column chromatography (methanol/dichloromethane) to provide ethyl 3-(4-((4-chloro-2-(difluoromethoxy)phenyl)carbamoyl)-4-(6,6-dimethyl-4,6-dihydro-1*H*-furo[3,4-c]pyrazol-1-yl)piperidin-1-yl)-2-(tetrahydro-2*H*-pyran-4-yl)propanoate. MS (ESI) *m*/*z*: 626.2 (M+H)⁺.

**Step 2: Compound 176.** To a stirred solution of ethyl 3-(4-((4-chloro-2-(difluoromethoxy)phenyl)carbamoyl)-4-(6,6-dimethyl-4,6-dihydro-1*H*-furo[3,4-*c*]pyrazol-1-yl)piperidin-1-yl)-2-(tetrahydro-2*H*-pyran-4-yl)propanoate (240 mg) in anhydrous methanol (1mL) and tetrahydrofuran (1mL), was added solid lithium hydroxide (46.9 mg). The reaction mixture was stirred at 45 °C for 16 hours. After completion, the solvent was removed under reduced pressure and hydrogen chloride (4 M in 1,4-dioxane) (1.0 mL) was added. The mixture was concentrated under reduced pressure and the crude product was purified by flash chromatography (methanol/dichloromethane) to provide a racemic mixture. The racemic mixture was then subjected to SFC chiral separation employing a CHIRALPAK^{®} IG, 5 µm, 21 mm x 250 mm column to provide the title compound. ¹H NMR (500 MHz, dimethyl sulfoxide-*d*₆) δ ppm 1.06 - 1.48 (m, 10H), 1.56 (d, *J* = 13.0 Hz, 1H), 1.64 - 1.74 (m, 1H), 2.09 (s, 1H), 2.20 - 2.41 (m, 5H), 2.51 - 2.56 (m, 2H), 2.70 (s, 1H), 2.92 (s, 1H), 3.23 (t, *J* = 11.5 Hz, 2H), 3.81 (d, *J* = 11.3 Hz, 2H), 4.68 (s, 2H), 7.05 - 7.33 (m, 2H), 7.36 (d, *J* = 2.3 Hz, 1H), 7.48 (s, 1H), 7.82 (s, 1H), 8.00 (t, *J* = 9.5 Hz, 1H), 12.32 (s, 1H). MS (ESI) *m*/*z:* 598.2 (M+H)⁺.

### Example #33: Compound 195: (2S)-3-[4-[[4-chloro-2-(difluoromethoxy)phenyl]carbamoyl]-4-(5-isopropylpyrazol-1-yl)-1-piperidyl]-2-[(2R,4r,6S)-2,6-dimethyltetrahydropyran-4-yl]propanoic acid

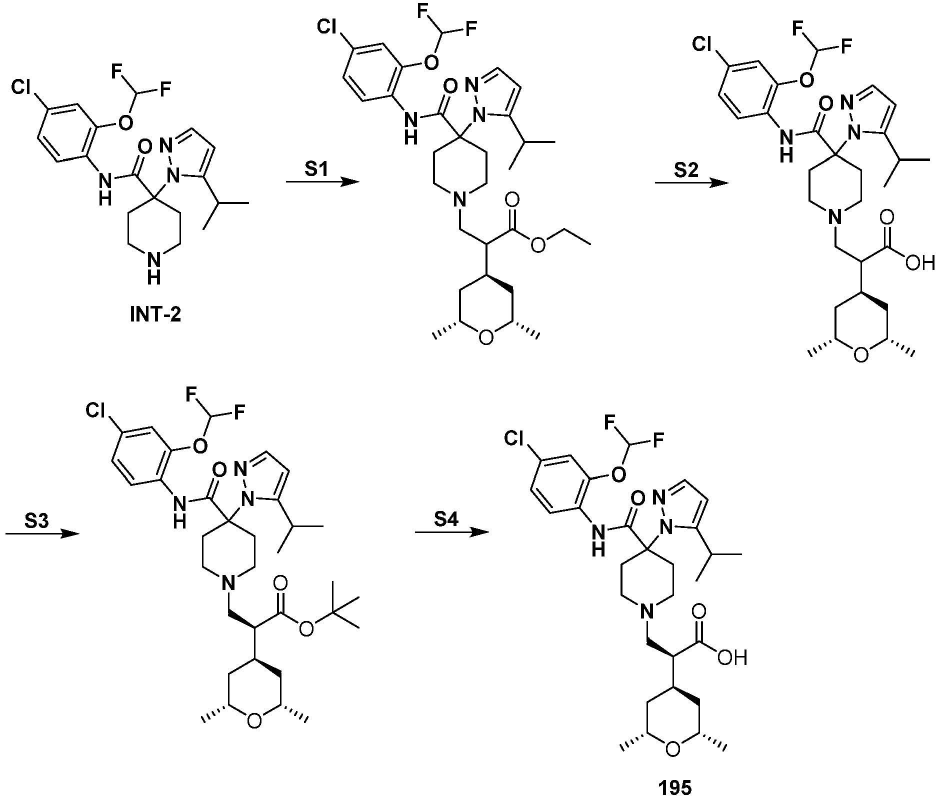

**Step 1: ethyl 3-(4-((4-chloro-2-(difluoromethoxy)phenyl)carbamoyl)-4-(5-isopropyl-1H-pyrazol-1-yl)piperidin-1-yl)-2-((2R,4r,6S)-2,6-dimethyltetrahydro-2H-pyran-4-yl)propanoate.** To a solution of N-(4-chloro-2-(difluoromethoxy)phenyl)-4-(5-isopropyl-1*H-*pyrazol-1-yl)piperidine-4-carboxamide, hydrochloric acid (**INT-2**, 300 mg), ethyl 2-((2R,6S)-2,6-dimethyltetrahydro-2*H*-pyran-4-yl)-3-oxopropanoate (230 mg) and *N,N-*diisopropylethylamine (240 µL) in dichloromethane (3 mL) was added tetramethylammonium triacetoxyhydroborate (350 mg) and the reaction mixture was stirred at ambient temperature for 16 hours. The mixture was quenched with saturated aqueous sodium hydrogen carbonate (20 mL) and extracted with dichloromethane (3 x 30 mL). The combined organics were washed with brine (50 mL), dried over magnesium sulfate, filtered and concentrated under reduced pressure. The crude product was purified by flash chromatography (ethyl acetate/iso-hexane) to provide the title compound. MS (ESI) *m*/*z:* 625.2 (M+H)⁺.

**Step 2: 3-(4-((4-chloro-2-(difluoromethoxy)phenyl)carbamoyl)-4-(5-isopropyl-1*H-*pyrazol-1-yl)piperidin-1-yl)-2-((2*R*,4*r*,6*S*)-2,6-dimethyltetrahydro-2*H*-pyran-4-yl)propanoic acid.** To a solution of ethyl 3-(4-((4-chloro-2-(difluoromethoxy)phenyl)carbamoyl)-4-(5-isopropyl-1*H*-pyrazol-1-yl)piperidin-1-yl)-2-((2*R*,4*r*,6*S*)-2,6-dimethyltetrahydro-2*H*-pyran-4-yl)propanoate (320 mg) in tetrahydrofuran (2 mL) and methanol (2 mL) was added lithium hydroxide (2 M in water) (3 mL) and the reaction mixture stirred at 45 °C for 18 hours. The reaction was then quenched with 4 M hydrogen chloride in 1,4-dioxane (3 mL) and concentrated under reduced pressure. The crude product was purified by flash chromatography (methanol/dichloromethane) to provide the title compound. MS (ESI) *m*/*z*: 597.2 (M+H)⁺.

**Step 3: tert-butyl (S)-3-(4-((4-chloro-2-(difluoromethoxy)phenyl)carbamoyl)-4-(5-isopropyl-1*H*-pyrazol-1-yl)piperidin-1-yl)-2-((2*R*,4*r*,6*S*)-2,6-dimethyltetrahydro-2*H*-pyran-4-yl)propanoate.** To a solution of 3-(4-((4-chloro-2-(difluoromethoxy)phenyl)carbamoyl)-4-(5-isopropyl-1*H*-pyrazol-1-yl)piperidin-1-yl)-2-((2*R*,4r,6*S*)-2,6-dimethyltetrahydro-2*H*-pyran-4-yl)propanoic acid (255 mg) in dichloromethane (5 mL) was added *tert*-butyl 2,2,2-trichloroacetimidate (300 µl) and the reaction mixture stirred at ambient temperature under inert atmosphere for 16 hours. Boron trifluoride etherate (10 µl) and additional *tert*-butyl 2,2,2-trichloroacetimidate (150 µl) were added and the reaction was stirred for a further 48 hours at ambient temperature. The reaction mixture was concentrated under reduced pressure and the crude product was purified by flash chromatography (methanol/dichloromethane) to provide a racemic mixture. The racemic mixture was then subjected to SFC chiral separation employing a CHIRALPAK^{®} IG, 5 µm, 21 mm x 250 mm column to provide the title compound. MS (ESI) *m*/*z*: 653.2 (M+H)⁺.

**Step 4: Compound 195.** To a stirred solution of (*S*)-*tert*-butyl 3-(4-((4-chloro-2-(difluoromethoxy)phenyl)carbamoyl)-4-(5-isopropyl-1*H*-pyrazol-1-yl)piperidin-1-yl)-2-((2*R*,4*r*,6,*S*)-2,6-dimethyltetrahydro-2*H*-pyran-4-yl)propanoate (16 mg) in dichloromethane (2 mL) was added trifluoroacetic acid (200 µl) and the reaction was stirred at ambient temperature for 3 hours. The mixture was concentrated under reduced pressure and the crude product was purified by flash chromatography (methanol/dichloromethane,) to provide the title compound. ¹H NMR (400 MHz, dimethyl sulfoxide-*d*₆) δ ppm 0.73 - 0.83 (m, 1H), 0.88 (q, *J* = 12.3 Hz, 1H), 1.05 *(d, J* = 6.1 Hz, 6H), 1.07 - 1.13 (m, 6H), 1.45 (d, *J* = 12.7 Hz, 1H), 1.56 - 1.66 (m, 1H), 1.76 (s, 1H), 2.18 - 2.28 (m, 1H), 2.27 - 2.40 (m, 4H), 2.41 - 2.53 (m, 2H), 2.56 - 2.69 (m, 1H), 2.80 - 2.88 (m, 1H), 2.92 (p, *J* = 6.6 Hz, 1H), 3.30 - 3.44 (m, 4H), 6.32 (d, *J* = 1.9 Hz, 1H), 6.94 - 7.36 (m, 1H), 7.32 (dd, *J* = 6.9, 3.2 Hz, 2H), 7.52 (d, *J* = 1.9 Hz, 1H), 7.79 (d, *J* = 9.4 Hz, 1H), 8.16 (s, 1H). COOH not observed. MS (ESI) *m*/*z*: 597.3 (M+H)⁺.

### Example #34: Compound 208: (2S)-2-[[4-[[4-chloro-2-(difluoromethoxy)phenyl]carbamoyl]-4-(6,6-dimethyl-4H-furo[3,4-c]pyrazol-1-yl)-1-piperidyl]methyl]-3-methoxy-propanoic acid

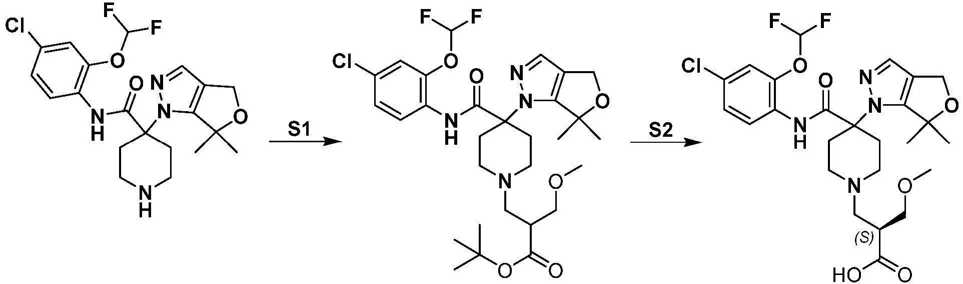

**Step 1: *tert*-butyl 3-(4-((4-chloro-2-(difluoromethoxy)phenyl)carbamoyl)-4-(6,6-dimethyl-4,6-dihydro-1*H*-furo[3,4-*c*]pyrazol-1-yl)piperidin-1-yl)-2-(methoxymethyl)propanoate.** To a solution of *N*-(4-chloro-2-(difluoromethoxy)phenyl)-4-(6,6-dimethyl-4,6-dihydro-1*H*-furo[3,4-*c*]pyrazol-1-yl)piperidine-4-carboxamide, hydrochloric acid (the product of **Step 6, Example #4**) (650 mg) and 1,8-diazabicyclo[5.4.0]undec-7-ene (0.620 mL) in dichloromethane (13 mL) was added a solution of *tert*-butyl 2-(methoxymethyl)acrylate (916 mg) in dichloromethane (4 mL). The mixture was stirred at ambient temperature for 18 hours. Additional 1,8-diazabicyclo[5.4.0]undec-7-ene (0.205 mL) and *tert*-butyl 2-(methoxymethyl)acrylate (257 mg) were added and the reaction was heated at 30 °C for 6 hours. Saturated aqueous ammonium chloride (5 mL) was added and the mixture was extracted with dichloromethane (3 x 5 mL). The combined organics were dried over magnesium sulfate, filtered and concentrated under reduced pressure. The crude product was purified by flash chromatography (methanol/dichloromethane) to provide the title compound. MS (ESI) *m*/*z*: 613.3 (M+H)⁺.

**Step 2: Compound 208.** To a stirred solution of *tert*-butyl 3-(4-((4-chloro-2-(difluoromethoxy)phenyl)carbamoyl)-4-(6,6-dimethyl-4,6-dihydro-1*H*-furo[3,4-*c*]pyrazol-1-yl)piperidin-1-yl)-2-(methoxymethyl)propanoate (0.620 g) in dichloromethane (15 mL) was added trifluoroacetic acid (1.6 mL) and the solution was stirred for 18 hours at 35 °C. The reaction mixture was cooled to ambient temperature and concentrated under reduced pressure. The crude material was purified by flash chromatography (acetonitrile:water 0.1% formic acid) to provide a racemic mixture. The racemic mixture was then subjected to SFC chiral separation employing a Phenomenex^{®} Luna^{®}-4, 21 x 250 mm, 5 µm column to provide the title compound. ¹H NMR (400 MHz, dimethyl sulfoxide-*d*₆) δ ppm 1.38 (s, 6H), 2.10 - 2.41 (m, 5H), 2.79 (d, *J =* 8.8 Hz, 2H), 2.90 (*d, J* = 11.6 Hz, 1H), 3.21 (s, 3H), 3.31 - 3.54 (m, 5H), 4.69 (s, 2H), 7.21 (t, *J =* 73.0 Hz, 1H), 7.31 (dd, *J* = 8.8, 2.3 Hz, 1H), 7.36 (d, *J* = 2.3 Hz, 1H), 7.48 (s, 1H), 7.82 - 7.90 (m, 1H), 7.98 (d, *J =* 8.8 Hz, 1H), 12.65 (s, 1H). MS (ESI) *m*/*z*: 557.2 (M+H)⁺.

### Example #35: Compound 211: (2R)-2-[[4-[[4-chloro-2-(difluoromethoxy)phenyl]carbamoyl]-4-(6,6-dimethyl-4H-furo[3,4-c]pyrazol-1-yl)-1-piperidyl]methyl]-3-methoxy-propanoic acid

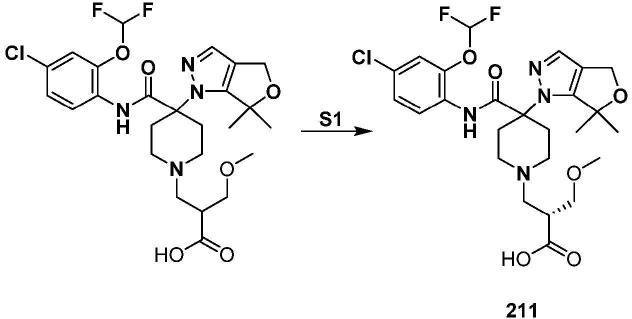

**Compound 211.** The racemic mixture (the product from **Step 2, Example #34**) was subjected to SFC chiral separation employing a Phenomenex^{®} Luna^{®}-4, 21 x 250 mm, 5 µm column to provide the title compound. ¹H NMR (400 MHz, dimethyl sulfoxide-*d*₆) δ ppm 1.38 (s, 6H), 2.07 - 2.44 (m, 5H), 2.79 (d, *J* = 9.2 Hz, 2H), 2.86 - 2.96 (m, 1H), 3.21 (s, 3H), 3.33 - 3.52 (m, 5H), 4.69 (s, 2H), 7.21 (t, *J =* 73.0 Hz, 1H), 7.31 (dd, *J =* 8.8, 2.3 Hz, 1H), 7.36 (d, *J =* 2.4 Hz, 1H), 7.48 (s, 1H), 7.87 (s, 1H), 7.98 *(d, J* = 8.8 Hz, 1H). COOH not observed. MS (ESI) *m*/*z:* 557.3 (M+H)⁺.

### Example #36: Compounds 197 and 198: (2S)-3-[4-[[4-chloro-2-(difluoromethoxy)phenyl]carbamoyl]-4-(4-chloro-5-isopropyl-pyrazol-1-yl)-1-piperidyl]-2-tetrahydropyran-4-yl-propanoic acid and (2R)-3-[4-[[4-chloro-2-(difluoromethoxy)phenyl]carbamoyl]-4-(4-chloro-5-isopropyl-pyrazol-1-yl)-1-piperidyl]-2-tetrahydropyran-4-yl-propanoic acid

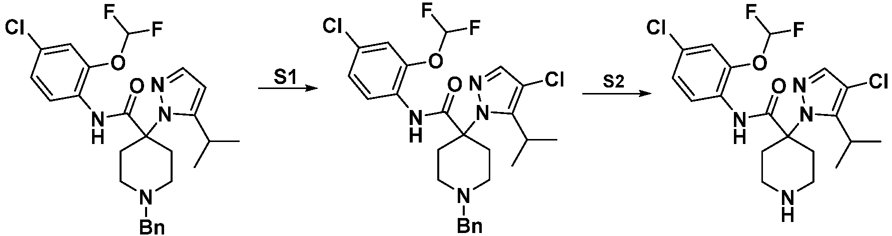

### Preparation #2, Step 4

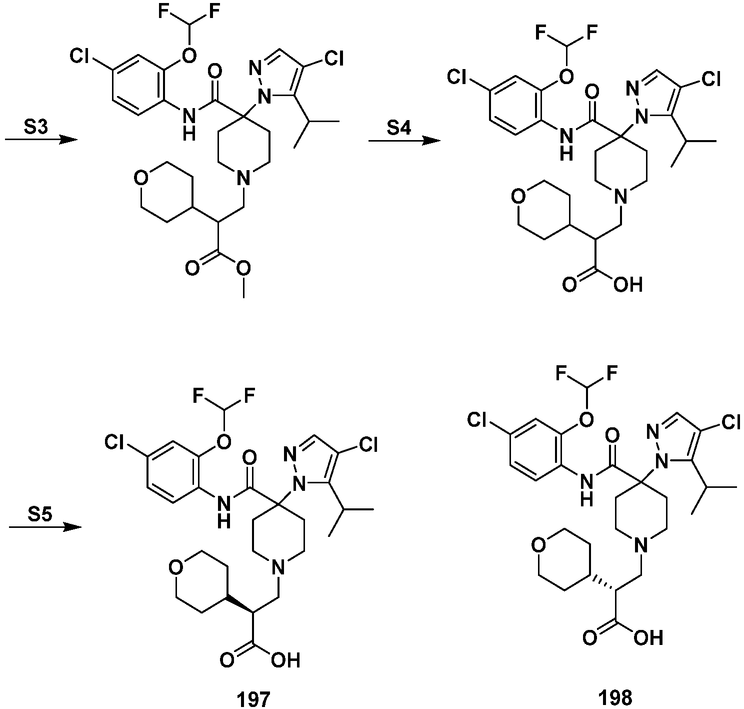

**Step 1: 1-benzyl-*N*-(4-chloro-2-(difluoromethoxy)phenyl)-4-(4-chloro-5-isopropyl-1*H*-pyrazol-1-yl)piperidine-4-carboxamide.** To a stirred mixture of 1-benzyl-N-(4-chloro-2-(difluoromethoxy)phenyl)-4-(5-isopropyl-1H-pyrazol-1-yl)piperidine-4-carboxamide **(Preparation #2, Step 4,** 3.00 g, 5.96 mmol) in acetonitrile (39.8 mL) was added HCl (4 M in dioxane) (2.98 mL, 11.93 mmol). The mixture was stirred for 30 minutes, and 1-(chloromethyl)-4-fluoro-1,4-diazabicyclo[2.2.2]octane-1,4-diium tetrafluoroborate (2.75 g, 7.75 mmol) was added. The mixture was stirred at ambient temperature for 4 days. The mixture was quenched with saturated aqueous sodium bicarbonate (30 mL) and ethyl acetate (100 mL) was added. The aqueous phase was extracted with ethyl acetate (2 x 50 mL), and the combined organics were dried over magnesium sulfate, filtered and concentrated under reduced pressure. The crude material was purified by column chromatography, eluting with ethyl acetate in heptanes to provide the title compound. ¹H NMR (499 MHz, pyridine-*d*₅) δ ppm 9.10 (s, 1H), 8.12 (d, *J =* 8.7 Hz, 1H), 7.57 (s, 1H), 7.51 - 7.36 (m, 5H), 7.35 - 7.26 (m, 3H), 3.48 (s, 2H), 3.37 (h, *J* = 6.9 Hz, 1H), 2.89 - 2.56 (m, 8H), 1.34 (d, *J* = 7.0 Hz, 6H). MS (APCI+) *m*/*z*: 538.3 (M+H)⁺.

**Step 2: *N*-(4-chloro-2-(difluoromethoxy)phenyl)-4-(4-chloro-5-isopropyl-1*H-*pyrazol-1-yl)piperidine-4-carboxamide.** 1-Chloroethyl carbonochloridate (0.699 mL, 6.48 mmol) was added into a solution of 1-benzyl-*N*-(4-chloro-2-(difluoromethoxy)phenyl)-4-(4-chloro-5-isopropyl-1*H*-pyrazol-1-yl)piperidine-4-carboxamide (2.90 g, 5.40 mmol) in 1,2-dichloroethane (DCE, 27.0 mL) at 0 °C. The reaction mixture was heated at 83 °C for 16 hours. The mixture was cooled to 60 °C, and methanol (~ 15 mL) was added. The mixture was heated at reflux for two hours, cooled to ambient temperature, and concentrated. Methanol (~ 20 mL) was added and the mixture was heated at reflux for 4 more hours, cooled to ambient temperature, and concentrated to provide the crude title compound. MS (APCI+) *m*/*z*: 448.2 (M+H)⁺.

**Step 3: methyl 3-(4-((4-chloro-2-(difluoromethoxy)phenyl)carbamoyl)-4-(4-chloro-5-isopropyl-1*H*-pyrazol-1-yl)piperidin-1-yl)-2-(tetrahydro-2*H*-pyran-4-yl)propanoate.** The crude *N*-(4-chloro-2-(difluoromethoxy)phenyl)-4-(4-chloro-5-isopropyl-1*H*-pyrazol-1-yl)piperidine-4-carboxamide (0.974 g, 2.177 mmol) was dissolved in 1,2-dichloroethane (18.15 mL). Methyl 3-oxo-2-(tetrahydro-2*H*-pyran-4-yl)propanoate (0.631 g, 3.05 mmol) and sodium triacetoxyborohydride (0.692 g, 3.27 mmol) were added and the mixture was stirred at ambient temperature overnight. Saturated sodium carbonate solution (30 mL) was added and the mixture was extracted with dichloromethane (150 mL). The dichloromethane layer was washed with brine, dried over Na₂SO₄, filtered, concentrated, and purified by column chromatography, eluting with ethyl acetate in heptanes to provide the title compound. ¹H NMR (600 MHz, dimethyl sulfoxide-*d*₆) δ ppm 8.75 (s, 1H), 7.59 (s, 1H), 7.55 (d, *J =* 8.3 Hz, 1H), 7.37 - 7.30 (m, 2H), 7.16 (s, 1H), 3.88 - 3.77 (m, 4H), 3.59 (s, 3H), 3.30 - 3.19 (m, 2H), 3.08 (h, *J =* 6.9 Hz, 1H), 2.77 (dd, *J =* 10.0, 5.4 Hz, 1H), 2.58 - 2.51 (m, 1H), 2.36-2.33 (m, 4H), 2.25 (s, 1H), 2.10 - 2.01 (m, 1H), 1.70 (dtt, *J =* 11.4, 7.5, 3.7 Hz, 1H), 1.57 (ddd, *J =* 13.2, 3.8, 2.0 Hz, 2H), 1.38-1.15(m, 9H). ). MS (APCI+) m/z: 618.5 (M+H)⁺.

**Step 4: *rac*-3-(4-((4-chloro-2-(difluoromethoxy)phenyl)carbamoyl)-4-(4-chloro-5-isopropyl-1*H*-pyrazol-1-yl)piperidin-1-yl)-2-(tetrahydro-2*H-*pyran-4-yl)propanoic acid.** Methyl 3-(4-((4-chloro-2-(difluoromethoxy)phenyl)carbamoyl)-4-(4-chloro-5-isopropyl-1*H-*pyrazol-1-yl)piperidin-1-yl)-2-(tetrahydro-2*H*-pyran-4-yl)propanoate (1.30 g, 2.105 mmol) was dissolved in a mixture of tetrahydrofuran (9.02 mL), methanol (9.02 mL) and water (3.01 mL). Lithium hydroxide monohydrate (0.353 g, 8.42 mmol) was added and the reaction mixture was stirred at 50 °C overnight. The reaction mixture was concentrated to remove some of the organic solvents. Dichloromethane (100 mL) was added and the pH was adjusted to ~ 3 by adding 1 N aqueous HCl. The aqueous layer was extracted with dichloromethane (2 x 40 mL). The combined organic layers were dried over Na₂SO₄, filtered, concentrated and purified by column chromatography, eluting with ethyl acetate in heptanes, then methanol in ethyl acetate to provide the title compound. MS (APCI+) *m*/*z*: 604.5 (M+H)⁺.

**Step 5: Compounds 197 and 198.** Racemic 3-(4-((4-Chloro-2-(difluoromethoxy)phenyl)carbamoyl)-4-(4-chloro-5-isopropyl-1*H*-pyrazol-1-yl)piperidin-1-yl)-2-(tetrahydro-2*H*-pyran-4-yl)propanoic acid was separated by preparative chiral supercritical fluid chromatography (CHIRALPAK^{®} IC, 250 x 30 mm; mobile phase: (A) CO₂ and (B) methanol). The fractions from the first eluting peak were concentrated and dissolved in 400 mL of dichloromethane. Water (10 mL) was added, and the pH was adjusted to about 3 with 1N aqueous HCl. The organics were collected, and the aqueous layer was extracted with dichloromethane (2 x 50 mL). The combined organics were dried over Na₂SO₄, filtered, and concentrated under reduced pressure. The residue was purified by column chromatography on silica gel, eluting with methanol in dichloromethane (0 to 10%) to provide **Compound 197.** ¹H NMR (499 MHz, dimethyl sulfoxide-*d*₆*)* δ ppm 8.81 (s, 1H), 7.61 (s, 1H), 7.58 - 7.52 (m, 1H), 7.36 - 7.30 (m, 1H), 7.32 (s, 1H), 7.17 ( s, 1H), 3.85 - 3.78 (m, 2H), 3.28 - 3.19 (m, 2H), 3.06 (h, *J* = 7.0 Hz, 1H), 2.86 (s, 1H), 2.66 - 2.56 (m, 2H), 2.43 - 2.34 (m, 8H), 1.71 (dtt, *J* = 11.2, 7.2, 3.6 Hz, 1H), 1.56 (ddt, *J* = 11.1, 4.2, 2.2 Hz, 1H), 1.42 (ddd, *J* = 13.1, 4.1, 2.1 Hz, 1H), 1.38 - 1.17 (m, 9H). The fractions from the second eluting peak were concentrated and dissolved in 400 mL of dichloromethane. Water (10 mL) was added, and the pH was adjusted to about 3 with 1N aqueous HCl. The organics were collected, and the aqueous layer was extracted with dichloromethane (2 x 50 mL). The combined organics were dried over Na₂SO₄, filtered, and concentrated under reduced pressure. The residue was purified by column chromatography on silica gel, eluting with methanol in dichloromethane to provide **Compound 198.** ¹H NMR (499 MHz, dimethyl sulfoxide-*d*₆) δ ppm 8.81 (s, 1H), 7.61 (s, 1H), 7.58 - 7.52 (m, 1H), 7.36 - 7.30 (m, 1H), 7.32 (s, 1H), 7.17(s, 1H), 3.85 - 3.78 (m, 2H), 3.24 (td, *J* = 11.9, 2.0 Hz, 2H), 3.06 (h, *J* = 7.0 Hz, 1H), 2.86 (s, 1H), 2.66 - 2.57 (m, 2H), 2.42 - 2.34 (m, 8H), 1.72 (tdt, *J* = 11.2, 7.1, 3.5 Hz, 1H), 1.56 (ddd, *J* = 13.2, 4.0, 2.2 Hz, 1H), 1.42 (ddd, *J* = 13.0, 4.0, 2.1 Hz, 1H), 1.38 - 1.17 (m, 9H).

### Example #37: Compounds 205 and 206: (2S)-2-[[4-[[4-chloro-2-(difluoromethoxy)phenyl]carbamoyl]-4-(4-chloro-5-isopropyl-pyrazol-1-yl)-1-piperidyl]methyl]-3-methoxy-propanoic acid and (2R)-2-[[4-[[4-chloro-2-(difluoromethoxy)phenyl]carbamoyl]-4-(4-chloro-5-isopropyl-pyrazol-1-yl)-1-piperidyl]methyl]-3-methoxy-propanoic acid

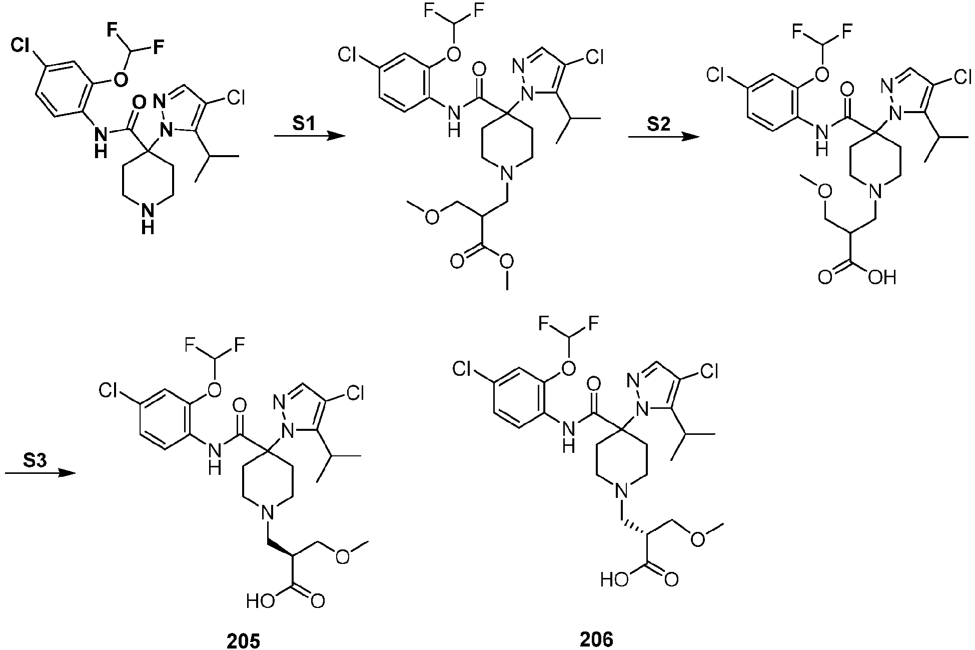

**Step** 1: **methyl 3-(4-((4-chloro-2-(difluoromethoxy)phenyl)carbamoyl)-4-(4-chloro-5-isopropyl-1*H*-pyrazol-1-yl)piperidin-1-yl)-2-(methoxymethyl)propanoate.** The crude *N*-(4-chloro-2-(difluoromethoxy)phenyl)-4-(4-chloro-5-isopropyl-1*H*-pyrazol-1-yl)piperidine-4-carboxamide, **(Example** #36, **Step 2,** 1.5 g, 2.68 mmol) was dissolved in dichloromethane (16.77 mL). Methyl 2-(methoxymethyl)prop-2-enoate (0.524 g, 4.02 mmol) was added followed by 1,8-diazabicyclo[5.4.0]undec-7-ene (DBU) (0.661 mL, 4.43 mmol) and the mixture was stirred at ambient temperature for 12 hours. Water (15 mL) and dichloromethane (50 mL) were added, and the organics were collected. The aqueous layer was extracted with dichloromethane (30 mL) and the combined organic layers were washed with brine, dried over Na₂SO₄, filtered, and concentrated under reduced pressure. The residue was purified by column chromatography, eluting with ethyl acetate in heptanes to provide the title compound. ¹H NMR (600 MHz, dimethyl sulfoxide-*d*₆) δ ppm 8.15 (s, 1H), 7.83 - 7.78 (m, 1H), 7.52 (d, *J =* 1.8 Hz, 1H), 7.34 - 7.29 (m, 2H), 6.32 (d, *J =* 1.9 Hz, 1H), 3.91-3.2 (br, 6H), 2.93 (hept, *J* = 6.7 Hz, 1H), 2.70 (p, *J =* 7.7 Hz, 1H), 2.62 (dt, *J =* 12.2, 4.5 Hz, 2H), 2.37 (dq, *J* = 13.4, 4.5 Hz, 2H), 2.20 - 2.17 (m, 2H), 2.17 - 2.11 (m, 2H), 1.88 (ddd, *J* = 9.7, 7.2, 2.6 Hz, 2H), 1.29 (s, 2H), 1.10 (d, *J* = 6.7 Hz, 6H). MS (APCI+) *m*/*z:* 578.5 (M+H)⁺.

**Step 2: rac-3-(4-((4-chloro-2-(difluoromethoxy)phenyl)carbamoyl)-4-(4-chloro-5-isopropyl-1H-pyrazol-1-yl)piperidin-1-yl)-2-(methoxymethyl)propanoic acid.** Methyl 3-(4-((4-chloro-2-(difluoromethoxy)phenyl)carbamoyl)-4-(4-chloro-5-isopropyl-1*H*-pyrazol-1-yl)piperidin-1-yl)-2-(methoxymethyl)propanoate (1.28 g, 2.217 mmol) was dissolved in a mixture of tetrahydrofuran (9.50 mL), methanol (9.50 mL) and water (3.17 mL). Lithium hydroxide monohydrate (0.372 g, 8.87 mmol) was added and the reaction mixture was stirred at 50 °C overnight. The reaction mixture was concentrated to remove some organic solvent, dichloromethane (100 mL) was added, and the pH was adjusted to ~ 3 with 1 N aqueous HCl. The aqueous layer was extracted with dichloromethane (2 x 40 mL), and the combined organic was dried over Na₂SO₄, filtered, and concentrated under reduced pressure. The residue was purified by column chromatography, eluting with ethyl acetate in heptanes then methanol in ethyl acetate to provide the title compound. MS (APCI+) *m*/*z:* 565.4 (M+H)⁺.

**Step 3: Compounds 205 and 206.** Racemic 3-(4-((4-chloro-2-(difluoromethoxy)phenyl)carbamoyl)-4-(4-chloro-5-isopropyl-1*H*-pyrazol-1-yl)piperidin-1-yl)-2-(methoxymethyl)propanoic acid was separated by preparative chiral supercritical fluid chromatography (CHIRALCEL^{®} OZ^{™}-H, 250 x 30 mm; mobile phase: (A) CO₂ and (B) methanol). Each set of peaks were pooled and re-purified by column chromatography (methanol in dichloromethane) to provide the title **Compound 206:** ¹H NMR (600 MHz, dimethyl sulfoxide-*d*₆) δ ppm 8.84 (s, 1H), 7.61 (s, 1H), 7.54 (d, *J* = 8.4 Hz, 1H), 7.36 - 7.31 (m, 2H), 7.17 (s, 1H), 3.46 (qd, *J* = 9.3, 6.1 Hz, 2H), 3.21 (s, 3H), 3.07 (p, *J* = 7.0 Hz, 2H), 2.83-2.65 (m,4H), 2.54 (m, 1H), 2.41 - 2.36 (m, 6H), 1.23 (dd, *J* = 6.9, 1.2 Hz, 6H). MS (APCI+) *m*/*z:* 565.4 (M+H)⁺. **Compound 205:** ¹H NMR (499 MHz, dimethyl sulfoxide-*d*₆) δ ppm 8.83 (s, 1H), 7.61 (s, 1H), 7.58 - 7.52 (m, 1H), 7.36 - 7.30 (m, 2H), 7.17 (s, 1H), 3.45 (qd, *J* = 9.3, 6.1 Hz, 2H), 3.21 (s, 3H), 3.07 (p, *J* = 6.9 Hz, 2H), 2.82 - 2.65 (m, 4H), 2.56-2.51 (m, 1H), 2.38 (q, *J* = 5.1 Hz, 6H), 1.28 - 1.17 (m, 6H). MS (APCI+) *m*/*z:* 565.4 (M+H)⁺.

### Example #38: Compound 51: 2-[4-[[4-[[4-chloro-2-(difluoromethoxy)phenyl]carbamoyl]-4-(5-isopropylpyrazol-1-yl)-1-piperidyl]methyl]tetrahydropyran-4-yl]acetic acid

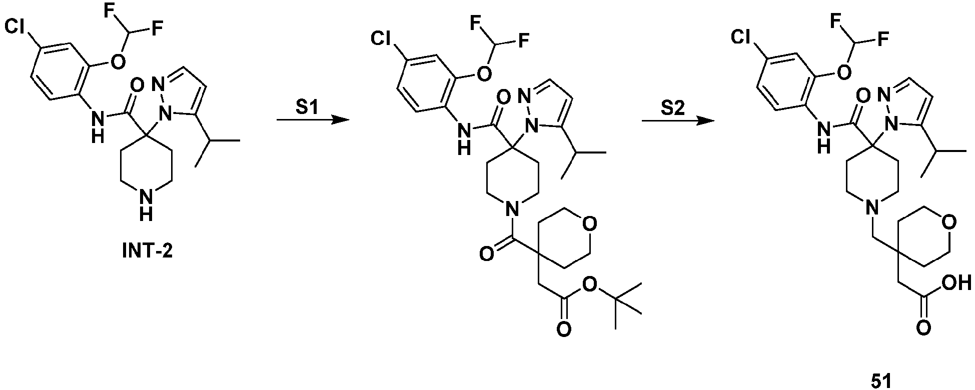

**Step 1: *tert*-butyl 2-(4-(4-((4-chloro-2-(difluoromethoxy)phenyl)carbamoyl)-4-(5-isopropyl-1*H*-pyrazol-1-yl)piperidine-1-carbonyl)tetrahydro-2*H*-pyran-4-yl)acetate.** To a solution of 4-(2-(*tert*-butoxy)-2-oxoethyl)tetrahydro-2*H*-pyran-4-carboxylic acid (110 mg) and *N,N-*diisopropylethylamine (0.25 mL) in dichloromethane (4 mL) was added 1-(chloro(pyrrolidin-1-yl)methylene)pyrrolidin-1-ium hexafluorophosphate(V) (155 mg) and the reaction was stirred at ambient temperature for 3 hours. **INT-2** (169 mg) was added and the reaction was stirred for a further 18 hours at ambient temperature. The reaction mixture was then quenched with saturated aqueous ammonium chloride (5 mL). The mixture was extracted with dichloromethane (3 x 5 mL). The combined organic layers were dried over magnesium sulfate, filtered and concentrated under reduced pressure. The crude product was purified by flash chromatography (ethyl acetate/isohexane) to provide the title compound. MS (ESI) *m*/*z*: 583.2 (M-*^{t}*Bu+H)⁺.

**Step 2: Compound 51.** 1,1,3,3-Tetramethyldisiloxane (0.4 mL) was added dropwise to a solution of *tert*-butyl 2-(4-(4-((4-chloro-2-(difluoromethoxy)phenyl)carbamoyl)-4-(5-isopropyl-1*H*-pyrazol-1-yl)piperidine-1-carbonyl)tetrahydro-2H-pyran-4-yl)acetate (150 mg) and carbonylchlorobis(triphenylphosphine)iridium(I) (17 mg) in dichloromethane (2.5 mL), and the reaction was stirred for 30 minutes at ambient temperature. Trifluoroacetic acid (0.05 mL) was added and the reaction was stirred at ambient temperature for 45 minutes. The reaction mixture was diluted with toluene (1 mL) and concentrated under reduced pressure. The material was partitioned between dichloromethane (3 mL) and water (1 mL). The pH of the aqueous layer was adjusted to ~12 with 2 N aqueous sodium hydroxide, and the mixture was washed with saturated aqueous ammonium chloride (5 mL). The organic layer was separated, and the aqueous layer was extracted with dichloromethane (3 x 5 mL). The combined organic extracts were dried over magnesium sulfate, filtered and concentrated under reduced pressure to provide the title compound. ¹H NMR (400 MHz, CD₃OD) δ ppm 1.22 (d, *J* = 6.7 Hz, 6H), 1.42 - 1.56 (m, 2H), 1.88 (t, *J* = 7.5 Hz, 2H), 2.02 - 2.14 (m, 2H), 2.67 - 2.81 (m, 2H), 2.81 - 2.95 (m, 2H), 2.95 - 3.07 (m, 3H), 3.07 - 3.22 (m, 2H), 3.41 - 3.54 (m, 2H), 3.54 - 3.69 (m, 2H), 3.72 - 3.88 (m, 2H), 6.42 (d, *J* = 2.0 Hz, 1H), 6.83 (t, *J* = 73.0 Hz, 1H), 7.28 (d, *J* = 7.8 Hz, 2H), 7.64 (d, *J* = 1.9 Hz, 1H), 7.99 (d, *J* = 8.7 Hz, 1H). Exchangeable protons not observed. MS (ESI) *m*/*z:* 569.3 (M+H)⁺.

### Example #39: Compound 42: 1-[[4-[[4-chloro-2-(difluoromethoxy)phenyl]carbamoyl]-4-(5-isopropylpyrazol-1-yl)-1-piperidyl]methyl]cyclohexanecarboxylic acid

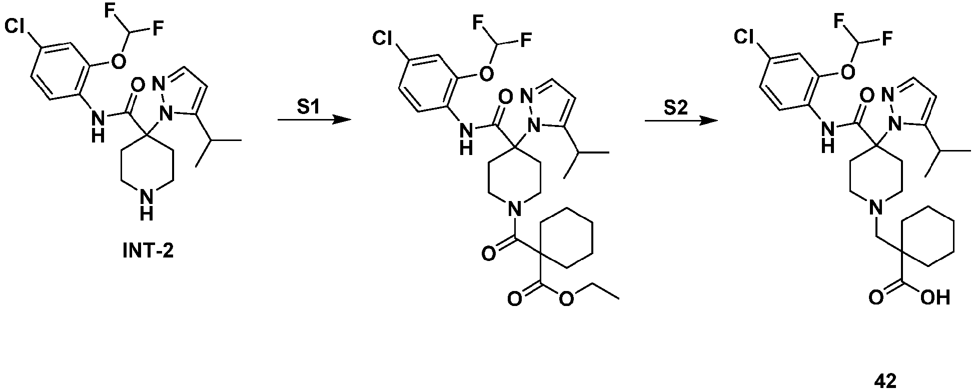

**Step 1: ethyl 1-((4-((4-chloro-2-(difluoromethoxy)phenyl)carbamoyl)-4-(5-isopropyl-1*H*-pyrazol-1-yl)piperidin-1-yl)methyl)cyclohexane-1-carboxylate.** To a solution of **INT-2** (200 mg, 0.484 mmol) in dichloromethane (4.5 mL) was added ethyl 1-formylcyclohexane-1-carboxylate (268 mg, 1.45 mmol), and tetramethylammonium triacetoxyborohydride (382 mg, 1.45 mmol). The resulting mixture was then stirred at 25 °C for 18 hours. The resultant mixture was diluted with water (10 mL) and extracted with dichloromethane (2 × 10 mL). The combined organic phase was dried over MgSO₄, filtered and concentrated under reduced pressure. The residue was purified by silica gel column chromatography using ethyl acetate in heptanes to provide the title compound. MS *m*/*z:* 581.3 [M+H]⁺.

**Step 2: Compound 42.** To ethyl 1-((4-((4-chloro-2-(difluoromethoxy)phenyl)carbamoyl)-4-(5-isopropyl-1*H*-pyrazol-1-yl)piperidin-1-yl)methyl)cyclohexane-1-carboxylate (212 mg, 0.365 mmol) dissolved in a mixture of tetrahydrofuran (3 mL), methanol (3 mL) and water (1 mL) was added lithium hydroxide monohydrate (153 mg, 3.65 mmol). The mixture was stirred at 65 °C for 18 hours. The resultant mixture was diluted with dichloromethane (10 mL) and extracted with half saturated aqueous ammonium chloride (2 × 5 mL). The combined organic phase was dried over MgSO₄, filtered and the filtrate was concentrated under reduced pressure. The residue was purified by silica gel column chromatography using methanol in dichloromethane to provide the title compound. ¹H NMR (400 MHz, dimethyl sulfoxide-*d*₆) δ ppm 8.11 (s, 1H), 7.81 (d, *J =* 9.4 Hz, 1H), 7.52 (d, *J =* 1.8 Hz, 1H), 7.31 (m, 2H), 7.15 (t, *J* = 73.2 Hz, 1H), 6.32 (d, *J* = 1.8 Hz, 1H), 3.17 (s, 2H), 2.91 (m, 1H), 2.71 (m, 2H), 2.38 (m, 6H), 1.89 (m, 2H), 1.49 (m, 3H), 1.33 (m, 2H), 1.22 (m, 3H), 1.09 (d, *J =* 6.7 Hz, 6H). MS *m*/*z:* 553.1 [M+H]⁺.

### Example #40: Compound 45: 1-[[4-[[4-chloro-2-(difluoromethoxy)phenyl]carbamoyl]-4-(5-isopropylpyrazol-1-yl)-1-piperidyl]methyl]cyclobutanecarboxylic acid

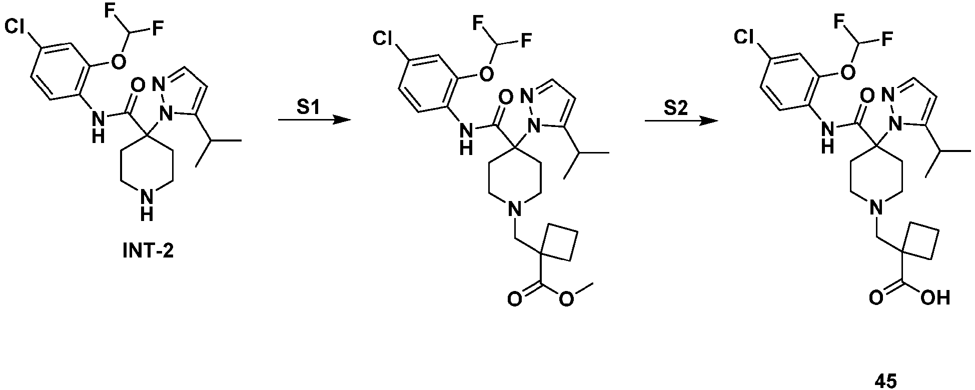

**Step 1: methyl 1-((4-((4-chloro-2-(difluoromethoxy)phenyl)carbamoyl)-4-(5-isopropyl-1*H*-pyrazol-1-yl)piperidin-1-yl)methyl)cyclobutane-1-carboxylate.** To a solution of **INT-2** (200 mg, 0.484 mmol) dissolved in dichloromethane (4.5 mL) was added methyl 1-formylcyclobutane-1-carboxylate (206 mg, 1.45 mmol), and tetramethylammonium triacetoxyborohydride (382 mg, 1.45 mmol). The mixture was then stirred at 25 °C for 18 hours. The resultant mixture was diluted with water (10 mL) and extracted with dichloromethane (2 × 10 mL). The combined organic phase was dried over MgSO₄, filtered, and the filtrate was concentrated under reduced pressure. The residue was purified by silica gel column chromatography using ethyl acetate in heptanes to provide the title compound. MS *m*/*z*: 539.4 [M+H]⁺.

**Step 2: Compound 45.** Methyl 1-((4-((4-chloro-2-(difluoromethoxy)phenyl)carbamoyl)-4-(5-isopropyl-1H-pyrazol-1-yl)piperidin-1-yl)methyl)cyclobutane-1-carboxylate (260 mg, 0.484 mmol) was dissolved in a mixture of tetrahydrofuran (3 mL), methanol (3 mL) and water (1 mL). Lithium hydroxide monohydrate (203 mg, 4.84 mmol) was added, and the mixture was stirred at 25 °C for 18 hours. The resultant mixture was diluted with dichloromethane (10 mL) and extracted with half saturated aqueous ammonium chloride (2 × 5 mL). The combined organic phase was dried over MgSO₄, filtered and the filtrate was concentrated under reduced pressure. The residue was purified by silica gel column chromatography using methanol in dichloromethane to provide the title compound. ¹H NMR (500 MHz, dimethyl sulfoxide-*d*₆) δ ppm 8.18 (s, 1H), 7.78 (d, *J* = 9.4 Hz, 1H), 7.53 (d, *J =* 1.8 Hz, 1H), 7.32 (m, 2H), 7.15 (t, *J =* 73.2 Hz, 1H), 6.33 (d, *J =* 1.8 Hz, 1H), 2.92 (m, 1H), 2.70 (m, 1H), 2.45 (m, 4H), 2.35 (m, 2H), 1.90 (m, 2H), 1.81 (m, 2H), 1.09 (d, *J =* 6.7 Hz, 6H). MS *m*/*z:* 525.2 [M+H]⁺.

### Example #41: Compound 52: 3-[4-[[4-chloro-2-(difluoromethoxy)phenyl]carbamoyl]-4-(5-isopropylpyrazol-1-yl)-1-piperidyl]-2-methyl-2-tetrahydropyran-4-yl-propanoic acid

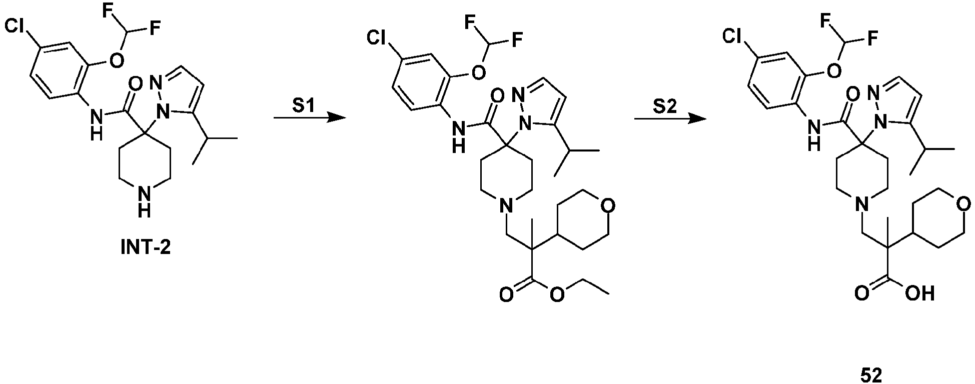

**Step 1: ethyl 3-(4-((4-chloro-2-(difluoromethoxy)phenyl)carbamoyl)-4-(5-isopropyl-1*H*-pyrazol-1-yl)piperidin-1-yl)-2-methyl-2-(tetrahydro-2*H*-pyran-4-yl)propanoate.** To a solution of **INT-2** (150 mg, 0.363 mmol) dissolved in 1,2-dichloroethane (3 mL) was added methyl ethyl 2-methyl-3-oxo-2-(tetrahydro-2*H*-pyran-4-yl)propanoate (117 mg, 0.545 mmol), and sodium triacetoxyborohydride (116 mg, 0.545 mmol). The mixture was then stirred at 25 °C for 18 hours. The resultant mixture was diluted with water (10 mL) and extracted with dichloromethane (2 × 10 mL). The combined organic phase was dried over MgSO₄, filtered and the filtrate was concentrated under reduced pressure. The residue was purified by silica gel column chromatography using ethyl acetate in heptanes to provide the title compound. MS (APCI+) *m*/*z:* 611.3 [M+H]⁺.

**Step 2: Compound** 52. To a solution of ethyl 3-(4-((4-chloro-2-(difluoromethoxy)phenyl)carbamoyl)-4-(5-isopropyl-1*H*-pyrazol-1-yl)piperidin-1-yl)-2-methyl-2-(tetrahydro-2*H*-pyran-4-yl)propanoate (85 mg, 0.139 mmol) was dissolved in a mixture of tetrahydrofuran (3 mL), methanol (3 mL) and water (1 mL). Lithium hydroxide monohydrate (58 mg, 1.39 mmol) was added, and the solution was stirred at 70 °C for 72 hours. The resultant mixture was diluted with dichloromethane (10 mL) and extracted with half saturated aqueous ammonium chloride (2 × 5 mL). The combined organic phase was dried over MgSO₄, filtered and the filtrate was concentrated under reduced pressure. The residue was purified by silica gel column chromatography using methanol in dichloromethane to provide the title compound. ¹H NMR (400 MHz, dimethyl sulfoxide-*d*₆) δ ppm 8.14 (bs, 1H), 7.80 (m, 1H), 7.52 (d, *J* = 1.8 Hz, 1H), 7.31 (m, 2H), 7.15 (t, *J* = 73.2 Hz, 1H), 6.32 (d, *J* = 2.1 Hz, 1H), 3.85 (m, 1H), 3.22 (m, 4H), 2.92 (m, 1H), 2.73 (m, 2H), 2.63, (m, 1H), 2.54 (m, 2H), 2.46 (m, 2H), 2.33 (m, 2H), 1.70 (m, 1H), 1.48 (m, 1H), 1.28 (m, 3H), 1.09 (d, *J =* 6.7 Hz, 6H), 1.05 (s, 3H). MS (APCI+) *m*/*z:* 585.2 [M+H]⁺.

### Example #42: Compound 63: 2-[[4-[[4-chloro-2-(difluoromethoxy)phenyl]carbamoyl]-4-(5-isopropylpyrazol-1-yl)-1-piperidyl]methyl]indane-2-carboxylic acid

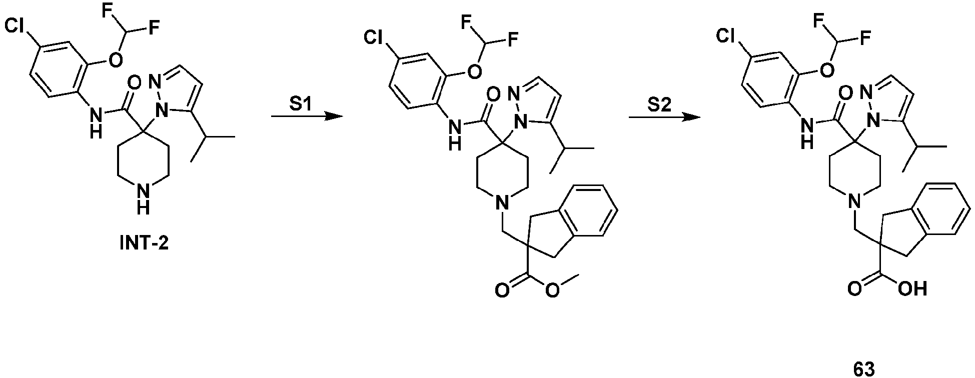

**Step 1: methyl 2-((4-((4-chloro-2-(difluoromethoxy)phenyl)carbamoyl)-4-(5-isopropyl-1*H*-pyrazol-1-yl)piperidin-1-yl)methyl)-2,3-dihydro-1*H*-indene-2-carboxylate.** To a solution of **INT-2** (200 mg, 0.484 mmol) dissolved in 1,2-dichloroethane (4.5 mL) was added methyl 2-formyl-2,3-dihydro-1*H*-indene-2-carboxylate (396 mg, 1.94 mmol), and tetramethylammonium triacetoxyborohydride (382 mg, 1.45 mmol). The mixture was then stirred at 25 °C for 18 hours. The resultant mixture was diluted with water (10 mL) and extracted with dichloromethane (2 × 10 mL). The combined organic phase was dried over MgSO₄, filtered and the filtrate was concentrated under reduced pressure. The residue was purified by silica gel column chromatography using ethyl acetate in heptanes to provide the title compound. MS (APCI+) *m*/*z*: 601.3 [M+H]⁺.

**Step 2: Compound 63.** To a solution of methyl 2-((4-((4-chloro-2-(difluoromethoxy)phenyl)carbamoyl)-4-(5-isopropyl-1*H*-pyrazol-1-yl)piperidin-1-yl)methyl)-2,3-dihydro-1*H*-indene-2-carboxylate (195 mg, 0.324 mmol) was dissolved in a mixture of tetrahydrofuran (3 mL), methanol (3 mL) and water (1 mL). Lithium hydroxide monohydrate (136 mg, 3.24 mmol) was added and the solution was stirred at 60 °C for 18 hours. The resultant mixture was diluted with dichloromethane (10 mL) and extracted with half saturated aqueous ammonium chloride (2 × 5 mL). The combined organic phase was dried over MgSO₄, filtered and the filtrate was concentrated under reduced pressure. The residue was purified by silica gel column chromatography using methanol in dichloromethane to provide the title compound. ¹H NMR (600 MHz, dimethyl sulfoxide-*d*₆) δ ppm 12.60 (bs, 1H), 8.13 (s, 1H), 7.80 (d, *J* = 9.4 Hz, 1H), 7.53 (d, *J* = 1.8 Hz, 1H), 7.32 (m, 2H), 7.18 (m, 2H), 7.15 (t, *J* = 73.3 Hz, 1H), 7.10 (m, 2H), 6.32 (d, *J* = 1.9 Hz, 1H), 3.29 (m, 2H), 2.93 (m, 3H), 2.74 (m, 2H), 2.62 (s, 2H), 2.46 (m, 3H), 2.36 (m, 2H), 1.10 (d, *J =* 6.7 Hz, 6H). MS (APCI+) *m*/*z:* 587.3 [M+H]⁺.

### Example #43: Compound 95: (1s,4s)-1-[[4-[[4-chloro-2-(difluoromethoxy)phenyl]carbamoyl]-4-(5-isopropylpyrazol-1-yl)-1-piperidyl]methyl]-4-hydroxy-cyclohexanecarboxylic acid

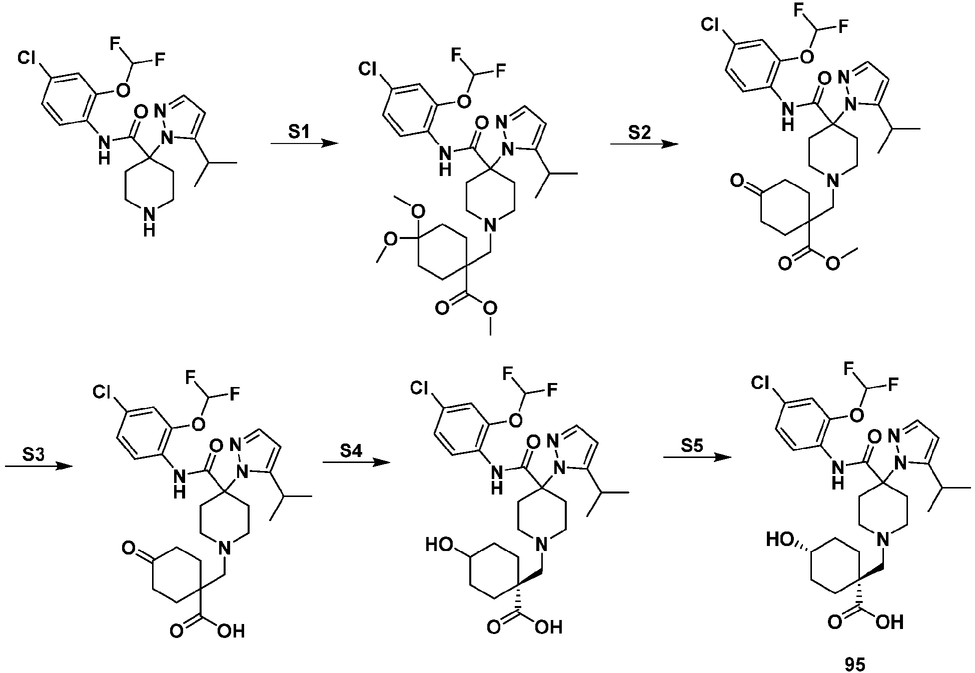

**Step 1: methyl 1-((4-((4-chloro-2-(difluoromethoxy)phenyl)carbamoyl)-4-(5-isopropyl-1*H*-pyrazol-1-yl)piperidin-1-yl)methyl)-4,4-dimethoxycyclohexane-1-carboxylate.** To a solution of **INT-2** (1000 mg, 2.42 mmol) dissolved in 1,2-dichloroethane (10 mL) was then added methyl 1-formyl-4,4-dimethoxycyclohexane-1-carboxylate (1674 mg, 7.26 mmol), and sodium triacetoxyborohydride (1540 mg, 7.26 mmol). The resulting mixture was then stirred at 25 °C for 18 hours. The resultant mixture was diluted with water (25 mL) and extracted with dichloromethane (2 × 25 mL). The combined organic phase was dried over MgSO₄, filtered and the filtrate was concentrated under reduced pressure. The residue was purified by silica gel column chromatography using ethyl acetate in heptanes to provide the title compound. MS (APCI+) *m*/*z:* 627.0 [M+H]⁺.

**Step 2: methyl 1-((4-((4-chloro-2-(difluoromethoxy)phenyl)carbamoyl)-4-(5-isopropyl-1*H*-pyrazol-1-yl)piperidin-1-yl)methyl)-4-oxocyclohexane-1-carboxylate.** To a solution of methyl 1-((4-((4-chloro-2-(difluoromethoxy)phenyl)carbamoyl)-4-(5-isopropyl-1*H-*pyrazol-1-yl)piperidin-1-yl)methyl)-4,4-dimethoxycyclohexane-1-carboxylate (1230 mg, 1.961 mmol) dissolved in tetrahydrofuran (25 mL) was added a 1 N aqueous solution of hydrochloric acid (9.81 mL, 9.81 mmol) and the mixture was stirred at room temperature for 22 hours. A solution of half saturated aqueous sodium bicarbonate (20 mL) was added, and the mixture was extracted with dichloromethane (25 mL). The organic layer was separated, dried over MgSO₄, filtered, and concentrated to provide the title material. MS (APCI+) *m*/*z*: 581.0 [M+H]⁺.

**Step 3: 1-((4-((4-chloro-2-(difluoromethoxy)phenyl)carbamoyl)-4-(5-isopropyl-1*H-*pyrazol-1-yl)piperidin-1-yl)methyl)-4-oxocyclohexane-1-carboxylic acid.** To methyl 1-((4-((4-chloro-2-(difluoromethoxy)phenyl)carbamoyl)-4-(5-isopropyl-1*H*-pyrazol-1-yl)piperidin-1-yl)methyl)-4-oxocyclohexane-1-carboxylate (380 mg, 0.654 mmol) dissolved in a mixture of tetrahydrofuran (3 mL), methanol (3 mL) and water (1 mL) was added lithium hydroxide monohydrate (274 mg, 6.54 mmol) and the solution stirred at 50 °C for 18 hours. The resultant mixture was diluted with dichloromethane (20 mL) and extracted with half saturated aqueous ammonium chloride (2 × 10 mL). The combined organic phase was dried over MgSO₄, filtered and the filtrate was concentrated under reduced pressure to provide the title compound. MS (APCI+) *m*/*z*: 567.0 [M+H]⁺.

**Step 4: 1-((4-((4-chloro-2-(difluoromethoxy)phenyl)carbamoyl)-4-(5-isopropyl-1*H-*pyrazol-1-yl)piperidin-1-yl)methyl)-4-hydroxycyclohexane-1-carboxylic acid.** To 1-((4-((4-chloro-2-(difluoromethoxy)phenyl)carbamoyl)-4-(5-isopropyl-1*H*-pyrazol-1-yl)piperidin-1-yl)methyl)-4-oxocyclohexane-1-carboxylic acid (315 mg, 0.556 mmol) dissolved in methanol (10 mL) was added solid sodium borohydride (31.5 mg, 0.833 mmol) portion wise over 2 minutes at room temperature. After allowing the mixture to stir for 1.5 hours, a solution of half saturated aqueous ammonium chloride (5 mL) was added and the mixture was extracted with dichloromethane (20 mL). The organic extract was then dried over magnesium sulfate, filtered and concentrated to provide the title compound. MS (APCI+) *m*/*z*: 569.2 [M+H]⁺.

**Step 5: Compound 95.** 220 mg of 1-((4-((4-chloro-2-(difluoromethoxy)phenyl)carbamoyl)-4-(5-isopropyl-1*H*-pyrazol-1-yl)piperidin-1-yl)methyl)-4-hydroxycyclohexane-1-carboxylic acid was subjected to SFC separation employing a CHIRAL ART Cellulose-SC (IC) column using methanol with 0.1% diethylamine added as eluent to provide the title compound as the second peak to elute from the column. ¹H NMR (400 MHz, dimethyl sulfoxide-*d*₆) δ ppm 8.10 (s, 1H), 7.81 (d, *J =* 9.4 Hz, 1H), 7.52 (d, *J =* 1.8 Hz, 1H), 7.32 (m, 2H), 7.14 (t, *J =* 73.2 Hz, 1H), 6.32 (d, *J =* 1.8 Hz, 1H), 4.42 (d, *J =* 4.5 Hz, 1H), 2.91 (m, 1H), 2.70 (m, 2H), 2.39 (m, 7H), 2.00 (m, 2H), 1.66 (m, 2H), 1.17 (m, 4H), 1.09 (d, *J =* 6.7 Hz, 6H). MS (APCI+) *m*/*z:* 569.2 [M+H]⁺.

### Example #44: Compound 175: (1r,4r)-1-[[4-[[4-chloro-2-(difluoromethoxy)phenyl]carbamoyl]-4-(5-isopropylpyrazol-1-yl)-1-piperidyl]methyl]-4-hydroxy-4-methyl-cyclohexanecarboxylic acid

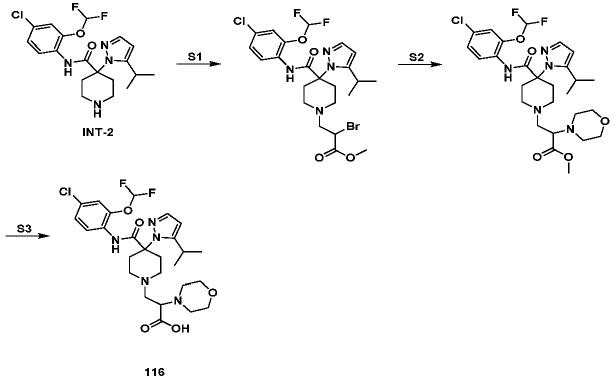

**Compound 175.** To a solution of 1-((4-((4-chloro-2-(difluoromethoxy)phenyl)carbamoyl)-4-(5-isopropyl-1*H*-pyrazol-1-yl)piperidin-1-yl)methyl)-4-oxocyclohexane-1-carboxylic acid **(Example #43, Step 3,** 50 mg, 0.088 mmol) dissolved in tetrahydrofuran (1.5 mL) was added a solution of methylmagnesium bromide (3.0 M in ether, 0.147 mL, 0.441 mmol) at room temperature. After allowing the mixture to stir for 0.5 hours, a solution of half saturated aqueous ammonium chloride (3 mL) was added. The mixture was extracted with dichloromethane (5 mL). The organic extract was dried over magnesium sulfate, filtered and concentrated. The residue was purified by HPLC to provide the title compound. ¹H NMR (500 MHz, dimethyl sulfoxide-*d*₆) δ ppm 7.81 (d, *J =* 9.4 Hz, 1H), 7.52 (d, *J =* 1.8 Hz, 1H), 7.31 (m, 2H), 7.14 (t, *J =* 73.2 Hz, 1H), 6.32 (d, *J =* 1.8 Hz, 1H), 2.91 (m, 1H), 2.75 (m, 2H), 2.54 (m, 2H), 2.46 (m, 4H), 2.35 (m, 2H), 1.91 (m, 2H), 1.38 (m, 4H), 1.31 (m, 2H), 1.08 (d, *J =* 6.7 Hz, 6H), 1.06 (s, 3H). MS (APCI+) *m*/*z:* 567.7 [M+H]⁺.

### Example #45: Compound 116: 3-[4-[[4-chloro-2-(difluoromethoxy)phenyl]carbamoyl]-4-(5-isopropylpyrazol-1-yl)-1-piperidyl]-2-morpholino-propanoic acid

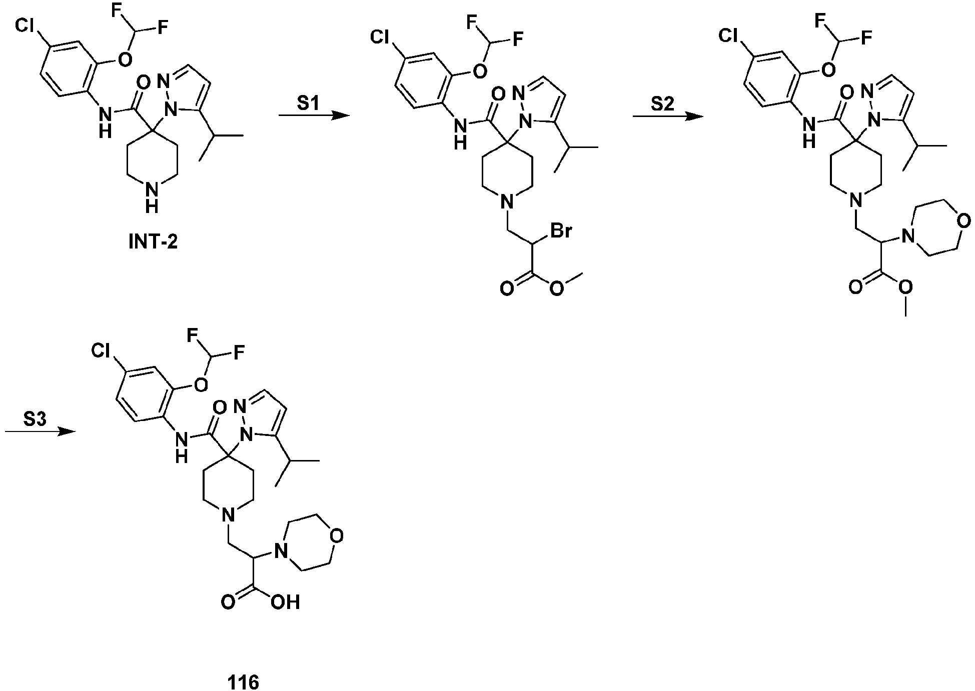

**Step 1: methyl 2-bromo-3-(4-((4-chloro-2-(difluoromethoxy)phenyl)carbamoyl)-4-(5-isopropyl-1*H*-pyrazol-1-yl)piperidin-1-yl)propanoate.** To a solution of **INT-2** (200 mg, 0.484 mmol) dissolved in dichloromethane (3 mL) was added methyl 2-bromoacrylate (120 mg, 0.727 mmol), and 1,8-diazabicyclo[5.4.0]undec-7-ene (118 mg, 0.775 mmol). The mixture was stirred at 25 °C for 2 hours. The resultant mixture was diluted with water (5 mL) and extracted with dichloromethane (2 × 10 mL). The combined organic phase was dried over MgSO₄, filtered and the filtrate was concentrated under reduced pressure. The residue was purified by silica gel column chromatography using ethyl acetate in heptanes to provide the title product. MS (APCI+) *m*/*z:* 577/579 [M+H]⁺.

**Step 2: methyl 3-(4-((4-chloro-2-(difluoromethoxy)phenyl)carbamoyl)-4-(5-isopropyl-1*H*-pyrazol-1-yl)piperidin-1-yl)-2-morpholinopropanoate.** To a solution of methyl 2-bromo-3-(4-((4-chloro-2-(difluoromethoxy)phenyl)carbamoyl)-4-(5-isopropyl-1*H*-pyrazol-1-yl)piperidin-1-yl)propanoate (80 mg, 0.138 mmol) dissolved in acetonitrile (2 mL) was added morpholine (0.060 mL, 0.692 mmol) and tripotassium phosphate (88 mg, 0.415 mmol). After allowing the mixture to stir at room temperature for 18 hours, water (5 mL) was added and the mixture was extracted with dichloromethane (10 mL). The organic extract was then dried over magnesium sulfate, filtered, and concentrated to provide the title compound that was used directly in the next reaction.

**Step 3: Compound 116.** To a solution of methyl 3-(4-((4-chloro-2-(difluoromethoxy)phenyl)carbamoyl)-4-(5-isopropyl-1*H*-pyrazol-1-yl)piperidin-1-yl)-2-morpholinopropanoate (80 mg, 0.137 mmol) dissolved in a mixture of tetrahydrofuran (3 mL), methanol (3 mL) and water (1 mL) was added lithium hydroxide monohydrate (58 mg, 1.37 mmol). The solution was stirred at ambient temperature for 72 hours. The resultant mixture was diluted with dichloromethane (10 mL) and extracted with half saturated aqueous ammonium chloride (2 × 5 mL). The combined organic phase was dried over MgSO₄, filtered and the filtrate was concentrated under reduced pressure. The residue was purified by silica gel column chromatography using methanol in dichloromethane to provide the title compound. ¹H NMR (600 MHz, dimethyl sulfoxide-*d*₆) δ ppm 8.19 (s, 1H), 7.81 (d, *J* = 9.4 Hz, 1H), 7.53 (d, *J* = 1.8 Hz, 1H), 7.32 (m, 2H), 7.15 (t, *J* = 73.2 Hz, 1H), 6.33 (d, *J* = 1.8 Hz, 1H), 3.53 (m, 4H), 3.30 (m, 1H), 2.90 (m, 2H), 2.74 (m, 2H), 2.59 (m, 4H), 2.47 (m, 4H), 2.37 (m, 3H), 1.10 (d, *J* = 6.7 Hz, 6H). MS (APCI+) *m*/*z:* 570.8 [M+H]⁺.

### Example #46: Compound 163: (2S)-2-[[4-[[4-chloro-2-(difluoromethoxy)phenyl]carbamoyl]-4-(5-isopropylpyrazol-1-yl)-1-piperidyl]methyl]-3-cyano-propanoic acid

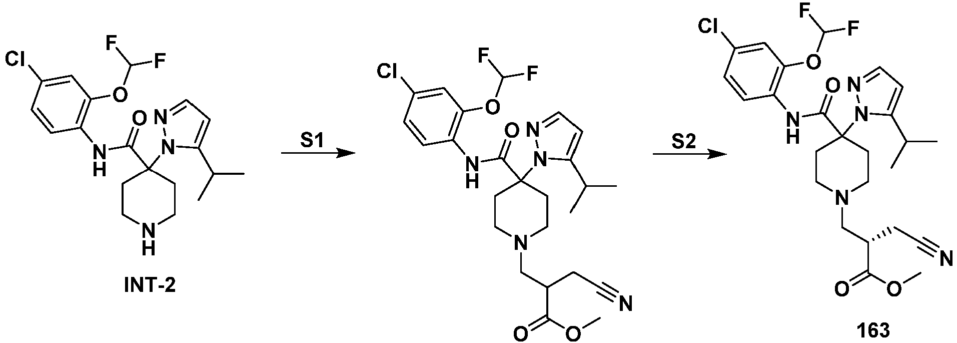

**Step 1:** methyl **3-(4-((4-chloro-2-(difluoromethoxy)phenyl)carbamoyl)-4-(5-isopropyl-1*H*-pyrazol-1-yl)piperidin-1-yl)-2-(cyanomethyl)propanoate.** To a solution of **INT-2** (300 mg, 0.727 mmol) dissolved in dichloromethane (10 mL) was added methyl 2-(cyanomethyl)acrylate (272 mg, 2.18 mmol), and 1,8-diazabicyclo[5.4.0]undec-7-ene (354 mg, 2.33 mmol). The mixture was stirred at 25 °C for 48 hours. The resultant mixture was diluted with water (5 mL) and extracted with dichloromethane (2 × 10 mL). The combined organic phase was dried over MgSO₄, filtered and the filtrate was concentrated under reduced pressure to provide a residue, which was purified by silica gel column chromatography using ethyl acetate in heptanes to provide the title compound. MS (APCI+) *m*/*z:* 538.9 [M+H]⁺.

**Step 2: Compound 163.** Methyl 3-(4-((4-chloro-2-(difluoromethoxy)phenyl)carbamoyl)-4-(5-isopropyl-1*H*-pyrazol-1-yl)piperidin-1-yl)-2-(cyanomethyl)propanoate (285 mg, 0.530 mmol) was dissolved in a mixture of tetrahydrofuran (3 mL), methanol (3 mL) and water (1 mL). Lithium hydroxide monohydrate (222 mg, 5.30 mmol) was added and the mixture was stirred at 25 °C for 2 hours. The resultant mixture was diluted with dichloromethane (10 mL) and extracted with half saturated aqueous ammonium chloride (2 × 5 mL). The combined organic phase was dried over MgSO₄, filtered and the filtrate was concentrated under reduced pressure. The residue was purified by silica gel column chromatography using methanol in dichloromethane to provide the racemic title compound. Racemic title compound (90 mg) was then subjected to SFC separation employing a Whelk-O^{®} (S,S) column using 1:1 isopropanol:methanol with 0.1% diethylamine added as eluent to provide the title compound as the first peak to elute from the column. ¹H NMR (600 MHz, dimethyl sulfoxide-*d*₆) δ ppm 8.23 (s, 1H), 7.78 (d, *J* = 9.4 Hz, 1H), 7.53 (d, *J =* 1.8 Hz, 1H), 7.32 (m, 2H), 7.15 (t, *J =* 73.2 Hz, 1H), 6.33 (d, *J =* 1.8 Hz, 1H), 3.17 (s, 1H), 2.93 (m, 2H), 2.80 (m, 2H), 2.69 (m, 2H), 2.58 (m, 2H), 2.50 (m, 4H), 2.41 (m, 2H), 1.10 (d, *J =* 6.7 Hz, 6H). MS (APCI+) *m*/*z:* 524.7 [M+H]⁺.

### Example #47: Compound 5: 1-acetyl-4-[[4-[[4-chloro-2-(difluoromethoxy)phenyl]carbamoyl]-4-(5-isopropylpyrazol-1-yl)-1-piperidyl]methyl]piperidine-4-carboxylic acid

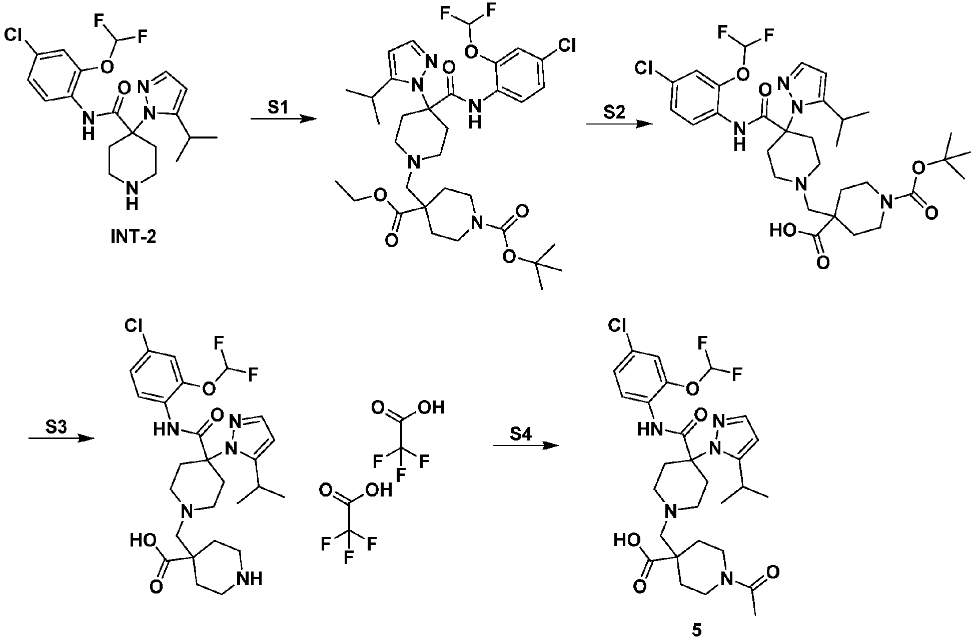

**Step** 1: **1-(*tert*-butyl) 4-ethyl 4-((4-((4-chloro-2-(difluoromethoxy)phenyl)carbamoyl)-4-(5-isopropyl-1*H*-pyrazol-1-yl)piperidin-1-yl)methyl)piperidine-1,4-dicarboxylate.** To a solution of **INT-2** (500 mg, 1.21 mmol) dissolved in dichloromethane (12 mL) was added 1-(*tert*-butyl) 4-ethyl 4-formylpiperidine-1,4-dicarboxylate (1036 mg, 3.64 mmol), and sodium triacetoxyborohydride (770 mg, 3.64 mmol). The resulting mixture was then stirred at 25 °C for 48 hours. The resultant mixture was diluted with water (10 mL) and extracted with dichloromethane (2 × 15 mL). The combined organic phase was dried over MgSO₄, filtered and the filtrate was concentrated under reduced pressure. The residue was purified by silica gel column chromatography using ethyl acetate in heptanes to provide the title product. MS (APCI+) *m*/*z*: 682.5 [M+H]⁺.

**Step 2: 1-(*tert*-butoxycarbonyl)-4-((4-((4-chloro-2-(difluoromethoxy)phenyl)carbamoyl)-4-(5-isopropyl-1H-pyrazol-1-yl)piperidin-1-yl)methyl)piperidine-4-carboxylic acid.** To a solution of 1-(*tert*-butyl) 4-ethyl 4-((4-((4-chloro-2-(difluoromethoxy)phenyl)carbamoyl)-4-(5-isopropyl-1*H*-pyrazol-1-yl)piperidin-1-yl)methyl)piperidine-1,4-dicarboxylate (349 mg, 0.512 mmol) dissolved in a mixture of tetrahydrofuran (3 mL), methanol (3 mL) and water (1 mL) was added lithium hydroxide monohydrate (215 mg, 5.12 mmol) and the solution stirred at 60 °C for 18 hours. The resultant mixture was diluted with dichloromethane (10 mL) and extracted with half saturated aqueous ammonium chloride (2 × 5 mL). The combined organic phase was dried over MgSO₄, filtered and the filtrate was concentrated under reduced pressure to provide the title compound. MS (APCI+) *m*/*z*: 654.4 [M+H]⁺.

**Step 3: 4-((4-((4-chloro-2-(difluoromethoxy)phenyl)carbamoyl)-4-(5-isopropyl-1*H-*pyrazol-1-yl)piperidin-1-yl)methyl)piperidine-4-carboxylic acid.** To 1-(*tert*-butoxycarbonyl)-4-((4-((4-chloro-2-(difluoromethoxy)phenyl)carbamoyl)-4-(5-isopropyl-1*H*-pyrazol-1-yl)piperidin-1-yl)methyl)piperidine-4-carboxylic acid (305 mg, 0.466 mmol) dissolved in dichloromethane (1 mL) was added trifluoroacetic acid (1.80 mL, 23.31 mmol) and the mixture was stirred at 25 °C for 1.5 hours. The resultant mixture was concentrated under reduced pressure to provide the title compound as a bis trifluoroacetic acid salt. MS (APCI+) *m*/*z:* 554.4 [M+H]⁺.

**Step 4: Compound 5.** To 4-((4-((4-chloro-2-(difluoromethoxy)phenyl)carbamoyl)-4-(5-isopropyl-1H-pyrazol-1-yl)piperidin-1-yl)methyl)piperidine-4-carboxylic acid bis trifluoroacetic acid salt (57 mg, 0.072 mmol) dissolved in a mixture of tetrahydrofuran (1.5 mL) was added triethylamine (0.050 mL, 0.361 mmol) and acetic anhydride (0.0102 mL, 0.108 mmol) and the solution stirred at ambient temperature for 15 minutes. The resultant mixture was diluted with dichloromethane (1 mL), and extracted with half saturated aqueous ammonium chloride (2 × 3 mL). The combined organic phase was dried over MgSO₄, filtered and the filtrate was concentrated under reduced pressure to provide a residue, which was purified by silica gel column chromatography using methanol in dichloromethane to provide the title compound. ¹H NMR (600 MHz, dimethyl sulfoxide-*d*₆) δ ppm 8.12 (s, 1H), 7.80 (d, *J* = 9.4 Hz, 1H), 7.52 (d, *J* = 1.8 Hz, 1H), 7.32 (m, 2H), 7.14 *(t, J* = 73.2 Hz, 1H), 6.32 (d, *J* = 1.8 Hz, 1H), 4.01 (m, 1H), 3.62 (m, 1H), 3.15 (m, 1H), 2.91 (m, 1H), 2.77 (m, 1H), 2.72 (m, 2H), 2.46 (m, 3H), 2.34 (m, 2H), 1.97 (s, 3H), 1.96 (m, 1H), 1.88 (m, 1H), 1.41 (m, 1H), 1.28 (m, 1H), 1.09 (d, *J* = 6.7 Hz, 6H). MS (APCI+) *m*/*z:* 626.4 [M+H]⁺.

### Example #48: Compound 119: (2R)-2-[[4-[[4-chloro-2-(difluoromethoxy)phenyl]carbamoyl]-4-(5-isopropylpyrazol-1-yl)-1-piperidyl]methyl]-3-methoxy-propanoic acid

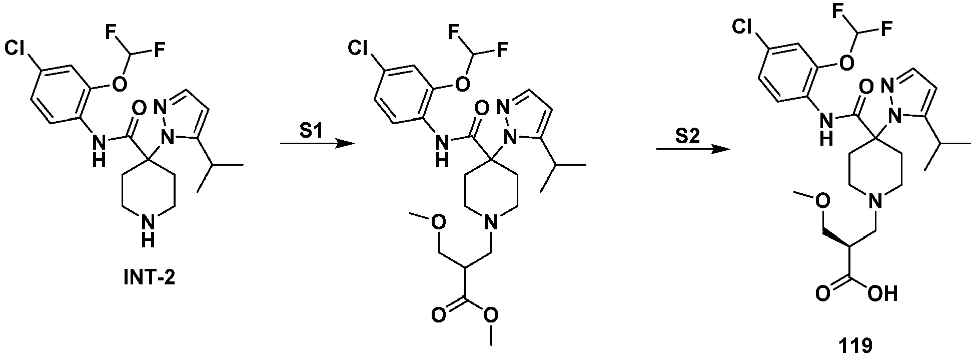

**Step 1: methyl 3-(4-((4-chloro-2-(difluoromethoxy)phenyl)carbamoyl)-4-(5-isopropyl-1*H*-pyrazol-1-yl)piperidin-1-yl)-2-(methoxymethyl)propanoate.** To a solution of **INT-2** (200 mg, 0.484 mmol) dissolved in dichloromethane (3 mL) was added methyl 2-(methoxymethyl)prop-2-enoate (95 mg, 0.727 mmol), and 1,8-diazabicyclo[5.4.0]undec-7-ene (118 mg, 0.775 mmol). The mixture was then stirred at 25 °C for 2.5 hours. The resultant mixture was diluted with water (5 mL) and extracted with dichloromethane (2 × 10 mL). The combined organic phase was dried over MgSO₄, filtered and the filtrate was concentrated under reduced pressure. The residue was purified by silica gel column chromatography using ethyl acetate in heptanes to provide the title compound. MS (APCI+) *m*/*z*: 543.7 [M+H]⁺.

**Step 2: Compound 119.** Methyl 3-(4-((4-chloro-2-(difluoromethoxy)phenyl)carbamoyl)-4-(5-isopropyl-1*H*-pyrazol-1-yl)piperidin-1-yl)-2-(methoxymethyl)propanoate (205 mg, 0.378 mmol) was dissolved in a mixture of tetrahydrofuran (3 mL), methanol (3 mL) and water (1 mL). Lithium hydroxide monohydrate (158 mg, 3.78 mmol) was added, and the solution was stirred at 25 °C for 24 hours. The resultant mixture was diluted with dichloromethane (10 mL) and extracted with half saturated aqueous ammonium chloride (2 × 5 mL). The combined organic phase was dried over MgSO₄, filtered and the filtrate was concentrated under reduced pressure. The residue was purified by silica gel column chromatography using methanol in dichloromethane to provide the racemic title compound . The racemic title compound was subjected to SFC separation employing a CHIRAL ART Cellulose SC (IC) column using ethanol with 0.1% diethylamine added as eluent to provide the title compound as the second peak to elute from the column. ¹H NMR (400 MHz, dimethyl sulfoxide-*d*₆) δ ppm 8.21 (s, 1H), 7.78 (d, *J* = 9.4 Hz, 1H), 7.53 (d, *J* = 1.8 Hz, 1H), 7.32 (m, 2H), 7.15 (t, *J* = 73.2 Hz, 1H), 6.33 (d, *J* = 1.8 Hz, 1H), 3.48 (m, 4H), 3.21 (s, 3H), 2.93 (m, 1H), 2.83 (m, 1H), 2.75 (m, 2H), 2.49 (m, 3H), 2.38 (m, 3H), 1.10 (d, *J =* 6.7 Hz, 6H). MS (APCI+) *m*/*z:* 529.7 [M+H]⁺.

### Example #49: Compound 78: (2S)-3-[4-[[4-chloro-2-(difluoromethoxy)phenyl]carbamoyl]-4-(5-isopropylpyrazol-1-yl)-1-piperidyl]-2-methyl-2-tetrahydropyran-4-yl-propanoic acid

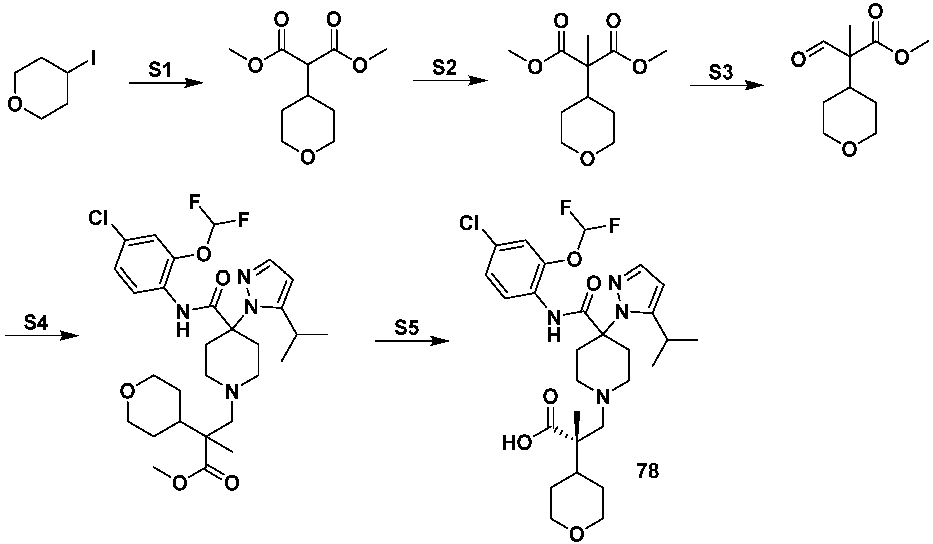

**Step 1: dimethyl 2-(tetrahydro-2*H*-pyran-4-yl)malonate.** To a solution of potassium *tert*-butoxide (1.02 g, 9.08 mmol, 1.2 equiv) in dimethyl sulfoxide (7.6 mL, 1M) was added dimethyl malonate (1 g, 7.57 mmol, 1 equiv) and 4-iodotetrahydro-2*H*-pyran (2.41 g, 11.35 mmol, 1.5 equiv). The reaction mixture was heated to 80 °C for 16 hours then cooled to room temperature and poured into 1N aqueous HCl. The mixture was extracted 3x with methyl *tert*-butyl ether, and washed with saturated sodium bicarbonate, 10% saturated Na₂S₃O₃, and brine. The organic layer was dried over Na₂SO₄, filtered, and concentrated. The residue was purified by flash chromatography using ethyl acetate in heptanes to provide the title compound. MS (ESI) *m*/*z* 217.0 (M+H) ⁺.

**Step 2: dimethyl 2-methyl-2-(tetrahydro-2*H*-pyran-4-yl)malonate.** A solution of dimethyl 2-(tetrahydro-2*H*-pyran-4-yl)malonate (2.3 g, 10.64 mmol, 1 equiv) in tetrahydrofuran (42.5 mL, 0.25 M) was cooled to 0 °C. NaH (60% dispersion in mineral oil, 638 mg, 15.96 mmol, 1.5 equiv) was added and the mixture was allowed to stir for 30 minutes. Iodomethane (3.02 g, 21.27 mmol, 2 equiv) was added and the reaction mixture was allowed to warm to ambient temperature. After 16 hours, saturated aqueous NH₄Cl was added and the mixture was extracted 3x with ethyl acetate, washed with brine, dried over Na₂SO₄, filtered, and concentrated. The residue was purified by flash chromatography, using ethyl acetate in heptanes, to provide the title compound. MS (ESI) *m*/*z* 231.6 (M+H)⁺.

**Step 3: methyl 2-methyl-3-oxo-2-(tetrahydro-2*H*-pyran-4-yl)propanoate.** A solution of dimethyl 2-methyl-2-(tetrahydro-2*H*-pyran-4-yl)malonate (1 g, 4.34 mmol, 1 equiv) in dichloromethane (8.7 mL, 0.5 M) was cooled to -78 °C. Diisobutylaluminium hydride (1M in dichloromethane, 8.69 mL, 8.69 mmol, 2 equiv) was added over 10 minutes. After 45 minutes, the reaction mixture was quenched by addition of 350 µL water, 350 µL 15% aqueous NaOH solution, and then 870 µL water. The mixture was diluted with methyl *tert*-butyl ether, dried over MgSO₄, filtered, and concentrated. The residue was purified by flash chromatography, using ethyl acetate in heptanes, to provide the title compound.

**Step 4: methyl 3-(4-((4-chloro-2-(difluoromethoxy)phenyl)carbamoyl)-4-(5-isopropyl-1H-pyrazol-1-yl)piperidin-1-yl)-2-methyl-2-(tetrahydro-2H-pyran-4-yl)propanoate.** A solution of methyl 2-methyl-3-oxo-2-(tetrahydro-2*H*-pyran-4-yl)propanoate (495 mg, 2.47 mmol, 2 equiv) and **INT-2** (510 mg, 1.24 mmol, 1 equiv) in 1,2-dichloroethane (6.2 mL, 0.2 M) was stirred at room temperature for 90 minutes. Tetramethylammonium triacetoxyborohydride (650 mg, 2.47 mmol, 2 equiv) was added. After 20 hours, the mixture was poured into saturated NH₄Cl and extracted 3x with dichloromethane. The organics were dried over Na₂SO₄, filtered, and concentrated. The residue was purified by flash chromatography, using ethyl acetate in heptanes, to provide the title compound. MS (ESI) *m*/*z* 597.2 (M+H) ⁺.

**Step 5: Compound 78** Methyl 3-(4-((4-chloro-2-(difluoromethoxy)phenyl)carbamoyl)-4-(5-isopropyl-1*H*-pyrazol-1-yl)piperidin-1-yl)-2-methyl-2-(tetrahydro-2*H*-pyran-4-yl)propanoate (161 mg, 0.27 mmol, 1 equiv) was dissolved in a mixture of tetrahydrofuran (1.2 mL), methanol (1.2 mL) and water (0.385 mL). Lithium hydroxide hydrate (113 mg, 2.7 mmol, 10 equivalents) was added and the mixture was heated to 70 °C. After 24 hours, the reaction mixture was poured into saturated aqueous NH₄Cl, extracted 3x with dichloromethane, dried over Na₂SO₄, filtered, and concentrated. The residue was purified by flash chromatography, using methanol in dichloromethane, to provide a racemic mixture. The enantiomers were separated using Chiral SFC using a CHIRALCEL^{®} OZ^{™}-H column. Both fractions were repurified using the initial flash chromatography conditions to provide the arbitrarily assigned R isomer, the first eluting peak, and the second eluting peak provided the title compound, arbitrarily assigned S. ¹H NMR (500 MHz, dimethyl sulfoxide-*d*₆) δ ppm 12.47 (br s, 1H), 8.12 (s, 1H), 7.80 (d, *J =* 9.4 Hz, 1H), 7.52 (d, *J =* 1.8 Hz, 1H), 7.35 - 7.30 (m, 1H), 7.14 (t, *J =* 73.2 Hz, 1H), 6.32 (d, *J =* 1.8 Hz, 1H), 3.84 (dt, *J =* 10.4, 5.1 Hz, 2H), 3.23 (dddd, *J =* 11.5, 9.8, 7.7, 2.1 Hz, 2H), 2.92 (hept, *J =* 6.7 Hz, 1H), 2.73 (ddt, *J =* 16.4, 10.3, 4.7 Hz, 2H), 2.63 (d, *J =* 13.4 Hz, 1H), 2.56 - 2.28 (m, 8H), 1.70 (ddt, *J =* 11.9, 7.0, 3.4 Hz, 1H), 1.48 (d, *J =* 12.8 Hz, 1H), 1.29 (dddd, *J =* 41.4, 25.6, 12.2, 8.0 Hz, 3H), 1.09 (d, *J =* 6.6 Hz, 6H), 1.02 (s, 3H). MS (ESI) *m*/*z* 583.0 (M+H)⁺.

### Example #50: Compound 3: 4-[[4-[[4-chloro-2-(difluoromethoxy)phenyl]carbamoyl]-4-(5-isopropylpyrazol-1-yl)-1-piperidyl]methyl]tetrahydropyran-4-carboxylic acid

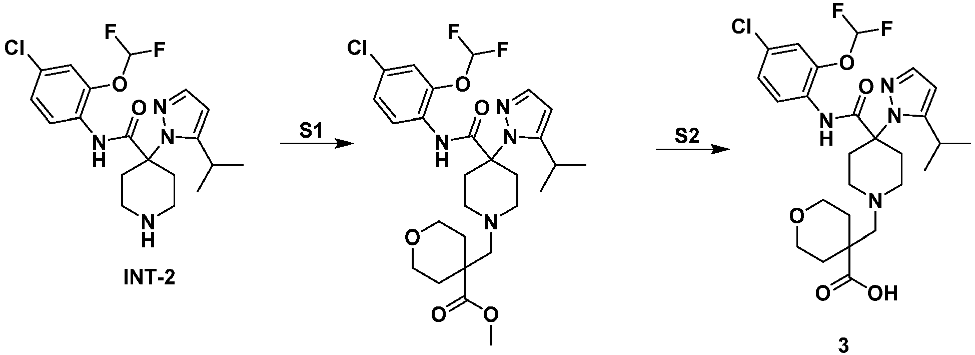

**Step 1: methyl 4-((4-((4-chloro-2-(difluoromethoxy)phenyl)carbamoyl)-4-(5-isopropyl-1*H*-pyrazol-1-yl)piperidin-1-yl)methyl)tetrahydro-2*H*-pyran-4-carboxylate.** To a solution of **INT-2** (100 mg, 0.242 mmol) dissolved in dichloromethane (3 mL) was added methyl 4-formyltetrahydro-2*H*-pyran-4-carboxylate (63 mg, 0.363 mmol) and sodium triacetoxyborohydride (77 mg, 0.363 mmol). The mixture was then stirred at 25 °C for 18 hours. The resultant mixture was diluted with water (10 mL) and extracted with dichloromethane (2 × 10 mL). The combined organic phase was dried over MgSO₄, filtered and the filtrate was concentrated under reduced pressure. The residue was purified by silica gel column chromatography using ethyl acetate in heptanes to provide the title compound. MS (APCI+) *m*/*z*: 569.4 [M+H]⁺.

**Step 2: Compound 3.** Methyl 4-((4-((4-chloro-2-(difluoromethoxy)phenyl)carbamoyl)-4-(5-isopropyl-1*H*-pyrazol-1-yl)piperidin-1-yl)methyl)tetrahydro-2*H*-pyran-4-carboxylate (75 mg, 0.132 mmol) was dissolved in a mixture of tetrahydrofuran (3 mL), methanol (3 mL) and water (1 mL) and lithium hydroxide monohydrate (55 mg, 1.32 mmol)was added. The mixture was stirred at 40 °C for 62 hours. The resultant mixture was diluted with dichloromethane (10 mL) and extracted with half saturated aqueous ammonium chloride (2 × 5 mL). The combined organic phase was dried over MgSO₄, filtered and the filtrate was concentrated under reduced pressure. The residue was purified by silica gel column chromatography using methanol in dichloromethane to provide the title compound. ¹H NMR (500 MHz, dimethyl sulfoxide-*d*₆) δ ppm 12.55 (bs, 1H), 8.11 (s, 1H), 7.80 (d, *J* = 9.4 Hz, 1H), 7.52 *(d, J* = 1.8 Hz, 1H), 7.32 (m, 2H), 7.14 (t, *J* = 73.2 Hz, 1H), 6.32 (d, *J =* 1.8 Hz, 1H), 3.69 (m, 2H), 3.37 (m, 4H), 2.91 (m, 1H), 2.72 (m, 2H), 2.45 (m, 4H), 2.35 (m, 2H), 1.87 (m, 2H), 1.46 (m, 2H), 1.09 (d, *J =* 6.7 Hz, 6H). MS (APCI+) *m*/*z:* 555.4 [M+H]⁺.

### Example #51: Compound 123: (1s,3s)-3-[4-[[4-chloro-2-(difluoromethoxy)phenyl]carbamoyl]-4-(4-fluoro-5-isopropyl-pyrazol-1-yl)-1-piperidyl]cyclobutanecarboxylic acid

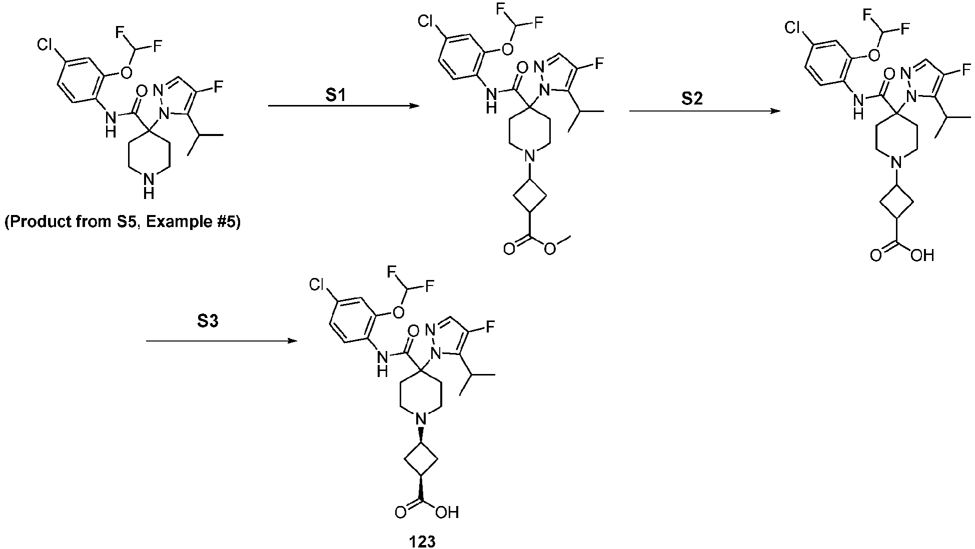

**Step 1: methyl 3-(4-((4-chloro-2-(difluoromethoxy)phenyl)carbamoyl)-4-(4-fluoro-5-isopropyl-1*H*-pyrazol-1-yl)piperidin-1-yl)cyclobutane-1-carboxylate.** A mixture of (product of **Step 5, Example #5**), N-(4-chloro-2-(difluoromethoxy)phenyl)-4-(4-fluoro-5-isopropyl-1*H-*pyrazol-1-yl)piperidine-4-carboxamide (1.38 g, 3.20 mmol) and methyl 3-oxocyclobutane-1-carboxylate (0.821 g, 6.41 mmol) were combined in a 40 mL pressure vial followed by the addition of dimethylsulfoxide (20 mL) and heated at 70 °C for 40 minutes to give a homogeneous solution. Formic acid (1.23 mL, 32.0 mmol) was then added and the mixture was stirred at 70 °C for 17 hours. The reaction mixture was concentrated to dryness. The residue was purified by flash silica gel chromatography (ethyl acetate in heptanes) to provide the title compound. MS (APCI+) *m*/*z*: *543* [M]⁺.

**Step 2: 3-(4-((4-chloro-2-(difluoromethoxy)phenyl)carbamoyl)-4-(4-fluoro-5-isopropyl-1*H*-pyrazol-1-yl)piperidin-1-yl)cyclobutane-1-carboxylic acid.** The product from **Step 1** (1.1 g, 2.03 mmol) was dissolved in tetrahydrofuran (30 mL) followed by the addition of potassium trimethylsilanolate (0.78 g, 6.08 mmol). The reaction mixture was heated to 60 °C for 30 minutes. The reaction mixture was cooled to 25 °C and diluted with dichloromethane (40 mL) followed by saturated ammonium chloride (30 mL). The organic extracts were dried over MgSO₄, filtered and concentrated. The residue was purified by flash silica gel chromatography (methanol in dichloromethane) to provide the title compound. MS (APCI+) *m*/*z*: 529 [M+H]⁺.

**Step 3: Compound 123.** The cis, trans product mixture from **Step 2** (0.721 g) was separated by preparative chiral supercritical fluid chromatography on CHIRALCEL^{®} OZ^{™}-H column, (30 x 250 mm, 5 µm) at ambient temperature, using a mobile phase of 25% 97:3 methanol (0.5% diethylamine)/water:CO₂. The fractions were collected based on UV detection at 254 nm with the desired second eluting peak detected at 12.65 minutes (B) as the cis isomer. Peak B was concentrated to dryness then was re-purified by silica gel chromatography to remove residual diethylamine additive from the SFC run eluting with methanol in dichloromethane to provide the title compound. ¹H NMR (500 MHz, dimethyl sulfoxide-*d*₆) δ ppm 8.62 (s, 1H), 7.63 - 7.59 (m, 1H), 7.56 (d, *J =* 4.2 Hz, 1H), 7.34 (d, *J =* 2.3 Hz, 1H), 7.33 (s, 1H), 7.17 (d, *J =* 73.2 Hz, 1H), 2.99 (hept, J = 7.0 Hz, 2H), 2.67 (tt, *J* = 9.8, 8.2 Hz, 2H), 2.57 (ddd, *J* = 15.3, 7.3, 3.8 Hz, 6H), 2.36 (d, *J* = 9.9 Hz, 2H), 2.25 - 2.14 (m, 2H), 1.95 - 1.85 (m, 1H), 1.16 (d, *J* = 6.8 Hz, 6H). MS (APCI+) *m*/*z*: 529 [M+H]⁺.

### Example #52: Compound 41: 4-[[4-[[4-chloro-2-(difluoromethoxy)phenyl]carbamoyl]-4-(5-isopropylpyrazol-1-yl)-1-piperidyl]methyl]-1-propanoyl-piperidine-4-carboxylic acid

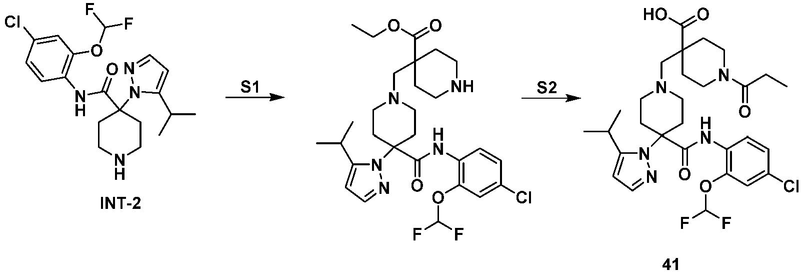

**Step 1: ethyl 4-((4-((4-chloro-2-(difluoromethoxy)phenyl)carbamoyl)-4-(5-isopropyl-1*H*-pyrazol-1-yl)piperidin-1-yl)methyl)piperidine-4-carboxylate.** 1-*tert*-Butyl 4-ethyl 4-formylpiperidine-1,4-dicarboxylate (83 mg, 0.291 mmol) was added to *N*-(4-chloro-2-(difluoromethoxy)phenyl)-4-(5-isopropyl-1*H*-pyrazol-1-yl)piperidine-4-carboxamide (**INT-2**, 60 mg, 0.145 mmol) in 1,2-dichloroethane (2 mL) followed by the addition of tetramethylammonium triacetoxyborohydride (76 mg, 0.291 mmol). The mixture was stirred at 40 °C overnight. The reaction was quenched with saturated aqueous NaHCO₃ and extracted with dichloromethane (10 mL x 2). The combined organic layers were washed with brine and concentrated under reduced pressure. The crude product was purified by column chromatography on silica gel (ethyl acetate in hexane) to provide 1*-tert*-butyl 4-ethyl 4-((4-((4-chloro-2-(difluoromethoxy)phenyl)carbamoyl)-4-(5-isopropyl-1*H*-pyrazol-1-yl)piperidin-1-yl)methyl)piperidine-1,4-dicarboxylate, which dissolved in dichloromethane (1 mL). 4M HCl in dioxane (4 mL) was added and the mixture was stirred at room temperature overnight. The mixture was concentrated under reduced pressure to yield the crude title compound. MS (APCI+) *m*/*z*: 582 (M+H)⁺ .

**Step 2: Compound 41.** Triethylamine (0.084 mL, 0.601 mmol) was added to a solution of ethyl 4-((4-((4-chloro-2-(difluoromethoxy)phenyl)carbamoyl)-4-(5-isopropyl-1*H*-pyrazol-1-yl)piperidin-1-yl)methyl)piperidine-4-carboxylate (70 mg, 0.120 mmol) in dichloromethane (2 mL). Propionyl chloride (11.13 mg, 0.120 mmol) was added to the mixture. The mixture was stirred at room temperature for 2 hours and concentrated under reduced pressure. The crude product was purified by column chromatography on silica gel (ethyl acetate in hexane) to provide ethyl 4-((4-((4-chloro-2-(difluoromethoxy)phenyl)carbamoyl)-4-(5-isopropyl-1*H*-pyrazol-1-yl)piperidin-1-yl)methyl)-1-propionylpiperidine-4-carboxylate, which dissolved in methanol (2 mL) and 5N aqueous NaOH (0.5 mL) and stirred at room temperature for overnight. The pH was adjusted to about 1 by adding 2N aqueous HCl. The mixture was concentrated under reduced pressure and the crude product was purified by HPLC to provide the title compound as a trifluoroacetic acid salt. ¹H NMR (600 MHz, dimethyl sulfoxide-*d*₆) δ ppm 7.65 (s, 1H), 7.61 (s, 1H), 7.34 (m, 2H), 7.18 (t, *J =* 73.2 Hz, 1H), 6.41 (s, 1H), 3.73 (s, 1H), 3.66 - 3.02 (m, 10H), 2.87 (s, 1H), 2.68 (s, 4H), 2.31 (p, *J* = 6.7 Hz, 2H), 1.95 (dd, *J =* 45.6, 13.7 Hz, 2H), 1.55 (d, *J =* 43.2 Hz, 2H), 1.13 (d, *J =* 6.6 Hz, 6H), 0.98 (t, *J =* 7.4 Hz, 3H). MS (APCI+) *m*/*z* 610 (M+H)⁺.

### Example #53: Compound 6: 4-[[4-[[4-chloro-2-(difluoromethoxy)phenyl]carbamoyl]-4-(5-isopropylpyrazol-1-yl)-1-piperidyl]methyl]-1-ethoxycarbonyl-piperidine-4-carboxylic acid

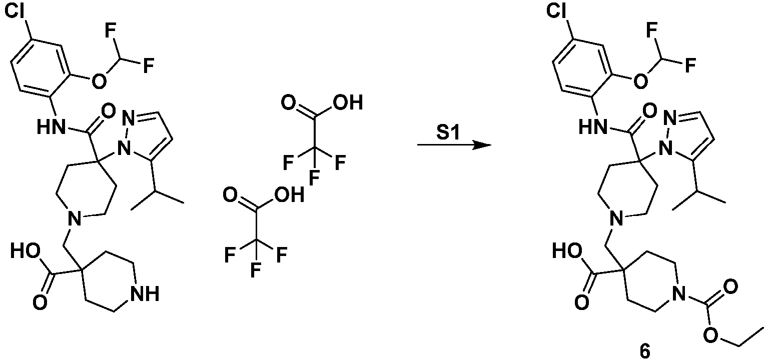

**Compound 6.** To a solution of 4-((4-((4-chloro-2-(difluoromethoxy)phenyl)carbamoyl)-4-(5-isopropyl-1*H*-pyrazol-1-yl)piperidin-1-yl)methyl)piperidine-4-carboxylic acid bis trifluroroacetic acid salt (**Example #47, Step 3**, 57 mg, 0.072 mmol) dissolved in a mixture of dichloromethane (1.5 mL) was added triethylamine (0.050 mL, 0.361 mmol) and acetic anhydride (0.0078 mL, 0.072 mmol) and the solution was stirred at ambient temperature for 10 minutes. The resultant mixture was diluted with dichloromethane (1 mL) and extracted with half saturated aqueous ammonium chloride (2 × 3 mL). The combined organic phase was dried over MgSO₄, filtered and the filtrate was concentrated under reduced pressure. The residue was purified by silica gel column chromatography using methanol in dichloromethane to provide the title compound. ¹H NMR (600 MHz, dimethyl sulfoxide-*d*₆) δ ppm 8.11 (s, 1H), 7.80 (d, *J =* 9.4 Hz, 1H), 7.52 (d, *J =* 1.8 Hz, 1H), 7.32 (m, 2H), 7.14 (t, *J =* 73.2 Hz, 1H), 6.32 (d, *J =* 1.8 Hz, 1H), 4.01 (d, *J =* 7.1 Hz, 2H), 3.73 (m, 2H), 3.17 (s, 2H), 2.95 (m, 2H), 2.92 (m, 1H), 2.72 (m, 2H), 2.44 (m, 4H), 2.35 (m, 2H), 1.90 (m, 2H), 1.35 (m, 2H), 1.16 (t, *J* = 7.1 Hz, 3H), 1.09 (d, *J* = 6.7 Hz, 6H). MS (APCI+) *m*/*z: 626.4* [M+H]⁺.

### Example #54: Inhibitory activity of compounds 15, 62, 77, 84 and 169 against LPAR1

The inhibitory activity of compounds 15, 62, 77, 84 and 169 against LPAR1 was measured using an Arrestin assay in U2OS cells expressing human LPAR1.

### Arrestin Assay Reagents

| **Reagent** | **Description** |
|---|---|
| TangoEDG2-bla U2OS Cell Line | U2OS cells expressing human LPAR1 (Invitrogen Cat K1519A) |
| FreeStyle293 Media | FreeStyle293 media for cell plating (Invitrogen Cat 12338-018) |
| HEPES | 1M 4-(2-hydroxyethyl)-1-piperazineethanesulfonic acid (HEPES) solution (Invitrogen Cat 15630-080) |
| HBSS | 1x Hank's Buffer Saline Solution (Invitrogen Cat 14025-076) |
| BSA | Fatty Acid Free Bovine Serum Albumin (BSA) powder (Sigma Cat A6003) |
| 18:1 LPA | Lyophilized Lysophosphatidic Acid; specifically 18:11-oleoyl-2-hydroxy-sn-glycero-3-phosphate (Avanti Cat 857130P) |
| LiveBLAzer FRET B/G | LiveBLAzer FRET B/G Kit (Invitrogen Cat K1096) |
| Solution D | Solution D is an anion transport inhibitor solution (Invitrogen Cat K1157) |
| Greiner 384 well black cell plates | 384 well black plates used to plate cell for experiments (Greiner Cat 781948) |
| Greiner 384 well plates, polypropylene | 384 well polypropylene plates used to make up antibody dilutions (Greiner Cat 781280) |

### Arrestin Assay Protocol

FreeStyle media was warmed in a 37 °C water bath. Cryo-preserved vial(s) of TangoEDG2-bla U2OS cells were rapidly thawed in a 37 °C water bath. The thawed cells were added to FreeStyle media and cells were pelleted at 300 x g for 5 minutes. The supernatant was aspirated, and cells were resuspended in FreeStyle media. Cell density was adjusted to 0.25 x 10⁶ cells/mL. Cells (1 x 10⁴ in 40 µL) were added to each well of Greiner 384 well black cell plate(s). Cell plate(s) were placed in a humidified 37 °C/5% CO₂ incubator for 18-20 hours. Dilutions of test compound were prepared at 6x final concentration; 12-point, 3-fold dilutions using HH Buffer (1x HBSS plus 20 mM HEPES). The 6x test compound was then added to the cell plate(s) at 10 µL/well. The final concentration ranged from 10 µM to 0.00006 µM. Cell plate(s) were incubated in a humidified 37 °C/5% CO₂ incubator for 1 hour. During the incubation, 6x LPA was prepared at 1.5 µM in 0.1 % BSA HH Buffer. After the 1-hour incubation of test compound with cells, the LPA was added to the cell plate(s) at 10 µL/well (final LPA concentration of 0.25 µM). Cell plate(s) were returned to a humidified 37 °C/5% CO₂ incubator for 3 hours. The Development solution was made up per the LiveBLAzerFRET B/G kit protocol. The Development solution was added to the cell plate(s) at 10 µL/well. Cell plates were incubated in the dark at room temperature for 2 hours. Plate(s) were read on an Envision Plate Reader (Perkin Elmer) to read fluorescence; excitation at 409 nm with emission at 460 nm and 530 nm. Raw Data for analysis is the ratio of emission at 460 nm divided by emission 530 nm. The formula used to determine % Inhibition is as follows (1 - (Raw Data - Low Average) / (High Average - Low Average)) * 100. Percent Inhibition was plotted to determine IC₅₀ value with GraphPad Prism 9.5.0 software using Variable slope 4-parameter curve fit analysis.

Results are shown below:

| **Compound Number** | **Arrestin Reporter EDG2-bla U2OS Human IC₅₀ (µM)** |
|---|---|
| 15 | 0.0478 |
| 62 | 0.0515 |
| 77 | 0.0436 |
| 84 | 0.0572 |
| 169 | 0.0262 |

### Example #55: Inhibitory activity of compounds against LPAR1

Additional compounds of the present disclosure were synthesized in a manner similar to the compounds described above and understood by a person of ordinary skill in the art. The inhibitory activity of the compounds of the present disclosure against LPAR1 was measured using the above-referenced Arrestin Assay protocol. Results are shown below:

| **Compound Number** | **Arrestin Reporter EDG2-bla U2OS Human Merged IC50 (µM)** | **Compound Number** | **Arrestin Reporter EDG2-bla U2OS Human Merged IC50 (µM)** |
|---|---|---|---|
| 1 | 0.0548 | 121 | 0.0488 |
| 2 | 0.0503 | 122 | 0.0239 |
| 3 | 0.0147 | 123 | 0.0389 |
| 4 | 0.0228 | 124 | 0.0493 |
| 5 | 0.0873 | 125 | 0.113 |
| 6 | 0.0247 | 126 | 0.0221 |
| 7 | 0.0508 | 127 | 0.0319 |
| 8 | 0.00331 | 128 | 0.056 |
| 9 | 0.128 | 129 | 0.0347 |
| 10 | 0.00753 | 130 | 0.0457 |
| 11 | 0.0526 | 131 | 0.134 |
| 12 | 0.0114 | 132 | 0.178 |
| 13 | 0.0112 | 133 | 0.0748 |
| 14 | 0.0339 | 134 | 0.0715 |
| 15 | 0.0478 | 135 | 0.0769 |
| 16 | 0.0298 | 136 | 0.0467 |
| 17 | 0.0145 | 137 | 0.0572 |
| 18 | 0.011 | 138 | 0.072 |
| 19 | 0.00956 | 139 | 0.0537 |
| 20 | 0.0288 | 140 | 0.132 |
| 21 | 0.0146 | 141 | 0.0257 |
| 22 | 0.0101 | 142 | 0.0379 |
| 23 | 0.0284 | 143 | 0.0322 |
| 24 | 0.0247 | 144 | 0.0559 |
| 25 | 0.028 | 145 | 0.0409 |
| 26 | 0.0245 | 146 | 0.0156 |
| 27 | 0.0142 | 147 | 0.167 |
| 28 | 0.00482 | 148 | 0.0504 |
| 29 | 0.00475 | 149 | 0.0345 |
| 30 | 0.0126 | 150 | 0.0995 |
| 31 | 0.00655 | 151 | 0.075 |
| 32 | 0.00311 | 152 | 0.0411 |
| 33 | 0.00513 | 153 | 0.0304 |
| 34 | 0.0333 | 154 | 0.0641 |
| 35 | 0.0436 | 155 | 0.125 |
| 36 | 0.0553 | 156 | 0.0497 |
| 37 | 0.0146 | 157 | 0.0532 |
| 38 | 0.0188 | 158 | 0.046 |
| 39 | 0.013 | 159 | 0.326 |
| 40 | 0.0225 | 160 | 0.0869 |
| 41 | 0.0228 | 161 | 0.212 |
| 42 | 0.0191 | 162 | 0.0518 |
| 43 | 0.0276 | 163 | 0.0385 |
| 44 | 0.0305 | 164 | 0.0724 |
| 45 | 0.0176 | 165 | 0.137 |
| 46 | 0.0627 | 166 | 0.12 |
| 47 | 0.0148 | 167 | 0.108 |
| 48 | 0.044 | 168 | 0.0288 |
| 49 | 0.0732 | 169 | 0.0262 |
| 50 | 0.0641 | 170 | 0.0331 |
| 51 | 0.0566 | 171 | 0.0926 |
| 52 | 0.0705 | 172 | 0.071 |
| 53 | 0.0135 | 173 | 0.031 |
| 54 | 0.0565 | 174 | 0.0502 |
| 55 | 0.00457 | 175 | 0.122 |
| 56 | 0.00673 | 176 | 0.0955 |
| 57 | 0.00683 | 177 | 0.0925 |
| 58 | 0.0756 | 178 | 0.123 |
| 59 | 0.0264 | 179 | 0.174 |
| 60 | 0.0912 | 180 | 0.0488 |
| 61 | 0.0345 | 181 | 0.0877 |
| 62 | 0.0515 | 182 | 0.0363 |
| 63 | 0.0045 | 183 | 0.0394 |
| 64 | 0.0515 | 184 | 0.0773 |
| 65 | 0.00987 | 185 | 0.0574 |
| 66 | 0.0228 | 186 | 0.0551 |
| 67 | 0.0209 | 187 | 0.0759 |
| 68 | 0.0656 | 188 | 0.0383 |
| 69 | 0.0148 | 189 | 0.0312 |
| 70 | 0.0114 | 190 | 0.0379 |
| 71 | 0.105 | 191 | 0.0334 |
| 72 | 0.0188 | 192 | 0.0656 |
| 73 | 0.0372 | 193 | 0.0313 |
| 74 | 0.0108 | 194 | 0.0906 |
| 75 | 0.0327 | 195 | 0.0676 |
| 76 | 0.0655 | 196 | 0.0347 |
| 77 | 0.0436 | 197 | 0.014 |
| 78 | 0.0483 | 198 | 0.0312 |
| 79 | 0.0157 | 199 | 0.0404 |
| 80 | 0.129 | 200 | 0.0377 |
| 81 | 0.043 | 201 | 0.0306 |
| 82 | 0.0219 | 202 | 0.046 |
| 83 | 0.0244 | 203 | 0.0666 |
| 84 | 0.0572 | 204 | 0.0469 |
| 85 | 0.0537 | 205 | 0.0179 |
| 86 | 0.0202 | 206 | 0.0169 |
| 87 | 0.0323 | 207 | 0.0937 |
| 88 | 0.0133 | 208 | 0.0944 |
| 89 | 0.00593 | 209 | 0.0336 |
| 90 | 0.00554 | 210 | 0.123 |
| 91 | 0.023 | 211 | 0.0873 |
| 92 | 0.119 | 212 | 0.0463 |
| 93 | 0.539 | 213 | 0.0222 |
| 94 | 0.0408 | 214 | 0.0758 |
| 95 | 0.0117 | 215 | 0.0185 |
| 96 | 0.0234 | 216 | 0.0284 |
| 97 | 0.122 | 217 | 0.118 |
| 98 | 0.121 | 218 | 0.0754 |
| 99 | 0.0321 | 219 | 0.0417 |
| 100 | 0.0785 | 220 | 0.0715 |
| 101 | 0.0809 | 221 | 0.0277 |
| 102 | 0.158 | 222 | 0.0479 |
| 103 | 0.164 | 223 | 0.0595 |
| 104 | 0.0447 | 224 | 0.0311 |
| 105 | 0.0269 | 225 | 0.0309 |
| 106 | 0.0481 | 226 | 0.0399 |
| 107 | 0.0401 | 227 | 0.0383 |
| 108 | 0.0558 | 228 | 0.0166 |
| 109 | 0.0949 | 229 | 0.0292 |
| 110 | 0.0755 | 230 | 0.0255 |
| 111 | 0.221 | 231 | 0.0142 |
| 112 | 0.309 | 232 | 0.00837 |
| 113 | 0.141 | 233 | 0.00894 |
| 114 | 0.088 | 234 | 0.00954 |
| 115 | 0.0825 | 235 | 0.0209 |
| 116 | 0.129 | 236 | 0.0648 |
| 117 | 0.049 | 237 | 0.0165 |
| 118 | 0.0208 | 238 | 0.126 |
| 119 | 0.0155 | 239 | 0.0623 |
| 120 | 0.0347 | 240 | 0.0238 |

### Example #56: Activity of Compound 15 against skin fibrosis

The activity of Compound 15 was measured using a bleomycin-evoked skin fibrosis model.

### Model Protocol

For the bleomycin model of skin fibrosis, C57B6J mice were purchased at 9 to 11 weeks of age. Upon arriving, mice were group-housed 10 per cage in individually ventilated cages, with a normal 12:12 hour light:dark cycle. Animals were acclimated to the animal facilities for a period of at least 10 days prior to initiation of bleomycin model studies; mouse ears were tagged for animal identification. At the initiation of bleomycin administration, 93M diet gel was also added to the cages of all mice and available ad libitum for the duration of the experiment. For all animals studied, food and water were available ad libitum.

The upper back region of the mouse was shaved and two circles (~1 cm diameter) were drawn onto the skin to identify the location for bleomycin injection. To induce skin fibrosis, mice received subcutaneous injections of bleomycin (10 units/kg) or phosphate-buffered saline (PBS) during Week 1 (D0 - D4) and week 2 (D7 - D10) at a volume of 100 µL into the two marked injection sites (for a total volume of 200 µL). Following the final dose of bleomycin, mice were then pseudorandomized into treatment groups so that all groups had comparable body weight loss prior to the onset of test compound dosing. For pharmacological assessment, mice received either vehicle or 1x, 3x, or 10x mg/kg of a dose of Compound 15 orally, twice daily, from Day 10 to 28. On Day 28, animals were humanely euthanized, and plasma and 2 to 8 mm back skin biopsy punches collected. The ALK5 inhibitor, SB-525334 was also included in the study as a positive control. SB-525334 was administered at a dose of 4.5x mg/kg following the same dosing regimen described for Compound 15. All treatments were administered at a volume of 1x mL/kg body weight.

For tissue gene expression measurement, an 8-mm skin biopsy punch was homogenized in 300 µL RLT buffer containing β-mercaptoethanol (1:100) and using a QIAgen TissueLyser II. RNA was then extracted using the RNeasy Fibrous Mini Kit and purified RNA analyzed for gene expression using TaqMan Kits (Thermofisher) with indicated probes (Table B). Expression data for each sample were normalized to the housekeeping gene (Rplp0) and expressed as fold change relative to the sham treated mice.

**Table B. Measured Genes and Corresponding TaqMan Probe/Primer**

| **Gene** | **TaqMan Probe/Primer** |
|---|---|
| Colla 1 | Mm00801666_g1 |
| Col6a3 | Mm00711678_m1 |
| Col8a 1 | Mm01344185_m1 |
| Fn 1 | Mm01256744_m1 |
| Timp 1 | Mm01341361_m1 |
| Mmp2 | Mm00439498_m1 |
| Serpine 1 | Mm00435858_m1 |
| Lpar 1 | Mm01346925_m1 |
| Rplpo | Mm00725448_s1 |

For protein measurement, ½ of an 8-mm biopsy punch was homogenized in 500 µL DI water containing extraction buffer and cell extraction enhancer solution, using a Bead Ruptor 24 with CryoCool (OMNI). ELISA assays were performed on the lysates to measure Fibronectin and Procollagen1a1 and a multiplexed MSD U-plex assay was performed to measure MCP-1 and CXCL1. All kits were run according to the manufacturers' instructions. Skin protein levels were normalized to total protein in the lysate and expressed per milligram of total protein.

Data from gene and protein measurements were analyzed using GraphPad Prism 7 (GraphPad Software, San Diego, CA, USA). Group comparisons were conducted by a one-way ANOVA, followed by Dunnett's multiple comparisons test.

### Bleomycin-Evoked Skin Fibrosis Model Results

In the subcutaneous bleomycin-induced skin fibrosis mouse model, Compound 15 was assessed after the skin fibrosis was established during Days 1 through 10. Compound 15 was administered twice daily by oral gavage from Day 10 through Day 28 as described in Table C.

**Table C. Systemic Bleomycin Model Study Design**

| **Group Number** | **PBS or Bleomycin (Bleo) QD (10 µ/kg SC)** | **Test Article** | **PO BID Dose (mg/kg)** | **N** |
|---|---|---|---|---|
| 1 | PBS D1 - D10 | Vehicle | - | 10 |
| 2 | Bleo D1 - D10 | Vehicle | - | 14 |
| 3 | Bleo D1 - D10 | Compound 15 | 1x | 12 |
| 4 | Bleo D1 - D10 | Compound 15 | 3x | 12 |
| 5 | Bleo D1 - D10 | Compound 15 | 10x | 12 |
| 6 | Bleo D1 - D10 | SB-525334 | 4.5x | 12 |

Treatment of mice with Compound 15 significantly reduced key measures of skin inflammation and fibrosis at test concentrations of 1x, 3x, and 10x mg/kg. Reductions in disease-relevant genes are summarized in Table D.

**Table D. Compound 15 Reduces Expression of Pro-Fibrotic and Pro-Inflammatory Genes in the Systemic Bleomycin Model of Skin Fibrosis**

| | **Fold-Change, Bleo vs Sham** | **% Inhibition by Concentration vs Bleo (mean ± SEM)** | | |
|---|---|---|---|---|
| **Gene** | **mean ± SEM** | **1x mg/kg** | **3x mg/kg** | **10x mg/kg** |
| Collal | 5.7 ± 0.9*** | 58 ± 12* | 77 ± 8*** | 88 + 6*** |
| Col6a3 | 2.4 ± 0.1 ** | 62 ± 24 | 108 ± 9** | 117 ± 8*** |
| Col8al | 7.5 ± 1.2*** | 16 ± 23 | 43 ±14 | 55 ± 11* |
| Fnl | 9.7 ± 2*** | 31 ± 20 | 76 ± 5** | 75 ± 4** |
| Timpl | 21.4 ± 5.4*** | 34 ± 21 | 81 ± 7** | 84 ± 5** |
| Mmp2 | 2.4 ± 0.2** | 39 ± 26 | 81 ± 15** | 55 ± 12 |
| Serpinel | 4.4 ± 1.0** | 28 ± 31 | 88 ± 5* | 75 ± 9* |
| Lparl | 1.7 ± 0.1 *** | 54 ± 24 | 97 ± 9*** | 95 ± 8*** |

| | | | | |
|---|---|---|---|---|
| *P < 0.05; **P < 0.01; ***P < 0.001 | | | | |

A reduction in disease-relevant skin proteins was also observed, including decreases in extracellular matrix proteins (pro-collagen 1 and fibronectin) and inflammatory protein expression (MCP-1 and CXCL6), as shown in Table E.

**Table E. Therapeutic Intervention with Compound 15 Reduces Disease-Relevant Proteins in the Systemic Bleomycin Model of Skin Fibrosis**

| **Protein** | **% Inhibition by Drug Treatment** | | | |
|---|---|---|---|---|
| | **Compound 15** | | | **SB-525334** |
| | **1x mg/kg** | **3x mg/kg** | **10x mg/kg** | |
| **Procollagen1a1** | 67*** | 74*** | 77*** | 69*** |
| **Fibronectin** | 29 | 46*** | 46*** | 82*** |
| **MCP-1** | 50** | 59** | 73*** | 17 |
| **CXCL1** | 56** | 71*** | 76*** | 49* |

| | | | | |
|---|---|---|---|---|
| *P < 0.05; **P < 0.01; ***P < 0.001 vs bleomycin/vehicle. | | | | |

### INCORPORATION BY REFERENCE

All of the U.S. patents and U.S. and PCT published patent applications cited herein are hereby incorporated by reference, except for any definitions, subject matter disclaimers or disavowals, and except to the extent that the incorporated material is inconsistent with the express disclosure herein, in which case the language in this disclosure controls.

### EQUIVALENTS

The foregoing written specification is considered to be sufficient to enable one skilled in the art to practice the invention. The present invention is not to be limited in scope by examples provided, since the examples are intend The foregoing written specification is considered to be sufficient to enable one skilled in the art to practice the invention. The present invention is not to be limited in scope by examples provided, since the examples are intended as a single illustration of one aspect of the invention and other functionally equivalent embodiments are within the scope of the invention. Various modifications of the invention in addition to those shown and described herein will become apparent to those skilled in the art from the foregoing description and fall within the scope of the appended claims. The advantages and objects of the invention are not necessarily encompassed by each embodiment of the invention. as a single illustration of one aspect of the invention and other functionally equivalent embodiments are within the scope of the invention. Various modifications of the invention in addition to those shown and described herein will become apparent to those skilled in the art from the foregoing description and fall within the scope of the appended claims. The advantages and objects of the invention are not necessarily encompassed by each embodiment of the invention.

The following are also aspects of the present invention.
1. A compound of Formula (I): 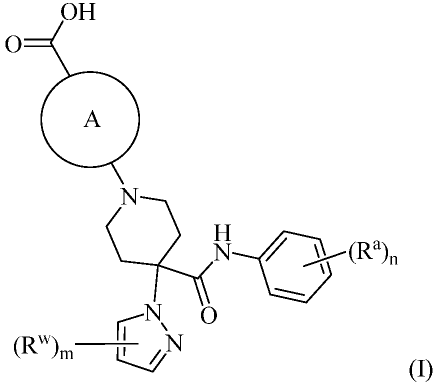 or a pharmaceutically acceptable salt thereof,
   wherein:
   each R^{a} is independently selected from the group consisting of halo, -O(C₁₋₆alkyl), -O(C₁₋₆haloalkyl), -C₁₋₆alkyl, and -C₁₋₆haloalkyl;
   n is 0, 1, or 2;
   each R^{W} is independently selected from the group consisting of halo, -OR^{W2}, -NZ₂, optionally substituted C₁₋₆alkyl, optionally substituted C₁₋₆haloalkyl, optionally substituted 3-6-membered carbocyclyl, and optionally substituted 3-6-membered heterocyclyl; or
      two vicinal R^{W} groups, taken together with the atoms to which they are attached, form a fused optionally substituted 4-6 membered carbocyclyl or fused optionally substituted 4-6 membered heterocyclyl;
   m is 0, 1, 2, or 3;
   Ring A is an optionally substituted non-aromatic 3-7-membered carbocyclylene or a non-aromatic 3-7-membered heterocyclylene;
   R^{W2} is hydrogen, optionally substituted C₁₋₆alkyl, optionally substituted C₁₋₆haloalkyl, optionally substituted 3-6-membered carbocyclyl, or optionally substituted 3-6-membered heterocyclyl; and
   each Z is independently selected from the group consisting of hydrogen, optionally substituted C₁₋₆alkyl, optionally substituted C₁₋₆haloalkyl, optionally substituted 3-6-membered carbocyclyl, and optionally substituted 3-6-membered heterocyclyl.
2. The compound of 1, wherein Ring A is an optionally substituted 4-membered carbocyclylene.
3. The compound of 1 or 2, wherein Ring A is a 4-membered carbocyclylene, optionally substituted with 1-3 substituents each independently selected from the group consisting of halo, C₁₋₆alkyl, C₁₋₆haloalkyl, -O(C₁₋₆alkyl), and -O(C₁₋₆haloalkyl).
4. A compound of Formula (II): 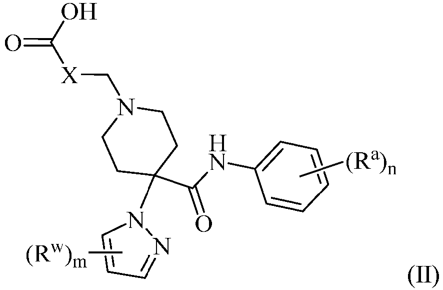 or a pharmaceutically acceptable salt thereof,
   wherein:
   each R^{a} is independently selected from the group consisting of halo, -O(C₁₋₆alkyl), -O(C₁₋₆haloalkyl), -C₁₋₆alkyl, and -C₁₋₆haloalkyl;
   n is 0, 1, or 2;
   each R^{W} is independently selected from the group consisting of halo, -OR^{W2}, -NZ₂, optionally substituted C₁₋₆alkyl, optionally substituted C₁₋₆haloalkyl, optionally substituted 3-6-membered carbocyclyl, and optionally substituted 3-6-membered heterocyclyl; or
      two vicinal R^{W} groups, taken together with the atoms to which they are attached, form a fused optionally substituted 4-6 membered carbocyclyl or fused optionally substituted 4-6 membered heterocyclyl;
   m is 0, 1, 2, or 3;
   X is a bond or an optionally substituted 3-7-membered carbocyclylene, 3-7-membered heterocyclylene, 3-7-membered arylene, 3-7-membered heteroarylene, C₁₋₆alkylene, C₂₋₆alkenylene, -CH(3-7-membered carbocyclyl)-, -CH(3-7-membered heterocyclyl)-, - CH(3-7-membered heterocyclyl)alkyl)-, -CH(3-7-membered carbocyclyl)alkyl)-, - CH(heteroaryl)-, -CH(aryl)-, -CH(heteroarylalkyl)-, -CH(arylalkyl)-, -CH(C₁₋₆alkyl)-, - CH(C₁₋₆haloalkyl)-, -CH(C₁₋₆hydroxyalkyl)-, -CH(C₁₋₆alkoxy)-, -CH(C₁₋₆alkoxyC₁₋₆alkyl)-, or -C(OH)(C₁₋₆alkyl)-;
   R^{W2} is hydrogen, optionally substituted C₁₋₆alkyl, optionally substituted C₁₋₆haloalkyl, optionally substituted 3-6-membered carbocyclyl, or optionally substituted 3-6-membered heterocyclyl; and
   each Z is independently selected from the group consisting of hydrogen, optionally substituted C₁₋₆alkyl, optionally substituted C₁₋₆haloalkyl, optionally substituted 3-6-membered carbocyclyl, and optionally substituted 3-6-membered heterocyclyl.
5. The compound of 4, wherein X is an optionally substituted 3-7-membered carbocyclylene, 3-7-membered heterocyclylene, -CH(3-7-membered carbocyclyl)-, -CH(3-7-membered heterocyclyl)-, -CH(C₁₋₆alkyl), -CH(C₁₋₆haloalkyl), or -CH(C₁₋₆alkoxyC₁₋₆alkyl).
6. The compound of 5, wherein X is an optionally substituted 3-7-membered heterocyclylene, -CH(3-7-membered heterocyclyl)-, or -CH(C₁₋₆alkoxyC₁₋₆alkyl).
7. The compound of any one of 1-6, wherein each R^{a} is independently selected from the group consisting of -Cl and -OCHF₂, and n is 2.
8. The compound of any one of 1-3 and 7, having the structure of Formula (Ib): 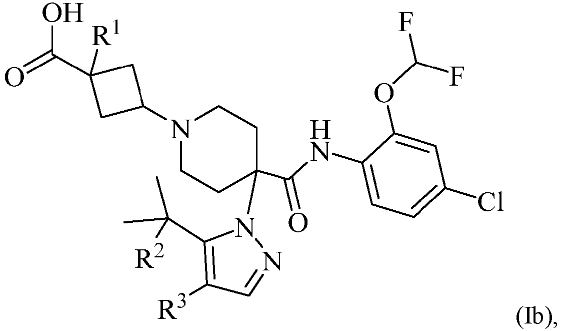 or a pharmaceutically acceptable salt thereof,
   wherein:
   R¹ is hydrogen or C₁₋₆alkyl; and
   R² and R³ are each independently hydrogen, halo, optionally substituted (C₁₋₃alkyl), or optionally substituted alkoxy(C₁₋₃alkyl); or
   R² and R³ taken together with the atoms to which they are attached, form a fused optionally substituted 4-6 membered carbocyclyl or fused optionally substituted 4-6 membered heterocyclyl.
9. The compound of 8, wherein R¹ is hydrogen or methyl.
10. The compound of 8 or 9, wherein R² and R³ are each hydrogen.
11. The compound of any one of 8-10, selected from the following table: 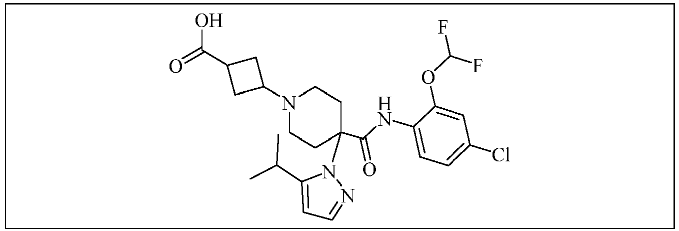 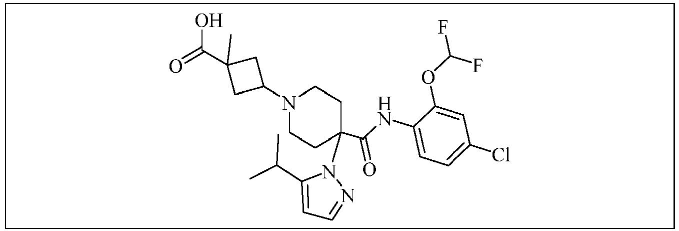
12. The compound of any one of 8-10, selected from the following table: 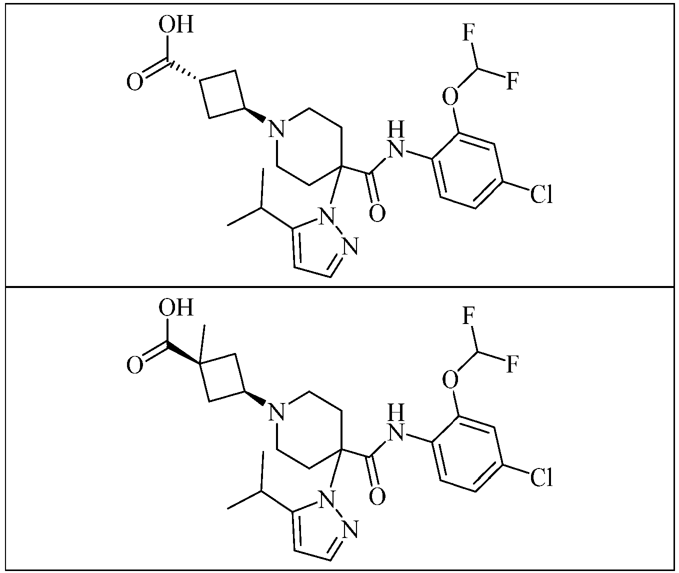
13. The compound of 11, wherein the compound is: 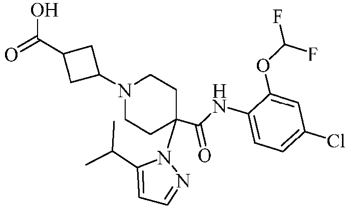 or a pharmaceutically acceptable salt thereof.
14. The compound of 11, wherein the compound is: 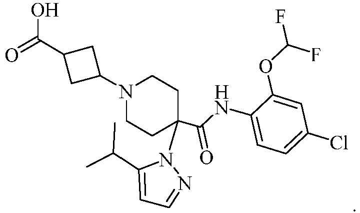
15. The compound of 12, wherein the compound is: 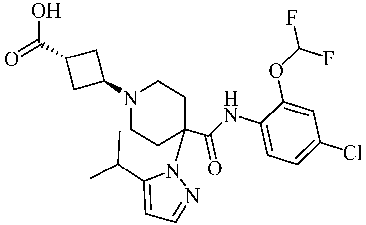 or a pharmaceutically acceptable salt thereof.
16. The compound of 12, wherein the compound is: 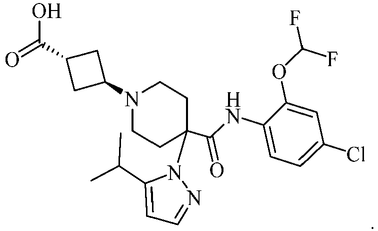
17. A pharmaceutically acceptable salt of the compound of 16.
18. The compound of any one of 4-7, having the structure of Formula (IIb): 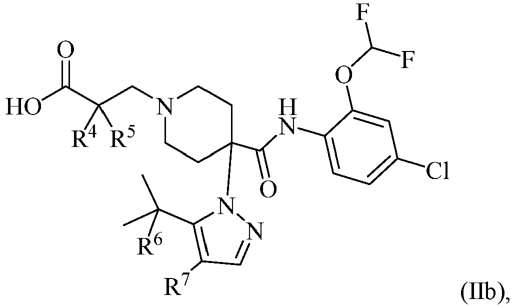 or a pharmaceutically acceptable salt thereof,
   wherein:
   R⁴ is hydrogen and R⁵ is an optionally substituted 3-7-membered carbocyclyl, 3-7-membered heterocyclyl, C₁₋₆alkyl, C₁₋₆haloalkyl, or C₁₋₆alkoxyC₁₋₆alkyl; or
   R⁴ and R⁵ taken together with the atom to which they are attached form an optionally substituted 3-7-membered carbocyclylene or an optionally susbstituted 3-7-membered heterocyclylene; and
   R⁶ and R⁷ are each independently hydrogen, halo, optionally substituted (C₁₋₃alkyl), or optionally substituted alkoxy(C₁₋₃alkyl); or
   R⁶ and R⁷ taken together with the atoms to which they are attached, form a fused optionally substituted 4-6 membered carbocyclyl or fused optionally substituted 4-6 membered heterocyclyl.
19. The compound of 18, wherein R⁴ is hydrogen and R⁵ is tetrahydropyranyl or -CH₂OCH₃; or
   R⁴ and R⁵ taken together with the atom to which they are attached form a tetrahydropyran ring.
20. The compound of 18 or 19, wherein R⁶ and R⁷ are each independently hydrogen or fluoro; or R⁶ and R⁷ taken together with the atoms to which they are attached form a fused 5-membered heterocyclyl.
21. The compound of any one of 18-20, selected from the following table: 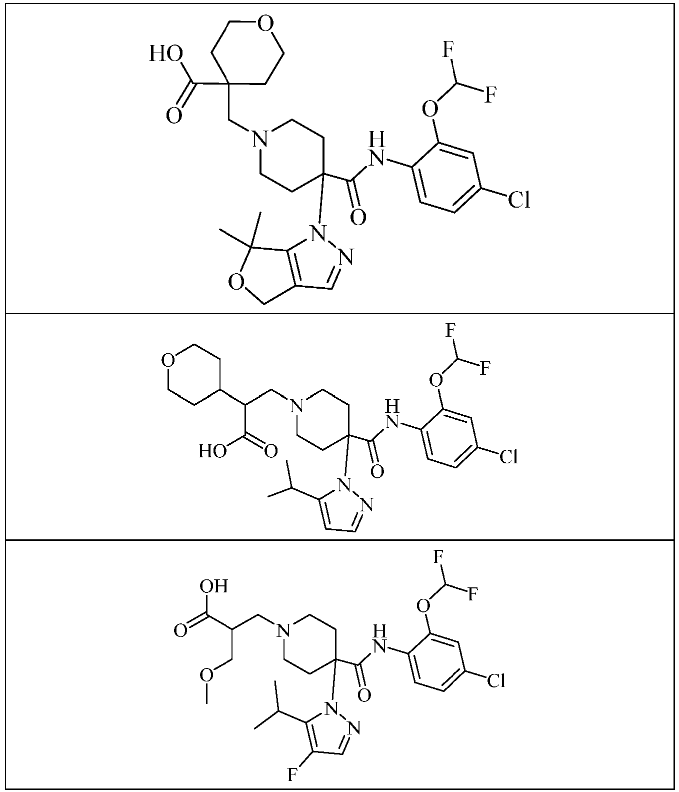
22. The compound of any one of 18-20, selected from the following table: 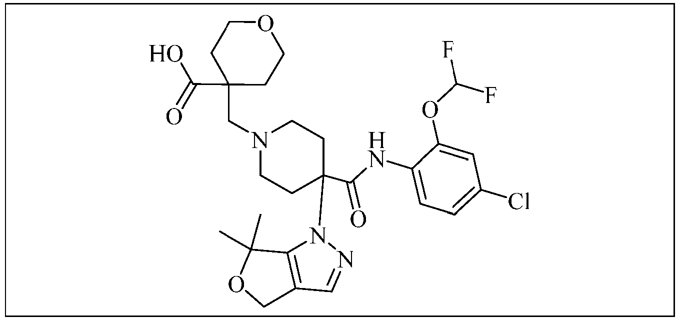 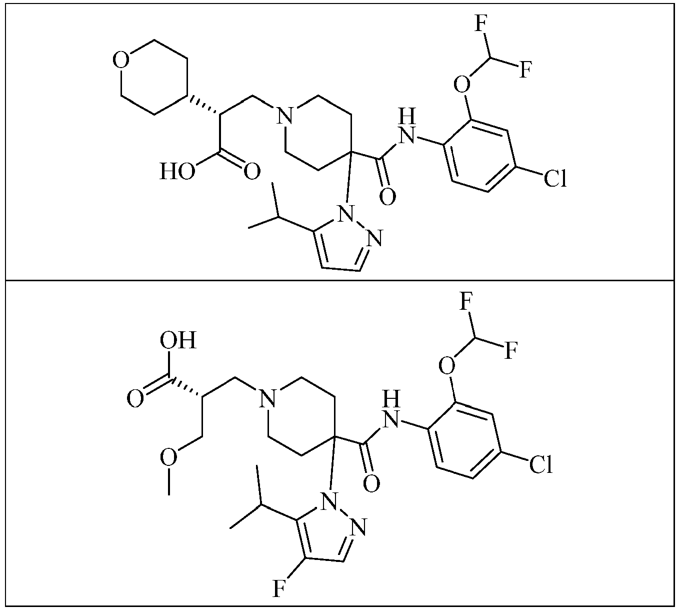
23. The compound of 21, wherein the compound is: 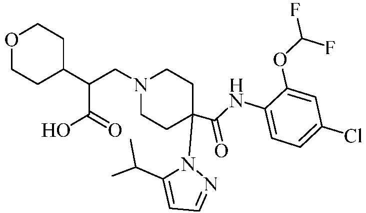 or a pharmaceutically acceptable salt thereof.
24. The compound of 21, wherein the compound is: 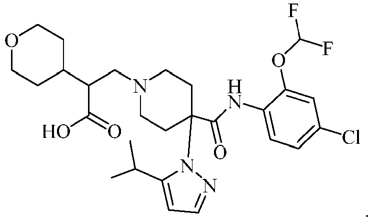
25. The compound of 22, wherein the compound is: 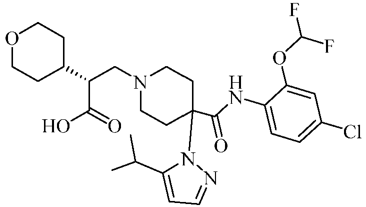 or a pharmaceutically acceptable salt thereof.
26. The compound of 22, wherein the compound is: 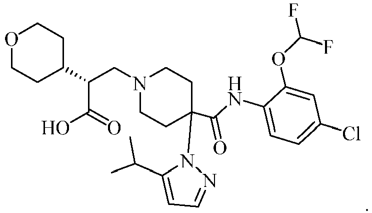
27. A pharmaceutically acceptable salt of the compound of 26.
28. The compound of 22, wherein the compound is: 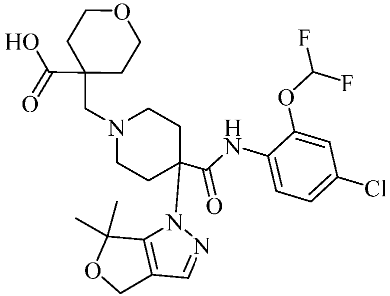 or a pharmaceutically acceptable salt thereof.
29. The compound of 22, wherein the compound is: 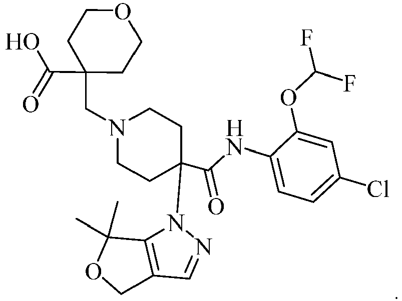
30. A pharmaceutically acceptable salt of the compound of 29.
31. A pharmaceutical composition, comprising a compound of any one of 1-30 and a pharmaceutically acceptable carrier.
32. A method of treating systemic sclerosis (SSc) in a subject in need thereof, comprising administering to the subject a therapeutically effective amount of a compound of any one of 1-30.

## Claims

1. A compound of Formula (I): 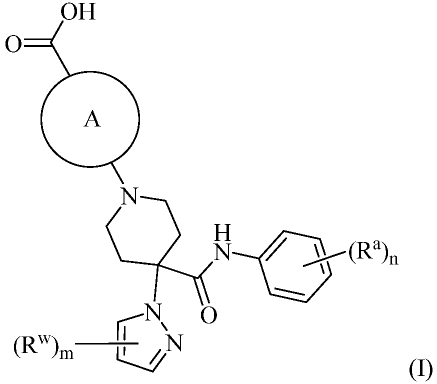 or a pharmaceutically acceptable salt thereof,
wherein:
each R^{a} is independently selected from the group consisting of halo, -O(C₁₋₆alkyl), -O(C₁₋₆haloalkyl), -C₁₋₆alkyl, and -C₁₋₆haloalkyl;
n is 0, 1, or 2;
each R^{W} is independently selected from the group consisting of halo, -OR^{W2}, -NZ₂, optionally substituted C₁₋₆alkyl, optionally substituted C₁₋₆haloalkyl, optionally substituted 3-6-membered carbocyclyl, and optionally substituted 3-6-membered heterocyclyl; or
two vicinal R^{W} groups, taken together with the atoms to which they are attached, form a fused optionally substituted 4-6 membered carbocyclyl or fused optionally substituted 4-6 membered heterocyclyl;
m is 0, 1, 2, or 3;
Ring A is an optionally substituted non-aromatic 3-7-membered carbocyclylene or a non-aromatic 3-7-membered heterocyclylene;
R^{W2} is hydrogen, optionally substituted C₁₋₆alkyl, optionally substituted C₁₋₆haloalkyl, optionally substituted 3-6-membered carbocyclyl, or optionally substituted 3-6-membered heterocyclyl; and
each Z is independently selected from the group consisting of hydrogen, optionally substituted C₁₋₆alkyl, optionally substituted C₁₋₆haloalkyl, optionally substituted 3-6-membered carbocyclyl, and optionally substituted 3-6-membered heterocyclyl.

2. The compound of claim 1,
(i) wherein Ring A is an optionally substituted 4-membered carbocyclylene, preferably wherein Ring A is a 4-membered carbocyclylene, optionally substituted with 1-3 substituents each independently selected from the group consisting of halo, C₁₋₆alkyl, C₁₋₆haloalkyl, -O(C₁₋₆alkyl), and -O(C₁₋₆haloalkyl), and/or
(ii) wherein each R^{a} is independently selected from the group consisting of -Cl and - OCHF₂, and n is 2.

3. A compound of Formula (II): 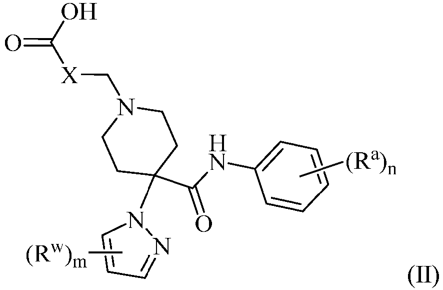 or a pharmaceutically acceptable salt thereof,
wherein:
each R^{a} is independently selected from the group consisting of halo, -O(C₁₋₆alkyl), -O(C₁₋₆haloalkyl), -C₁₋₆alkyl, and -C₁₋₆haloalkyl;
n is 0, 1, or 2;
each R^{W} is independently selected from the group consisting of halo, -OR^{W2}, -NZ₂, optionally substituted C₁₋₆alkyl, optionally substituted C₁₋₆haloalkyl, optionally substituted 3-6-membered carbocyclyl, and optionally substituted 3-6-membered heterocyclyl; or
two vicinal R^{W} groups, taken together with the atoms to which they are attached, form a fused optionally substituted 4-6 membered carbocyclyl or fused optionally substituted 4-6 membered heterocyclyl;
m is 0, 1, 2, or 3;
X is a bond or an optionally substituted 3-7-membered carbocyclylene, 3-7-membered heterocyclylene, 3-7-membered arylene, 3-7-membered heteroarylene, C₁₋₆alkylene, C₂₋₆alkenylene, -CH(3-7-membered carbocyclyl)-, -CH(3-7-membered heterocyclyl)-, - CH(3-7-membered heterocyclyl)alkyl)-, -CH(3-7-membered carbocyclyl)alkyl)-, - CH(heteroaryl)-, -CH(aryl)-, -CH(heteroarylalkyl)-, -CH(arylalkyl)-, -CH(C₁₋₆alkyl)-, - CH(C₁₋₆haloalkyl)-, -CH(C₁₋₆hydroxyalkyl)-, -CH(C₁₋₆alkoxy)-, -CH(C₁₋₆alkoxyC₁₋₆alkyl)-, or -C(OH)(C₁₋₆alkyl)-;
R^{W2} is hydrogen, optionally substituted C₁₋₆alkyl, optionally substituted C₁₋₆haloalkyl, optionally substituted 3-6-membered carbocyclyl, or optionally substituted 3-6-membered heterocyclyl; and
each Z is independently selected from the group consisting of hydrogen, optionally substituted C₁₋₆alkyl, optionally substituted C₁₋₆haloalkyl, optionally substituted 3-6-membered carbocyclyl, and optionally substituted 3-6-membered heterocyclyl.

4. The compound of claim 3,
(i) wherein X is an optionally substituted 3-7-membered carbocyclylene, 3-7-membered heterocyclylene, -CH(3-7-membered carbocyclyl)-, -CH(3-7-membered heterocyclyl)-, - CH(C₁₋₆alkyl), -CH(C₁₋₆haloalkyl), or -CH(C₁₋₆alkoxyC₁₋₆alkyl), preferably wherein X is an optionally substituted 3-7-membered heterocyclylene, -CH(3-7-membered heterocyclyl)-, or - CH(C₁₋₆alkoxyC₁₋₆alkyl), and/or
(ii) wherein each R^{a} is independently selected from the group consisting of -Cl and - OCHF₂, and n is 2.

5. The compound of any one of claims1-2, having the structure of Formula (Ib): 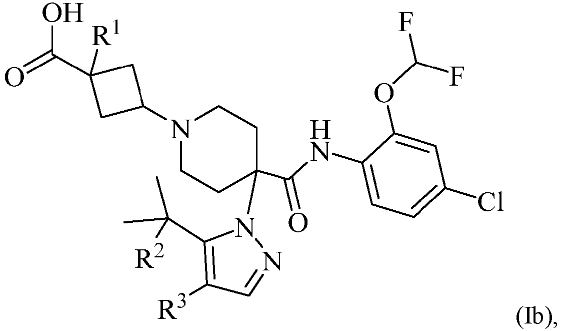 or a pharmaceutically acceptable salt thereof,
wherein:
R¹ is hydrogen or C₁₋₆alkyl; and
R² and R³ are each independently hydrogen, halo, optionally substituted (C₁₋₃alkyl), or optionally substituted alkoxy(C₁₋₃alkyl); or
R² and R³ taken together with the atoms to which they are attached, form a fused optionally substituted 4-6 membered carbocyclyl or fused optionally substituted 4-6 membered heterocyclyl.

6. The compound of claim 5, wherein
(i) R¹ is hydrogen or methyl, and/or
(ii) R² and R³ are each hydrogen, and/or
(iii) the compound is selected from the following table: 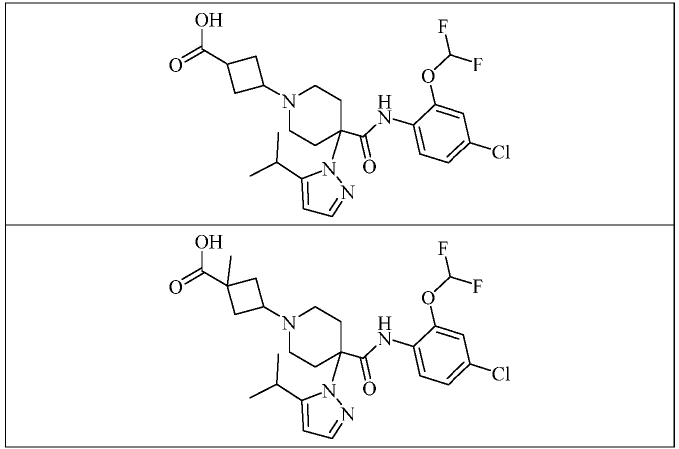 , or
the compound is selected from the following table:

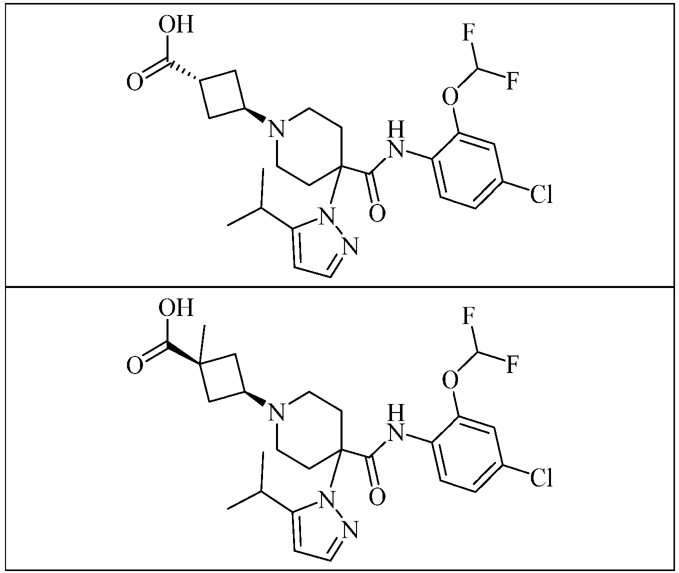

7. The compound of claim 6, wherein the compound is: 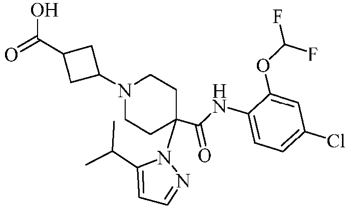 or a pharmaceutically acceptable salt thereof.

8. The compound of claim 6, wherein the compound is: 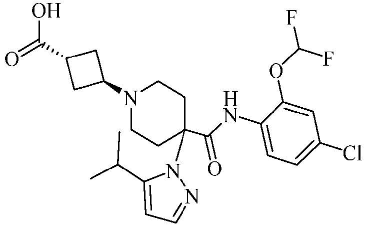 or a pharmaceutically acceptable salt thereof.

9. The compound of any one of claims 3-4, having the structure of Formula (IIb): 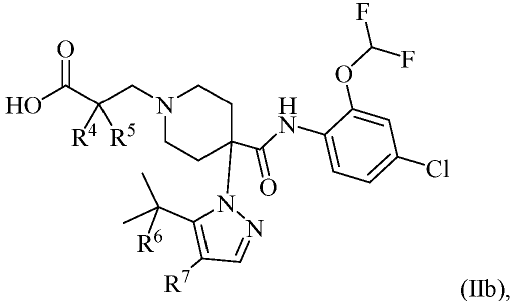 or a pharmaceutically acceptable salt thereof,
wherein:
R⁴ is hydrogen and R⁵ is an optionally substituted 3-7-membered carbocyclyl, 3-7-membered heterocyclyl, C₁₋₆alkyl, C₁₋₆haloalkyl, or C₁₋₆alkoxyC₁₋₆alkyl; or
R⁴ and R⁵ taken together with the atom to which they are attached form an optionally substituted 3-7-membered carbocyclylene or an optionally susbstituted 3-7-membered heterocyclylene; and
R⁶ and R⁷ are each independently hydrogen, halo, optionally substituted (C₁₋₃alkyl), or optionally substituted alkoxy(C₁₋₃alkyl); or
R⁶ and R⁷ taken together with the atoms to which they are attached, form a fused optionally substituted 4-6 membered carbocyclyl or fused optionally substituted 4-6 membered heterocyclyl.

10. The compound of claim 9,
(i) wherein R⁴ is hydrogen and R⁵ is tetrahydropyranyl or -CH₂OCH₃; or
R⁴ and R⁵ taken together with the atom to which they are attached form a tetrahydropyran ring, and/or
(ii) wherein R⁶ and R⁷ are each independently hydrogen or fluoro; or R⁶ and R⁷ taken together with the atoms to which they are attached form a fused 5-membered heterocyclyl, and/or
(iii) wherein the compound is selected from the following table:
, or 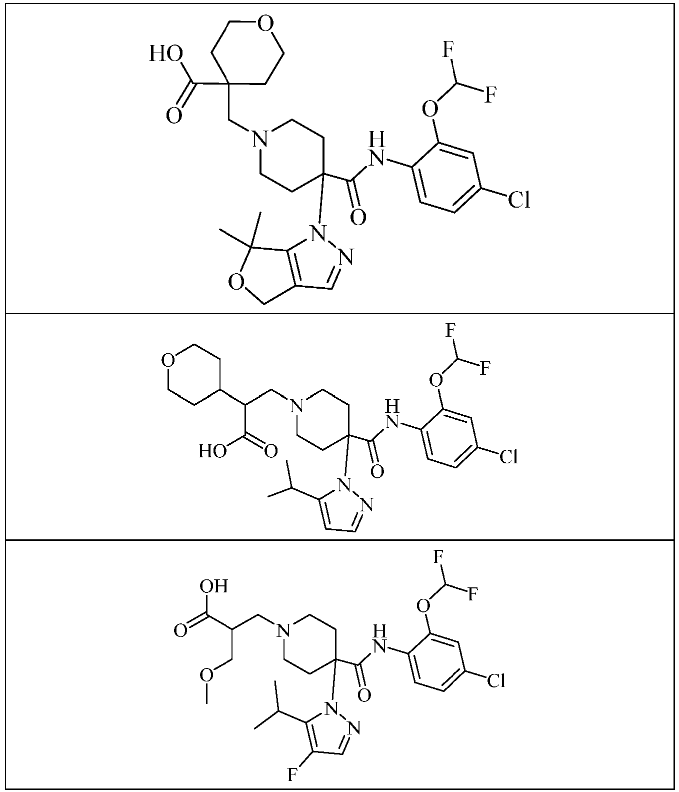 the compound is selected from the following table:

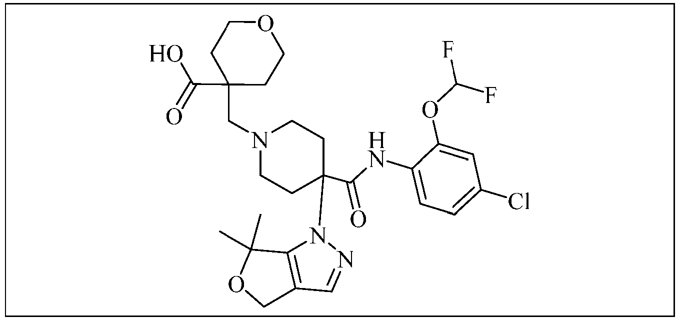

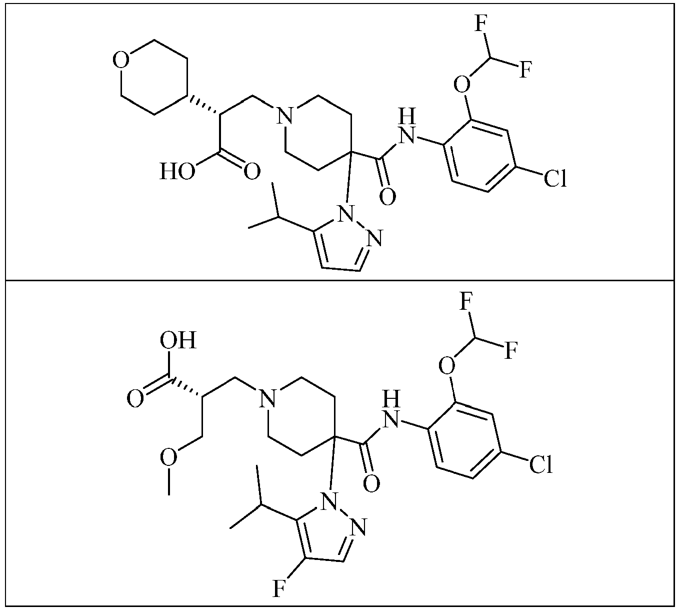

11. The compound of claim 10, wherein the compound is: 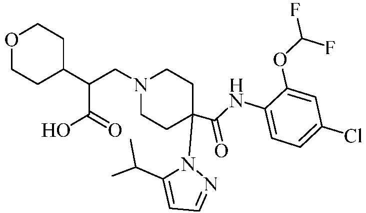 or a pharmaceutically acceptable salt thereof.

12. The compound of claim 10, wherein the compound is: 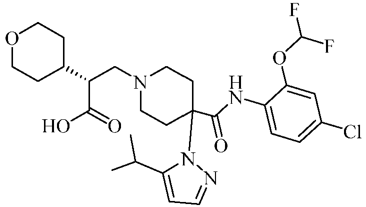 or a pharmaceutically acceptable salt thereof.

13. The compound of claim 10, wherein the compound is: 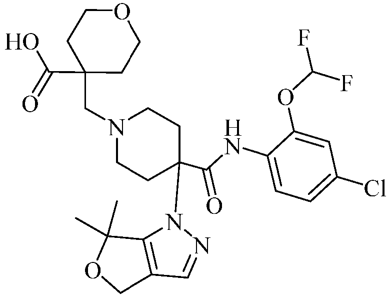 or a pharmaceutically acceptable salt thereof.

14. A pharmaceutical composition, comprising a compound of any one of claims 1-13 and a pharmaceutically acceptable carrier.

15. A compound of any one of claims 1-13 for use in a method of treating systemic sclerosis (SSc) in a subject in need thereof, comprising administering to the subject a therapeutically effective amount of the compound.
